# EUROPEAN PATENT APPLICATION

(11) **EP 2 116 136 A1**
(43) Date of publication of application: **11.11.2009**
(21) Application number: 08155927.0
(22) Date of filing: 08.05.2008
(51) Int. Cl.: A23K 1/165, C12N 9/16, C12N 15/55, C12N 1/15

(54) **Novel phytases**

(71) Applicant: Nederlandse Organisatie voor Toegepast- Natuurwetenschappelijk Onderzoek TNO, 2628 VK Delft (NL)
(72) Inventor: Punt, Peter Jan, 3994 XT Houten (NL); van Zeijl, Cornelia Maria Johanna, 3451 EP Vleuten (NL)
(74) Representative: Hatzmann, Martin

(57) **Abstract**

The present invention provides novel polypeptides having phytase activity, recombinant nuclei acid molecules and vectors encoding said novel phytases, and to host cells transformed with said recombinant nuclei acid molecules and vectors. Also provided is a method of transforming a suitable host cell with polynucleotides encoding the phytases, expression of the transformed polynucleotides and the use of the expressed phytase in feed as a supplement.

## Description

### FIELD OF THE INVENTION

The present invention is in the field of food and feed supplements. In particular, the present invention relates to phytase enzymes useful as a feed supplement, and to methods of producing said phytase enzymes by genetic engineering techniques.

### BACKGROUND TO THE INVENTION

Phytic acid (known as inositol hexakisphosphate (IP6), or phytate when in salt form) is the principal storage form of phosphorus in many plant tissues, especially bran and seeds. Phosphorus in phytate form is, in general, not bioavailable to non-ruminant animals because they lack the digestive enzyme phytase, which is required to separate phosphorus from the phytate molecule. On the other hand, ruminants readily utilize phytate because of the phytase produced by rumen microorganisms.

In most commercial agriculture, non-ruminant livestock such as swine and poultry are fed mainly grains such as soybeans and maize. Because phytate from these grains is unavailable for absorption, the unabsorbed phytate passes through the gastrointestinal tract, elevating the amount of phosphorus in the manure. Excess phosphorus excretion can lead to environmental problems such as eutrophication. The bioavailability of phytate phosphorus can be increased by supplementation of the diet with phytase enzyme.

Phytases are by far the most industrially relevant feed enzymes due to their nutritional and environment benefits in intensive animal breeding, and applications in fish feed are rapidly developing.

From the plant kingdom, e.g. a wheat-bran phytase is known (Thomlinson et al., Biochemistry 1 (1962), 166-171). An alkaline phytase from Lilly pollen has been described by Barrientos et al., Plant Physiol.106 (1994), 1489-1495. Amongst the bacteria, phytases have been described which are derived from *Bacillus subtilis* (Paver and Jagannathan, Journal of Bacteriology 151 (1982), 1102-1108) and *Pseudomonas* (Cosgrove, Australian Journal of Biological Sciences 23 (1970), 1207-1220).

There are several descriptions of phytase producing filamentous fungi. In particular, there are several references to phytase producing ascomycetes of the Aspergillus genus such as *Aspergillus terreus* (Yamada et al., Agric. Biol. Chem. 322 (1986), 1275-1282). Also, the cloning and expression of the phytase gene from *Aspergillus niger* var. *awamori* has been described (Piddington et al., Gene 133 (1993), 55-62).

EP 0 420 358 describes the cloning and expression of a phytase of *Aspergillus ficuum* (*niger*). EP 0 684 313 describes the cloning and expression of phytases of the ascomycetes *Myceliophthora thermophila* and *Aspergillus terreus*.

EP 897 010 entitled "Modified phytases" discloses, i.a., certain variants of an *Aspergillus fumigatus* phytase. EP 897 985 entitled "Consensus phytases" discloses, i.a., a fungal consensus phytase which may be designed on the basis of, i.a., a multiple alignment of several ascomycete phytases. WO 99/48380 entitled "Thermostable phytases in feed preparation and plant expression" relates to certain aspects of using thermostable phytases. WO 00/143503 entitled "Improved phytases" relates *inter alia* to certain phytase variants of increased thermo-stability, which may be designed by a process similar to the one described in EP 897985.

A phytase derived from *Peniophora lycii* is disclosed in WO 98/28408, and certain variants thereof in WO 99/49022, as well as in WO 03/066847.

Phytase producing yeasts are also described: For example, EP 0 699 762 A2 describes the cloning and expression of a phytase of the yeast *Schwanniomyces occidentalis.*

There is however still need for additional sources of phytase. In addition there is need for phytases having improved properties, such as increased activity.

### SUMMARY OF THE INVENTION

The present inventors have now discovered novel fungal phytases dissimilar to previously identified phytases, including two different groups.

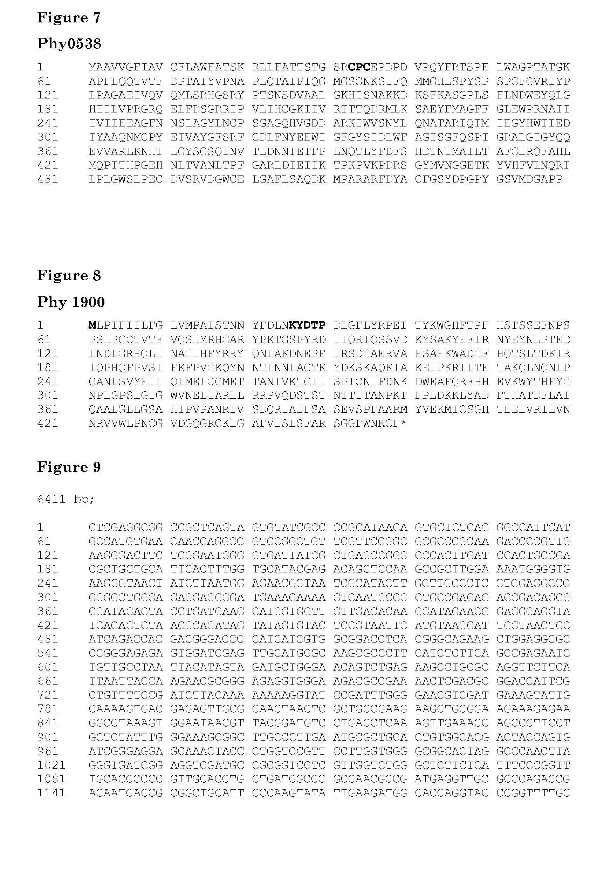

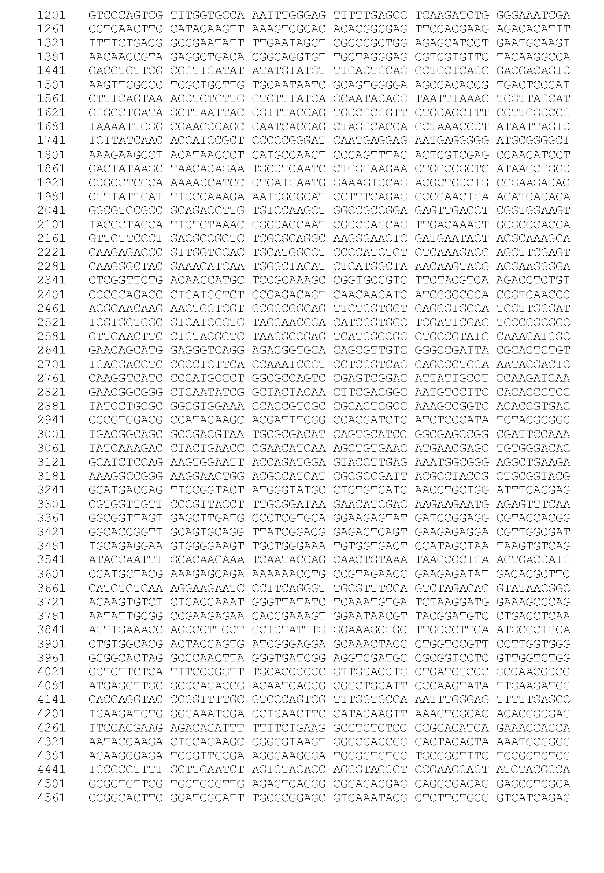

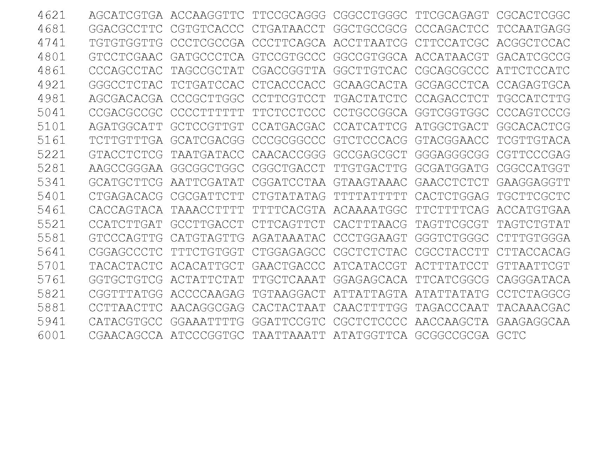

One group including Phy 538 and Phy1900 (Figure 7, 8, 13, 14) consists of a new subclass of phytases with very low similarity to previously know fungal phytases.

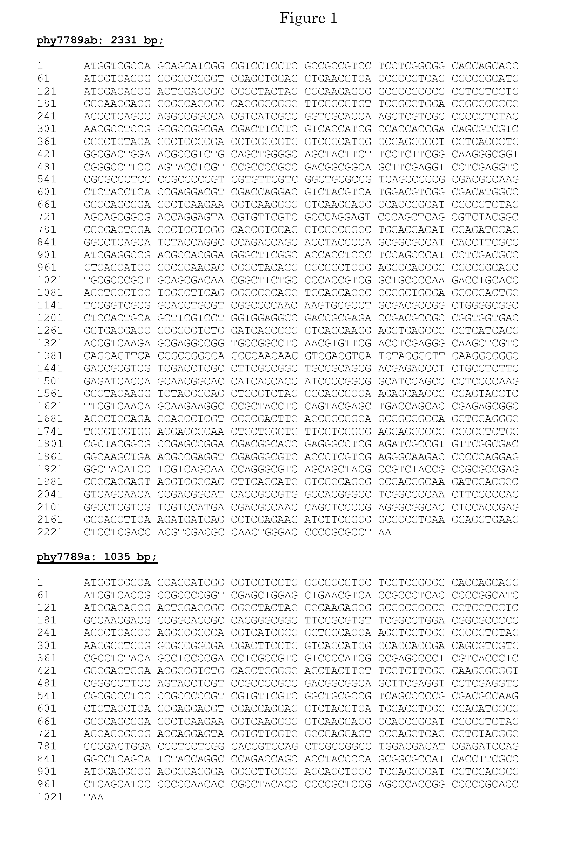

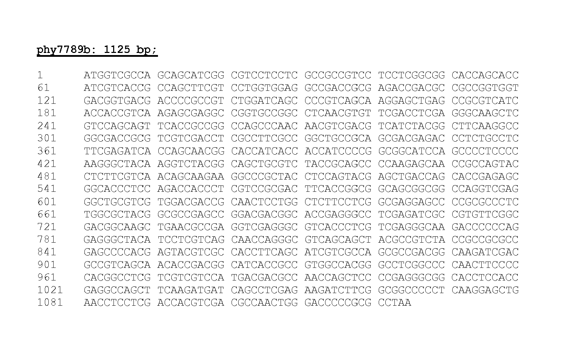

The other class consists of a fungal gene having two tandemly linked phytase domains (such as Phy7789ab Fig 1, 2-5, 10). Both of these domains have high levels of homology to phytases, proteins to date only characterized from bacterial sources. The structure of these phytases is homologous to known bacterial phytases (*vide* Paver and Jagannathan *supra*, and Cosgrove, *supra*). Thus, the present inventors discovered the existence of a completely new class of fungal proteins with homology to bacterial phytases.

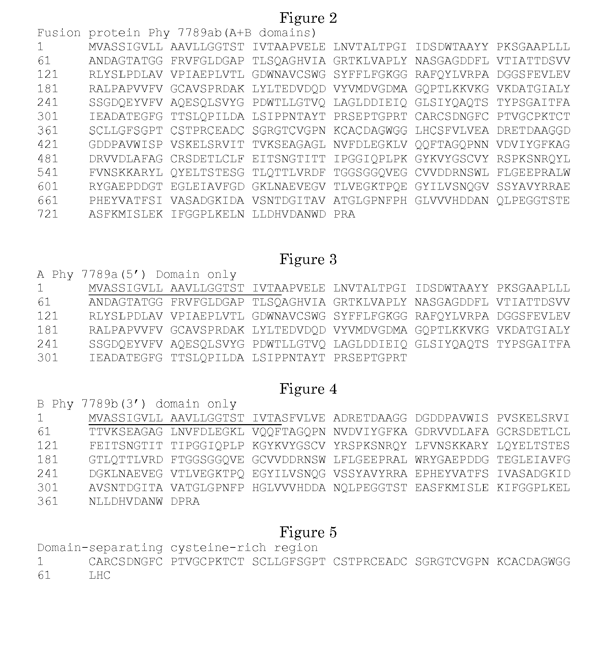

In a first aspect, the present invention provides an isolated polypeptide selected from the group consisting of:
(a) polypeptides having the amino acid sequence as shown in FIG. 2;
(b) polypeptides having an amino acid sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 2 and having phytase activity; and
(c) fragments of the polypeptide of (a) or (b) having phytase activity.

In a preferred embodiment of a polypeptide of the present invention, said polypeptide is a double domain phytase comprising a first and second phytase domain,
- said first phytase domain having an amino acid sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 3, and
- said second phytase domain having an amino acid sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 4.

In a further preferred embodiment of a polypeptide of the present invention, said first and second phytase domains are separated by a cysteine-rich region having a sequence which is at least 40, preferably at least 50% more preferably at least 55%, 60%, 65%, 70%, 75%, 80%, 85%, 90%, 95%, 96%, 97%, 98% or 99% identical to the amino acid sequence shown in FIG. 5.

In yet another aspect, the present invention provides an isolated nucleic acid molecule encoding a protein having phytase activity selected from the group consisting of:
a) a nucleic acid molecule having a nucleotide sequence which is at least 50% identical to the nucleotide sequence shown in Fig. 1;
b) a nucleic acid molecule hybridizing under stringent conditions to the nucleotide sequence shown in Fig. 1;
c) a nucleic acid molecule encoding a polypeptide having the amino acid sequence which is at least 50% identical to the amino acid sequence shown in Fig. 2 and having phytase activity;
d) a nucleic acid molecule encoding a naturally occurring allelic variant of the polypeptide having the amino acid sequence shown in Fig.2;
e) a nucleic acid molecule complementary to the nucleic acid molecules of any one of a) to d).

In yet another aspect, the present invention provides a recombinant nucleic acid molecule comprising a polynucleotide selected from the group consisting of:
(a) polynucleotides comprising a nucleotide sequence as disclosed in FIG. 1;
(b) polynucleotides comprising a nucleotide sequence encoding a polypeptide as defined above;
(c) polynucleotides comprising a nucleotide sequence the complementary strand of which hybridizes to the polynucleotide of (a) or (b), wherein said nucleotide sequence encodes a polypeptide having phytase activity; and
(d) polynucleotides comprising a nucleotide sequence that deviates from the nucleotide sequence defined in (c) by the degeneracy of the genetic code.

As indicated above a further embodiment of the present invention is provided by two new classes of fungal phytases, exemplified by Phy538 and Phy1900. Also included in said class are homologous proteins that are preferably at least 40, preferably at least 50% more preferably at least 55%, 60%, 65%, 70%, 75%, 80%, 85%, 90%, 95%, 96%, 97%, 98% or 99% identical to the amino acid sequence depicted in Fig. 7 or 8.

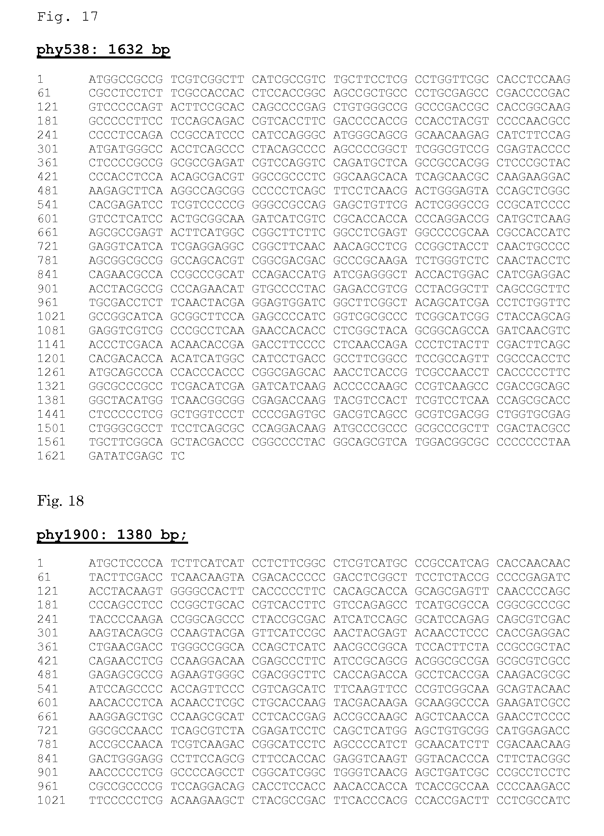

In yet another aspect, the present invention provides an isolated nucleic acid molecule encoding a protein having phytase activity selected from the group consisting of:
a) a nucleic acid molecule having a nucleotide sequence which is at least 40, preferably at least 50% more preferably at least 55%, 60%, 65%, 70%, 75%, 80%, 85%, 90%, 95%, 96%, 97%, 98% or 99% identical to identical to the nucleotide sequence shown in Fig. 17 or 18;
b) a nucleic acid molecule hybridizing under stringent conditions to the nucleotide sequence shown in Fig. 17 or 18;
c) a nucleic acid molecule encoding a polypeptide having the amino acid sequence which is at least 40, preferably at least 50% more preferably at least 55%, 60%, 65%, 70%, 75%, 80%, 85%, 90%, 95%, 96%, 97%, 98% or 99% identical to identical to the amino acid sequence shown in Fig. 7 or 8 and having phytase activity;
d) a nucleic acid molecule encoding a naturally occurring allelic variant of the polypeptide having the amino acid sequence shown in Fig.7 or 8;
e) a nucleic acid molecule complementary to the nucleic acid molecules of any one of a) to d).

In yet another aspect, the present invention provides a recombinant nucleic acid molecule comprising a polynucleotide selected from the group consisting of:
(a) polynucleotides comprising a nucleotide sequence as disclosed in FIG. 17 or 18;
(b) polynucleotides comprising a nucleotide sequence encoding a polypeptide as defined above;
(c) polynucleotides comprising a nucleotide sequence the complementary strand of which hybridizes to the polynucleotide of (a) or (b), wherein said nucleotide sequence encodes a polypeptide having phytase activity; and
(d) polynucleotides comprising a nucleotide sequence that deviates from the nucleotide sequence defined in (c) by the degeneracy of the genetic code.

In a preferred embodiment of a recombinant nucleic acid molecule of the present invention, said recombinant nucleic acid molecule further comprises expression control sequences operably linked to said polynucleotide.

In yet another aspect, the present invention provides a vector comprising the recombinant nucleic acid molecule of the invention as described above.

In yet another aspect, the present invention provides a method for producing genetically engineered host cells comprising introducing the recombinant nucleic acid molecule of the present invention or the vector of the present invention into a host cell.

In yet another aspect, the present invention provides a host cell that is genetically engineered with the recombinant nucleic acid molecule or the vector of the present invention or a host cell that is obtainable by the method of the present invention.

In a preferred embodiment, said host cell is a bacterial, yeast, fungus, plant or animal cell, most preferably a fungus.

In yet another aspect, the present invention provides a method for the production of a polypeptide having phytase activity, comprising cultivating the host cell of the present invention under conditions that allow for the expression of the polynucleotide, and in which the polypeptide encoded by said polynucleotide is isolated from the cells and/or the culture medium.

In yet another aspect, the present invention provides a polypeptide having phytase activity obtainable by a method of the present invention.

In yet another aspect, the present invention provides an antibody specifically recognizing the polypeptide of the present invention.

In yet another aspect, the present invention provides a composition comprising a polypeptide of the present invention. In a preferred embodiment, said composition is a feed, food or an additive for a feed or a food.

In yet another aspect, the present invention provides a process for preparing a feed or a food comprising the step of adding a polypeptide of the present invention to the feed or food components.

In yet another aspect, the present invention provides the use of a polypeptide of the present invention for liberating inorganic phosphate from phytic acid.

### BRIEF DESCRIPTION OF THE DRAWINGS:

Figure 1 shows the complete nucleotide sequence of the ORF encoding the double domain phytase protein of the present invention. Also separately presented are the nucleotide sequences of the two domains.
Figure 2 shows the complete amino acid sequence of the double domain phytase protein of the present invention encoded by the gene in Fig. 1.
Figure 3 shows the sequence of the first phytase domain of the double domain phytase protein of the present invention the complete amino acid sequence of which is displayed in Fig. 2.
Figure 4 shows the sequence of the second phytase domain of the double domain phytase protein of the present invention.
Figure 5 shows the sequence of the cysteine-rich region that separates first and second phytase domains.
**Figure 6****:** Homology tree of selected fungal phytases. The boxed entries are *Magnaporthe* sequences selected for expression.
Figure 7 shows the amino acid sequence based on a manually corrected annotation of a DNA sequence of Phy0538 from *Magnaporthe grisea.*
Figure 8 shows the amino acid sequence of Phy1900 based on the annotation of a DNA sequence from *Magnaporthe grisea.*
Figure 9. Plug-in expression cassette based on the *cbh1* promoter and terminator sequences of *Chrysosporium lucknowense*.
Figure 10. Synthetic expression cassette for phy7789ab.
Figure 11. Synthetic expression cassette for phy7789a.
Figure 12. Synthetic expression cassette for phy7789b.
Figure 13. Synthetic expression cassette for phy0538.
Figure 14. Synthetic expression cassette for phy1900.
Figure 15. Glucoamylase phytase fusion protein (glucoamylase phy7789ab).
Figure 16. DNA sequence coding for the protein of Fig. 15.
Figure 17. Nucleotide sequence of coding region of phy0538.
Figure 18. Nucleotide sequence of coding region of phy1900.

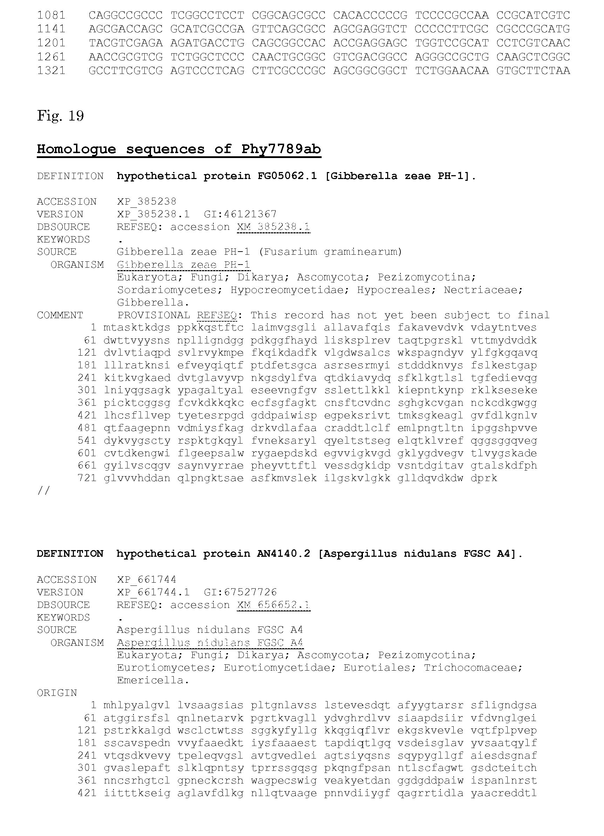

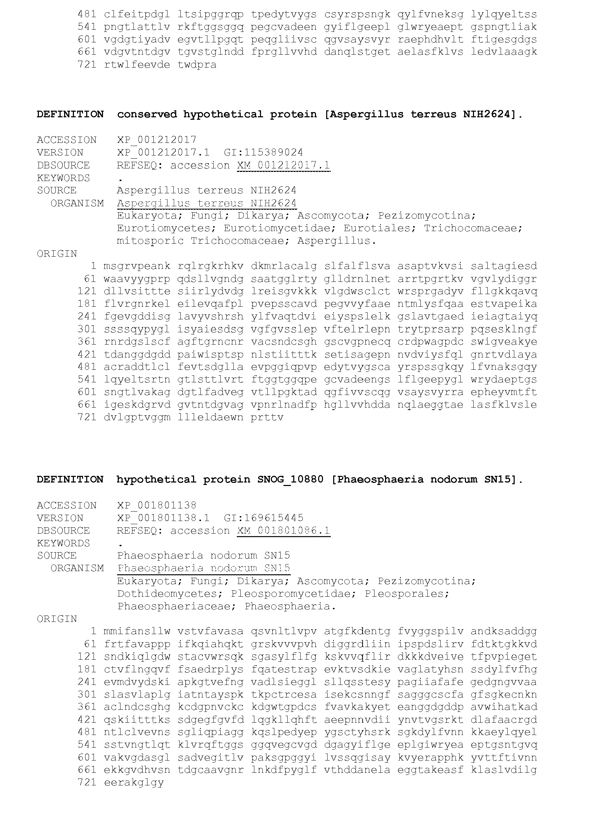

Figure 19. Homologue protein sequences of phy7789ab
Figure 20. Homologue protein sequences of phy0538

### DETAILED DESCRIPTION OF THE INVENTION

### Definitions

The terms "polypeptide", "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The essential nature of such analogues of naturally occurring amino acids is that, when incorporated into a protein, that protein is specifically reactive to antibodies elicited to the same protein but consisting entirely of naturally occurring amino acids. The terms "polypeptide", "peptide" and "protein" are also inclusive of modifications including, but not limited to, glycosylation, lipid attachment, sulfation, gamma-carboxylation of glutamic acid residues, hydroxylation and ADP-ribosylation.

The term "sequence identity," as used herein, is generally expressed as a percentage and refers to the percent of amino acid residues or nucleotides, as appropriate, that are identical as between two sequences when optimally aligned. For the purposes of this invention, sequence identity means the sequence identity determined using the well-known Basic Local Alignment Search Tool (BLAST), which is publicly available through the National Cancer Institute/National Institutes of Health (Bethesda, Maryland) and has been described in printed publications (see, e.g., Altschul et al., J. MoI. Biol, 215(3), 403-10 (1990)). Preferred parameters for amino acid sequences comparison using BLASTP are gap open 11.0, gap extend 1, Blosum 62 matrix.

The term "phytase" is defined herein as a protein or polypeptide which is capable of catalysing the hydrolysis of phytate (inositol hexakisphosphate (IP6) or phytic acid and releasing inorganic phosphate. The term encompasses both 3-phytase (E.C.3.1.3.8), 4-phytase (also referred to as 6-phytase, E.C.3.1.3.26), or 5-phytase (E. C.3.1.3.72) as classified in accordance with the Nomenclature Committee of the International Union of Biochemistry and Molecular Biology. Preferably, the phytase is 3-phytase (E.C.3.1.3.8), 4-phytase (E.C.3.1.3.26) or 5-phytase (E.C.3.1.3.72). The term phytase includes reference to the protein that exhibits peroxidase activity when the vanadate ion is incorporated into the active site of the phytase enzyme. (Van de Velde et al. 2000, Biotech. Bioeng. 67:87-96).

The term "phytase activity" as used herein, refers to the specific enzyme activity of a phytase to catalysing the hydrolysis of phytate or phytic acid expressed in µmol substrate/min/mg protein.

The term "double domain" as used herein, refers to the presence of two related domains in a single protein molecule resulting in an active protein.

As used herein, the term "cysteine-rich region" refers to a protein domain which has an amino acid sequence of about 10-200, preferably about 40-90, more preferably about 50-80 amino acid residues, and even more preferably at least about 60-65 amino acids of which at least about 8-30, preferably about 10-15, and more preferably about 11, 12, 13, or 14 of the amino acids are cysteine residues.

The term "signal peptide" or "signal sequence" as used herein, refers to an amino acid sequence, typically located at the amino terminus of an immature protein or polypeptide (e.g., prior to secretion from a cell and associated processing and cleavage), which directs the secretion of the protein or polypeptide from the cell in which it is produced. The signal peptide typically is removed from an immature protein or polypeptide prior to or during secretion and, thus, is not present in the mature, secreted polypeptide.

As used herein, the term "recombinant nucleic acid molecule" refers to a recombinant DNA molecule or a recombinant RNA molecule. A recombinant nucleic acid molecule is any synthetic nucleic acid construct or nucleic acid molecule containing joined nucleic acid molecules from different original sources or and not naturally occurring or attached together and prepared by using recombinant DNA techniques.

The term "recombinant host cell" as used herein, refers to a host cell strain containing nucleic acid not naturally occurring in that strain and which has been introduced into that strain using recombinant DNA techniques.

The term "nucleic acid" as used herein, includes reference to a deoxyribonucleotide or ribonucleotide polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e. g., peptide nucleic acids). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" (a polymeric form of nucleotides, either ribonucleotides or deoxyribonucleotides, double- or single-stranded of any length) as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including among other things, simple and complex cells.

Every nucleic acid sequence herein that encodes a polypeptide also, by reference to the genetic code, describes every possible silent variation of the nucleic acid. The term "conservatively modified variants" applies to both amino acid and nucleic acid sequences. With respect to particular nucleic acid sequences, conservatively modified variants refer to those nucleic acids which encode identical or conservatively modified variants of the amino acid sequences due to the degeneracy of the genetic code.

The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation.

The term "gene", as used herein, refers to a nucleic acid sequence containing a template for a nucleic acid polymerase, in eukaryotes, RNA polymerase II. Genes are transcribed into mRNAs that are then translated into protein.

"Expression" refers to the transcription of a gene into structural RNA (rRNA, tRNA) or messenger RNA (mRNA) with subsequent translation into a protein.

The term "complementary", as used herein, refers to a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'. As used herein, "substantially complementary" means that two nucleic acid sequences have at least about 40, preferably about 50% more preferably at least 55%, more preferably about 60%, more preferably about 70%, more preferably about 80%, even more preferably 90%, and most preferably about 98%, sequence complementarity to each other.

The term "hybridise" refers to the process by which single strands of nucleic acid sequences form double-helical segments through hydrogen bonding between complementary nucleotides.

As used herein, the term "expression control sequence" refers to a nucleic acid sequence that regulates the transcription and translation of a gene to which it is operatively linked. An expression control sequence is "operatively linked" to a gene when the expression control sequence controls and regulates the transcription and, where appropriate, translation of the gene. The term "operatively linked" includes the provision of an appropriate start codon (e.g. ATG), in front of a polypeptide-encoding gene and maintaining the correct reading frame of that gene to permit proper translation of the mRNA.

As used herein, the term "operably linked" refers to a juxtaposition wherein the components so described are in a relationship permitting them to function in their intended manner. A control sequence "operably linked" to another control sequence and/or to a coding sequence is ligated in such a way that transcription and/or expression of the coding sequence is achieved under conditions compatible with the control sequence. Generally, operably linked means that the nucleic acid sequences being linked are contiguous and, where necessary to join two protein coding regions, contiguous and in the same reading frame.

The term "vector" as used herein, includes reference to an autosomal expression vector and to an integration vector used for integration into the chromosome.

The term "expression vector" refers to a DNA molecule, linear or circular, that comprises a segment encoding a polypeptide of interest under the control of (i.e., operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular an expression vector comprises a nucleotide sequence that comprises in the 5' to 3' direction and operably linked: (a) a fungal-recognized transcription and translation initiation region, (b) a coding sequence for a polypeptide of interest, and (c) a fungal-recognized transcription and translation termination region. "Plasmid" refers to autonomously replicating extrachromosomal DNA which is not integrated into a microorganism's genome and is usually circular in nature.

An "integration vector" refers to a DNA molecule, linear or circular, that can be incorporated in a microorganism's genome and provides for stable inheritance of a gene encoding a polypeptide of interest. The integration vector generally comprises one or more segments comprising a gene sequence encoding a polypeptide of interest under the control of (i.e., operably linked to) additional nucleic acid segments that provide for its transcription. Such additional segments may include promoter and terminator sequences, and one or more segments that drive the incorporation of the gene of interest into the genome of the target cell, usually by the process of homologous recombination. Typically, the integration vector will be one which can be transferred into the host cell, but which has a replicon which is nonfunctional in that organism. Integration of the segment comprising the gene of interest may be selected if an appropriate marker is included within that segment.

"Transformation" and "transforming", as used herein, refers to the insertion of an exogenous polynucleotide into a host cell, irrespective of the method used for the insertion, for example, direct uptake, transduction, f-mating or electroporation. The exogenous polynucleotide may be maintained as a non-integrated vector, for example, a plasmid, or alternatively, may be integrated into the host cell genome.

By "host cell" is meant a cell which contains a vector or recombinant nucleic acid molecule and supports the replication and/or expression of the vector or recombinant nucleic acid molecule. Host cells may be prokaryotic cells such as E. coli, or eukaryotic cells such as yeast, fungus, plant, insect, amphibian, or mammalian cells. Preferably, host cells are fungal cells.

The term "fungus" or "fungi" includes a wide variety of nucleated, spore-bearing organisms which are devoid of chlorophyll. Examples of fungi include yeasts, mildews, molds, rusts and mushrooms. Preferred fungi in aspects of the present invention are organisms of the genera Aspergillus, Trichoderma, Myceliophtora, Rhizopus, Magnaporthe, Chrysosporium, Gibberella, Phaeosphaeria, Chaetomium , Coprinopsis, Ajellomyces, Penicillium, Thielavia, Corynascus, Agrocybe, Peniophora, Trametes, Laccaria, Ceriphora, Neurospora, Humicola, Talaromyces, Neosartorya.

The terms "isolated" or "purified" as used herein refer to a nucleic acid or protein or peptide that is removed from at least one component with which it is naturally associated. In the present invention, an isolated nucleic acid can include a vector comprising the nucleic acid. Purified as used herein to describe a polypeptide produced by cultivation of a recombinant host cell refers to removing that polypeptide from at least one component with which it is naturally associated in the host cell or culture medium.

### Description of the preferred embodiments

The amino acid sequence of the double domain phytase protein of the present invention is, in a preferred embodiment, the polypeptide as shown in FIG. 2.

This double domain protein resembles a phytase with very low levels of sequence homology (sequence identity to Bacillus sequence in US6638746 is 36%) with the phytases presently known. Despite being encoded by a fungal gene, the protein exhibits the highest level of sequence homology with *Bacillus* phytases. The finding that genes in the natural fungal genome are present that encode proteins that resemble bacterial-type phytases is a surprising finding. The expression of such bacterial phytase genes in fungi has hitherto not been described. The gene as presently identified and presented in FIG. 1, comprises two phytase domains in a single open reading frame and phytase activity has yet only been observed for the double domain protein. It is, however, expressly noticed that the individual phytase domains are part of the present invention. Although known in bacteria, phytase genes having a double phytase domain structure are poorly studied and have hitherto not been reported in the fungal genome.

The phytases of the present invention may be expressed in any host cell, but are preferably expressed in fungal cells.

Phytase producing organisms may not only be obtained by using recombinant techniques and expression vectors. Alternatively, DNA fragments may be generated using restriction-endonuclease digestion or PCR the expression of the endogenous gene in an organism may be controlled by methods known in the art, which expression may be altered by any available method for genetic modification, for instance via alteration of the regulation of the expression.

In another preferred embodiment, a polypeptide of the present invention has an amino acid sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 2 wherein said polypeptide exhibits phytase activity.

The skilled person is well aware how phytase activity can be determined for such polypeptides. For instance, the polypeptide can be synthesized or expressed in a host cell and the optionally purified polypeptide can be brought in contact with a phytate or phytic acid substrate under conditions of temperature, salinity, pH and ions and the like that allow for the enzymatic hydrolysis of the substrate by phytases. As a reference enzyme a commercially available bacterial or fungal phytase can be used. The hydrolysis of phytate or phytic acid may be determined by measuring the liberation of inorganic phosphates by spectrophotometric, chromatographic or chemical assay methods.

In another preferred embodiment, a polypeptide of the present invention may be a fragment of the polypeptide as shown in FIG. 2, such as for instance the fragment shown in FIG. 3 or the fragment shown in FIG. 4, preferably having phytase activity, or a polypeptide of the present invention may be a fragment of the polypeptide having an amino acid sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 2 as described above, having phytase activity.

Whether a polypeptide or fragment thereof exhibits phytase activity as required by the present invention may be established by using a suitable enzymatic assay. One suitable assay for the measurement or detection of phytase activity is provided in the Examples below.

In a highly preferred embodiment, a polypeptide of the present invention is a double domain phytase comprising a first and second phytase domain, wherein said first phytase domain has an amino acid sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 3, and wherein said second phytase domain has an amino acid sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 4. Alternatively, the double domain phytase comprises two or more copies of one of these domains.

The first phytase domain as described above is preferably located at the 5' end, while the second phytase domain as described above is preferably located at the 3' end of the gene encoding the phytase of the invention. It should be noted that the order in which the domains are ordered is not limiting, the domain ordering may be in principle be AB, BA, AA and BB (wherein A and B denote the first and second domain). Even proteins with more than two domains would be considered covered, and of course any permutation of these domains is intended.

The present invention also contemplates the use of fusion proteins, accomplished by the use of a gene fusion, for instance with a carrier protein such as glucoamylase in order to increase secretion levels of the phytase protein.

The first and second phytase domains may be separated by a cysteine-rich region. Such a region is generally characterized by an amino acid sequence having an unusual high level of cysteine residues, for instance more than 10%, preferably around 20%, of the amino acid residues being cysteine. The cysteine-rich region serves as a region connecting and separating the two phytase domains of the double domain phytase of the present invention. This region may suitably be between 10 and 200 amino acid residues in length. A fungal double domain phytase of the present invention preferably comprises a cysteine-rich region having a sequence which is at least 40, preferably at least 50% more preferably at least 55%, 60%, 65%, 70%,75%, 80%, 85%, 90%, 95%, 96%, 97%, 98% or 99% identical to the amino acid sequence shown in FIG. 5. Most preferably the region consists of the amino acid sequence shown in FIG. 5. It should be noted that all uncharacterized database examples of fungal proteins are proteins with double domains carrying a Cysteine linker. There are very few uncharacterized bacterial proteins **from** *Pseudomonas* or *Pseudoalteromonas* with double domain but without Cysteine domain; e.g. emb|CAI85536.1| putative phytase domain protein [*Pseudoalteromonas haloplanhtis*].

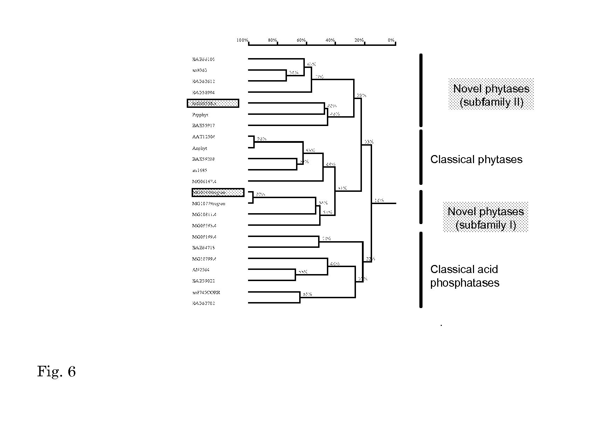

In another embodiment the present invention comprises two new classes of phytases, characterized by the two exemplary sequences phy0538 and phy1900 (of which the amino acid sequence is given in Figures 7 and 8 respectively, and of which the nucleotide sequence is given in Fig. 17 and 18, respectively). Both protein sequences are derived from the fungus *Magnaporthe grisea*. As indicated in the experimental part, these phytases do not resemble the currently known phytases. Further they form, together with the orthologues indicated in Example 1 two separate , new groups of phytases, as is indicated in Figure 6.

Although the orthologues listed have a relatively low identity with the sequences of phy538 and phy1900, they can be classified in distinct groups since the sequence identity with the classical phytases and acid phosphatases is even much lower (see fig. 6).

As indicated above a further embodiment of the present invention is provided by two new classes of fungal phytases, exemplified by Phy538 and Phy1900. Also included in said class are homologous proteins that are preferably at least 40, preferably at least 50% more preferably at least 55%, 60%, 65%, 70%, 75%, 80%, 85%, 90%, 95%, 96%, 97%, 98% or 99% identical to the amino acid sequence depicted in Fig. 7 or 8.

In yet another aspect, the present invention provides an isolated nucleic acid molecule encoding a protein having phytase activity selected from the group consisting of:
a) a nucleic acid molecule having a nucleotide sequence which is at least 40, preferably at least 50% more preferably at least 55%, 60%, 65%, 70%, 75%, 80%, 85%, 90%, 95%, 96%, 97%, 98% or 99% identical to identical to the nucleotide sequence shown in Fig. 17 or 18;
b) a nucleic acid molecule hybridizing under stringent conditions to the nucleotide sequence shown in Fig. 17 or 18;
c) a nucleic acid molecule encoding a polypeptide having the amino acid sequence which is at least 40, preferably at least 50% more preferably at least 55%, 60%, 65%, 70%, 75%, 80%, 85%, 90%, 95%, 96%, 97%, 98% or 99% identical to identical to the amino acid sequence shown in Fig. 7 or 8 and having phytase activity;
d) a nucleic acid molecule encoding a naturally occurring allelic variant of the polypeptide having the amino acid sequence shown in Fig.7 or 8;
e) a nucleic acid molecule complementary to the nucleic acid molecules of any one of a) to d).

In yet another aspect, the present invention provides a recombinant nucleic acid molecule comprising a polynucleotide selected from the group consisting of:
(a) polynucleotides comprising a nucleotide sequence as disclosed in FIG. 17 or 18;
(b) polynucleotides comprising a nucleotide sequence encoding a polypeptide as defined above;
(c) polynucleotides comprising a nucleotide sequence the complementary strand of which hybridizes to the polynucleotide of (a) or (b), wherein said nucleotide sequence encodes a polypeptide having phytase activity; and
(d) polynucleotides comprising a nucleotide sequence that deviates from the nucleotide sequence defined in (c) by the degeneracy of the genetic code.

A polypeptide of the present invention may comprise a signal sequence, or may be fused to additional polypeptide sequences, such as for instance to a signal peptide, which signal sequence or peptide may contribute to excretion of the produced polypeptide from the host cell of the invention. A preferred signal sequence is the signal sequence as shown in FIGS. 2, 3 and 4.

Recombinant nucleic acid molecules of the present invention comprise polynucleotides that encode for polypeptides and phytases of the present invention.

One such polynucleotide comprises a nucleotide sequence as disclosed in FIG. 1. This polynucleotide encodes a polypeptide according to FIG. 2. The skilled person will understand that other polypeptides will also encode a functional phytase as described in FIG. 2, for instance polynucleotides comprising a nucleotide sequence that deviates from the nucleotide sequence shown in FIG. 1 by the degeneracy of the genetic code will also encode a functional enzyme.

In general all recombinant nucleic acid molecules comprising a polynucleotide comprising a nucleotide sequence encoding a polypeptide or phytase of the present invention as defined above are part of the present invention.

Also part of the present invention are recombinant nucleic acid molecules comprising a polynucleotide comprising a nucleotide sequence the complementary strand of which hybridizes to the polynucleotide encodes a polypeptide according to FIG. 2 or deviating therefrom due to the degeneracy of the genetic code.

Also part of the present invention are recombinant nucleic acid molecules comprising a polynucleotide comprising a nucleotide sequence encoding a polypeptide having phytase activity according to the present invention and polynucleotides deviating therefrom by the degeneracy of the genetic code.

The recombinant nucleic acid molecule of the present invention may further comprise one or more expression control sequences, such as a promoter, a ribosome binding site, a transcription initiation region, a transcription termination region, operably linked to said polynucleotide. As used herein "promoter" is a DNA sequence that directs the transcription of a gene. Typically, a promoter is located in the 5'-region of a gene, proximal to the transcriptional start site of a gene. Promoter sequences useful in aspects of the present invention may be constitutive, inducible or repressible. If a promoter is an inducible promoter, then the rate of transcription increases in response to an inducing agent. Preferred promoter sequences are sequences functional in fungi.

In order to achieve optimal expression in the host cell of interest, such as a fungal cell, the codon (pair) usage of the polynucleotides encoding the polypeptides and phytases of the present invention may be optimized by using any one of a variety of synthetic gene design software packages, for instance GeneOptimizer® from Geneart AG (Regensburg, Germany) for codon usage optimization or for codon pair usage optimization. Such adaptation of codon usage ensures that the gene, which is essentially of fungal origin, is effectively processed by the projected host cell's transcription and translation machinery. Optimization of codon (pair) usage will generally result in enhanced protein expression in the host cell.

The recombinant nucleic acid molecule of the present invention may for instance be cloned into a high-copy-number plasmid or other suitable vector system for transformation of and subsequent expression in the host cell.

Recombinant host cells can be obtained using methods known in the art for providing cells with recombinant nucleic acids. These include transformation, transconjugation, transfection or electroporation of a host cell with a suitable plasmid or vector comprising the nucleic acid construct of interest operationally coupled to a promoter sequence to drive expression. Host cells of the invention are preferably transformed with a recombinant nucleic acid molecule of the present invention and may comprise a single but preferably comprise multiple copies of the nucleic acid construct. The nucleic acid construct may be maintained episomally and thus comprise a sequence for autonomous replication, such as an ARS sequence. Suitable episomal nucleic acid constructs may e.g. be based on the yeast 2µ or pKD1 (Fleer et al., 1991, Biotechnology 9: 968-975) plasmids. Preferably, however, the nucleic acid construct is integrated in one or more copies into the genome of the host cell. Integration into the host cell's genome may occur at random by illegitimate recombination but preferably the nucleic acid construct is integrated into the host cell's genome by homologous recombination as is well known in the art of fungal molecular genetics (see e.g. WO 90/14423, EP-A-0 481 008, EP-A-0 635 574 and US 6,265,186).

Transformation of host cells with the nucleic acid constructs of the invention and additional genetic modification of the fungal host cells of the invention as described above may be carried out by methods well known in the art. Such methods are e.g. known from standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

Promotors suitable to drive expression of the genes in the hosts of the invention include e.g. promoters from glycolytic genes (e.g. from a glyceraldehyde-3-phosphate dehydrogenase gene), ribosomal protein encoding gene promoters, alcohol dehydrogenase promoters (ADH1, ADH4, and the like), promoters from genes encoding amylo- or cellulolytic enzymes (glucoamylase, TAKA-amylase and cellobiohydrolase). Other promoters, both constitutive and inducible and enhancers or upstream activating sequences will be known to those of skill in the art. The promoters used in the nucleic acid constructs of the present invention may be modified, if desired, to affect their control characteristics. Preferably, the promoter used in the nucleic acid construct for expression of the genes is homologous to the host cell in which genes are expressed.

In the nucleic acid construct, the 3'-end of the coding nucleotide acid sequence(s) preferably is/are operably linked to a transcription terminator sequence. Preferably the terminator sequence is operable in a host cell of choice. In any case the choice of the terminator is not critical; it may e.g. be from any fungal gene, although terminators may sometimes work if from a non-fungal, eukaryotic, gene. The transcription termination sequence further preferably comprises a polyadenylation signal.

Optionally, a selectable marker may be present in the nucleic acid construct. As used herein, the term "marker" refers to a gene encoding a trait or a phenotype which permits the selection of, or the screening for, a host cell containing the marker. A variety of selectable marker genes are available for use in the transformation of fungi. Suitable markers include auxotrophic marker genes involved in amino acid or nucleotide metabolism, such as e.g. genes encoding ornithine-transcarbamylases (argB), orotidine-5'-decaboxylases (pyrG, URA3) or glutamine-amido-transferase indoleglycerol-phosphate-synthase phosphoribosyl-anthranilate isomerases (trpC), or involved in carbon or nitrogen metabolism, such e.g. niaD or facA, and antibiotic resistance markers such as genes providing resistance against phleomycin, bleomycin or neomycin (G418). Preferably, bidirectional selection markers are used for which both a positive and a negative genetic selection is possible. Examples of such bidirectional markers are the pyrG (URA3, pyr4), pyrE (ura5, pyr5) facA and amdS genes. Due to their bidirectionality these markers can be deleted from transformed filamentous fungus while leaving the introduced recombinant DNA molecule in place, in order to obtain fungi that do not contain selectable markers. This essence of this MARKER GENE FREE™ transformation technology is disclosed in EP-A-0 635 574, which is herein incorporated by reference. Of these selectable markers the use of dominant and bidirectional selectable markers such as acetamidase genes like the amdS genes of *A. nidulans, A. niger* and *P. chrysogenum* is most preferred. In addition to their bidirectionality these markers provide the advantage that they are dominant selectable markers that, the use of which does not require mutant (auxotrophic) strains, but which can be used directly in wild type strains.

Optional further elements that may be present in the nucleic acid constructs of the invention include, but are not limited to, one or more leader sequences, enhancers, integration factors, and/or reporter genes, intron sequences, centromers, telomers and/or matrix attachment (MAR) sequences. The nucleic acid constructs of the invention may further comprise a sequence for autonomous replication, such as an ARS sequence. Suitable episomal nucleic acid constructs may e.g. be based on the yeast 2µ or pKD1 (Fleer et al., 1991, Biotechnology 9: 968-975) plasmids. Alternatively the nucleic acid construct may comprise sequences for integration, preferably by homologous recombination (see e.g. WO98/46772). Such sequences may thus be sequences homologous to the target site for integration in the host cell's genome. The nucleic acid constructs of the invention can be provided in a manner known per se, which generally involves techniques such as restricting and linking nucleic acids/nucleic acid sequences, for which reference is made to the standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987).

The transformed host cell comprising the recombinant nucleic acid molecule or the vector of the present invention can be used to produce the polypeptides of the present invention. As stated above, the host cell is preferably a fungal cell, such as a yeast cell, but may also be a bacterial cell. The polypeptide of the present invention can then be produced by cultivating the host cell under conditions that allow said host cell to replicate and express the polynucleotide encoding the polypeptide of the invention. Such conditions depend on the specific host cell applied, but are generally are well known to those of skill in the art. The cultivation may be on solid media, but is preferably in liquid medium, the cells may be freely suspended or may, alternatively, be immobilized to a support. Suitably, the cultivation medium may comprise agents that induce the expression of the polynucleotide in the host cell.

A method for the production of a polypeptide having phytase activity further comprises the step of isolating the polypeptide from the cells and/or the culture medium, depending on whether the polypeptide is excreted or remains associated with the host cells. When a suitable signal sequence, such as MVASSIGVLLAAVLLGGTSTIVTA or other suitable signal sequence, is incorporated at for instance the N-terminus of the polypeptide, the polypeptide may be transported to the endoplasmatic reticulum and excreted from the cells.

Once excreted to the culture medium, the polypeptide can be easily purified therefrom by using chromatographic or immunocapture techniques well known in the art. Chromatographic techniques may include liquid chromatography such as HPLC, size exclusion chromatography or affinity chromatography.

If not excreted to the medium the polypeptides of the invention may be isolated form the host cell by disrupting or lysing the host cells, separating the membrane fragments from the cell contents and purifying the polypeptide from either of these using the same techniques as described above. Suitable alternative protein purification techniques are well known to the skilled person and may be employed in methods of the present invention.

Once purified, the polypeptide of the present invention may be used for immunization of a mammal and the antibodies raised may in the mammal may be isolated and purified, optionally after producing hybridoma cell lines with isolated spleen cells producing specific antibodies, to produce polyclonal or monoclonal antibodies against the polypeptides of the invention.

The phytases of the present invention can be incorporated into any composition, preferably an animal feed, a human food product, or can be used as an additive or supplement for a feed or food product, most preferably a feed for non-ruminant animals, most preferably poultry or swine. An effective amount of phytase in food or feed is from about 10 to 20,000 FTU/kg; preferably from about 10 to 15,000 FTU/kg, more preferably from about 10 to 10,000 FTU/kg, in particular from about 100 to 5,000 FTU/kg, especially from about 100 to about 2,000 FTU/kg feed or food. The analytical method for determining microbial phytase activity and the definition of a phytase unit has been published by Engelen et al. (Journal of AOAC International 77 (3): 760-764 (1994).

In addition to its major application in animal nutrition (including livestock and crustaceans and fish), phytase is also used for processing of human food. Research in this field focuses on better mineral absorption and technical improvement of food processing such as bread making, production of plant protein isolates, corn wet milling and the fractionation of cereal bran. Also applications relating to biodefense and to biomass conversion for fuels development are foreseen. The protein may be provided in solution or in dry enzyme preparations.

The invention will now be illustrated in more detail by the following non-limiting Examples.

### Examples

### Example 1 Genome mining

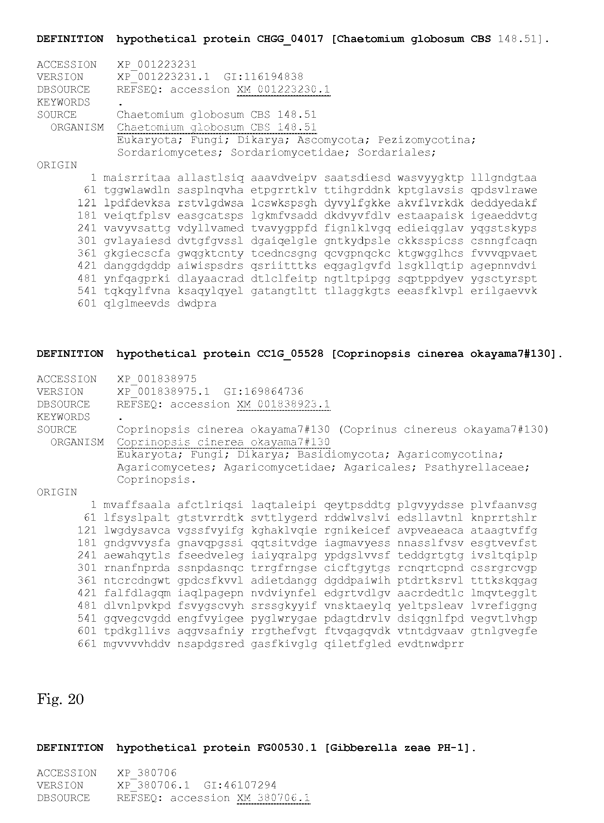

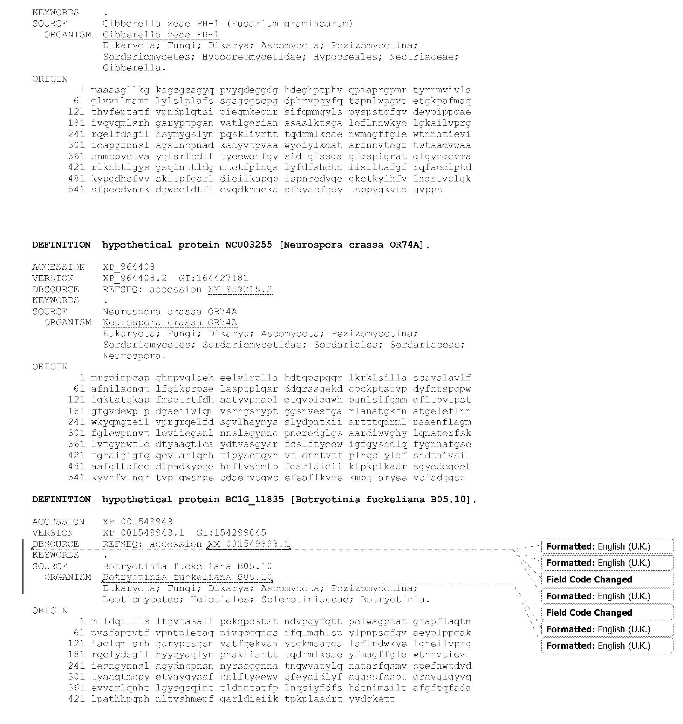

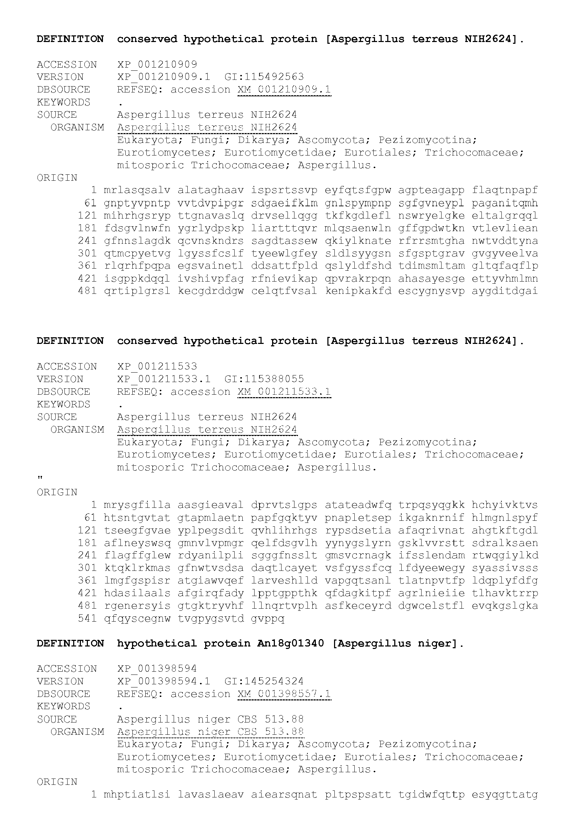

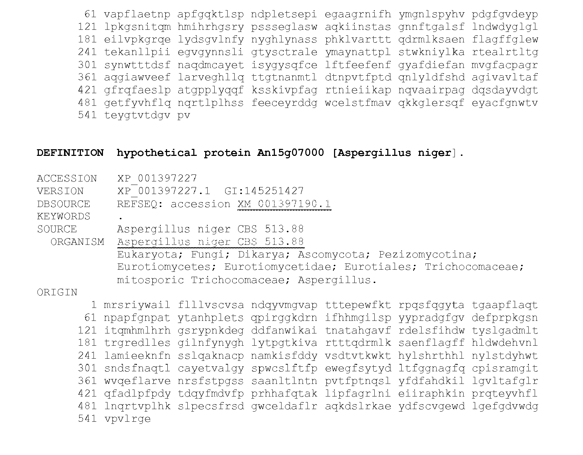

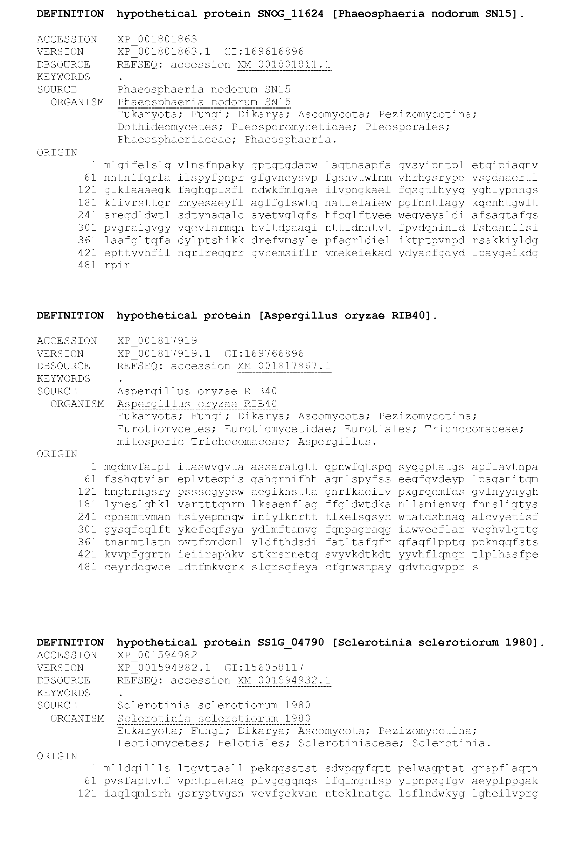

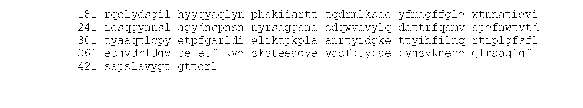

Genome mining in *Magnaporthe grisea* with phytase of *Aspergillus* and of *Bacillus* resulted in the identification of three new classes of fungal phytases:
1) a completely new class of fungal phytases, resembling bacterial phytases. This phytase consist of a double phytase domain fused by a linker region. Further genome mining showed that also the genomes from *Giberella zeae* (hypothetical protein FG05062.1 Identities = 414/765 (54%), Positives = 508/765 (66%)), *Aspergillus nidulans*, (hypothetical protein AN4140.2 (Identities = 365/752 (48%), Positives = 486/752 (64%)), *Aspergillus terreus* (XP_001212017, Identities = 368/757 (48%), Positives = 484/757 (63%), *Phaeosphaeria nodorum* (hypothetical protein SNOG_10880 Identities = 330/737 (44%), Positives = 429/737 (58%), *Chaetomium globosum* (hypothetical protein CHGG_04017 Identities = 288/590 (48%), Positives = 371/590 (62%)) and *Coprinopsis cinerea* (hypothetical protein CC1G_05528, Identities = 301/744 (40%), Positives = 408/744 (54%), carry a similar gene. None of these genes have been previously described. The *Magnaporthe* gene was manually annotated. This polypeptide sequence is shown in Figure 2 (phy7789ab).
(2) In addition also two diverse fungal type phytases were identified from *Magnaporthe grisea* (Phy538 and Phy1900, Fig 6, 7 and 8). From Phy538, homologous proteins are present in *Giberella zeae* (hypothetical protein FG00530.1,Identities = 350/549 (63%), Positives = 404/549 (73%), *Neurospora crassa*, (hypothetical protein NCU03255, Identities = 324/502 (64%), Positives = 381/502 (75%), *Botryotinia fuckeliana*, (hypothetical protein BC1G_11835, Identities = 302/450 (67%), Positives = 350/450 (77%), *Aspergillus terreus* (XP_001210909.1, Identities = 263/544 (48%), Positives = 343/544; XP_001211533, Identities = 236/506 (46%), Positives = 320/506 (63%), *Aspergillus niger* (hypothetical protein An18g01340, Identities = 248/531 (46%), Positives = 334/531 (62%); hypothetical protein An15g07000 , Identities = 240/528 (45%), Positives = 313/528 (59%), *Phaeosphaeria nodorum* (hypothetical protein SNOG_11624, *A.oryzae* (BAE55917, Identities = 234/521 (44%), Positives = 312/521 (59%), and *Sclerotinia sclerotiorum*, (hypothetical protein SS1G_04790, Identities = 181/284 (63%), Positives = 214/284 (75%). See also Fig. 20.

Phy1900 is very dissimilar to all known phytases. In blasting the sequence, the highest scoring sequences were present in *Sclerotinia sclerotiorum* (hypothetical protein SS1G_09959, Identities = 202/468 (43%), Positives = 282/468 (60%), *Botryotinia fuckeliana*, hypothetical protein BC1G_07284, Identities = 197/442 (44%), Positives = 272/442 (61%), *Aspergillus fumigatus*, (CAD12029, Identities = 176/436 (40%), Positives = 253/436 (58%), *Thielavia heterothallica* , (Identities = 187/463 (40%), Positives = 257/463 (55%), *Aspergillus niger* (Identities = 190/468 (40%), Positives = 269/468 (57%); XP_001401713 , (Identities = 186/467 (39%), Positives = 265/467 (56%)), *Aspergillus ficuum*, (Identities = 186/467 (39%), Positives = 265/467 (56%)), *Aspergillus awamori* , P34753 (Identities = 186/466 (39%), Positives = 262/466 (56%)), *Aspergillus oryzae*, (Identities = 176/463 (38%), Positives = 264/463 (57%),

From *Magnaporthe grisea* Phy 538 and Phy 1900 were annotated (Figure 7 (phy0538) and Figure 8 (phy1900)).

### Example 2 Phytase expression cassettes

A plug-in expression cassette based on the *cbh1* promoter and terminator sequences of *Chrysosporium lucknowense* was synthesized in vivo (GENEART). The *NcoI* and the *EcoRV* sites were present as cloning sites. Hundred fifty base pairs of the 3' end of the *cbh1* promoter (from NcoI until ATG) are introduced with the gene of interest. Fifteen hundred base pairs upstream the *NcoI* site a synthetic *amdS* gene was synthesized, based on the *Aspergillus nidulans amdS* gene. *C. lucknowense* strains are naturally *amdS-*negative. The *amdS* gene encodes an amidase, and introduction of this gene into *C. lucknowense* allows the strain to grow on acetamide as sole nitrogen source. The introns and most of the six-base restriction enzymes were removed in this *amdS* gene. Fivehundred basepairs of the cbh1 promoter upstream the amdS were also introduced downstream the *amdS* marker to create a repeat flanked selection marker. This repeat flanked *amdS* marker can be removed with *StuI*. The sequence of this expression cassette including the *amdS* marker is shown in Figure 9.

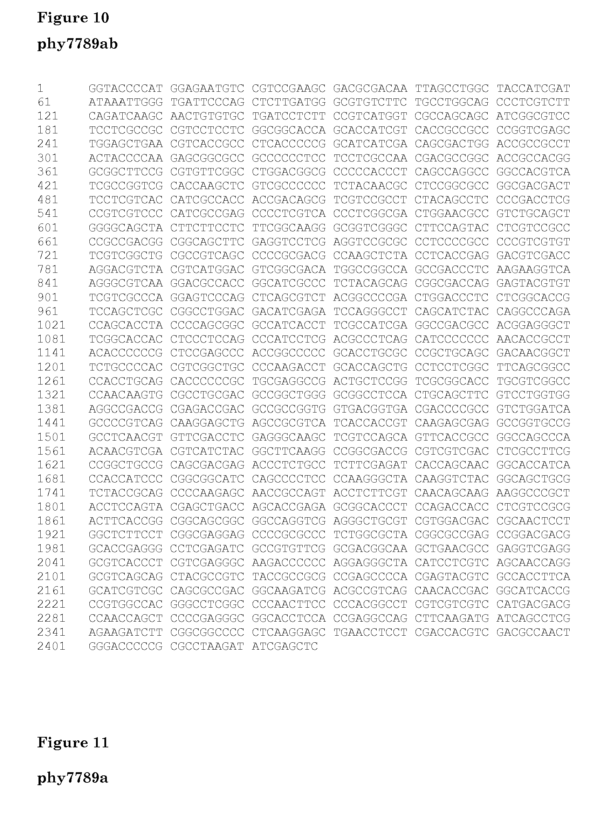

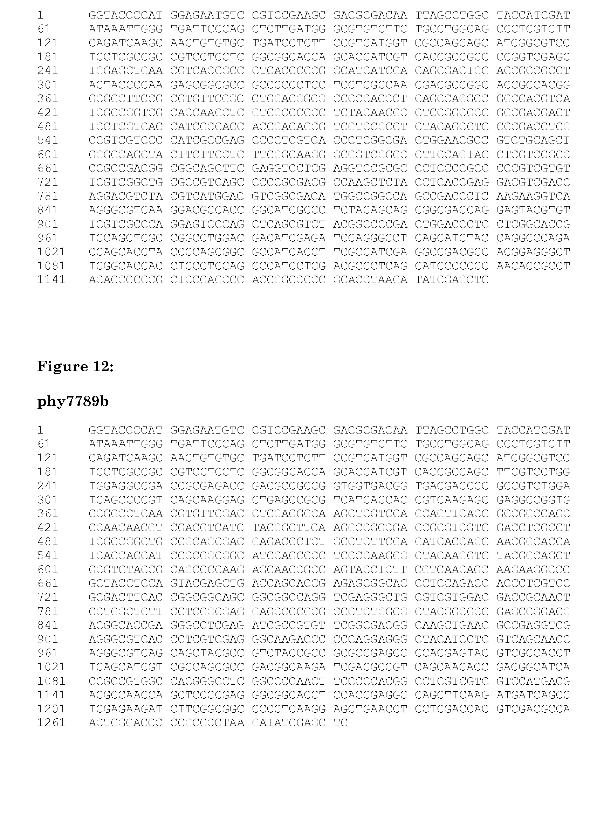

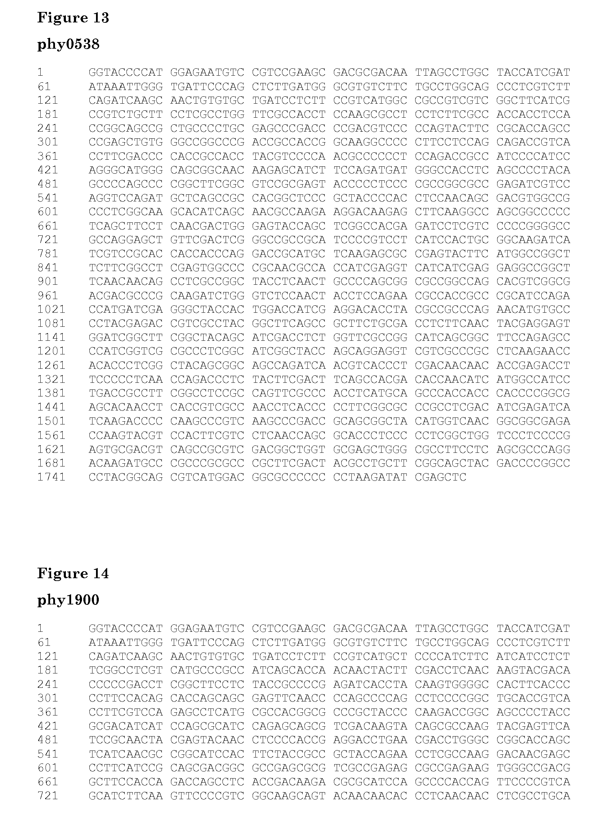

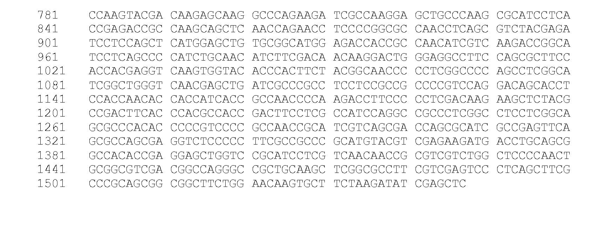

From the polypeptide sequence of the bacillus type phytase phy7789ab three gene versions were designed and synthesized in vivo (GENEART). The codons were optimized for *C. lucknowense*. The double domain (phy7789ab, Figure 10)) and each of the separate mono-domain genes (phy7789a, (Figure 11) and phy7789b (Figure 12)) were made in all cases carrying the native signal sequence. The nucleotide sequence of these three genes can be found in Figure 10, 11 and 12. The genes for phy0538 and phy1900 were designed and synthesized in vivo (GENEART). The codons were optimized for *C. lucknowense*. The nucleotide sequence of these genes can be found in Figure 13, respectively Figure 14. To facilitate the construction of the final expression cassette 150 bp of the *cbh1* promoter were also synthesized at the 3' of the gene starting with an *NcoI* site. At the 5' end of the gene an *EcoRV* site was introduced for cloning purposes.

The phytase genes were cloned with *NcoI* and *EcoRV* in the expression vector. The expression cassettes were digested with *NotII* and isolated to obtain E.coli DNA free fragments for transformation of *C. lucknowense*.

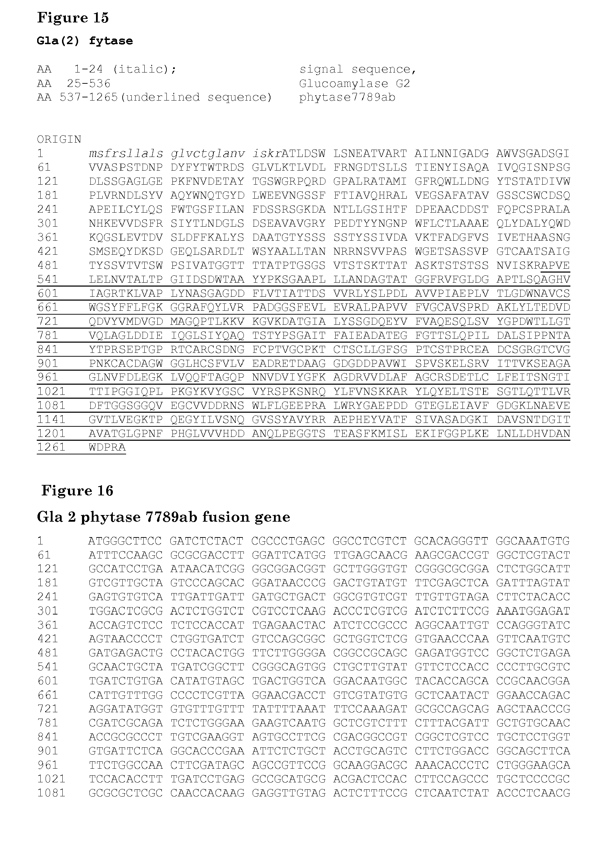

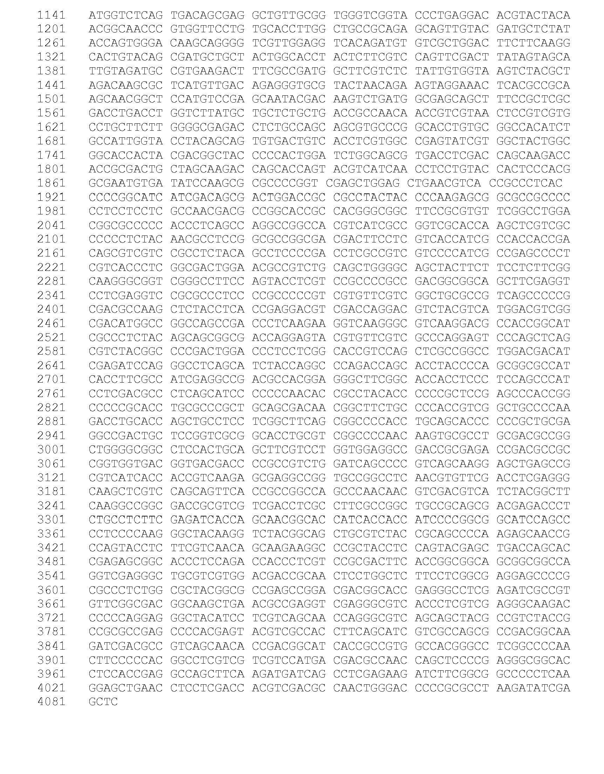

Another non-limiting example of this invention encompasses these phytases linked to glucoamylase with the KEX processing site to increase the expression and production of these phytases. In Heerikhuisen (Heerikhuisen, M., van den Hondel, C.A.M.J.J. and P.J. Punt (2005) Aspergillus sojae. In: Production of Recombinant Proteins. Novel microbial and eukaryotic expression systems. (Gerd Gellissen, Ed). Wiley-VCH Verlag, Weinheim. pp. 191-214) is described that fusion of heterologous proteins to glucoamylase results in higher production of heterologous proteins. An example of a glucoamylase phytase fusion protein (glucoamylase phy7789ab) before processing of both KEX-sites is shown in Figure 15. The nucleotide sequence encoding this protein is shown in Figure 16.

### Example 3 Transformation of Chrysosporium lucknownense

For transformation of *Chrysoporium lucknowense* low protease mutants were used. These protease mutants may be obtained not only by disrupting actual protease encoding genes. Exemplary low protease strains include, but are not limited to, a mutant of strain UV18-25, which has been mutated with ultraviolet light and selected for low protease activity, denoted herein as UV18#100.f; a strain denoted UV18#100.f Δpyr5 Δalp1, in which the protease gene alp1 has been selectively disrupted; and a strain denoted UV18#100.f Δpyr5 Δalp1 Δpep4 Δalp2, in which three protease genes, alp1, pep4, and alp2 have been disrupted.

The protocol is based on a procedure originally used for *Aspergillus* transformation (P. Punt, C. van den Hondel, Methods in Enzymology 1992 216:447-457). Rich medium (250 ml) was inoculated with 10⁶ spores/ml of a *Chrysosporium lucknowense* protease mutant in a1L Erlenmeyer flask. The culture was grown for 24-36 hours at 35 °C in an air incubator 25 (300 rpm). The mycelium was filtered through a sterile Miracloth(TM) filter (Calbiochem) and washed with ca. 100 ml 1700 mOsmol NaCl/CaCl₂ (0.27 M CaCl₂/ 0.6 M NaCl). The mycelium was weighed and then kept on ice. Caylase(TM) (Cayla) was added (20 mg per gram mycelium) and 1700 mOsmol NaCl/CaCl₂ (3.3ml/g mycelium) and the suspension was incubated in a 33 °C air incubator (100 rpm). The protoplasting was followed under the microscope. After 1-3 hours of incubation, most of the mycelium was digested, leaving mostly protoplasts in the microscopic view of the preparation. The protoplasts were filtered through a sterile Myracloth filter and the filter was washed with 1 volume cold STC1700 (1.2 M sorbitol/ 10 mM Tris.HCl pH 7.5/ 50 mM CaCl₂/ 35 mM NaCl). The protoplasts were spun down at 2500 rpm for 10 minutes at 4 °C. The pellet was resuspended in STC1700 and centrifuged again. After resuspending the pellet in STC1700, the protoplasts were counted. STC1700 was added to a final concentration of 2 x 10⁸ protoplasts per ml.

DNA fragment of the phytase expression vector (5-10 µg) was pipetted into the bottom of a sterile tube and 1 µl 1M ATA (aurintricarbonic acid) and 100 µl protoplasts (ca. 2 x 107) were added to the DNA. A minus DNA negative control was included in the experiment. After mixing, the protoplasts were incubated at room temperature for 25 minutes. 1350 µl PEG6000 (60% PEG/50 mM CaCl₂/ 10 mM Tris pH 7.5) was added drop by drop and mixed. The solution was kept at room temperature for 20 minutes. The tubes were then filled with 8 ml STC1700, mixed and centrifuged at 2500 rpm for 10 minutes at 4°C, and the pellet was suspended in 250 µl STC1700. Aliquots of the sample were used for plating on selective medium. For *amdS* + selection, plates were prepared containing 1.5% Oxoid agar, 1.2 M sorbitol, 2mM MgSO₄.7 H₂O, 1x trace elements, 1% glucose, 1x AspA without nitrate, 15mM CsCl and 10mM acetamide. The plates were incubated at 30 or 35°C.

The spores and viable protoplasts before and after PEG6000 treatment were counted by plating dilutions in STC1700 on minimal medium plates with nitrate and with or without sorbitol. 100 µl of 10⁻¹, 10⁻² and 10⁻³ dilutions were plated on plates without sorbitol to count for spores and 100 µl of 10⁻², 10⁻³, 10⁻⁴ and 10⁻⁵ dilutions were plated on plates with sorbitol to count the viable protoplasts.

### Example 4 Selection of transformants

Twelve transformants of UV18#100.f Δpyr5 Δalp1 Δpep4 Δalp2 Phy7789ab, were purified. The transformants were grown in 3 ml cultures. Phytase activity (as described in Piddington et al, (1993) Gene 133. 55-62) was measured in these medium samples. Activity could be measured in some medium samples of the transformants. A transformant was selected for controlled fermentation (UV18#100.f Δpyr5 Δalp1 Δpep4 Δalp2 Phy7789ab#3). Southern analysis showed that 2-3 phytase expression copies were integrated in each transformant.

### Example 4 determination of phytase activity

The transformant was grown in controlled fed batch fermentation. Phytase activity was determined in end-of-fermentation samples as described in Piddington et al at pH5.5 and at pH 7.0 with the addition of 1mMCaCl₂.

| | Phytase pH7 +1mMCaCl₂ U/ml | Phytase pH 4.5 U/ml |
|---|---|---|
| Phytase 7789ab | | |
| UV18#100.f Δalp1Δpep4Δalp2 #87-2 | | |
| 7789ab #3 | 0,23 | 0.09 |
| | 0.20 | |
| Parent strain | 0.01 | 0.05 |
| | 0.01 | 0.05 |

In another non-limiting example of this invention the phytase activity from phytase produced by glucoamylase phytase fusions was measured.

## Claims

1. An isolated polypeptide selected from the group consisting of:
(a) polypeptides having the amino acid sequence as shown in FIG. 2;
(b) polypeptides having an amino acid sequence which is at least 40% identical to the amino acid sequence shown in FIG. 2 and having phytase activity;
(c) fragments of the polypeptide of (a) or (b) having phytase activity.

2. Polypeptide according to claim 1, wherein said polypeptide is a double domain phytase comprising a first and second phytase domain,
- said first phytase domain having an amino acid sequence which is at least 40% identical to the amino acid sequence shown in FIG. 3, and
- said second phytase domain having an amino acid sequence which is at least 40% identical to the amino acid sequence shown in FIG. 4.

3. Polypeptide according to claim 2, wherein said first and second phytase domains are separated by a cysteine-rich region having a sequence which is at least 40% identical to the amino acid sequence shown in FIG. 5.

4. An isolated nucleic acid molecule encoding a protein having phytase activity selected from the group consisting of:
a) a nucleic acid molecule having a nucleotide sequence which is at least 40% identical to the nucleotide sequence shown in Fig. 1;
b) a nucleic acid molecule hybridizing under stringent conditions to the nucleotide sequence shown in Fig. 1;
c) a nucleic acid molecule encoding a polypeptide having the amino acid sequence which is at least 40% identical to the amino acid sequence shown in Fig. 2 and having phytase activity;
d) a nucleic acid molecule encoding a naturally occurring allelic variant of the polypeptide having the amino acid sequence shown in Fig.2;
e) a nucleic acid molecule complementary to the nucleic acid molecules of any one of a) to d).

5. A recombinant nucleic acid molecule comprising a polynucleotide selected from the group consisting of:
(a) polynucleotides comprising a nucleotide sequence as disclosed in FIG. 1;
(b) polynucleotides comprising a nucleotide sequence encoding a polypeptide as defined in any one of claims 1-3;
(c) polynucleotides comprising a nucleotide sequence the complementary strand of which hybridizes to the polynucleotide of (a) or (b), wherein said nucleotide sequence encodes a polypeptide having phytase activity; and
(d) polynucleotides comprising a nucleotide sequence that deviates from the nucleotide sequence defined in (a), (b) or (c) by the degeneracy of the genetic code.

6. An isolated polypeptide selected from the group consisting of:
(a) polypeptides having the amino acid sequence as shown in FIG. 7;
(b) polypeptides having an amino acid sequence which is at least 40% identical to the amino acid sequence shown in FIG. 7 and having phytase activity;
(c) fragments of the polypeptide of (a) or (b) having phytase activity.

7. An isolated nucleic acid molecule encoding a protein having phytase activity selected from the group consisting of:
a) a nucleic acid molecule having a nucleotide sequence which is at least 40% identical to the nucleotide sequence shown in Fig. 17;
b) a nucleic acid molecule hybridizing under stringent conditions to the nucleotide sequence shown in Fig. 17;
c) a nucleic acid molecule encoding a polypeptide having the amino acid sequence which is at least 40% identical to the amino acid sequence shown in Fig. 7 and having phytase activity;
d) a nucleic acid molecule encoding a naturally occurring allelic variant of the polypeptide having the amino acid sequence shown in Fig. 7;
e) a nucleic acid molecule complementary to the nucleic acid molecules of any one of a) to d).

8. A recombinant nucleic acid molecule comprising a polynucleotide selected from the group consisting of:
(a) polynucleotides comprising a nucleotide sequence as disclosed in FIG. 17;
(b) polynucleotides comprising a nucleotide sequence encoding a polypeptide as defined in claim 6;
(c) polynucleotides comprising a nucleotide sequence the complementary strand of which hybridizes to the polynucleotide of (a) or (b), wherein said nucleotide sequence encodes a polypeptide having phytase activity; and
(d) polynucleotides comprising a nucleotide sequence that deviates from the nucleotide sequence defined in (a), (b) or (c) by the degeneracy of the genetic code.

9. An isolated polypeptide selected from the group consisting of:
(a) polypeptides having the amino acid sequence as shown in FIG. 8;
(b) polypeptides having an amino acid sequence which is at least 50% identical to the amino acid sequence shown in FIG. 8 and having phytase activity;
(c) fragments of the polypeptide of (a) or (b) having phytase activity.

10. An isolated nucleic acid molecule encoding a protein having phytase activity selected from the group consisting of:
a) a nucleic acid molecule having a nucleotide sequence which is at least 50% identical to the nucleotide sequence shown in Fig. 18;
b) a nucleic acid molecule hybridizing under stringent conditions to the nucleotide sequence shown in Fig. 18;
c) a nucleic acid molecule encoding a polypeptide having the amino acid sequence which is at least 50% identical to the amino acid sequence shown in Fig. 8 and having phytase activity;
d) a nucleic acid molecule encoding a naturally occurring allelic variant of the polypeptide having the amino acid sequence shown in Fig.8;
e) a nucleic acid molecule complementary to the nucleic acid molecules of any one of a) to d).

11. A recombinant nucleic acid molecule comprising a polynucleotide selected from the group consisting of:
(a) polynucleotides comprising a nucleotide sequence as disclosed in FIG. 18;
(b) polynucleotides comprising a nucleotide sequence encoding a polypeptide as defined in claim 9;
(c) polynucleotides comprising a nucleotide sequence the complementary strand of which hybridizes to the polynucleotide of (a) or (b), wherein said nucleotide sequence encodes a polypeptide having phytase activity; and
(d) polynucleotides comprising a nucleotide sequence that deviates from the nucleotide sequence defined in (a), (b) or (c) by the degeneracy of the genetic code.

12. Recombinant nucleic acid molecule according to claim 4, 8 or 11 further comprising expression control sequences operably linked to said polynucleotide.

13. A vector comprising the recombinant nucleic acid molecule of claim 4, 8, 11 or 12.

14. A method for producing a recombinant host cell comprising transforming a host cell with the recombinant nucleic acid molecule of claim 4, 8, 11 or 12 or the vector of claim 13.

15. A host cell transformed with the recombinant nucleic acid molecule of claim 4, 8, 11 or 12 or the vector of claim 13, preferably wherein said host cell is a bacterial, yeast, fungus, plant or animal cell.

16. A method for the production of a polypeptide having phytase activity, comprising cultivating the host cell of claim 15 under conditions allowing for the expression of the polynucleotide and in which the polypeptide encoded by said polynucleotide is isolated from the cells and/or the culture medium.

17. A purified polypeptide having phytase activity obtainable by the method of claim 11.

18. An antibody specifically recognizing the polypeptide of any one of claims 1-3, 6, 9 or 17.

19. A composition comprising a polypeptide of any one of claims 1-3, 6, 9 or 17, preferably wherein said composition is a feed, food or an additive for a feed or a food.

20. A process for preparing a feed or a food comprising the step of adding a polypeptide of any one of claims 1-3, 6, 9 or 17 to the feed or food components.

21. Use of a polypeptide of any one of claims 1-3, 6, 9 or 17 for liberating inorganic phosphate from phytic acid.


SEQUENCE LISTING

<110>  Nederlandse Organisatie voor
       toegepast-natuurwetenschappelijk onderzoek TNO

<120>  Novel phytases

<130>  P83267EP00

<160>  34

<170>  PatentIn version 3.3

<210>  1
<211>  24
<212>  PRT
<213>  Artificial

<220>
<223>  signal sequence

<400>  1

Met Val Ala Ser Ser Ile Gly Val Leu Leu Ala Ala Val Leu Leu Gly
1               5                   10                  15

Gly Thr Ser Thr Ile Val Thr Ala
            20

<210>  2
<211>  2262
<212>  DNA
<213>  Magnaporthe grisea

<400>  2
atggtcgcca gcagcatcgg cgtcctcctc gccgccgtcc tcctcggcgg caccagcacc      60

atcgtcaccg ccgccccggt cgagctggag ctgaacgtca ccgccctcac ccccggcatc     120

atcgacagcg actggaccgc cgcctactac cccaagagcg gcgccgcccc cctcctcctc     180

gccaacgacg ccggcaccgc cacgggcggc ttccgcgtgt tcggcctgga cggcgccccc     240

accctcagcc aggccggcca cgtcatcgcc ggtcgcacca agctcgtcgc ccccctctac     300

aacgcctccg gcgccggcga cgacttcctc gtcaccatcg ccaccaccga cagcgtcgtc     360

cgcctctaca gcctccccga cctcgccgtc gtccccatcg ccgagcccct cgtcaccctc     420

ggcgactgga acgccgtctg cagctggggc agctacttct tcctcttcgg caagggcggt     480

cgggccttcc agtacctcgt ccgccccgcc gacggcggca gcttcgaggt cctcgaggtc     540

cgcgccctcc ccgcccccgt cgtgttcgtc ggctgcgccg tcagcccccg cgacgccaag     600

ctctacctca ccgaggacgt cgaccaggac gtctacgtca tggacgtcgg cgacatggcc 660

ggccagccga ccctcaagaa ggtcaagggc gtcaaggacg ccaccggcat cgccctctac 720

agcagcggcg accaggagta cgtgttcgtc gcccaggagt cccagctcag cgtctacggc 780

cccgactgga ccctcctcgg caccgtccag ctcgccggcc tggacgacat cgagatccag 840

ggcctcagca tctaccaggc ccagaccagc acctacccca gcggcgccat caccttcgcc 900

atcgaggccg acgccacgga gggcttcggc accacctccc tccagcccat cctcgacgcc 960

ctcagcatcc cccccaacac cgcctacacc ccccgctccg agcccaccgg cccccgcacc 1020

tgcgcccgct gcagcgacaa cggcttctgc cccaccgtcg gctgccccaa gacctgcacc 1080

agctgcctcc tcggcttcag cggccccacc tgcagcaccc cccgctgcga ggccgactgc 1140

tccggtcgcg gcacctgcgt cggccccaac aagtgcgcct gcgacgccgg ctggggcggc 1200

ctccactgca gcttcgtcct ggtggaggcc gaccgcgaga ccgacgccgc cggtggtgac 1260

ggtgacgacc ccgccgtctg gatcagcccc gtcagcaagg agctgagccg cgtcatcacc 1320

accgtcaaga gcgaggccgg tgccggcctc aacgtgttcg acctcgaggg caagctcgtc 1380

cagcagttca ccgccggcca gcccaacaac gtcgacgtca tctacggctt caaggccggc 1440

gaccgcgtcg tcgacctcgc cttcgccggc tgccgcagcg acgagaccct ctgcctcttc 1500

gagatcacca gcaacggcac catcaccacc atccccggcg gcatccagcc cctccccaag 1560

ggctacaagg tctacggcag ctgcgtctac cgcagcccca agagcaaccg ccagtacctc 1620

ttcgtcaaca gcaagaaggc ccgctacctc cagtacgagc tgaccagcac cgagagcggc 1680

accctccaga ccaccctcgt ccgcgacttc accggcggca gcggcggcca ggtcgagggc 1740

tgcgtcgtgg acgaccgcaa ctcctggctc ttcctcggcg aggagccccg cgccctctgg 1800

cgctacggcg ccgagccgga cgacggcacc gagggcctcg agatcgccgt gttcggcgac 1860

ggcaagctga acgccgaggt cgagggcgtc accctcgtcg agggcaagac cccccaggag 1920

ggctacatcc tcgtcagcaa ccagggcgtc agcagctacg ccgtctaccg ccgcgccgag 1980

ccccacgagt acgtcgccac cttcagcatc gtcgccagcg ccgacggcaa gatcgacgcc 2040

gtcagcaaca ccgacggcat caccgccgtg gccacgggcc tcggccccaa cttcccccac 2100

ggcctcgtcg tcgtccatga cgacgccaac cagctccccg agggcggcac ctccaccgag 2160

gccagcttca agatgatcag cctcgagaag atcttcggcg gccccctcaa ggagctgaac 2220

ctcctcgacc acgtcgacgc caactgggac ccccgcgcct aa 2262

<210>  3
<211>  753
<212>  PRT
<213>  Magnaporthe grisea

<400>  3

Met Val Ala Ser Ser Ile Gly Val Leu Leu Ala Ala Val Leu Leu Gly
1               5                   10                  15

Gly Thr Ser Thr Ile Val Thr Ala Ala Pro Val Glu Leu Glu Leu Asn
            20                  25                  30

Val Thr Ala Leu Thr Pro Gly Ile Ile Asp Ser Asp Trp Thr Ala Ala
        35                  40                  45

Tyr Tyr Pro Lys Ser Gly Ala Ala Pro Leu Leu Leu Ala Asn Asp Ala
    50                  55                  60

Gly Thr Ala Thr Gly Gly Phe Arg Val Phe Gly Leu Asp Gly Ala Pro
65                  70                  75                  80

Thr Leu Ser Gln Ala Gly His Val Ile Ala Gly Arg Thr Lys Leu Val
                85                  90                  95

Ala Pro Leu Tyr Asn Ala Ser Gly Ala Gly Asp Asp Phe Leu Val Thr
            100                 105                 110

Ile Ala Thr Thr Asp Ser Val Val Arg Leu Tyr Ser Leu Pro Asp Leu
        115                 120                 125

Ala Val Val Pro Ile Ala Glu Pro Leu Val Thr Leu Gly Asp Trp Asn
    130                 135                 140

Ala Val Cys Ser Trp Gly Ser Tyr Phe Phe Leu Phe Gly Lys Gly Gly
145                 150                 155                 160

Arg Ala Phe Gln Tyr Leu Val Arg Pro Ala Asp Gly Gly Ser Phe Glu
                165                 170                 175

Val Leu Glu Val Arg Ala Leu Pro Ala Pro Val Val Phe Val Gly Cys
            180                 185                 190

Ala Val Ser Pro Arg Asp Ala Lys Leu Tyr Leu Thr Glu Asp Val Asp
        195                 200                 205

Gln Asp Val Tyr Val Met Asp Val Gly Asp Met Ala Gly Gln Pro Thr
    210                 215                 220

Leu Lys Lys Val Lys Gly Val Lys Asp Ala Thr Gly Ile Ala Leu Tyr
225                 230                 235                 240

Ser Ser Gly Asp Gln Glu Tyr Val Phe Val Ala Gln Glu Ser Gln Leu
                245                 250                 255

Ser Val Tyr Gly Pro Asp Trp Thr Leu Leu Gly Thr Val Gln Leu Ala
            260                 265                 270

Gly Leu Asp Asp Ile Glu Ile Gln Gly Leu Ser Ile Tyr Gln Ala Gln
        275                 280                 285

Thr Ser Thr Tyr Pro Ser Gly Ala Ile Thr Phe Ala Ile Glu Ala Asp
    290                 295                 300

Ala Thr Glu Gly Phe Gly Thr Thr Ser Leu Gln Pro Ile Leu Asp Ala
305                 310                 315                 320

Leu Ser Ile Pro Pro Asn Thr Ala Tyr Thr Pro Arg Ser Glu Pro Thr
                325                 330                 335

Gly Pro Arg Thr Cys Ala Arg Cys Ser Asp Asn Gly Phe Cys Pro Thr
            340                 345                 350

Val Gly Cys Pro Lys Thr Cys Thr Ser Cys Leu Leu Gly Phe Ser Gly
        355                 360                 365

Pro Thr Cys Ser Thr Pro Arg Cys Glu Ala Asp Cys Ser Gly Arg Gly
    370                 375                 380

Thr Cys Val Gly Pro Asn Lys Cys Ala Cys Asp Ala Gly Trp Gly Gly
385                 390                 395                 400

Leu His Cys Ser Phe Val Leu Val Glu Ala Asp Arg Glu Thr Asp Ala
                405                 410                 415

Ala Gly Gly Asp Gly Asp Asp Pro Ala Val Trp Ile Ser Pro Val Ser
            420                 425                 430

Lys Glu Leu Ser Arg Val Ile Thr Thr Val Lys Ser Glu Ala Gly Ala
        435                 440                 445

Gly Leu Asn Val Phe Asp Leu Glu Gly Lys Leu Val Gln Gln Phe Thr
    450                 455                 460

Ala Gly Gln Pro Asn Asn Val Asp Val Ile Tyr Gly Phe Lys Ala Gly
465                 470                 475                 480

Asp Arg Val Val Asp Leu Ala Phe Ala Gly Cys Arg Ser Asp Glu Thr
                485                 490                 495

Leu Cys Leu Phe Glu Ile Thr Ser Asn Gly Thr Ile Thr Thr Ile Pro
            500                 505                 510

Gly Gly Ile Gln Pro Leu Pro Lys Gly Tyr Lys Val Tyr Gly Ser Cys
        515                 520                 525

Val Tyr Arg Ser Pro Lys Ser Asn Arg Gln Tyr Leu Phe Val Asn Ser
    530                 535                 540

Lys Lys Ala Arg Tyr Leu Gln Tyr Glu Leu Thr Ser Thr Glu Ser Gly
545                 550                 555                 560

Thr Leu Gln Thr Thr Leu Val Arg Asp Phe Thr Gly Gly Ser Gly Gly
                565                 570                 575

Gln Val Glu Gly Cys Val Val Asp Asp Arg Asn Ser Trp Leu Phe Leu
            580                 585                 590

Gly Glu Glu Pro Arg Ala Leu Trp Arg Tyr Gly Ala Glu Pro Asp Asp
        595                 600                 605

Gly Thr Glu Gly Leu Glu Ile Ala Val Phe Gly Asp Gly Lys Leu Asn
    610                 615                 620

Ala Glu Val Glu Gly Val Thr Leu Val Glu Gly Lys Thr Pro Gln Glu
625                 630                 635                 640

Gly Tyr Ile Leu Val Ser Asn Gln Gly Val Ser Ser Tyr Ala Val Tyr
                645                 650                 655

Arg Arg Ala Glu Pro His Glu Tyr Val Ala Thr Phe Ser Ile Val Ala
            660                 665                 670

Ser Ala Asp Gly Lys Ile Asp Ala Val Ser Asn Thr Asp Gly Ile Thr
        675                 680                 685

Ala Val Ala Thr Gly Leu Gly Pro Asn Phe Pro His Gly Leu Val Val
    690                 695                 700

Val His Asp Asp Ala Asn Gln Leu Pro Glu Gly Gly Thr Ser Thr Glu
705                 710                 715                 720

Ala Ser Phe Lys Met Ile Ser Leu Glu Lys Ile Phe Gly Gly Pro Leu
                725                 730                 735

Lys Glu Leu Asn Leu Leu Asp His Val Asp Ala Asn Trp Asp Pro Arg
            740                 745                 750

Ala

<210>  4
<211>  340
<212>  PRT
<213>  Magnaporthe grisea

<400>  4

Met Val Ala Ser Ser Ile Gly Val Leu Leu Ala Ala Val Leu Leu Gly
1               5                   10                  15

Gly Thr Ser Thr Ile Val Thr Ala Ala Pro Val Glu Leu Glu Leu Asn
            20                  25                  30

Val Thr Ala Leu Thr Pro Gly Ile Ile Asp Ser Asp Trp Thr Ala Ala
        35                  40                  45

Tyr Tyr Pro Lys Ser Gly Ala Ala Pro Leu Leu Leu Ala Asn Asp Ala
    50                  55                  60

Gly Thr Ala Thr Gly Gly Phe Arg Val Phe Gly Leu Asp Gly Ala Pro
65                  70                  75                  80

Thr Leu Ser Gln Ala Gly His Val Ile Ala Gly Arg Thr Lys Leu Val
                85                  90                  95

Ala Pro Leu Tyr Asn Ala Ser Gly Ala Gly Asp Asp Phe Leu Val Thr
            100                 105                 110

Ile Ala Thr Thr Asp Ser Val Val Arg Leu Tyr Ser Leu Pro Asp Leu
        115                 120                 125

Ala Val Val Pro Ile Ala Glu Pro Leu Val Thr Leu Gly Asp Trp Asn
    130                 135                 140

Ala Val Cys Ser Trp Gly Ser Tyr Phe Phe Leu Phe Gly Lys Gly Gly
145                 150                 155                 160

Arg Ala Phe Gln Tyr Leu Val Arg Pro Ala Asp Gly Gly Ser Phe Glu
                165                 170                 175

Val Leu Glu Val Arg Ala Leu Pro Ala Pro Val Val Phe Val Gly Cys
            180                 185                 190

Ala Val Ser Pro Arg Asp Ala Lys Leu Tyr Leu Thr Glu Asp Val Asp
        195                 200                 205

Gln Asp Val Tyr Val Met Asp Val Gly Asp Met Ala Gly Gln Pro Thr
    210                 215                 220

Leu Lys Lys Val Lys Gly Val Lys Asp Ala Thr Gly Ile Ala Leu Tyr
225                 230                 235                 240

Ser Ser Gly Asp Gln Glu Tyr Val Phe Val Ala Gln Glu Ser Gln Leu
                245                 250                 255

Ser Val Tyr Gly Pro Asp Trp Thr Leu Leu Gly Thr Val Gln Leu Ala
            260                 265                 270

Gly Leu Asp Asp Ile Glu Ile Gln Gly Leu Ser Ile Tyr Gln Ala Gln
        275                 280                 285

Thr Ser Thr Tyr Pro Ser Gly Ala Ile Thr Phe Ala Ile Glu Ala Asp
    290                 295                 300

Ala Thr Glu Gly Phe Gly Thr Thr Ser Leu Gln Pro Ile Leu Asp Ala
305                 310                 315                 320

Leu Ser Ile Pro Pro Asn Thr Ala Tyr Thr Pro Arg Ser Glu Pro Thr
                325                 330                 335

Gly Pro Arg Thr
            340

<210>  5
<211>  374
<212>  PRT
<213>  Magnaporthe grisea

<400>  5

Met Val Ala Ser Ser Ile Gly Val Leu Leu Ala Ala Val Leu Leu Gly
1               5                   10                  15

Gly Thr Ser Thr Ile Val Thr Ala Ser Phe Val Leu Val Glu Ala Asp
            20                  25                  30

Arg Glu Thr Asp Ala Ala Gly Gly Asp Gly Asp Asp Pro Ala Val Trp
        35                  40                  45

Ile Ser Pro Val Ser Lys Glu Leu Ser Arg Val Ile Thr Thr Val Lys
    50                  55                  60

Ser Glu Ala Gly Ala Gly Leu Asn Val Phe Asp Leu Glu Gly Lys Leu
65                  70                  75                  80

Val Gln Gln Phe Thr Ala Gly Gln Pro Asn Asn Val Asp Val Ile Tyr
                85                  90                  95

Gly Phe Lys Ala Gly Asp Arg Val Val Asp Leu Ala Phe Ala Gly Cys
            100                 105                 110

Arg Ser Asp Glu Thr Leu Cys Leu Phe Glu Ile Thr Ser Asn Gly Thr
        115                 120                 125

Ile Thr Thr Ile Pro Gly Gly Ile Gln Pro Leu Pro Lys Gly Tyr Lys
    130                 135                 140

Val Tyr Gly Ser Cys Val Tyr Arg Ser Pro Lys Ser Asn Arg Gln Tyr
145                 150                 155                 160

Leu Phe Val Asn Ser Lys Lys Ala Arg Tyr Leu Gln Tyr Glu Leu Thr

                165                 170                 175

Ser Thr Glu Ser Gly Thr Leu Gln Thr Thr Leu Val Arg Asp Phe Thr
            180                 185                 190

Gly Gly Ser Gly Gly Gln Val Glu Gly Cys Val Val Asp Asp Arg Asn
        195                 200                 205

Ser Trp Leu Phe Leu Gly Glu Glu Pro Arg Ala Leu Trp Arg Tyr Gly
    210                 215                 220

Ala Glu Pro Asp Asp Gly Thr Glu Gly Leu Glu Ile Ala Val Phe Gly
225                 230                 235                 240

Asp Gly Lys Leu Asn Ala Glu Val Glu Gly Val Thr Leu Val Glu Gly
                245                 250                 255

Lys Thr Pro Gln Glu Gly Tyr Ile Leu Val Ser Asn Gln Gly Val Ser
            260                 265                 270

Ser Tyr Ala Val Tyr Arg Arg Ala Glu Pro His Glu Tyr Val Ala Thr
        275                 280                 285

Phe Ser Ile Val Ala Ser Ala Asp Gly Lys Ile Asp Ala Val Ser Asn
    290                 295                 300

Thr Asp Gly Ile Thr Ala Val Ala Thr Gly Leu Gly Pro Asn Phe Pro
305                 310                 315                 320

His Gly Leu Val Val Val His Asp Asp Ala Asn Gln Leu Pro Glu Gly
                325                 330                 335

Gly Thr Ser Thr Glu Ala Ser Phe Lys Met Ile Ser Leu Glu Lys Ile
            340                 345                 350

Phe Gly Gly Pro Leu Lys Glu Leu Asn Leu Leu Asp His Val Asp Ala
        355                 360                 365

Asn Trp Asp Pro Arg Ala
    370

<210> 6
<211> 63

<212>  PRT
<213>  Magnaporthe grisea

<400>  6

Cys Ala Arg Cys Ser Asp Asn Gly Phe Cys Pro Thr Val Gly Cys Pro
1               5                   10                  15

Lys Thr Cys Thr Ser Cys Leu Leu Gly Phe Ser Gly Pro Thr Cys Ser
            20                  25                  30

Thr Pro Arg Cys Glu Ala Asp Cys Ser Gly Arg Gly Thr Cys Val Gly
        35                  40                  45

Pro Asn Lys Cys Ala Cys Asp Ala Gly Trp Gly Gly Leu His Cys
    50                  55                  60

<210>  7
<211>  539
<212>  PRT
<213>  Magnaporthe grisea

<400>  7

Met Ala Ala Val Val Gly Phe Ile Ala Val Cys Phe Leu Ala Trp Phe
1               5                   10                  15

Ala Thr Ser Lys Arg Leu Leu Phe Ala Thr Thr Ser Thr Gly Ser Arg
            20                  25                  30

Cys Pro Cys Glu Pro Asp Pro Asp Val Pro Gln Tyr Phe Arg Thr Ser
        35                  40                  45

Pro Glu Leu Trp Ala Gly Pro Thr Ala Thr Gly Lys Ala Pro Phe Leu
    50                  55                  60

Gln Gln Thr Val Thr Phe Asp Pro Thr Ala Thr Tyr Val Pro Asn Ala
65                  70                  75                  80

Pro Leu Gln Thr Ala Ile Pro Ile Gln Gly Met Gly Ser Gly Asn Lys
                85                  90                  95

Ser Ile Phe Gln Met Met Gly His Leu Ser Pro Tyr Ser Pro Ser Pro
            100                 105                 110

Gly Phe Gly Val Arg Glu Tyr Pro Leu Pro Ala Gly Ala Glu Ile Val

115                 120                 125

Gln Val Gln Met Leu Ser Arg His Gly Ser Arg Tyr Pro Thr Ser Asn
    130                 135                 140

Ser Asp Val Ala Ala Leu Gly Lys His Ile Ser Asn Ala Lys Lys Asp
145                 150                 155                 160

Lys Ser Phe Lys Ala Ser Gly Pro Leu Ser Phe Leu Asn Asp Trp Glu
                165                 170                 175

Tyr Gln Leu Gly His Glu Ile Leu Val Pro Arg Gly Arg Gln Glu Leu
            180                 185                 190

Phe Asp Ser Gly Arg Arg Ile Pro Val Leu Ile His Cys Gly Lys Ile
        195                 200                 205

Ile Val Arg Thr Thr Thr Gln Asp Arg Met Leu Lys Ser Ala Glu Tyr
    210                 215                 220

Phe Met Ala Gly Phe Phe Gly Leu Glu Trp Pro Arg Asn Ala Thr Ile
225                 230                 235                 240

Glu Val Ile Ile Glu Glu Ala Gly Phe Asn Asn Ser Leu Ala Gly Tyr
                245                 250                 255

Leu Asn Cys Pro Ser Gly Ala Gly Gln His Val Gly Asp Asp Ala Arg
            260                 265                 270

Lys Ile Trp Val Ser Asn Tyr Leu Gln Asn Ala Thr Ala Arg Ile Gln
        275                 280                 285

Thr Met Ile Glu Gly Tyr His Trp Thr Ile Glu Asp Thr Tyr Ala Ala
    290                 295                 300

Gln Asn Met Cys Pro Tyr Glu Thr Val Ala Tyr Gly Phe Ser Arg Phe
305                 310                 315                 320

Cys Asp Leu Phe Asn Tyr Glu Glu Trp Ile Gly Phe Gly Tyr Ser Ile
                325                 330                 335

Asp Leu Trp Phe Ala Gly Ile Ser Gly Phe Gln Ser Pro Ile Gly Arg
            340                 345                 350

Ala Leu Gly Ile Gly Tyr Gln Gln Glu Val Val Ala Arg Leu Lys Asn
        355                 360                 365

His Thr Leu Gly Tyr Ser Gly Ser Gln Ile Asn Val Thr Leu Asp Asn
    370                 375                 380

Asn Thr Glu Thr Phe Pro Leu Asn Gln Thr Leu Tyr Phe Asp Phe Ser
385                 390                 395                 400

His Asp Thr Asn Ile Met Ala Ile Leu Thr Ala Phe Gly Leu Arg Gln
                405                 410                 415

Phe Ala His Leu Met Gln Pro Thr Thr His Pro Gly Glu His Asn Leu
            420                 425                 430

Thr Val Ala Asn Leu Thr Pro Phe Gly Ala Arg Leu Asp Ile Glu Ile
        435                 440                 445

Ile Lys Thr Pro Lys Pro Val Lys Pro Asp Arg Ser Gly Tyr Met Val
    450                 455                 460

Asn Gly Gly Glu Thr Lys Tyr Val His Phe Val Leu Asn Gln Arg Thr
465                 470                 475                 480

Leu Pro Leu Gly Trp Ser Leu Pro Glu Cys Asp Val Ser Arg Val Asp
                485                 490                 495

Gly Trp Cys Glu Leu Gly Ala Phe Leu Ser Ala Gln Asp Lys Met Pro
            500                 505                 510

Ala Arg Ala Arg Phe Asp Tyr Ala Cys Phe Gly Ser Tyr Asp Pro Gly
        515                 520                 525

Pro Tyr Gly Ser Val Met Asp Gly Ala Pro Pro
    530                 535

<210>  8
<211>  459
<212>  PRT
<213>  Magnaporthe grisea

<400>  8

Met Leu Pro Ile Phe Ile Ile Leu Phe Gly Leu Val Met Pro Ala Ile
1               5                   10                  15

Ser Thr Asn Asn Tyr Phe Asp Leu Asn Lys Tyr Asp Thr Pro Asp Leu
            20                  25                  30

Gly Phe Leu Tyr Arg Pro Glu Ile Thr Tyr Lys Trp Gly His Phe Thr
        35                  40                  45

Pro Phe His Ser Thr Ser Ser Glu Phe Asn Pro Ser Pro Ser Leu Pro
    50                  55                  60

Gly Cys Thr Val Thr Phe Val Gln Ser Leu Met Arg His Gly Ala Arg
65                  70                  75                  80

Tyr Pro Lys Thr Gly Ser Pro Tyr Arg Asp Ile Ile Gln Arg Ile Gln
                85                  90                  95

Ser Ser Val Asp Lys Tyr Ser Ala Lys Tyr Glu Phe Ile Arg Asn Tyr
            100                 105                 110

Glu Tyr Asn Leu Pro Thr Glu Asp Leu Asn Asp Leu Gly Arg His Gln
        115                 120                 125

Leu Ile Asn Ala Gly Ile His Phe Tyr Arg Arg Tyr Gln Asn Leu Ala
    130                 135                 140

Lys Asp Asn Glu Pro Phe Ile Arg Ser Asp Gly Ala Glu Arg Val Ala
145                 150                 155                 160

Glu Ser Ala Glu Lys Trp Ala Asp Gly Phe His Gln Thr Ser Leu Thr
                165                 170                 175

Asp Lys Thr Arg Ile Gln Pro His Gln Phe Pro Val Ser Ile Phe Lys
            180                 185                 190

Phe Pro Val Gly Lys Gln Tyr Asn Asn Thr Leu Asn Asn Leu Ala Cys
        195                 200                 205

Thr Lys Tyr Asp Lys Ser Lys Ala Gln Lys Ile Ala Lys Glu Leu Pro
    210                 215                 220

Lys Arg Ile Leu Thr Glu Thr Ala Lys Gln Leu Asn Gln Asn Leu Pro

225                 230                 235                 240

Gly Ala Asn Leu Ser Val Tyr Glu Ile Leu Gln Leu Met Glu Leu Cys
                245                 250                 255

Gly Met Glu Thr Thr Ala Asn Ile Val Lys Thr Gly Ile Leu Ser Pro
            260                 265                 270

Ile Cys Asn Ile Phe Asp Asn Lys Asp Trp Glu Ala Phe Gln Arg Phe
        275                 280                 285

His His Glu Val Lys Trp Tyr Thr His Phe Tyr Gly Asn Pro Leu Gly
    290                 295                 300

Pro Ser Leu Gly Ile Gly Trp Val Asn Glu Leu Ile Ala Arg Leu Leu
305                 310                 315                 320

Arg Arg Pro Val Gln Asp Ser Thr Ser Thr Asn Thr Thr Ile Thr Ala
                325                 330                 335

Asn Pro Lys Thr Phe Pro Leu Asp Lys Lys Leu Tyr Ala Asp Phe Thr
            340                 345                 350

His Ala Thr Asp Phe Leu Ala Ile Gln Ala Ala Leu Gly Leu Leu Gly
        355                 360                 365

Ser Ala His Thr Pro Val Pro Ala Asn Arg Ile Val Ser Asp Gln Arg
    370                 375                 380

Ile Ala Glu Phe Ser Ala Ser Glu Val Ser Pro Phe Ala Ala Arg Met
385                 390                 395                 400

Tyr Val Glu Lys Met Thr Cys Ser Gly His Thr Glu Glu Leu Val Arg
                405                 410                 415

Ile Leu Val Asn Asn Arg Val Val Trp Leu Pro Asn Cys Gly Val Asp
            420                 425                 430

Gly Gln Gly Arg Cys Lys Leu Gly Ala Phe Val Glu Ser Leu Ser Phe
        435                 440                 445

Ala Arg Ser Gly Gly Phe Trp Asn Lys Cys Phe
    450                 455

<210> 9
<211> 6054
<212> DNA
<213> Artificial

<220>
<223> plug-in expression cassette

<400> 9
ctcgaggcgg ccgctcagta gtgtatcgcc ccgcataaca gtgctctcac ggccattcat 60

gccatgtgaa caaccaggcc gtccggctgt tcgttccggc gcgcccgcaa gaccccgttg 120

aagggacttc tcggaatggg gtgattatcg ctgagccggg cccacttgat ccactgccga 180

cgctgctgca ttcactttgg tgcatacgag acagctccaa gccgcttgga aaatggggtg 240

aagggtaact atcttaatgg agaacggtaa tcgcatactt gcttgccctc gtcgaggccc 300

ggggctggga gaggagggga tgaaacaaaa gtcaatgccg ctgccgagag accgacagcg 360

cgatagacta cctgatgaag catggtggtt gttgacacaa ggatagaacg gagggaggta 420

tcacagtcta acgcagatag tatagtgtac tccgtaattc atgtaaggat tggtaactgc 480

atcagaccac gacgggaccc catcatcgtg gcggacctca cgggcagaag ctggaggcgc 540

ccgggagaga gtggatcgag ttgcatgcgc aagcgccctt catctcttca gccgagaatc 600

tgttgcctaa ttacatagta gatgctggga acagtctgag aagcctgcgc aggttcttca 660

ttaattacca agaacgcggg agaggtggga agacgccgaa aactcgacgc ggaccattcg 720

ctgttttccg atcttacaaa aaaaaggtat ccgatttggg gaacgtcgat gaaagtattg 780

caaaagtgac gagagttgcg caactaactc gctgccgaag aagctgcgga agaaagagaa 840

ggcctaaagt ggaataacgt tacggatgtc ctgacctcaa agttgaaacc agcccttcct 900

gctctatttg ggaaagcggc ttgcccttga atgcgctgca ctgtggcacg actaccagtg 960

atcgggagga gcaaactacc ctggtccgtt ccttggtggg gcggcactag gcccaactta 1020

gggtgatcgg aggtcgatgc cgcggtcctc gttggtctgg gctcttctca tttcccggtt 1080

tgcacccccc gttgcacctg ctgatcgccc gccaacgccg atgaggttgc gcccagaccg 1140

acaatcaccg cggctgcatt cccaagtata ttgaagatgg caccaggtac ccggttttgc 1200

gtcccagtcg tttggtgcca aatttgggag tttttgagcc tcaagatctg gggaaatcga 1260

cctcaacttc catacaagtt aaagtcgcac acacggcgag ttccacgaag agacacattt 1320

ttttctgacg gccgaatatt ttgaatagct cgcccgctgg agagcatcct gaatgcaagt 1380

aacaaccgta gaggctgaca cggcaggtgt tgctagggag cgtcgtgttc tacaaggcca 1440

gacgtcttcg cggttgatat atatgtatgt ttgactgcag gctgctcagc gacgacagtc 1500

aagttcgccc tcgctgcttg tgcaataatc gcagtgggga agccacaccg tgactcccat 1560

ctttcagtaa agctctgttg gtgtttatca gcaatacacg taatttaaac tcgttagcat 1620

ggggctgata gcttaattac cgtttaccag tgccgcggtt ctgcagcttt ccttggcccg 1680

taaaattcgg cgaagccagc caatcaccag ctaggcacca gctaaaccct ataattagtc 1740

tcttatcaac accatccgct cccccgggat caatgaggag aatgaggggg atgcggggct 1800

aaagaagcct acataaccct catgccaact cccagtttac actcgtcgag ccaacatcct 1860

gactataagc taacacagaa tgcctcaatc ctgggaagaa ctggccgctg ataagcgggc 1920

ccgcctcgca aaaaccatcc ctgatgaatg gaaagtccag acgctgcctg cggaagacag 1980

cgttattgat ttcccaaaga aatcgggcat cctttcagag gccgaactga agatcacaga 2040

ggcgtccgcc gcagaccttg tgtccaagct ggccgccgga gagttgacct cggtggaagt 2100

tacgctagca ttctgtaaac gggcagcaat cgcccagcag ttgacaaact gcgcccacga 2160

gttcttccct gacgccgctc tcgcgcaggc aagggaactc gatgaatact acgcaaagca 2220

caagagaccc gttggtccac tgcatggcct ccccatctct ctcaaagacc agcttcgagt 2280

caagggctac gaaacatcaa tgggctacat ctcatggcta aacaagtacg acgaagggga 2340

ctcggttctg acaaccatgc tccgcaaagc cggtgccgtc ttctacgtca agacctctgt 2400

cccgcagacc ctgatggtct gcgagacagt caacaacatc atcgggcgca ccgtcaaccc 2460

acgcaacaag aactggtcgt gcggcggcag ttctggtggt gagggtgcca tcgttgggat 2520

tcgtggtggc gtcatcggtg taggaacgga catcggtggc tcgattcgag tgccggcggc 2580

gttcaacttc ctgtacggtc taaggccgag tcatgggcgg ctgccgtatg caaagatggc 2640

gaacagcatg gagggtcagg agacggtgca cagcgttgtc gggccgatta cgcactctgt 2700

tgaggacctc cgcctcttca ccaaatccgt cctcggtcag gagccctgga aatacgactc 2760

caaggtcatc cccatgccct ggcgccagtc cgagtcggac attattgcct ccaagatcaa 2820

gaacggcggg ctcaatatcg gctactacaa cttcgacggc aatgtccttc cacaccctcc 2880

tatcctgcgc ggcgtggaaa ccaccgtcgc cgcactcgcc aaagccggtc acaccgtgac 2940

cccgtggacg ccatacaagc acgatttcgg ccacgatctc atctcccata tctacgcggc 3000

tgacggcagc gccgacgtaa tgcgcgacat cagtgcatcc ggcgagccgg cgattccaaa 3060

tatcaaagac ctactgaacc cgaacatcaa agctgtgaac atgaacgagc tgtgggacac 3120

gcatctccag aagtggaatt accagatgga gtaccttgag aaatggcggg aggctgaaga 3180

aaaggccggg aaggaactgg acgccatcat cgcgccgatt acgcctaccg ctgcggtacg 3240

gcatgaccag ttccggtact atgggtatgc ctctgtcatc aacctgctgg atttcacgag 3300

cgtggttgtt cccgttacct ttgcggataa gaacatcgac aagaagaatg agagtttcaa 3360

ggcggttagt gagcttgatg ccctcgtgca ggaagagtat gatccggagg cgtaccacgg 3420

ggcaccggtt gcagtgcagg ttatcggacg gagactcagt gaagagagga cgttggcgat 3480

tgcagaggaa gtggggaagt tgctgggaaa tgtggtgact ccatagctaa taagtgtcag 3540

atagcaattt gcacaagaaa tcaataccag caactgtaaa taagcgctga agtgaccatg 3600

ccatgctacg aaagagcaga aaaaaacctg ccgtagaacc gaagagatat gacacgcttc 3660

catctctcaa aggaagaatc ccttcagggt tgcgtttcca gtctagacac gtataacggc 3720

acaagtgtct ctcaccaaat gggttatatc tcaaatgtga tctaaggatg gaaagcccag 3780

aatattgcgg ccgaagagaa caccgaaagt ggaataacgt tacggatgtc ctgacctcaa 3840

agttgaaacc agcccttcct gctctatttg ggaaagcggc ttgcccttga atgcgctgca 3900

ctgtggcacg actaccagtg atcgggagga gcaaactacc ctggtccgtt ccttggtggg 3960

gcggcactag gcccaactta gggtgatcgg aggtcgatgc cgcggtcctc gttggtctgg 4020

gctcttctca tttcccggtt tgcacccccc gttgcacctg ctgatcgccc gccaacgccg 4080

atgaggttgc gcccagaccg acaatcaccg cggctgcatt cccaagtata ttgaagatgg 4140

caccaggtac ccggttttgc gtcccagtcg tttggtgcca aatttgggag tttttgagcc 4200

tcaagatctg gggaaatcga cctcaacttc catacaagtt aaagtcgcac acacggcgag 4260

ttccacgaag agacacattt ttttctgaag gcctctctcc ccgcacatca gaaaccacca 4320

aataccaaga ctgcagaagc cggggtaagt gggccaccgg gactacacta aaatgcgggg 4380

agaagcgaga tccgttgcga agggaaggga tggggtgtgc tgcggctttc tccgctctcg 4440

tgcgcctttt gcttgaatct agtgtacacc agggtaggct ccgaaggagt atctacggca 4500

gcgctgttcg tgctgcgttg agagtcaggg cggagacgag caggcgacag gagcctcgca 4560

ccggcacttc ggatcgcatt tgcgcggagc gtcaaatacg ctcttctgcg gtcatcagag 4620

agcatcgtga accaaggttc ttccgcaggg cggcctgggc ttcgcagagt cgcactcggc 4680

ggacgccttc cgtgtcaccc ctgataacct ggctgccgcg cccagactcc tccaatgagg 4740

tgtgtggttg ccctcgccga cccttcagca accttaatcg cttccatcgc acggctccac 4800

gtcctcgaac gatgccctca gtccgtgccc ggccgtggca accataacgt gacatcgccg 4860

cccagcctac tagccgctat cgaccggtta ggcttgtcac cgcagcgccc attctccatc 4920

gggcctctac tctgatccac ctcacccacc gcaagcacta gcgagcctca ccagagtgca 4980

agcgacacga cccgcttggc ccttcgtcct tgactatctc ccagacctct tgccatcttg 5040

ccgacgccgc cccctttttt ttctcctccc cctgccggca ggtcggtggc cccagtcccg 5100

agatggcatt gctccgttgt ccatgacgac ccatcattcg atggctgact ggcacactcg 5160

tcttgtttga gcatcgacgg cccgcggccc gtctcccacg gtacggaacc tcgttgtaca 5220

gtacctctcg taatgatacc caacaccggg gccgagcgct gggagggcgg cgttcccgag 5280

aagccgggaa ggcggctggc cggctgacct ttgtgacttg gcgatggatg cggccatggt 5340

gcatgcttcg aattcgatat cggatcctaa gtaagtaaac gaacctctct gaaggaggtt 5400

ctgagacacg cgcgattctt ctgtatatag ttttattttt cactctggag tgcttcgctc 5460

caccagtaca taaacctttt ttttcacgta acaaaatggc ttcttttcag accatgtgaa 5520

ccatcttgat gccttgacct cttcagttct cactttaacg tagttcgcgt tagtctgtat 5580

gtcccagttg catgtagttg agataaatac ccctggaagt gggtctgggc ctttgtggga 5640

cggagccctc tttctgtggt ctggagagcc cgctctctac cgcctacctt cttaccacag 5700

tacactactc acacattgct gaactgaccc atcataccgt actttatcct gttaattcgt 5760

ggtgctgtcg actattctat ttgctcaaat ggagagcaca ttcatcggcg cagggataca 5820

cggtttatgg accccaagag tgtaaggact attattagta atattatatg cctctaggcg 5880

ccttaacttc aacaggcgag cactactaat caacttttgg tagacccaat tacaaacgac 5940

catacgtgcc ggaaattttg ggattccgtc cgctctcccc aaccaagcta gaagaggcaa 6000

cgaacagcca atcccggtgc taattaaatt atatggttca gcggccgcga gctc 6054

<210> 10
<211> 2429
<212> DNA
<213> Artificial

<220>
<223> synthetic expression cassette for phy7789ab

<400> 10
ggtaccccat ggagaatgtc cgtccgaagc gacgcgacaa ttagcctggc taccatcgat 60

ataaattggg tgattcccag ctcttgatgg gcgtgtcttc tgcctggcag ccctcgtctt 120

cagatcaagc aactgtgtgc tgatcctctt ccgtcatggt cgccagcagc atcggcgtcc 180

tcctcgccgc cgtcctcctc ggcggcacca gcaccatcgt caccgccgcc ccggtcgagc 240

tggagctgaa cgtcaccgcc ctcacccccg gcatcatcga cagcgactgg accgccgcct 300

actaccccaa gagcggcgcc gcccccctcc tcctcgccaa cgacgccggc accgccacgg 360

gcggcttccg cgtgttcggc ctggacggcg cccccaccct cagccaggcc ggccacgtca 420

tcgccggtcg caccaagctc gtcgcccccc tctacaacgc ctccggcgcc ggcgacgact 480

tcctcgtcac catcgccacc accgacagcg tcgtccgcct ctacagcctc cccgacctcg 540

ccgtcgtccc catcgccgag cccctcgtca ccctcggcga ctggaacgcc gtctgcagct 600

ggggcagcta cttcttcctc ttcggcaagg gcggtcgggc cttccagtac ctcgtccgcc 660

ccgccgacgg cggcagcttc gaggtcctcg aggtccgcgc cctccccgcc cccgtcgtgt 720

tcgtcggctg cgccgtcagc ccccgcgacg ccaagctcta cctcaccgag gacgtcgacc 780

aggacgtcta cgtcatggac gtcggcgaca tggccggcca gccgaccctc aagaaggtca 840

agggcgtcaa ggacgccacc ggcatcgccc tctacagcag cggcgaccag gagtacgtgt 900

tcgtcgccca ggagtcccag ctcagcgtct acggccccga ctggaccctc ctcggcaccg 960

tccagctcgc cggcctggac gacatcgaga tccagggcct cagcatctac caggcccaga 1020

ccagcaccta ccccagcggc gccatcacct tcgccatcga ggccgacgcc acggagggct 1080

tcggcaccac ctccctccag cccatcctcg acgccctcag catccccccc aacaccgcct 1140

acaccccccg ctccgagccc accggccccc gcacctgcgc ccgctgcagc gacaacggct 1200

tctgccccac cgtcggctgc cccaagacct gcaccagctg cctcctcggc ttcagcggcc 1260

ccacctgcag caccccccgc tgcgaggccg actgctccgg tcgcggcacc tgcgtcggcc 1320

ccaacaagtg cgcctgcgac gccggctggg gcggcctcca ctgcagcttc gtcctggtgg 1380

aggccgaccg cgagaccgac gccgccggtg gtgacggtga cgaccccgcc gtctggatca 1440

gccccgtcag caaggagctg agccgcgtca tcaccaccgt caagagcgag gccggtgccg 1500

gcctcaacgt gttcgacctc gagggcaagc tcgtccagca gttcaccgcc ggccagccca 1560

acaacgtcga cgtcatctac ggcttcaagg ccggcgaccg cgtcgtcgac ctcgccttcg 1620

ccggctgccg cagcgacgag accctctgcc tcttcgagat caccagcaac ggcaccatca 1680

ccaccatccc cggcggcatc cagcccctcc ccaagggcta caaggtctac ggcagctgcg 1740

tctaccgcag ccccaagagc aaccgccagt acctcttcgt caacagcaag aaggcccgct 1800

acctccagta cgagctgacc agcaccgaga gcggcaccct ccagaccacc ctcgtccgcg 1860

acttcaccgg cggcagcggc ggccaggtcg agggctgcgt cgtggacgac cgcaactcct 1920

ggctcttcct cggcgaggag ccccgcgccc tctggcgcta cggcgccgag ccggacgacg 1980

gcaccgaggg cctcgagatc gccgtgttcg gcgacggcaa gctgaacgcc gaggtcgagg 2040

gcgtcaccct cgtcgagggc aagacccccc aggagggcta catcctcgtc agcaaccagg 2100

gcgtcagcag ctacgccgtc taccgccgcg ccgagcccca cgagtacgtc gccaccttca 2160

gcatcgtcgc cagcgccgac ggcaagatcg acgccgtcag caacaccgac ggcatcaccg 2220

ccgtggccac gggcctcggc cccaacttcc cccacggcct cgtcgtcgtc catgacgacg 2280

ccaaccagct ccccgagggc ggcacctcca ccgaggccag cttcaagatg atcagcctcg 2340

agaagatctt cggcggcccc ctcaaggagc tgaacctcct cgaccacgtc gacgccaact 2400

gggacccccg cgcctaagat atcgagctc 2429

<210> 11
<211> 1190
<212> DNA
<213> Artificial

<220>
<223> synthetic expression cassette for phy7789a

<400> 11
ggtaccccat ggagaatgtc cgtccgaagc gacgcgacaa ttagcctggc taccatcgat 60

ataaattggg tgattcccag ctcttgatgg gcgtgtcttc tgcctggcag ccctcgtctt 120

cagatcaagc aactgtgtgc tgatcctctt ccgtcatggt cgccagcagc atcggcgtcc 180

tcctcgccgc cgtcctcctc ggcggcacca gcaccatcgt caccgccgcc ccggtcgagc 240

tggagctgaa cgtcaccgcc ctcacccccg gcatcatcga cagcgactgg accgccgcct 300

actaccccaa gagcggcgcc gcccccctcc tcctcgccaa cgacgccggc accgccacgg 360

gcggcttccg cgtgttcggc ctggacggcg cccccaccct cagccaggcc ggccacgtca 420

tcgccggtcg caccaagctc gtcgcccccc tctacaacgc ctccggcgcc ggcgacgact 480

tcctcgtcac catcgccacc accgacagcg tcgtccgcct ctacagcctc cccgacctcg 540

ccgtcgtccc catcgccgag cccctcgtca ccctcggcga ctggaacgcc gtctgcagct 600

ggggcagcta cttcttcctc ttcggcaagg gcggtcgggc cttccagtac ctcgtccgcc 660

ccgccgacgg cggcagcttc gaggtcctcg aggtccgcgc cctccccgcc cccgtcgtgt 720

tcgtcggctg cgccgtcagc ccccgcgacg ccaagctcta cctcaccgag gacgtcgacc 780

aggacgtcta cgtcatggac gtcggcgaca tggccggcca gccgaccctc aagaaggtca 840

agggcgtcaa ggacgccacc ggcatcgccc tctacagcag cggcgaccag gagtacgtgt 900

tcgtcgccca ggagtcccag ctcagcgtct acggccccga ctggaccctc ctcggcaccg 960

tccagctcgc cggcctggac gacatcgaga tccagggcct cagcatctac caggcccaga 1020

ccagcaccta ccccagcggc gccatcacct tcgccatcga ggccgacgcc acggagggct 1080

tcggcaccac ctccctccag cccatcctcg acgccctcag catccccccc aacaccgcct 1140

acaccccccg ctccgagccc accggccccc gcacctaaga tatcgagctc 1190

<210> 12
<211> 1292
<212> DNA
<213> Artificial

<220>
<223> synthetic expression cassette for phy7789b

<400> 12
ggtaccccat ggagaatgtc cgtccgaagc gacgcgacaa ttagcctggc taccatcgat 60

ataaattggg tgattcccag ctcttgatgg gcgtgtcttc tgcctggcag ccctcgtctt 120

cagatcaagc aactgtgtgc tgatcctctt ccgtcatggt cgccagcagc atcggcgtcc 180

tcctcgccgc cgtcctcctc ggcggcacca gcaccatcgt caccgccagc ttcgtcctgg 240

tggaggccga ccgcgagacc gacgccgccg gtggtgacgg tgacgacccc gccgtctgga 300

tcagccccgt cagcaaggag ctgagccgcg tcatcaccac cgtcaagagc gaggccggtg 360

ccggcctcaa cgtgttcgac ctcgagggca agctcgtcca gcagttcacc gccggccagc 420

ccaacaacgt cgacgtcatc tacggcttca aggccggcga ccgcgtcgtc gacctcgcct 480

tcgccggctg ccgcagcgac gagaccctct gcctcttcga gatcaccagc aacggcacca 540

tcaccaccat ccccggcggc atccagcccc tccccaaggg ctacaaggtc tacggcagct 600

gcgtctaccg cagccccaag agcaaccgcc agtacctctt cgtcaacagc aagaaggccc 660

gctacctcca gtacgagctg accagcaccg agagcggcac cctccagacc accctcgtcc 720

gcgacttcac cggcggcagc ggcggccagg tcgagggctg cgtcgtggac gaccgcaact 780

cctggctctt cctcggcgag gagccccgcg ccctctggcg ctacggcgcc gagccggacg 840

acggcaccga gggcctcgag atcgccgtgt tcggcgacgg caagctgaac gccgaggtcg 900

agggcgtcac cctcgtcgag ggcaagaccc cccaggaggg ctacatcctc gtcagcaacc 960

agggcgtcag cagctacgcc gtctaccgcc gcgccgagcc ccacgagtac gtcgccacct 1020

tcagcatcgt cgccagcgcc gacggcaaga tcgacgccgt cagcaacacc gacggcatca 1080

ccgccgtggc cacgggcctc ggccccaact tcccccacgg cctcgtcgtc gtccatgacg 1140

acgccaacca gctccccgag ggcggcacct ccaccgaggc cagcttcaag atgatcagcc 1200

tcgagaagat cttcggcggc cccctcaagg agctgaacct cctcgaccac gtcgacgcca 1260

actgggaccc ccgcgcctaa gatatcgagc tc 1292

<210> 13
<211> 1787
<212> DNA
<213> Artificial

<220>
<223> synthetic expression cassette for phy538

<400> 13
ggtaccccat ggagaatgtc cgtccgaagc gacgcgacaa ttagcctggc taccatcgat 60

ataaattggg tgattcccag ctcttgatgg gcgtgtcttc tgcctggcag ccctcgtctt 120

cagatcaagc aactgtgtgc tgatcctctt ccgtcatggc cgccgtcgtc ggcttcatcg 180

ccgtctgctt cctcgcctgg ttcgccacct ccaagcgcct cctcttcgcc accacctcca 240

ccggcagccg ctgcccctgc gagcccgacc ccgacgtccc ccagtacttc cgcaccagcc 300

ccgagctgtg ggccggcccg accgccaccg gcaaggcccc cttcctccag cagaccgtca 360

ccttcgaccc caccgccacc tacgtcccca acgcccccct ccagaccgcc atccccatcc 420

agggcatggg cagcggcaac aagagcatct tccagatgat gggccacctc agcccctaca 480

gccccagccc cggcttcggc gtccgcgagt accccctccc cgccggcgcc gagatcgtcc 540

aggtccagat gctcagccgc cacggctccc gctaccccac ctccaacagc gacgtggccg 600

ccctcggcaa gcacatcagc aacgccaaga aggacaagag cttcaaggcc agcggccccc 660

tcagcttcct caacgactgg gagtaccagc tcggccacga gatcctcgtc cccgggggcc 720

gccaggagct gttcgactcg ggccgccgca tccccgtcct catccactgc ggcaagatca 780

tcgtccgcac caccacccag gaccgcatgc tcaagagcgc cgagtacttc atggccggct 840

tcttcggcct cgagtggccc cgcaacgcca ccatcgaggt catcatcgag gaggccggct 900

tcaacaacag cctcgccggc tacctcaact gccccagcgg cgccggccag cacgtcggcg 960

acgacgcccg caagatctgg gtctccaact acctccagaa cgccaccgcc cgcatccaga 1020

ccatgatcga gggctaccac tggaccatcg aggacaccta cgccgcccag aacatgtgcc 1080

cctacgagac cgtcgcctac ggcttcagcc gcttctgcga cctcttcaac tacgaggagt 1140

ggatcggctt cggctacagc atcgacctct ggttcgccgg catcagcggc ttccagagcc 1200

ccatcggtcg cgccctcggc atcggctacc agcaggaggt cgtcgcccgc ctcaagaacc 1260

acaccctcgg ctacagcggc agccagatca acgtcaccct cgacaacaac accgagacct 1320

tccccctcaa ccagaccctc tacttcgact tcagccacga caccaacatc atggccatcc 1380

tgaccgcctt cggcctccgc cagttcgccc acctcatgca gcccaccacc caccccggcg 1440

agcacaacct caccgtcgcc aacctcaccc ccttcggcgc ccgcctcgac atcgagatca 1500

tcaagacccc caagcccgtc aagcccgacc gcagcggcta catggtcaac ggcggcgaga 1560

ccaagtacgt ccacttcgtc ctcaaccagc gcaccctccc cctcggctgg tccctccccg 1620

agtgcgacgt cagccgcgtc gacggctggt gcgagctggg cgccttcctc agcgcccagg 1680

acaagatgcc cgcccgcgcc cgcttcgact acgcctgctt cggcagctac gaccccggcc 1740

cctacggcag cgtcatggac ggcgcccccc cctaagatat cgagctc 1787

<210> 14
<211> 1547
<212> DNA
<213> Artificial

<220>
<223> synthetic expression cassette for phy1900

<400> 14
ggtaccccat ggagaatgtc cgtccgaagc gacgcgacaa ttagcctggc taccatcgat 60

ataaattggg tgattcccag ctcttgatgg gcgtgtcttc tgcctggcag ccctcgtctt 120

cagatcaagc aactgtgtgc tgatcctctt ccgtcatgct ccccatcttc atcatcctct 180

tcggcctcgt catgcccgcc atcagcacca acaactactt cgacctcaac aagtacgaca 240

cccccgacct cggcttcctc taccgccccg agatcaccta caagtggggc cacttcaccc 300

ccttccacag caccagcagc gagttcaacc ccagccccag cctccccggc tgcaccgtca 360

ccttcgtcca gagcctcatg cgccacggcg cccgctaccc caagaccggc agcccctacc 420

gcgacatcat ccagcgcatc cagagcagcg tcgacaagta cagcgccaag tacgagttca 480

tccgcaacta cgagtacaac ctccccaccg aggacctgaa cgacctgggc cggcaccagc 540

tcatcaacgc cggcatccac ttctaccgcc gctaccagaa cctcgccaag gacaacgagc 600

ccttcatccg cagcgacggc gccgagcgcg tcgccgagag cgccgagaag tgggccgacg 660

gcttccacca gaccagcctc accgacaaga cgcgcatcca gccccaccag ttccccgtca 720

gcatcttcaa gttccccgtc ggcaagcagt acaacaacac cctcaacaac ctcgcctgca 780

ccaagtacga caagagcaag gcccagaaga tcgccaagga gctgcccaag cgcatcctca 840

ccgagaccgc caagcagctc aaccagaacc tccccggcgc caacctcagc gtctacgaga 900

tcctccagct catggagctg tgcggcatgg agaccaccgc caacatcgtc aagaccggca 960

tcctcagccc catctgcaac atcttcgaca acaaggactg ggaggccttc cagcgcttcc 1020

accacgaggt caagtggtac acccacttct acggcaaccc cctcggcccc agcctcggca 1080

tcggctgggt caacgagctg atcgcccgcc tcctccgccg ccccgtccag gacagcacct 1140

ccaccaacac caccatcacc gccaacccca agaccttccc cctcgacaag aagctctacg 1200

ccgacttcac ccacgccacc gacttcctcg ccatccaggc cgccctcggc ctcctcggca 1260

gcgcccacac ccccgtcccc gccaaccgca tcgtcagcga ccagcgcatc gccgagttca 1320

gcgccagcga ggtctccccc ttcgccgccc gcatgtacgt cgagaagatg acctgcagcg 1380

gccacaccga ggagctggtc cgcatcctcg tcaacaaccg cgtcgtctgg ctccccaact 1440

gcggcgtcga cggccagggc cgctgcaagc tcggcgcctt cgtcgagtcc ctcagcttcg 1500

cccgcagcgg cggcttctgg aacaagtgct tctaagatat cgagctc 1547

<210> 15
<211> 1265
<212> PRT
<213> Artificial

<220>
<223> glucoamylase phytase fusion protein

<400> 15

Met Ser Phe Arg Ser Leu Leu Ala Leu Ser Gly Leu Val Cys Thr Gly
1               5                   10                  15

Leu Ala Asn Val Ile Ser Lys Arg Ala Thr Leu Asp Ser Trp Leu Ser
            20                  25                  30

Asn Glu Ala Thr Val Ala Arg Thr Ala Ile Leu Asn Asn Ile Gly Ala
        35                  40                  45

Asp Gly Ala Trp Val Ser Gly Ala Asp Ser Gly Ile Val Val Ala Ser
    50                  55                  60

Pro Ser Thr Asp Asn Pro Asp Tyr Phe Tyr Thr Trp Thr Arg Asp Ser
65                  70                  75                  80

Gly Leu Val Leu Lys Thr Leu Val Asp Leu Phe Arg Asn Gly Asp Thr
                85                  90                  95

Ser Leu Leu Ser Thr Ile Glu Asn Tyr Ile Ser Ala Gln Ala Ile Val
            100                 105                 110

Gln Gly Ile Ser Asn Pro Ser Gly Asp Leu Ser Ser Gly Ala Gly Leu
        115                 120                 125

Gly Glu Pro Lys Phe Asn Val Asp Glu Thr Ala Tyr Thr Gly Ser Trp
    130                 135                 140

Gly Arg Pro Gln Arg Asp Gly Pro Ala Leu Arg Ala Thr Ala Met Ile
145                 150                 155                 160

Gly Phe Arg Gln Trp Leu Leu Asp Asn Gly Tyr Thr Ser Thr Ala Thr
                165                 170                 175

Asp Ile Val Trp Pro Leu Val Arg Asn Asp Leu Ser Tyr Val Ala Gln
            180                 185                 190

Tyr Trp Asn Gln Thr Gly Tyr Asp Leu Trp Glu Glu Val Asn Gly Ser
        195                 200                 205

Ser Phe Phe Thr Ile Ala Val Gln His Arg Ala Leu Val Glu Gly Ser
    210                 215                 220

Ala Phe Ala Thr Ala Val Gly Ser Ser Cys Ser Trp Cys Asp Ser Gln
225                 230                 235                 240

Ala Pro Glu Ile Leu Cys Tyr Leu Gln Ser Phe Trp Thr Gly Ser Phe
                245                 250                 255

Ile Leu Ala Asn Phe Asp Ser Ser Arg Ser Gly Lys Asp Ala Asn Thr
            260                 265                 270

Leu Leu Gly Ser Ile His Thr Phe Asp Pro Glu Ala Ala Cys Asp Asp
        275                 280                 285

Ser Thr Phe Gln Pro Cys Ser Pro Arg Ala Leu Ala Asn His Lys Glu
    290                 295                 300

Val Val Asp Ser Phe Arg Ser Ile Tyr Thr Leu Asn Asp Gly Leu Ser
305                 310                 315                 320

Asp Ser Glu Ala Val Ala Val Gly Arg Tyr Pro Glu Asp Thr Tyr Tyr
                325                 330                 335

Asn Gly Asn Pro Trp Phe Leu Cys Thr Leu Ala Ala Ala Glu Gln Leu
            340                 345                 350

Tyr Asp Ala Leu Tyr Gln Trp Asp Lys Gln Gly Ser Leu Glu Val Thr
        355                 360                 365

Asp Val Ser Leu Asp Phe Phe Lys Ala Leu Tyr Ser Asp Ala Ala Thr
    370                 375                 380

Gly Thr Tyr Ser Ser Ser Ser Ser Thr Tyr Ser Ser Ile Val Asp Ala
385                 390                 395                 400

Val Lys Thr Phe Ala Asp Gly Phe Val Ser Ile Val Glu Thr His Ala
                405                 410                 415

Ala Ser Asn Gly Ser Met Ser Glu Gln Tyr Asp Lys Ser Asp Gly Glu
            420                 425                 430

Gln Leu Ser Ala Arg Asp Leu Thr Trp Ser Tyr Ala Ala Leu Leu Thr
        435                 440                 445

Ala Asn Asn Arg Arg Asn Ser Val Val Pro Ala Ser Trp Gly Glu Thr
    450                 455                 460

Ser Ala Ser Ser Val Pro Gly Thr Cys Ala Ala Thr Ser Ala Ile Gly
465                 470                 475                 480

Thr Tyr Ser Ser Val Thr Val Thr Ser Trp Pro Ser Ile Val Ala Thr
                485                 490                 495

Gly Gly Thr Thr Thr Thr Ala Thr Pro Thr Gly Ser Gly Ser Val Thr
            500                 505                 510

Ser Thr Ser Lys Thr Thr Ala Thr Ala Ser Lys Thr Ser Thr Ser Thr
        515                 520                 525

Ser Ser Asn Val Ile Ser Lys Arg Ala Pro Val Glu Leu Glu Leu Asn
    530                 535                 540

Val Thr Ala Leu Thr Pro Gly Ile Ile Asp Ser Asp Trp Thr Ala Ala
545                 550                 555                 560

Tyr Tyr Pro Lys Ser Gly Ala Ala Pro Leu Leu Leu Ala Asn Asp Ala
                565                 570                 575

Gly Thr Ala Thr Gly Gly Phe Arg Val Phe Gly Leu Asp Gly Ala Pro
            580                 585                 590

Thr Leu Ser Gln Ala Gly His Val Ile Ala Gly Arg Thr Lys Leu Val
        595                 600                 605

Ala Pro Leu Tyr Asn Ala Ser Gly Ala Gly Asp Asp Phe Leu Val Thr
    610                 615                 620

Ile Ala Thr Thr Asp Ser Val Val Arg Leu Tyr Ser Leu Pro Asp Leu
625                 630                 635                 640

Ala Val Val Pro Ile Ala Glu Pro Leu Val Thr Leu Gly Asp Trp Asn
                645                 650                 655

Ala Val Cys Ser Trp Gly Ser Tyr Phe Phe Leu Phe Gly Lys Gly Gly
            660                 665                 670

Arg Ala Phe Gln Tyr Leu Val Arg Pro Ala Asp Gly Gly Ser Phe Glu
        675                 680                 685

Val Leu Glu Val Arg Ala Leu Pro Ala Pro Val Val Phe Val Gly Cys
    690                 695                 700

Ala Val Ser Pro Arg Asp Ala Lys Leu Tyr Leu Thr Glu Asp Val Asp
705                 710                 715                 720

Gln Asp Val Tyr Val Met Asp Val Gly Asp Met Ala Gly Gln Pro Thr
                725                 730                 735

Leu Lys Lys Val Lys Gly Val Lys Asp Ala Thr Gly Ile Ala Leu Tyr
            740                 745                 750

Ser Ser Gly Asp Gln Glu Tyr Val Phe Val Ala Gln Glu Ser Gln Leu

        755                 760                 765

Ser Val Tyr Gly Pro Asp Trp Thr Leu Leu Gly Thr Val Gln Leu Ala
    770                 775                 780

Gly Leu Asp Asp Ile Glu Ile Gln Gly Leu Ser Ile Tyr Gln Ala Gln
785                 790                 795                 800

Thr Ser Thr Tyr Pro Ser Gly Ala Ile Thr Phe Ala Ile Glu Ala Asp
                805                 810                 815

Ala Thr Glu Gly Phe Gly Thr Thr Ser Leu Gln Pro Ile Leu Asp Ala
            820                 825                 830

Leu Ser Ile Pro Pro Asn Thr Ala Tyr Thr Pro Arg Ser Glu Pro Thr
        835                 840                 845

Gly Pro Arg Thr Cys Ala Arg Cys Ser Asp Asn Gly Phe Cys Pro Thr
    850                 855                 860

Val Gly Cys Pro Lys Thr Cys Thr Ser Cys Leu Leu Gly Phe Ser Gly
865                 870                 875                 880

Pro Thr Cys Ser Thr Pro Arg Cys Glu Ala Asp Cys Ser Gly Arg Gly
                885                 890                 895

Thr Cys Val Gly Pro Asn Lys Cys Ala Cys Asp Ala Gly Trp Gly Gly
            900                 905                 910

Leu His Cys Ser Phe Val Leu Val Glu Ala Asp Arg Glu Thr Asp Ala
        915                 920                 925

Ala Gly Gly Asp Gly Asp Asp Pro Ala Val Trp Ile Ser Pro Val Ser
    930                 935                 940

Lys Glu Leu Ser Arg Val Ile Thr Thr Val Lys Ser Glu Ala Gly Ala
945                 950                 955                 960

Gly Leu Asn Val Phe Asp Leu Glu Gly Lys Leu Val Gln Gln Phe Thr
                965                 970                 975

Ala Gly Gln Pro Asn Asn Val Asp Val Ile Tyr Gly Phe Lys Ala Gly
            980                 985                 990

Asp Arg Val Val Asp Leu Ala Phe Ala Gly Cys Arg Ser Asp Glu Thr
        995                 1000                1005

Leu Cys Leu Phe Glu Ile Thr Ser Asn Gly Thr Ile Thr Thr Ile
    1010                1015                1020

Pro Gly Gly Ile Gln Pro Leu Pro Lys Gly Tyr Lys Val Tyr Gly
    1025                1030                1035

Ser Cys Val Tyr Arg Ser Pro Lys Ser Asn Arg Gln Tyr Leu Phe
    1040                1045                1050

Val Asn Ser Lys Lys Ala Arg Tyr Leu Gln Tyr Glu Leu Thr Ser
    1055                1060                1065

Thr Glu Ser Gly Thr Leu Gln Thr Thr Leu Val Arg Asp Phe Thr
    1070                1075                1080

Gly Gly Ser Gly Gly Gln Val Glu Gly Cys Val Val Asp Asp Arg
    1085                1090                1095

Asn Ser Trp Leu Phe Leu Gly Glu Glu Pro Arg Ala Leu Trp Arg
    1100                1105                1110

Tyr Gly Ala Glu Pro Asp Asp Gly Thr Glu Gly Leu Glu Ile Ala
    1115                1120                1125

Val Phe Gly Asp Gly Lys Leu Asn Ala Glu Val Glu Gly Val Thr
    1130                1135                1140

Leu Val Glu Gly Lys Thr Pro Gln Glu Gly Tyr Ile Leu Val Ser
    1145                1150                1155

Asn Gln Gly Val Ser Ser Tyr Ala Val Tyr Arg Arg Ala Glu Pro
    1160                1165                1170

His Glu Tyr Val Ala Thr Phe Ser Ile Val Ala Ser Ala Asp Gly
    1175                1180                1185

Lys Ile Asp Ala Val Ser Asn Thr Asp Gly Ile Thr Ala Val Ala
    1190                1195                1200

Thr Gly Leu Gly Pro Asn Phe Pro His Gly Leu Val Val Val His
    1205                1210                1215

Asp Asp Ala Asn Gln Leu Pro Glu Gly Gly Thr Ser Thr Glu Ala
    1220                1225                1230

Ser Phe Lys Met Ile Ser Leu Glu Lys Ile Phe Gly Gly Pro Leu
    1235                1240                1245

Lys Glu Leu Asn Leu Leu Asp His Val Asp Ala Asn Trp Asp Pro
    1250                1255                1260

Arg Ala
    1265

<210> 16
<211> 4083
<212> DNA
<213> Artificial

<220>
<223> glucoamylase phytase fusion gene

<400> 16
atgggcttcc gatctctact cgccctgagc ggcctcgtct gcacagggtt ggcaaatgtg      60

atttccaagc gcgcgacctt ggattcatgg ttgagcaacg aagcgaccgt ggctcgtact     120

gccatcctga ataacatcgg ggcggacggt gcttgggtgt cgggcgcgga ctctggcatt     180

gtcgttgcta gtcccagcac ggataacccg gactgtatgt ttcgagctca gatttagtat     240

gagtgtgtca ttgattgatt gatgctgact ggcgtgtcgt ttgttgtaga cttctacacc     300

tggactcgcg actctggtct cgtcctcaag accctcgtcg atctcttccg aaatggagat     360

accagtctcc tctccaccat tgagaactac atctccgccc aggcaattgt ccagggtatc     420

agtaacccct ctggtgatct gtccagcggc gctggtctcg gtgaacccaa gttcaatgtc     480

gatgagactg cctacactgg ttcttgggga cggccgcagc gagatggtcc ggctctgaga     540

gcaactgcta tgatcggctt cgggcagtgg ctgcttgtat gttctccacc cccttgcgtc     600

tgatctgtga catatgtagc tgactggtca ggacaatggc tacaccagca ccgcaacgga     660

cattgtttgg cccctcgtta ggaacgacct gtcgtatgtg gctcaatact ggaaccagac     720

aggatatggt gtgtttgttt tattttaaat ttccaaagat gcgccagcag agctaacccg     780

cgatcgcaga tctctgggaa gaagtcaatg gctcgtcttt ctttacgatt gctgtgcaac     840

accgcgccct tgtcgaaggt agtgccttcg cgacggccgt cggctcgtcc tgctcctggt 900
gtgattctca ggcacccgaa attctctgct acctgcagtc cttctggacc ggcagcttca 960
ttctggccaa cttcgatagc agccgttccg gcaaggacgc aaacaccctc ctgggaagca 1020
tccacacctt tgatcctgag gccgcatgcg acgactccac cttccagccc tgctccccgc 1080
gcgcgctcgc caaccacaag gaggttgtag actctttccg ctcaatctat accctcaacg 1140
atggtctcag tgacagcgag gctgttgcgg tgggtcggta ccctgaggac acgtactaca 1200
acggcaaccc gtggttcctg tgcaccttgg ctgccgcaga gcagttgtac gatgctctat 1260
accagtggga caagcagggg tcgttggagg tcacagatgt gtcgctggac ttcttcaagg 1320
cactgtacag cgatgctgct actggcacct actcttcgtc cagttcgact tatagtagca 1380
ttgtagatgc cgtgaagact ttcgccgatg gcttcgtctc tattgtggta agtctacgct 1440
agacaagcgc tcatgttgac agagggtgcg tactaacaga agtaggaaac tcacgccgca 1500
agcaacggct ccatgtccga gcaatacgac aagtctgatg gcgagcagct ttccgctcgc 1560
gacctgacct ggtcttatgc tgctctgctg accgccaaca accgtcgtaa ctccgtcgtg 1620
cctgcttctt ggggcgagac ctctgccagc agcgtgcccg gcacctgtgc ggccacatct 1680
gccattggta cctacagcag tgtgactgtc acctcgtggc cgagtatcgt ggctactggc 1740
ggcaccacta cgacggctac ccccactgga tctggcagcg tgacctcgac cagcaagacc 1800
accgcgactg ctagcaagac cagcaccagt acgtcatcaa cctcctgtac cactcccacg 1860
gcgaatgtga tatccaagcg cgccccggtc gagctggagc tgaacgtcac cgccctcacc 1920
cccggcatca tcgacagcga ctggaccgcc gcctactacc ccaagagcgg cgccgccccc 1980
ctcctcctcg ccaacgacgc cggcaccgcc acgggcggct tccgcgtgtt cggcctggac 2040
ggcgccccca ccctcagcca ggccggccac gtcatcgccg gtcgcaccaa gctcgtcgcc 2100
cccctctaca acgcctccgg cgccggcgac gacttcctcg tcaccatcgc caccaccgac 2160
agcgtcgtcc gcctctacag cctccccgac ctcgccgtcg tccccatcgc cgagcccctc 2220
gtcaccctcg gcgactggaa cgccgtctgc agctggggca gctacttctt cctcttcggc 2280
aagggcggtc gggccttcca gtacctcgtc cgccccgccg acggcggcag cttcgaggtc 2340
ctcgaggtcc gcgccctccc cgcccccgtc gtgttcgtcg gctgcgccgt cagcccccgc 2400
gacgccaagc tctacctcac cgaggacgtc gaccaggacg tctacgtcat ggacgtcggc 2460
gacatggccg gccagccgac cctcaagaag gtcaaggcgg tcaaggacgc caccggcatc 2520

gccctctaca gcagcggcga ccaggagtac gtgttcgtcg cccaggagtc ccagctcagc 2580

gtctacggcc ccgactggac cctcctcggc accgtccagc tcgccggcct ggacgacatc 2640

gagatccagg gcctcagcat ctaccaggcc cagaccagca cctaccccag cggcgccatc 2700

accttcgcca tcgaggccga cgccacggag ggcttcggca ccacctccct ccagcccatc 2760

ctcgacgccc tcagcatccc ccccaacacc gcctacaccc cccgctccga gcccaccggc 2820

ccccgcacct gcgcccgctg cagcgacaac ggcttctgcc ccaccgtcgg ctgccccaag 2880

acctgcacca gctgcctcct cggcttcagc ggccccacct gcagcacccc ccgctgcgag 2940

gccgactgct ccggtcgcgg cacctgcgtc ggccccaaca agtgcgcctg cgacgccggc 3000

tggggcggcc tccactgcag cttcgtcctg gtggaggccg accgcgagac cgacgccgcc 3060

ggtggtgacg gtgacgaccc cgccgtctgg atcagccccg tcagcaagga gctgagccgc 3120

gtcatcacca ccgtcaagag cgaggccggt gccggcctca acgtgttcga cctcgagggc 3180

aagctcgtcc agcagttcac cgccggccag cccaacaacg tcgacgtcat ctacggcttc 3240

aaggccggcg accgcgtcgt cgacctcgcc ttcgccggct gccgcagcga cgagaccctc 3300

tgcctcttcg agatcaccag caacggcacc atcaccacca tccccggcgg catccagccc 3360

ctccccaagg gctacaaggt ctacggcagc tgcgtctacc gcagccccaa gagcaaccgc 3420

cagtacctct tcgtcaacag caagaaggcc cgctacctcc agtacgagct gaccagcacc 3480

gagagcggca ccctccagac caccctcgtc cgcgacttca ccggcggcag cggcggccag 3540

gtcgagggct gcgtcgtgga cgaccgcaac tcctggctct tcctcggcga ggagccccgc 3600

gccctctggc gctacggcgc cgagccggac gacggcaccg agggcctcga gatcgccgtg 3660

ttcggcgacg gcaagctgaa cgccgaggtc gagggcgtca ccctcgtcga gggcaagacc 3720

ccccaggagg gctacatcct cgtcagcaac cagggcgtca gcagctacgc cgtctaccgc 3780

cgcgccgagc cccacgagta cgtcgccacc ttcagcatcg tcgccagcgc cgacggcaag 3840

atcgacgccg tcagcaacac cgacggcatc accgccgtgg ccacgggcct cggccccaac 3900

ttcccccacg gcctcgtcgt cgtccatgac gacgccaacc agctccccga gggcggcacc 3960

tccaccgagg ccagcttcaa gatgatcagc ctcgagaaga tcttcggcgg ccccctcaag 4020

gagctgaacc tcctcgacca cgtcgacgcc aactgggacc cccgcgccta agatatcgag 4080

ctc 4083

<210> 17
<211> 4083

<212>  DNA
<213>  Magnaporthe grisea

<400>  17
atgggcttcc gatctctact cgccctgagc ggcctcgtct gcacagggtt ggcaaatgtg      60

atttccaagc gcgcgacctt ggattcatgg ttgagcaacg aagcgaccgt ggctcgtact     120

gccatcctga ataacatcgg ggcggacggt gcttgggtgt cgggcgcgga ctctggcatt     180

gtcgttgcta gtcccagcac ggataacccg gactgtatgt ttcgagctca gatttagtat     240

gagtgtgtca ttgattgatt gatgctgact ggcgtgtcgt ttgttgtaga cttctacacc     300

tggactcgcg actctggtct cgtcctcaag accctcgtcg atctcttccg aaatggagat     360

accagtctcc tctccaccat tgagaactac atctccgccc aggcaattgt ccagggtatc     420

agtaacccct ctggtgatct gtccagcggc gctggtctcg gtgaacccaa gttcaatgtc     480

gatgagactg cctacactgg ttcttgggga cggccgcagc gagatggtcc ggctctgaga     540

gcaactgcta tgatcggctt cgggcagtgg ctgcttgtat gttctccacc cccttgcgtc     600

tgatctgtga catatgtagc tgactggtca ggacaatggc tacaccagca ccgcaacgga     660

cattgtttgg cccctcgtta ggaacgacct gtcgtatgtg gctcaatact ggaaccagac     720

aggatatggt gtgtttgttt tattttaaat ttccaaagat gcgccagcag agctaacccg     780

cgatcgcaga tctctgggaa gaagtcaatg gctcgtcttt ctttacgatt gctgtgcaac     840

accgcgccct tgtcgaaggt agtgccttcg cgacggccgt cggctcgtcc tgctcctggt     900

gtgattctca ggcacccgaa attctctgct acctgcagtc cttctggacc ggcagcttca     960

ttctggccaa cttcgatagc agccgttccg gcaaggacgc aaacaccctc ctgggaagca    1020

tccacacctt tgatcctgag gccgcatgcg acgactccac cttccagccc tgctccccgc    1080

gcgcgctcgc caaccacaag gaggttgtag actctttccg ctcaatctat accctcaacg    1140

atggtctcag tgacagcgag gctgttgcgg tgggtcggta ccctgaggac acgtactaca    1200

acggcaaccc gtggttcctg tgcaccttgg ctgccgcaga gcagttgtac gatgctctat    1260

accagtggga caagcagggg tcgttggagg tcacagatgt gtcgctggac ttcttcaagg    1320

cactgtacag cgatgctgct actggcacct actcttcgtc cagttcgact tatagtagca    1380

ttgtagatgc cgtgaagact ttcgccgatg gcttcgtctc tattgtggta agtctacgct    1440

agacaagcgc tcatgttgac agagggtgcg tactaacaga agtaggaaac tcacgccgca    1500

agcaacggct ccatgtccga gcaatacgac aagtctgatg gcgagcagct ttccgctcgc    1560

gacctgacct ggtcttatgc tgctctgctg accgccaaca accgtcgtaa ctccgtcgtg    1620

cctgcttctt ggggcgagac ctctgccagc agcgtgcccg gcacctgtgc ggccacatct 1680
gccattggta cctacagcag tgtgactgtc acctcgtggc cgagtatcgt ggctactggc 1740
ggcaccacta cgacggctac ccccactgga tctggcagcg tgacctcgac cagcaagacc 1800
accgcgactg ctagcaagac cagcaccagt acgtcatcaa cctcctgtac cactcccacg 1860
gcgaatgtga tatccaagcg cgccccggtc gagctggagc tgaacgtcac cgccctcacc 1920
cccggcatca tcgacagcga ctggaccgcc gcctactacc ccaagagcgg cgccgccccc 1980
ctcctcctcg ccaacgacgc cggcaccgcc acgggcggct tccgcgtgtt cggcctggac 2040
ggcgccccca ccctcagcca ggccggccac gtcatcgccg gtcgcaccaa gctcgtcgcc 2100
cccctctaca acgcctccgg cgccggcgac gacttcctcg tcaccatcgc caccaccgac 2160
agcgtcgtcc gcctctacag cctccccgac ctcgccgtcg tccccatcgc cgagcccctc 2220
gtcaccctcg gcgactggaa cgccgtctgc agctggggca gctacttctt cctcttcggc 2280
aagggcggtc gggccttcca gtacctcgtc cgccccgccg acggcggcag cttcgaggtc 2340
ctcgaggtcc gcgccctccc cgcccccgtc gtgttcgtcg gctgcgccgt cagcccccgc 2400
gacgccaagc tctacctcac cgaggacgtc gaccaggacg tctacgtcat ggacgtcggc 2460
gacatggccg gccagccgac cctcaagaag gtcaagggcg tcaaggacgc caccggcatc 2520
gccctctaca gcagcggcga ccaggagtac gtgttcgtcg cccaggagtc ccagctcagc 2580
gtctacggcc ccgactggac cctcctcggc accgtccagc tcgccggcct ggacgacatc 2640
gagatccagg gcctcagcat ctaccaggcc cagaccagca cctaccccag cggcgccatc 2700
accttcgcca tcgaggccga cgccacggag ggcttcggca ccacctccct ccagcccatc 2760
ctcgacgccc tcagcatccc ccccaacacc gcctacaccc cccgctccga gcccaccggc 2820
ccccgcacct gcgcccgctg cagcgacaac ggcttctgcc ccaccgtcgg ctgccccaag 2880
acctgcacca gctgcctcct cggcttcagc ggccccacct gcagcacccc ccgctgcgag 2940
gccgactgct ccggtcgcgg cacctgcgtc ggccccaaca agtgcgcctg cgacgccggc 3000
tggggcggcc tccactgcag cttcgtcctg gtggaggccg accgcgagac cgacgccgcc 3060
ggtggtgacg gtgacgaccc cgccgtctgg atcagccccg tcagcaagga gctgagccgc 3120
gtcatcacca ccgtcaagag cgaggccggt gccggcctca acgtgttcga cctcgagggc 3180
aagctcgtcc agcagttcac cgccggccag cccaacaacg tcgacgtcat ctacggcttc 3240
aaggccggcg accgcgtcgt cgacctcgcc ttcgccggct gccgcagcga cgagaccctc 3300

tgcctcttcg agatcaccag caacggcacc atcaccacca tccccggcgg catccagccc 3360

ctccccaagg gctacaaggt ctacggcagc tgcgtctacc gcagccccaa gagcaaccgc 3420

cagtacctct tcgtcaacag caagaaggcc cgctacctcc agtacgagct gaccagcacc 3480

gagagcggca ccctccagac caccctcgtc cgcgacttca ccggcggcag cggcggccag 3540

gtcgagggct gcgtcgtgga cgaccgcaac tcctggctct tcctcggcga ggagccccgc 3600

gccctctggc gctacggcgc cgagccggac gacggcaccg agggcctcga gatcgccgtg 3660

ttcggcgacg gcaagctgaa cgccgaggtc gagggcgtca ccctcgtcga gggcaagacc 3720

ccccaggagg gctacatcct cgtcagcaac cagggcgtca gcagctacgc cgtctaccgc 3780

cgcgccgagc cccacgagta cgtcgccacc ttcagcatcg tcgccagcgc cgacggcaag 3840

atcgacgccg tcagcaacac cgacggcatc accgccgtgg ccacgggcct cggccccaac 3900

ttcccccacg gcctcgtcgt cgtccatgac gacgccaacc agctccccga gggcggcacc 3960

tccaccgagg ccagcttcaa gatgatcagc ctcgagaaga tcttcggcgg ccccctcaag 4020

gagctgaacc tcctcgacca cgtcgacgcc aactgggacc cccgcgccta agatatcgag 4080

ctc 4083

<210> 18
<211> 1380
<212> DNA
<213> Magnaporthe grisea

<400> 18
atgctcccca tcttcatcat cctcttcggc ctcgtcatgc ccgccatcag caccaacaac 60

tacttcgacc tcaacaagta cgacaccccc gacctcggct tcctctaccg ccccgagatc 120

acctacaagt ggggccactt cacccccttc cacagcacca gcagcgagtt caaccccagc 180

cccagcctcc ccggctgcac cgtcaccttc gtccagagcc tcatgcgcca cggcgcccgc 240

taccccaaga ccggcagccc ctaccgcgac atcatccagc gcatccagag cagcgtcgac 300

aagtacagcg ccaagtacga gttcatccgc aactacgagt acaacctccc caccgaggac 360

ctgaacgacc tgggccggca ccagctcatc aacgccggca tccacttcta ccgccgctac 420

cagaacctcg ccaaggacaa cgagcccttc atccgcagcg acggcgccga gcgcgtcgcc 480

gagagcgccg agaagtgggc cgacggcttc caccagacca gcctcaccga caagacgcgc 540

atccagcccc accagttccc cgtcagcatc ttcaagttcc ccgtcggcaa gcagtacaac 600

aacaccctca acaacctcgc ctgcaccaag tacgacaaga gcaaggccca gaagatcgcc 660

aaggagctgc ccaagcgcat cctcaccgag accgccaagc agctcaacca gaacctcccc 720

ggcgccaacc tcagcgtcta cgagatcctc cagctcatgg agctgtgcgg catggagacc 780

accgccaaca tcgtcaagac cggcatcctc agccccatct gcaacatctt cgacaacaag 840

gactgggagg ccttccagcg cttccaccac gaggtcaagt ggtacaccca cttctacggc 900

aaccccctcg gccccagcct cggcatcggc tgggtcaacg agctgatcgc ccgcctcctc 960

cgccgccccg tccaggacag cacctccacc aacaccacca tcaccgccaa ccccaagacc 1020

ttccccctcg acaagaagct ctacgccgac ttcacccacg ccaccgactt cctcgccatc 1080

caggccgccc tcggcctcct cggcagcgcc cacacccccg tccccgccaa ccgcatcgtc 1140

agcgaccagc gcatcgccga gttcagcgcc agcgaggtct cccccttcgc cgcccgcatg 1200

tacgtcgaga agatgacctg cagcggccac accgaggagc tggtccgcat cctcgtcaac 1260

aaccgcgtcg tctggctccc caactgcggc gtcgacggcc agggccgctg caagctcggc 1320

gccttcgtcg agtccctcag cttcgcccgc agcggcggct tctggaacaa gtgcttctaa 1380

<210> 19
<211> 774
<212> PRT
<213> Gibberella zeae

<400> 19

Met Thr Ala Ser Lys Thr Lys Asp Gly Ser Pro Pro Lys Lys Gln Ser
1               5                   10                  15

Thr Phe Thr Cys Leu Ala Ile Met Val Gly Ser Gly Leu Ile Ala Leu
            20                  25                  30

Leu Ala Val Ala Phe Gln Ile Ser Phe Ala Lys Ala Val Glu Val Asp
        35                  40                  45

Val Lys Val Asp Ala Tyr Thr Asn Thr Val Glu Ser Asp Trp Thr Thr
    50                  55                  60

Val Tyr Tyr Ser Asn Ser Asn Pro Leu Leu Ile Gly Asn Asp Gly Gly
65                  70                  75                  80

Pro Asp Lys Gly Gly Phe His Ala Tyr Asp Leu Ile Ser Lys Ser Pro
                85                  90                  95

Leu Arg Glu Val Thr Ala Gln Thr Pro Gly Arg Ser Lys Leu Val Thr

            100                 105                 110

Thr Met Tyr Asp Val Asp Asp Lys Asp Val Leu Val Thr Ile Ala Gln
        115                 120                 125

Pro Asp Ser Val Leu Arg Val Tyr Lys Met Pro Glu Phe Lys Gln Ile
    130                 135                 140

Lys Asp Ala Asp Phe Lys Val Leu Gly Asp Trp Ser Ala Leu Cys Ser
145                 150                 155                 160

Trp Lys Ser Pro Ala Gly Asn Asp Tyr Val Tyr Leu Phe Gly Lys Gly
                165                 170                 175

Gln Ala Val Gln Leu Leu Leu Arg Ala Thr Lys Asn Ser Ile Glu Phe
            180                 185                 190

Val Glu Tyr Gln Ile Gln Thr Phe Pro Thr Asp Phe Glu Thr Ser Gly
        195                 200                 205

Cys Ala Ala Ser Arg Ser Glu Ser Arg Met Tyr Ile Ser Thr Asp Asp
    210                 215                 220

Asp Lys Asn Val Tyr Ser Phe Ser Leu Lys Glu Ser Thr Gly Ala Pro
225                 230                 235                 240

Lys Ile Thr Lys Val Gly Lys Ala Glu Asp Asp Val Thr Gly Leu Ala
                245                 250                 255

Val Tyr Val Pro Asn Lys Gly Ser Asp Tyr Leu Phe Val Ala Gln Thr
            260                 265                 270

Asp Lys Ile Ala Val Tyr Asp Gln Ser Phe Lys Leu Lys Gly Thr Leu
        275                 280                 285

Ser Leu Thr Gly Phe Glu Asp Ile Glu Val Gln Gly Leu Asn Ile Tyr
    290                 295                 300

Gln Gly Ser Ala Gly Lys Tyr Pro Ala Gly Ala Leu Thr Tyr Ala Leu
305                 310                 315                 320

Glu Ser Glu Glu Val Asn Gly Phe Gly Val Ser Ser Leu Glu Thr Thr
                325                 330                 335

Leu Lys Lys Leu Lys Ile Glu Pro Asn Thr Lys Tyr Asn Pro Arg Lys
            340                 345                 350

Leu Lys Ser Glu Ser Glu Lys Glu Pro Ile Cys Lys Thr Cys Gly Gly
        355                 360                 365

Ser Gly Phe Cys Val Lys Asp Lys Lys Gln Lys Cys Glu Cys Phe Ser
    370                 375                 380

Gly Phe Ala Gly Lys Thr Cys Asn Ser Phe Thr Cys Val Asp Asn Cys
385                 390                 395                 400

Ser Gly His Gly Lys Cys Val Gly Ala Asn Asn Cys Lys Cys Asp Lys
                405                 410                 415

Gly Trp Gly Gly Leu His Cys Ser Phe Leu Leu Val Glu Pro Thr Tyr
            420                 425                 430

Glu Thr Glu Ser Arg Pro Gly Asp Gly Asp Asp Pro Ala Ile Trp Ile
        435                 440                 445

Ser Pro Glu Gly Pro Glu Lys Ser Arg Ile Val Thr Thr Met Lys Ser
    450                 455                 460

Gly Lys Glu Ala Gly Leu Gly Val Phe Asp Leu Lys Gly Asn Leu Val
465                 470                 475                 480

Gln Thr Phe Ala Ala Gly Glu Pro Asn Asn Val Asp Met Ile Tyr Ser
                485                 490                 495

Phe Lys Ala Gly Asp Arg Lys Val Asp Leu Ala Phe Ala Ala Cys Arg
            500                 505                 510

Ala Asp Asp Thr Leu Cys Leu Phe Glu Met Leu Pro Asn Gly Thr Leu
        515                 520                 525

Thr Asn Ile Pro Gly Gly Ser His Pro Val Val Glu Asp Tyr Lys Val
    530                 535                 540

Tyr Gly Ser Cys Thr Tyr Arg Ser Pro Lys Thr Gly Lys Gln Tyr Leu
545                 550                 555                 560

Phe Val Asn Glu Lys Ser Ala Arg Tyr Leu Gln Tyr Glu Leu Thr Ser
                565                 570                 575

Thr Ser Glu Gly Glu Leu Gln Thr Lys Leu Val Arg Glu Phe Gln Gly
            580                 585                 590

Gly Ser Gly Gly Gln Val Glu Gly Cys Val Thr Asp Lys Glu Asn Gly
        595                 600                 605

Trp Ile Phe Leu Gly Glu Glu Pro Ser Ala Leu Trp Arg Tyr Gly Ala
    610                 615                 620

Glu Pro Asp Ser Lys Asp Glu Gly Val Val Ile Gly Lys Val Gly Asp
625                 630                 635                 640

Gly Lys Leu Tyr Gly Asp Val Glu Gly Val Thr Leu Val Tyr Gly Ser
                645                 650                 655

Lys Ala Asp Glu Gly Tyr Ile Leu Val Ser Cys Gln Gly Val Ser Ala
            660                 665                 670

Tyr Asn Val Tyr Arg Arg Ala Glu Pro His Glu Tyr Val Thr Thr Phe
        675                 680                 685

Thr Leu Val Glu Ser Ser Asp Gly Lys Ile Asp Pro Val Ser Asn Thr
    690                 695                 700

Asp Gly Ile Thr Ala Val Gly Thr Ala Leu Ser Lys Asp Phe Pro His
705                 710                 715                 720

Gly Leu Val Val Val His Asp Asp Ala Asn Gln Leu Pro Asn Gly Lys
                725                 730                 735

Thr Ser Ala Glu Ala Ser Phe Lys Met Val Ser Leu Glu Lys Ile Leu
            740                 745                 750

Gly Ser Lys Val Leu Gly Lys Lys Gly Leu Leu Asp Gln Val Asp Lys
        755                 760                 765

Asp Trp Asp Pro Arg Lys
    770

<210>  20
<211>  736
<212>  PRT
<213>  Aspergillus nidulans

<400>  20

Met His Leu Pro Tyr Ala Leu Gly Val Leu Leu Val Ser Ala Ala Gly
1               5                   10                  15

Ser Ile Ala Ser Pro Leu Thr Gly Asn Leu Ala Val Ser Ser Leu Ser
            20                  25                  30

Thr Glu Val Glu Ser Asp Gln Thr Ala Phe Tyr Tyr Gly Thr Ala Arg
        35                  40                  45

Ser Arg Ser Phe Leu Ile Gly Asn Asp Gly Ser Ala Ala Thr Gly Gly
    50                  55                  60

Ile Arg Ser Phe Ser Leu Gln Asn Leu Asn Glu Thr Ala Arg Val Lys
65                  70                  75                  80

Pro Gly Arg Thr Lys Val Ala Gly Leu Leu Tyr Asp Val Gly His Arg
                85                  90                  95

Asp Leu Val Val Ser Ile Ala Ala Pro Asp Ser Ile Ile Arg Val Phe
            100                 105                 110

Asp Val Asn Gly Leu Gly Glu Ile Pro Ser Thr Arg Lys Lys Ala Leu
        115                 120                 125

Gly Asp Trp Ser Cys Leu Cys Thr Trp Thr Ser Ser Ser Gly Gly Lys
    130                 135                 140

Tyr Phe Tyr Leu Leu Gly Lys Lys Gln Gly Ile Gln Phe Leu Val Arg
145                 150                 155                 160

Glu Lys Gly Ser Lys Val Glu Val Leu Glu Val Gln Thr Phe Pro Leu
                165                 170                 175

Pro Val Glu Pro Ser Ser Cys Ala Val Ser Pro Glu Asp Asn Val Val
            180                 185                 190

Tyr Phe Ala Ala Glu Asp Lys Thr Ile Tyr Ser Phe Ala Ala Ala Glu
        195                 200                 205

Ser Thr Thr Ala Pro Asp Ile Gln Thr Leu Gly Gln Val Ser Asp Glu
    210                 215                 220

Ile Ser Gly Leu Ala Val Tyr Val Ser Ala Ala Thr Gln Tyr Leu Phe
225                 230                 235                 240

Val Thr Gln Ser Asp Lys Val Glu Val Tyr Thr Pro Glu Leu Glu Gln
                245                 250                 255

Val Gly Ser Leu Ala Val Thr Gly Val Glu Asp Leu Glu Ile Ala Gly
            260                 265                 270

Thr Ser Ile Tyr Gln Ser Asn Ser Ser Gln Tyr Pro Tyr Gly Leu Leu
        275                 280                 285

Gly Phe Ala Ile Glu Ser Asp Ser Gly Asn Ala Phe Gly Val Ala Ser
    290                 295                 300

Leu Glu Pro Ala Phe Thr Ser Leu Lys Leu Gln Pro Asn Thr Ser Tyr
305                 310                 315                 320

Thr Pro Arg Arg Ser Ser Gly Gln Ser Gly Pro Lys Gln Asn Gly Phe
                325                 330                 335

Pro Ser Ala Asn Asn Thr Leu Ser Cys Phe Ala Gly Trp Thr Gly Ser
            340                 345                 350

Asp Cys Thr Glu Ile Thr Cys His Asn Asn Cys Ser Arg His Gly Thr
        355                 360                 365

Cys Leu Gly Pro Asn Glu Cys Lys Cys Arg Ser His Trp Ala Gly Pro
    370                 375                 380

Glu Cys Ser Trp Ile Gly Val Glu Ala Lys Tyr Glu Thr Asp Ala Asn
385                 390                 395                 400

Gly Gly Asp Gly Asp Asp Pro Ala Ile Trp Ile Ser Pro Ala Asn Leu
                405                 410                 415

Asn Arg Ser Thr Ile Ile Thr Thr Thr Lys Ser Glu Ile Gly Ala Gly
            420                 425                 430

Leu Ala Val Phe Asp Leu Lys Gly Asn Leu Leu Gln Thr Val Ala Ala
        435                 440                 445

Gly Glu Pro Asn Asn Val Asp Ile Ile Tyr Gly Phe Gln Ala Gly Arg
    450                 455                 460

Arg Thr Ile Asp Leu Ala Tyr Ala Ala Cys Arg Glu Asp Asp Thr Leu
465                 470                 475                 480

Cys Leu Phe Glu Ile Thr Pro Asp Gly Leu Leu Thr Ser Ile Pro Gly
                485                 490                 495

Gly Arg Gln Pro Thr Pro Glu Asp Tyr Thr Val Tyr Gly Ser Cys Ser
            500                 505                 510

Tyr Arg Ser Pro Ser Asn Gly Lys Gln Tyr Leu Phe Val Asn Glu Lys
        515                 520                 525

Ser Gly Leu Tyr Leu Gln Tyr Glu Leu Thr Ser Ser Pro Asn Gly Thr
    530                 535                 540

Leu Ala Thr Thr Leu Val Arg Lys Phe Thr Gly Gly Ser Gly Gly Gln
545                 550                 555                 560

Pro Glu Gly Cys Val Ala Asp Glu Glu Asn Gly Tyr Ile Phe Leu Gly
                565                 570                 575

Glu Glu Pro Leu Gly Leu Trp Arg Tyr Glu Ala Glu Pro Thr Gly Ser
            580                 585                 590

Pro Asn Gly Thr Leu Ile Ala Lys Val Gly Asp Gly Thr Ile Tyr Ala
        595                 600                 605

Asp Val Glu Gly Val Thr Leu Leu Pro Gly Gln Thr Pro Glu Gln Gly
    610                 615                 620

Leu Ile Ile Val Ser Cys Gln Gly Val Ser Ala Tyr Ser Val Tyr Arg
625                 630                 635                 640

Arg Ala Glu Pro His Asp His Val Leu Thr Phe Thr Ile Gly Glu Ser
                645                 650                 655

Gly Asp Gly Ser Val Asp Gly Val Thr Asn Thr Asp Gly Val Thr Gly
            660                 665                 670

Val Ser Thr Gly Leu Asn Asp Asp Phe Pro Arg Gly Leu Leu Val Val
        675                 680                 685

His Asp Asp Ala Asn Gln Leu Ser Thr Gly Glu Thr Ala Glu Leu Ala
    690                 695                 700

Ser Phe Lys Leu Val Ser Leu Glu Asp Val Leu Ala Ala Ala Gly Lys
705                 710                 715                 720

Arg Thr Trp Leu Phe Glu Glu Val Asp Glu Thr Trp Asp Pro Arg Ala
                725                 730                 735

<210>  21
<211>  745
<212>  PRT
<213>  aspergillus terreus

<400>  21

Met Ser Gly Arg Val Pro Glu Ala Asn Lys Arg Gln Leu Arg Gly Lys
1               5                   10                  15

Arg His Lys Val Asp Lys Met Arg Leu Ala Cys Ala Leu Gly Ser Leu
            20                  25                  30

Phe Ala Leu Phe Leu Ser Val Ala Ala Ser Ala Pro Thr Val Lys Val
        35                  40                  45

Ser Ile Ser Ala Leu Thr Ala Gly Ile Glu Ser Asp Trp Ala Ala Val
    50                  55                  60

Tyr Tyr Gly Pro Arg Pro Gln Asp Ser Leu Leu Val Gly Asn Asp Gly
65                  70                  75                  80

Ser Ala Ala Thr Gly Gly Leu Arg Thr Tyr Gly Leu Leu Asp Arg Asn
                85                  90                  95

Leu Asn Glu Thr Ala Arg Arg Thr Pro Gly Arg Thr Lys Val Val Gly
            100                 105                 110

Val Leu Tyr Asp Ile Gly Gly Arg Asp Leu Leu Val Ser Ile Thr Thr
        115                 120                 125

Thr Glu Ser Ile Ile Arg Leu Tyr Asp Val Asp Gly Leu Arg Glu Ile
    130                 135                 140

Ser Gly Val Lys Lys Lys Val Leu Gly Asp Trp Ser Cys Leu Cys Thr
145                 150                 155                 160

Trp Arg Ser Pro Arg Gly Ala Asp Tyr Val Phe Leu Leu Gly Lys Lys
                165                 170                 175

Gln Ala Val Gln Phe Leu Val Arg Gly Asn Arg Lys Glu Leu Glu Ile
            180                 185                 190

Leu Glu Val Gln Ala Phe Pro Leu Pro Val Glu Pro Ser Ser Cys Ala
        195                 200                 205

Val Asp Pro Glu Gly Val Val Tyr Phe Ala Ala Glu Asn Thr Met Leu
    210                 215                 220

Tyr Ser Phe Gln Ala Ala Glu Ser Thr Val Ala Pro Glu Ile Lys Ala
225                 230                 235                 240

Phe Gly Glu Val Gly Asp Asp Ile Ser Gly Leu Ala Val Tyr Val Ser
                245                 250                 255

His Arg Ser His Tyr Leu Phe Val Ala Gln Thr Asp Val Ile Glu Ile
            260                 265                 270

Tyr Ser Pro Ser Leu Glu Leu Lys Gly Ser Leu Ala Val Thr Gly Ala
        275                 280                 285

Glu Asp Ile Glu Ile Ala Gly Thr Ala Ile Tyr Gln Ser Ser Ser Ser
    290                 295                 300

Gln Tyr Pro Tyr Gly Leu Ile Ser Tyr Ala Ile Glu Ser Asp Ser Gly
305                 310                 315                 320

Val Gly Phe Gly Val Ser Ser Leu Glu Pro Val Phe Thr Glu Leu Arg
                325                 330                 335

Leu Glu Pro Asn Thr Arg Tyr Thr Pro Arg Ser Ala Arg Pro Pro Gln
            340                 345                 350

Ser Glu Ser Lys Leu Asn Gly Phe Arg Asn Arg Asp Gly Ser Leu Ser
        355                 360                 365

Cys Phe Ala Gly Phe Thr Gly Arg Asn Cys Asn Arg Val Ala Cys Ser
    370                 375                 380

Asn Asp Cys Ser Gly His Gly Ser Cys Val Gly Pro Asn Glu Cys Gln
385                 390                 395                 400

Cys Arg Asp Pro Trp Ala Gly Pro Asp Cys Ser Trp Ile Gly Val Glu
                405                 410                 415

Ala Lys Tyr Glu Thr Asp Ala Asn Gly Gly Asp Gly Asp Asp Pro Ala
            420                 425                 430

Ile Trp Ile Ser Pro Thr Ser Pro Asn Leu Ser Thr Ile Ile Thr Thr
        435                 440                 445

Thr Lys Ser Glu Thr Ile Ser Ala Gly Glu Pro Asn Asn Val Asp Val
    450                 455                 460

Ile Tyr Ser Phe Gln Leu Gly Asn Arg Thr Val Asp Leu Ala Tyr Ala
465                 470                 475                 480

Ala Cys Arg Ala Asp Asp Thr Leu Cys Leu Phe Glu Val Thr Ser Asp
                485                 490                 495

Gly Leu Leu Ala Glu Val Pro Gly Gly Ile Gln Pro Val Pro Glu Asp
            500                 505                 510

Tyr Thr Val Tyr Gly Ser Cys Ala Tyr Arg Ser Pro Ser Ser Gly Lys
        515                 520                 525

Gln Tyr Leu Phe Val Asn Ala Lys Ser Gly Gln Tyr Leu Gln Tyr Glu
    530                 535                 540

Leu Thr Ser Arg Thr Asn Gly Thr Leu Ser Thr Thr Leu Val Arg Thr
545                 550                 555                 560

Phe Thr Gly Gly Thr Gly Gly Gln Pro Glu Gly Cys Val Ala Asp Glu
                565                 570                 575

Glu Asn Gly Ser Leu Phe Leu Gly Glu Glu Pro Tyr Gly Leu Trp Arg
            580                 585                 590

Tyr Asp Ala Glu Pro Thr Gly Ser Ser Asn Gly Thr Leu Val Ala Lys
        595                 600                 605

Ala Gly Asp Gly Thr Leu Phe Ala Asp Val Glu Gly Val Thr Leu Leu
    610                 615                 620

Pro Gly Lys Thr Ala Asp Gln Gly Phe Ile Val Val Ser Cys Gln Gly
625                 630                 635                 640

Val Ser Ala Tyr Ser Val Tyr Arg Arg Ala Glu Pro His Glu Tyr Val
                645                 650                 655

Met Thr Phe Thr Ile Gly Glu Ser Lys Asp Gly Arg Val Asp Gly Val
            660                 665                 670

Thr Asn Thr Asp Gly Val Ala Gly Val Pro Asn Arg Leu Asn Ala Asp
        675                 680                 685

Phe Pro His Gly Leu Leu Val Val His Asp Asp Ala Asn Gln Leu Ala
    690                 695                 700

Glu Gly Gly Thr Ala Glu Leu Ala Ser Phe Lys Leu Val Ser Leu Glu
705                 710                 715                 720

Asp Val Leu Gly Pro Thr Val Gly Gly Met Leu Leu Leu Glu Leu Asp
                725                 730                 735

Ala Glu Trp Asn Pro Arg Thr Thr Val
            740                 745

<210>  22
<211>  729
<212>  PRT
<213>  Phaeosphaeria nodorum

<400>  22

Met Met Ile Phe Ala Asn Ser Leu Leu Trp Val Ser Thr Val Phe Ala
1               5                   10                  15

Val Ala Ser Ala Gln Ser Val Asn Leu Thr Leu Val Pro Val Ala Thr
            20                  25                  30

Gly Phe Lys Asp Glu Asn Thr Gly Phe Val Tyr Gly Gly Ser Pro Ile
        35                  40                  45

Leu Val Ala Asn Asp Lys Ser Ala Asp Asp Gly Gly Phe Arg Thr Phe
    50                  55                  60

Ala Val Ala Pro Pro Pro Ile Phe Lys Gln Ile Ala His Gln Lys Thr
65                  70                  75                  80

Gly Arg Ser Lys Val Val Val Pro Val His Asp Ile Gly Gly Arg Asp
                85                  90                  95

Leu Ile Ile Asn Ile Pro Ser Pro Asp Ser Leu Ile Arg Val Phe Asp
            100                 105                 110

Thr Lys Thr Gly Lys Lys Val Asp Ser Asn Asp Lys Ile Gln Leu Gly
        115                 120                 125

Asp Trp Ser Thr Ala Cys Val Trp Arg Ser Gln Lys Ser Gly Ala Ser
    130                 135                 140

Tyr Leu Phe Leu Phe Gly Lys Ser Lys Val Val Gln Phe Leu Ile Arg
145                 150                 155                 160

Asp Lys Lys Lys Asp Val Glu Ile Val Glu Thr Phe Pro Val Pro Ile
                165                 170                 175

Glu Gly Glu Thr Cys Thr Val Phe Leu Asn Gly Gln Val Phe Phe Ser
            180                 185                 190

Ala Glu Asp Arg Pro Leu Tyr Ser Phe Gln Ala Thr Glu Ser Thr Arg
        195                 200                 205

Ala Pro Glu Val Lys Thr Val Ser Asp Lys Ile Glu Val Ala Gly Leu
    210                 215                 220

Ala Thr Tyr His Ser Asn Ser Ser Asp Tyr Leu Phe Val Phe His Gly
225                 230                 235                 240

Glu Val Met Asp Val Tyr Asp Ser Lys Ile Ala Pro Lys Gly Thr Val
                245                 250                 255

Glu Phe Asn Gly Val Ala Asp Leu Ser Ile Glu Gly Gly Leu Ser Leu
            260                 265                 270

Leu Gln Ser Ser Thr Glu Ser Tyr Pro Ala Gly Ile Ile Ala Phe Ala
        275                 280                 285

Phe Glu Gly Glu Asp Gly Asn Gly Val Val Ala Ala Ser Leu Ala Ser
    290                 295                 300

Val Leu Ala Pro Leu Gly Ile Ala Thr Asn Thr Ala Tyr Ser Pro Lys
305                 310                 315                 320

Thr Lys Pro Cys Thr Arg Cys Glu Ser Ala Ile Ser Glu Lys Cys Ser
                325                 330                 335

Asn Asn Gly Phe Ser Ala Gly Gly Gly Cys Ser Cys Phe Ala Gly Phe
            340                 345                 350

Ser Gly Lys Glu Cys Asn Lys Asn Ala Cys Leu Asn Asp Cys Ser Gly
        355                 360                 365

His Gly Lys Cys Asp Gly Pro Asn Val Cys Lys Cys Lys Asp Gly Trp
    370                 375                 380

Thr Gly Pro Asp Cys Ser Phe Val Ala Val Lys Ala Lys Tyr Glu Thr
385                 390                 395                 400

Glu Ala Asn Gly Gly Asp Gly Asp Asp Pro Ala Val Trp Ile His Ala
                405                 410                 415

Thr Lys Ala Asp Gln Ser Lys Ile Ile Thr Thr Thr Lys Ser Ser Asp
            420                 425                 430

Gly Glu Gly Phe Gly Val Phe Asp Leu Gln Gly Lys Leu Leu Gln His
        435                 440                 445

Phe Thr Ala Glu Glu Pro Asn Asn Val Asp Ile Ile Tyr Asn Val Thr
    450                 455                 460

Val Gly Ser Arg Lys Thr Asp Leu Ala Phe Ala Ala Cys Arg Gly Asp
465                 470                 475                 480

Asn Thr Leu Cys Leu Val Glu Val Asn Ser Ser Gly Leu Ile Gln Pro
                485                 490                 495

Ile Ala Gly Gly Lys Gln Ser Leu Pro Glu Asp Tyr Glu Pro Tyr Gly
            500                 505                 510

Ser Cys Thr Tyr His Ser Arg Lys Ser Gly Lys Asp Tyr Leu Phe Val
        515                 520                 525

Asn Asn Lys Lys Ala Glu Tyr Leu Gln Tyr Glu Leu Ser Ser Thr Val
    530                 535                 540

Asn Gly Thr Leu Gln Thr Lys Leu Val Arg Gln Phe Thr Gly Gly Ser
545                 550                 555                 560

Gly Gly Gln Val Glu Gly Cys Val Gly Asp Asp Gly Ala Gly Tyr Ile
                565                 570                 575

Phe Leu Gly Glu Glu Pro Leu Gly Ile Trp Arg Tyr Glu Ala Glu Pro
            580                 585                 590

Thr Gly Ser Asn Thr Gly Val Gln Val Ala Lys Val Gly Asp Ala Ser
        595                 600                 605

Gly Leu Ser Ala Asp Val Glu Gly Ile Thr Leu Val Pro Ala Lys Ser
    610                 615                 620

Gly Pro Gly Gly Tyr Ile Leu Val Ser Ser Gln Gly Ile Ser Ala Tyr
625                 630                 635                 640

Lys Val Tyr Glu Arg Ala Pro Pro His Lys Tyr Val Thr Thr Phe Thr
                645                 650                 655

Ile Val Asn Asn Glu Lys Lys Gly Val Asp His Val Ser Asn Thr Asp
            660                 665                 670

Gly Cys Ala Ala Val Gly Asn Arg Leu Asn Lys Asp Phe Pro Tyr Gly
        675                 680                 685

Leu Phe Val Thr His Asp Asp Ala Asn Glu Leu Ala Glu Gly Gly Thr
    690                 695                 700

Ala Lys Glu Ala Ser Phe Lys Leu Ala Ser Leu Val Asp Ile Leu Gly

705                 710                 715                 720

Glu Glu Arg Ala Lys Gly Leu Gly Tyr
                725

<210>  23
<211>  616
<212>  PRT
<213>  Chaetomium globosum

<400>  23

Met Ala Ile Ser Arg Arg Ile Thr Ala Ala Ala Leu Leu Ala Ser Thr
1               5                   10                  15

Leu Ser Ile Gln Ala Ala Ala Val Asp Val Glu Ile Pro Val Ser Ala
            20                  25                  30

Ala Thr Ser Asp Ile Glu Ser Asp Trp Ala Ser Val Tyr Tyr Gly Lys
        35                  40                  45

Thr Pro Leu Leu Leu Gly Asn Asp Gly Thr Ala Ala Thr Gly Gly Trp
    50                  55                  60

Leu Ala Trp Asp Leu Asn Ser Ala Ser Pro Leu Asn Gln Val His Ala
65                  70                  75                  80

Glu Thr Pro Gly Arg Arg Thr Lys Leu Val Thr Thr Ile His Gly Arg
                85                  90                  95

Asp Asp Asn Lys Lys Pro Thr Gly Leu Ala Val Ser Ile Ser Gln Pro
            100                 105                 110

Asp Ser Val Leu Arg Ala Trp Glu Leu Pro Asp Phe Asp Glu Val Lys
        115                 120                 125

Ser Ala Arg Ser Thr Val Leu Gly Asp Trp Ser Ala Leu Cys Ser Trp
    130                 135                 140

Lys Ser Pro Ser Gly His Asp Tyr Val Tyr Leu Phe Gly Lys Lys Glu
145                 150                 155                 160

Ala Lys Val Phe Leu Val Arg Lys Asp Lys Asp Glu Asp Asp Tyr Glu
                165                 170                 175

Asp Ala Lys Phe Val Glu Ile Gln Thr Phe Pro Leu Ser Val Glu Ala
            180                 185                 190

Ser Gly Cys Ala Thr Ser Pro Ser Leu Gly Lys Met Phe Val Ser Ala
        195                 200                 205

Asp Asp Asp Lys Asp Val Tyr Val Phe Asp Leu Val Glu Ser Thr Ala
    210                 215                 220

Ala Pro Ala Ile Ser Lys Ile Gly Glu Ala Glu Asp Asp Val Thr Gly
225                 230                 235                 240

Val Ala Val Tyr Val Ser Ala Thr Thr Gly Val Asp Tyr Leu Leu Val
                245                 250                 255

Ala Met Glu Asp Thr Val Ala Val Tyr Gly Pro Pro Phe Asp Phe Ile
            260                 265                 270

Gly Asn Leu Lys Leu Val Gly Gln Glu Asp Ile Glu Ile Gln Gly Leu
        275                 280                 285

Ala Val Tyr Gln Gly Ser Thr Ser Lys Tyr Pro Ser Gly Val Leu Ala
    290                 295                 300

Tyr Ala Ile Glu Ser Asp Asp Val Thr Gly Phe Gly Val Ser Ser Leu
305                 310                 315                 320

Asp Gly Ala Ile Gln Glu Leu Gly Leu Glu Gly Asn Thr Lys Tyr Asp
                325                 330                 335

Pro Ser Leu Glu Cys Lys Lys Ser Ser Pro Ile Cys Ser Ser Cys Ser
            340                 345                 350

Asn Asn Gly Phe Cys Ala Gln Asn Gly Lys Gly Ile Glu Cys Ser Cys
        355                 360                 365

Phe Ala Gly Trp Gln Gly Lys Thr Cys Asn Thr Tyr Thr Cys Glu Asp
    370                 375                 380

Asn Cys Ser Gly Asn Gly Gln Cys Val Gly Pro Asn Gln Cys Lys Cys
385                 390                 395                 400

Lys Thr Gly Trp Gly Gly Leu His Cys Ser Phe Val Val Val Gln Pro
                405                 410                 415

Val Ala Glu Thr Asp Ala Asn Gly Gly Asp Gly Asp Asp Pro Ala Ile
            420                 425                 430

Trp Ile Ser Pro Ser Asp Arg Ser Gln Ser Arg Ile Ile Thr Thr Thr
        435                 440                 445

Lys Ser Glu Gln Gly Ala Gly Leu Gly Val Phe Asp Leu Ser Gly Lys
    450                 455                 460

Leu Leu Gln Thr Ile Pro Ala Gly Glu Pro Asn Asn Val Asp Val Ile
465                 470                 475                 480

Tyr Asn Phe Gln Ala Gly Pro Arg Lys Ile Asp Leu Ala Tyr Ala Ala
                485                 490                 495

Cys Arg Ala Asp Asp Thr Leu Cys Leu Phe Glu Ile Thr Pro Asn Gly
            500                 505                 510

Thr Leu Thr Pro Ile Pro Gly Gly Ser Gln Pro Thr Pro Pro Asp Tyr
        515                 520                 525

Glu Val Tyr Gly Ser Cys Thr Tyr Arg Ser Pro Thr Thr Gln Lys Gln
    530                 535                 540

Tyr Leu Phe Val Asn Ala Lys Ser Ala Gln Tyr Leu Gln Tyr Glu Leu
545                 550                 555                 560

Gly Ala Thr Ala Asn Gly Thr Leu Thr Thr Thr Leu Leu Ala Gly Gly
                565                 570                 575

Lys Gly Thr Ser Glu Glu Ala Ser Phe Lys Leu Val Pro Leu Glu Arg
            580                 585                 590

Ile Leu Gly Ala Glu Val Val Lys Gln Leu Gly Leu Met Glu Glu Val
        595                 600                 605

Asp Ser Asp Trp Asp Pro Arg Ala
    610                 615

<210>  24

<211>  710
<212>  PRT
<213>  Coprinus cinereus

<400>  24

Met Val Ala Phe Phe Ser Ala Ala Leu Ala Ala Phe Cys Thr Leu Arg
1               5                   10                  15

Ile Gln Ser Ile Leu Ala Gln Thr Ala Leu Glu Ile Pro Ile Gln Glu
            20                  25                  30

Tyr Thr Pro Ser Asp Asp Thr Gly Pro Leu Gly Val Tyr Tyr Asp Ser
        35                  40                  45

Ser Glu Pro Leu Val Phe Ala Ala Asn Val Ser Gly Leu Phe Ser Tyr
    50                  55                  60

Ser Leu Pro Ala Leu Thr Gly Thr Ser Thr Val Arg Arg Asp Thr Lys
65                  70                  75                  80

Ser Val Thr Thr Leu Tyr Gly Glu Arg Asp Arg Asp Asp Trp Leu Val
                85                  90                  95

Ser Leu Val Ile Glu Asp Ser Leu Leu Ala Val Thr Asn Leu Lys Asn
            100                 105                 110

Pro Arg Arg Thr Ser His Leu Arg Leu Trp Gly Asp Tyr Ser Ala Val
        115                 120                 125

Cys Ala Val Gly Ser Ser Phe Val Tyr Ile Phe Gly Lys Gly His Ala
    130                 135                 140

Lys Leu Val Gln Ile Glu Arg Gly Asn Ile Lys Glu Ile Cys Glu Phe
145                 150                 155                 160

Ala Val Pro Val Glu Ala Glu Ala Cys Ala Ala Thr Ala Ala Gly Thr
                165                 170                 175

Val Phe Phe Gly Gly Asn Asp Gly Val Val Tyr Ser Phe Ala Gly Asn
            180                 185                 190

Ala Val Gln Pro Gly Ser Ser Ile Gln Gln Thr Ser Ile Thr Val Asp
        195                 200                 205

Gly Glu Ile Ala Gly Met Ala Val Tyr Glu Ser Ser Asn Asn Ala Ser
    210                 215                 220

Ser Leu Phe Val Ser Val Glu Ser Gly Thr Val Glu Val Phe Ser Thr
225                 230                 235                 240

Ala Glu Trp Ala His Gln Tyr Thr Leu Ser Phe Ser Glu Glu Asp Val
                245                 250                 255

Glu Leu Glu Gly Ile Ala Ile Tyr Gln Arg Ala Leu Pro Gly Tyr Pro
            260                 265                 270

Asp Gly Ser Leu Val Val Ser Phe Thr Glu Asp Asp Gly Arg Thr Gly
        275                 280                 285

Thr Gly Ile Val Ser Leu Thr Gln Ile Pro Leu Pro Arg Asn Ala Asn
    290                 295                 300

Phe Asn Pro Arg Asp Ala Ser Ser Asn Pro Asp Ala Ser Asn Gln Cys
305                 310                 315                 320

Thr Arg Arg Gly Phe Arg Asn Gly Ser Glu Cys Ile Cys Phe Thr Gly
                325                 330                 335

Tyr Thr Gly Ser Arg Cys Asn Gln Arg Thr Cys Pro Asn Asp Cys Ser
            340                 345                 350

Ser Arg Gly Arg Cys Val Gly Pro Asn Thr Cys Arg Cys Asp Asn Gly
        355                 360                 365

Trp Thr Gly Pro Asp Cys Ser Phe Lys Val Val Leu Ala Asp Ile Glu
    370                 375                 380

Thr Asp Ala Asn Gly Gly Asp Gly Asp Asp Pro Ala Ile Trp Ile His
385                 390                 395                 400

Pro Thr Asp Arg Thr Lys Ser Arg Val Leu Thr Thr Thr Lys Ser Lys
                405                 410                 415

Gln Gly Ala Gly Phe Ala Leu Phe Asp Leu Ala Gly Gln Met Ile Ala
            420                 425                 430

Gln Leu Pro Ala Gly Glu Pro Asn Asn Val Asp Val Ile Tyr Asn Phe
        435                 440                 445

Glu Leu Glu Asp Gly Arg Thr Val Asp Leu Gly Val Ala Ala Cys Arg
    450                 455                 460

Asp Glu Asp Thr Leu Cys Leu Met Gln Val Thr Glu Gly Gly Leu Thr
465                 470                 475                 480

Asp Leu Val Asn Leu Pro Val Lys Pro Asp Phe Ser Val Tyr Gly Ser
                485                 490                 495

Cys Val Tyr His Ser Arg Ser Ser Gly Lys Tyr Tyr Ile Phe Val Asn
            500                 505                 510

Ser Lys Thr Ala Glu Tyr Leu Gln Tyr Glu Leu Thr Pro Ser Leu Glu
        515                 520                 525

Ala Val Leu Val Arg Glu Phe Ile Gly Gly Asn Gly Gly Gln Val Glu
    530                 535                 540

Gly Cys Val Gly Asp Asp Glu Asn Gly Phe Val Tyr Ile Gly Glu Glu
545                 550                 555                 560

Pro Tyr Gly Leu Trp Arg Tyr Gly Ala Glu Pro Asp Ala Gly Thr Asp
                565                 570                 575

Arg Val Leu Val Asp Ser Ile Gln Gly Asn Leu Phe Pro Asp Val Glu
            580                 585                 590

Gly Val Thr Leu Val His Gly Pro Thr Pro Asp Lys Gly Leu Leu Ile
        595                 600                 605

Val Ser Ala Gln Gly Val Ser Ala Phe Asn Ile Tyr Arg Arg Gly Thr
    610                 615                 620

His Glu Phe Val Gly Thr Phe Thr Val Gln Ala Gly Gln Val Asp Lys
625                 630                 635                 640

Val Thr Asn Thr Asp Gly Val Ala Ala Val Gly Thr Asn Leu Gly Val
                645                 650                 655

Glu Gly Phe Glu Met Gly Val Val Val Val His Asp Asp Val Asn Ser

            660                 665                 670

Ala Pro Asp Gly Ser Arg Glu Asp Gly Ala Ser Phe Lys Ile Val Gly
        675                 680                 685

Leu Gly Gln Ile Leu Glu Thr Phe Gly Leu Glu Asp Glu Val Asp Thr
    690                 695                 700

Asn Trp Asp Pro Arg Arg
705                 710

<210> 25
<211> 595
<212> PRT
<213> Gibberella zeae

<400> 25

Met Ala Ala Ala Ser Gly Leu Leu Lys Gly Lys Ala Gly Ser Gly Ser
1               5                   10                  15

Ala Gly Tyr Gln Pro Val Tyr Gln Asp Glu Gly Gly Asp Gly His Asp
            20                  25                  30

Glu Gly His Pro Thr Pro His Val Gln Pro Ile Ala Pro Arg Gly Pro
        35                  40                  45

Met Arg Thr Tyr Arg Asn Met Val Ile Val Leu Ser Gly Leu Val Val
    50                  55                  60

Ile Leu Met Ala Met Asn Leu Tyr Leu Ser Leu Pro Leu Ala Phe Ser
65                  70                  75                  80

Ser Gly Ser Gly Ser Gly Ser Cys Pro Gly Asp Pro His Arg Val Pro
                85                  90                  95

Gln Tyr Phe Gln Thr Ser Pro Asn Leu Trp Pro Gly Val Thr Glu Thr
            100                 105                 110

Gly Lys Pro Ala Phe Met Ala Gln Thr His Val Phe Glu Pro Thr Ala
        115                 120                 125

Thr Phe Val Pro Asn Asp Pro Leu Gln Thr Ser Ile Pro Ile Glu Gly
    130                 135                 140

Met Lys Glu Gly Asn Arg Ser Ile Phe Gln Met Met Gly Tyr Leu Ser
145                 150                 155                 160

Pro Tyr Ser Pro Ser Thr Gly Phe Gly Val Asp Glu Tyr Pro Ile Pro
                165                 170                 175

Pro Gly Ala Glu Ile Val Gln Val Gln Met Leu Ser Arg His Gly Ala
            180                 185                 190

Arg Tyr Pro Thr Pro Gly Ala Asn Val Ala Thr Leu Gly Glu Arg Ile
        195                 200                 205

Ala Asn Ala Ser Ala Ser Leu Lys Thr Ser Gly Ala Leu Glu Phe Leu
    210                 215                 220

Asn Asn Trp Lys Tyr Glu Leu Gly Lys Ala Ile Leu Val Pro Arg Gly
225                 230                 235                 240

Arg Gln Glu Leu Phe Asp Ser Gly Ile Leu His Ser Tyr Met Tyr Gly
                245                 250                 255

Ser Leu Tyr Asn Pro Gln Ser Lys Leu Ile Val Arg Thr Thr Thr Gln
            260                 265                 270

Asp Arg Met Leu Lys Ser Ala Glu Asn Trp Met Ala Gly Phe Phe Gly
        275                 280                 285

Leu Glu Trp Thr Asn Asn Ala Thr Ile Glu Val Ile Ile Glu Ala Pro
    290                 295                 300

Gly Phe Asn Asn Ser Leu Ala Gly Ser Leu Asn Cys Pro Asn Ala Asp
305                 310                 315                 320

Lys Ala Asp Tyr Val Thr Pro Val Ala Ala Trp Tyr Glu Ile Tyr Leu
                325                 330                 335

Lys Asp Ala Thr Ala Arg Phe Asn Asn Val Thr Glu Gly Phe Thr Trp
            340                 345                 350

Thr Ala Ala Asp Val Trp Ala Ala Gln Asn Met Cys Pro Tyr Glu Thr
        355                 360                 365

Val Ala Tyr Gly Phe Ser Arg Phe Cys Asp Leu Phe Thr Tyr Glu Glu
    370                 375                 380

Trp Glu His Phe Gly Tyr Ser Ile Asp Leu Gly Phe Ser Ser Gly Ala
385                 390                 395                 400

Gly Phe Gln Ser Pro Ile Gly Arg Ala Thr Gly Leu Gly Tyr Gln Gln
                405                 410                 415

Glu Val Met Ala Arg Leu Lys Asn His Thr Leu Gly Tyr Ser Gly Ser
            420                 425                 430

Gln Ile Asn Thr Thr Leu Asp Gly Met Thr Glu Thr Phe Pro Leu Asn
        435                 440                 445

Gln Ser Leu Tyr Phe Asp Phe Ser His Asp Thr Asn Ile Ile Ser Ile
    450                 455                 460

Leu Thr Ala Phe Gly Phe Arg Gln Phe Ala Glu Asp Leu Pro Thr Asp
465                 470                 475                 480

Lys Tyr Pro Gly Asp His Glu Phe Val Val Ser Lys Ile Thr Pro Phe
                485                 490                 495

Gly Ala Arg Leu Asp Ile Glu Ile Ile Lys Ala Pro Gln Pro Ile Ser
            500                 505                 510

Pro Asn Arg Glu Asp Tyr Gln Glu Gly Lys Glu Thr Lys Tyr Ile His
        515                 520                 525

Phe Val Leu Asn Gln Arg Thr Val Pro Leu Gly Lys Ser Phe Pro Glu
    530                 535                 540

Cys Asp Val Asn Arg Lys Asp Gly Trp Cys Glu Leu Asp Thr Phe Ile
545                 550                 555                 560

Glu Val Gln Asp Lys Met Ala Glu Lys Ala Gln Phe Asp Tyr Ala Cys
                565                 570                 575

Phe Gly Asp Tyr Thr Ser Pro Pro Tyr Gly Lys Val Thr Asp Gly Val
            580                 585                 590

Pro Pro Ser

        595

<210>  26
<211>  599
<212>  PRT
<213>  Neurospora crassa

<400>  26

Met Arg Ser Pro Ile Asn Pro Gln Ala Pro Gly His Asn Pro Val Gly
1               5                   10                  15

Leu Ala Glu Lys Glu Glu Leu Val Leu Arg Pro Leu Leu Ala His Asp
            20                  25                  30

Thr Gln Pro Ser Pro Gly Gln Arg Leu Lys Arg Lys Leu Ser Ile Leu
        35                  40                  45

Leu Ala Ser Cys Ala Val Ser Leu Ala Val Leu Phe Ala Phe Asn Ile
    50                  55                  60

Leu Ala Cys Asn Gly Thr Leu Phe Gly Ile Lys Pro Arg Pro Ser Glu
65                  70                  75                  80

Leu Ala Ser Pro Thr Pro Leu Gln Ala Arg Asp Asp Gln Arg Ser Ser
                85                  90                  95

Gly Glu Lys Asp Cys Pro Cys Lys Pro Thr Ser Thr Val Pro Asp Tyr
            100                 105                 110

Phe Asn Thr Ser Pro Gly Pro Trp Ile Gly Lys Thr Ala Thr Gly Lys
        115                 120                 125

Ala Pro Phe Met Ala Gln Thr Arg Thr Phe Asp His Ala Ala Thr Tyr
    130                 135                 140

Val Pro Asn Ala Pro Leu Gln Thr Gln Val Pro Ile Gln Gly Trp His
145                 150                 155                 160

Pro Gly Asn Leu Ser Ile Phe Gly Met Met Gly Phe Leu Thr Pro Tyr
                165                 170                 175

Thr Pro Ser Thr Gly Phe Gly Val Asp Glu Trp Pro Leu Pro Asp Gly
            180                 185                 190

Ala Glu Ile Ile Trp Leu Gln Met Val Ser Arg His Gly Ser Arg Tyr
        195                 200                 205

Pro Thr Gly Gly Ser Asn Val Glu Ser Phe Gly Ala Arg Leu Ala Asn
    210                 215                 220

Ala Thr Gly Lys Phe Asn Ala Thr Gly Glu Leu Glu Phe Leu Asn Asn
225                 230                 235                 240

Trp Lys Tyr Gln Met Gly Thr Glu Ile Leu Val Pro Arg Gly Arg Gln
                245                 250                 255

Glu Leu Phe Asp Ser Gly Val Leu His Ala Tyr Met Tyr Ser Ser Leu
            260                 265                 270

Tyr Asp Pro Asn Thr Lys Ile Ile Ala Arg Thr Thr Thr Gln Asp Arg
        275                 280                 285

Met Leu Arg Ser Ala Glu Asn Phe Leu Ala Gly Met Phe Gly Leu Glu
    290                 295                 300

Trp Pro Asn Asn Val Thr Leu Glu Val Ile Ile Glu Gly Ser Asn Leu
305                 310                 315                 320

Asn Asn Ser Leu Ala Gly Tyr Met Asn Cys Pro Asn Glu Arg Glu Asp
                325                 330                 335

Gly Leu Gly Ser Ala Ala Arg Asp Ile Trp Val Gly His Tyr Leu Gln
            340                 345                 350

Asn Ala Thr Glu Arg Phe Ser Lys Leu Val Thr Gly Tyr Asn Trp Thr
        355                 360                 365

Leu Asp Asp Thr Tyr Ala Ala Gln Thr Leu Cys Ala Tyr Asp Thr Val
    370                 375                 380

Ala Ser Gly Tyr Ser Arg Phe Cys Ser Leu Phe Thr Tyr Glu Glu Trp
385                 390                 395                 400

Ile Gly Phe Gly Tyr Ser His Asp Leu Gln Phe Tyr Gly Asn Asn Ala
                405                 410                 415

Phe Gly Ser Glu Thr Gly Arg Ala Ile Gly Ile Gly Phe Gln Gln Glu
            420                 425                 430

Val Leu Ala Arg Leu Gln Asn His Thr Ile Pro Tyr Ser Glu Thr Gln
        435                 440                 445

Val Asn Val Thr Leu Asp Asn Asn Thr Val Thr Phe Pro Leu Asn Gln
    450                 455                 460

Ser Leu Tyr Leu Asp Phe Ser His Asp Thr Asn Ile Val Ser Ile Leu
465                 470                 475                 480

Ala Ala Phe Gly Leu Thr Gln Phe Glu Glu Asp Leu Pro Ala Asp Lys
                485                 490                 495

Tyr Pro Gly Glu His Asn Phe Thr Val Ser His Met Thr Pro Phe Gly
            500                 505                 510

Ala Arg Leu Asp Ile Glu Ile Ile Lys Thr Pro Lys Pro Leu Lys Ala
        515                 520                 525

Asp Arg Ser Gly Tyr Glu Asp Glu Gly Glu Glu Thr Lys Tyr Val His
    530                 535                 540

Phe Val Leu Asn Gln Arg Thr Val Pro Leu Gly Trp Ser His Pro Glu
545                 550                 555                 560

Cys Asp Ala Glu Arg Val Asp Gly Trp Cys Glu Phe Glu Ala Phe Leu
                565                 570                 575

Lys Val Gln Glu Lys Met Pro Gly Leu Ala Arg Tyr Glu Glu Val Cys
            580                 585                 590

Phe Ala Asp Gly Gly Ser Pro
        595

<210>  27
<211>  468
<212>  PRT
<213>  Botryotinia fuckeliana

<400>  27

Met Leu Leu Asp Gln Ile Leu Leu Leu Ser Leu Thr Gly Val Thr Ala
1               5                   10                  15

Ser Ala Leu Leu Pro Glu Lys Gln Pro Cys Ser Thr Ser Thr Asn Asp
            20                  25                  30

Val Pro Gln Tyr Phe Gln Thr Thr Pro Glu Leu Trp Ala Gly Pro Thr
        35                  40                  45

Ala Thr Gly Arg Ala Pro Phe Leu Ala Gln Thr Asn Pro Val Ser Phe
    50                  55                  60

Ala Pro Thr Val Thr Phe Val Pro Asn Thr Pro Leu Glu Thr Ala Gln
65                  70                  75                  80

Pro Ile Val Gly Gln Gly Gln Asn Gly Ser Ile Phe Gln Leu Met Gly
                85                  90                  95

His Leu Ser Pro Tyr Leu Pro Asn Pro Ser Gly Phe Gly Val Ala Glu
            100                 105                 110

Tyr Pro Leu Pro Pro Gly Ala Lys Ile Ala Gln Leu Gln Met Leu Ser
        115                 120                 125

Arg His Gly Ala Arg Tyr Pro Thr Ser Gly Ser Asn Val Ala Thr Phe
    130                 135                 140

Gly Glu Lys Val Ala Asn Tyr Thr Gly Lys Met Asp Ala Thr Gly Ala
145                 150                 155                 160

Leu Ser Phe Leu Asn Asp Trp Lys Tyr Glu Leu Gly His Glu Ile Leu
                165                 170                 175

Val Pro Arg Gly Arg Gln Glu Leu Tyr Asp Ser Gly Ile Leu His Tyr
            180                 185                 190

Tyr Gln Tyr Ala Gln Leu Tyr Asn Pro His Ser Lys Ile Ile Ala Arg
        195                 200                 205

Thr Thr Thr Gln Asp Arg Met Leu Lys Ser Ala Glu Tyr Phe Met Ala
    210                 215                 220

Gly Phe Phe Gly Leu Glu Trp Thr Asn Asn Val Thr Ile Glu Val Ile
225                 230                 235                 240

Ile Glu Ser Asn Gly Tyr Asn Asn Ser Leu Ala Gly Tyr Asp Asn Cys
                245                 250                 255

Pro Asn Ser Asn Asn Tyr Arg Ser Ala Gly Gly Asn Asn Ala Thr Asn
            260                 265                 270

Gln Trp Val Ala Thr Tyr Leu Gln Asn Ala Thr Ala Arg Phe Gln Ser
        275                 280                 285

Met Val Ser Pro Glu Phe Asn Trp Thr Asp Val Asp Thr Tyr Ala Ala
    290                 295                 300

Gln Thr Met Cys Pro Tyr Glu Thr Val Ala Tyr Gly Tyr Ser Ala Phe
305                 310                 315                 320

Cys Asn Leu Phe Thr Tyr Glu Glu Trp Val Gly Phe Glu Tyr Ala Ile
                325                 330                 335

Asp Leu Tyr Phe Ala Gly Gly Ser Ser Phe Ala Ser Pro Thr Gly Arg
            340                 345                 350

Ala Val Gly Ile Gly Tyr Val Gln Glu Val Val Ala Arg Leu Gln Asn
        355                 360                 365

His Thr Leu Gly Tyr Ser Gly Ser Gln Ile Asn Thr Thr Leu Asp Asn
    370                 375                 380

Asn Thr Ala Thr Phe Pro Leu Asn Gln Ser Ile Tyr Phe Asp Phe Ser
385                 390                 395                 400

His Asp Thr Asn Ile Met Ser Ile Leu Thr Ala Phe Gly Phe Thr Gln
                405                 410                 415

Phe Ala Asp Ala Leu Pro Ala Thr His His Pro Gly Pro His Asn Leu
            420                 425                 430

Thr Val Ser His Met Glu Pro Phe Gly Ala Arg Leu Asp Ile Glu Ile
        435                 440                 445

Ile Lys Thr Pro Lys Pro Leu Ala Ala Asp Arg Thr Tyr Val Asp Gly
    450                 455                 460

Lys Glu Thr Thr
465

<210> 28
<211> 540
<212> PRT
<213> aspergillus terreus

<400> 28

Met Arg Leu Ala Ser Gln Ser Ala Leu Val Ala Leu Ala Thr Ala Gly
1               5                   10                  15

His Ala Ala Val Ile Ser Pro Ser Arg Thr Ser Ser Val Pro Glu Tyr
            20                  25                  30

Phe Gln Thr Ser Phe Gly Pro Trp Ala Gly Pro Thr Glu Ala Gly Ala
        35                  40                  45

Pro Pro Phe Leu Ala Gln Thr Asn Pro Ala Pro Phe Gly Asn Pro Thr
    50                  55                  60

Tyr Val Pro Asn Thr Pro Val Val Thr Asp Val Pro Ile Pro Gly Arg
65                  70                  75                  80

Ser Asp Gly Ala Glu Ile Phe Lys Leu Met Gly Asn Leu Ser Pro Tyr
                85                  90                  95

Met Pro Asn Pro Ser Gly Phe Gly Val Asn Glu Tyr Pro Leu Pro Ala
            100                 105                 110

Gly Ala Asn Ile Thr Gln Met His Met Ile His Arg His Gly Ser Arg
        115                 120                 125

Tyr Pro Thr Thr Gly Asn Ala Val Ala Ser Leu Gln Asp Arg Val Ser
    130                 135                 140

Glu Leu Leu Gln Gly Gly Thr Lys Phe Lys Gly Asp Leu Glu Phe Leu
145                 150                 155                 160

Asn Ser Trp Arg Tyr Glu Leu Gly Lys Glu Glu Leu Thr Ala Leu Gly
                165                 170                 175

Arg Gln Gln Leu Phe Asp Ser Gly Val Leu Asn Trp Phe Asn Tyr Gly
            180                 185                 190

Arg Leu Tyr Asp Pro Ser Lys Pro Leu Ile Ala Arg Thr Thr Thr Gln
        195                 200                 205

Val Arg Met Leu Gln Ser Ala Glu Asn Trp Leu Asn Gly Phe Phe Gly
    210                 215                 220

Pro Asp Trp Thr Lys Asn Val Thr Leu Glu Val Leu Ile Glu Ala Asn
225                 230                 235                 240

Gly Phe Asn Asn Ser Leu Ala Gly Asp Lys Gln Cys Val Asn Ser Lys
                245                 250                 255

Asn Asp Arg Ser Ser Ala Gly Asp Thr Ala Ser Ser Glu Trp Gln Lys
            260                 265                 270

Ile Tyr Leu Lys Asn Ala Thr Glu Arg Phe Arg Arg Ser Met Thr Gly
        275                 280                 285

His Ala Asn Trp Thr Val Asp Asp Thr Tyr Asn Ala Gln Thr Met Cys
    290                 295                 300

Pro Tyr Glu Thr Val Gly Leu Gly Tyr Ser Ser Phe Cys Ser Leu Phe
305                 310                 315                 320

Thr Tyr Glu Glu Trp Leu Gly Phe Glu Tyr Ser Leu Asp Leu Ser Tyr
                325                 330                 335

Tyr Gly Ser Asn Ser Phe Gly Ser Pro Thr Gly Arg Ala Val Gly Val
            340                 345                 350

Gly Tyr Val Glu Glu Leu Val Ala Arg Leu Gln Arg His Phe Pro Gln
        355                 360                 365

Pro Ala Glu Gly Ser Val Ala Ile Asn Glu Thr Leu Asp Asp Ser Ala
    370                 375                 380

Thr Thr Phe Pro Leu Asp Gln Ser Leu Tyr Leu Asp Phe Ser His Asp
385                 390                 395                 400

Thr Asp Ile Met Ser Met Leu Thr Ala Met Gly Leu Thr Gln Phe Ala
                405                 410                 415

Gln Phe Leu Pro Ile Ser Gly Pro Pro Lys Asp Gln Gln Leu Ile Val
            420                 425                 430

Ser His Ile Val Pro Phe Ala Gly Arg Phe Asn Ile Glu Val Ile Lys
        435                 440                 445

Ala Pro Gln Pro Val Arg Ala Lys Arg Pro Gln Asn Ala His Ala Ser
    450                 455                 460

Ala Tyr Glu Ser Gly Glu Glu Thr Thr Tyr Val His Met Leu Met Asn
465                 470                 475                 480

Gln Arg Thr Ile Pro Leu Gly Arg Ser Leu Lys Glu Cys Gly Asp Arg
                485                 490                 495

Asp Asp Gly Trp Cys Glu Leu Gln Thr Phe Val Ser Ala Leu Lys Glu
            500                 505                 510

Asn Ile Pro Lys Ala Lys Phe Asp Glu Ser Cys Tyr Gly Asn Tyr Ser
        515                 520                 525

Val Pro Ala Tyr Gly Asp Ile Thr Asp Gly Ala Ile
    530                 535                 540

<210>  29
<211>  565
<212>  PRT
<213>  aspergillus terreus

<400>  29

Met Arg Tyr Ser Gly Phe Ile Leu Leu Ala Ala Ala Ser Gly Ile Glu
1               5                   10                  15

Ala Val Ala Leu Asp Pro Arg Val Thr Ser Leu Gly Pro Ser Ala Thr
            20                  25                  30

Ala Thr Glu Ala Asp Trp Phe Gln Thr Arg Pro Gln Ser Tyr Gln Gly
        35                  40                  45

Lys Lys His Cys His Tyr Ile Val Lys Thr Val Ser His Thr Ser Asn
    50                  55                  60

Thr Gly Val Thr Ala Thr Gly Thr Ala Pro Met Leu Ala Glu Thr Asn

65                  70                  75                  80

Pro Ala Pro Phe Gly Gln Lys Thr Tyr Val Pro Asn Ala Pro Leu Glu
                85                  90                  95

Thr Ser Glu Pro Ile Lys Gly Ala Lys Asn Arg Asn Ile Phe His Leu
            100                 105                 110

Met Gly Asn Leu Ser Pro Tyr Phe Thr Ser Glu Glu Gly Phe Gly Val
        115                 120                 125

Ala Glu Tyr Pro Leu Pro Glu Gly Ser Asp Ile Thr Gln Val His Leu
    130                 135                 140

Ile His Arg His Gly Ser Arg Tyr Pro Ser Asp Ser Glu Thr Ile Ala
145                 150                 155                 160

Ala Phe Ala Gln Arg Ile Val Asn Ala Thr Ala His Gly Thr Lys Phe
                165                 170                 175

Thr Gly Asp Leu Ala Phe Leu Asn Glu Tyr Ser Trp Ser Gln Gly Met
            180                 185                 190

Asn Val Leu Val Pro Met Gly Arg Gln Glu Leu Phe Asp Ser Gly Val
        195                 200                 205

Leu His Tyr Tyr Asn Tyr Gly Ser Leu Tyr Arg Asn Gly Ser Lys Leu
    210                 215                 220

Val Val Arg Ser Thr Thr Ser Asp Arg Ala Leu Lys Ser Ala Glu Asn
225                 230                 235                 240

Phe Leu Ala Gly Phe Phe Gly Leu Glu Trp Arg Asp Tyr Ala Asn Ile
                245                 250                 255

Leu Pro Leu Ile Ser Gly Gly Gly Phe Asn Ser Ser Leu Thr Gly Met
            260                 265                 270

Ser Val Cys Arg Asn Ala Gly Lys Ile Phe Ser Ser Leu Glu Asn Asp
        275                 280                 285

Ala Met Arg Thr Trp Gln Gly Ile Tyr Leu Lys Asp Lys Thr Gln Lys
    290                 295                 300

Leu Arg Lys Met Ala Ser Gly Phe Asn Trp Thr Val Ser Asp Ser Ala
305                 310                 315                 320

Asp Ala Gln Thr Leu Cys Ala Tyr Glu Thr Val Ser Phe Gly Tyr Ser
                325                 330                 335

Ser Phe Cys Gln Leu Phe Asp Tyr Glu Glu Trp Glu Gly Tyr Ser Tyr
            340                 345                 350

Ala Ser Ser Ile Val Ser Ser Ser Leu Met Gly Phe Gly Ser Pro Ile
        355                 360                 365

Ser Arg Ala Thr Gly Ile Ala Trp Val Gln Glu Phe Leu Ala Arg Val
    370                 375                 380

Glu Ser His Leu Leu Asp Val Ala Pro Gly Gln Thr Ser Ala Asn Leu
385                 390                 395                 400

Thr Leu Ala Thr Asn Pro Val Thr Phe Pro Leu Asp Gln Pro Leu Tyr
                405                 410                 415

Phe Asp Phe Gly His Asp Ala Ser Ile Leu Ala Ala Leu Ser Ala Phe
            420                 425                 430

Gly Ile Arg Gln Phe Ala Asp Tyr Leu Pro Pro Thr Gly Pro Pro Thr
        435                 440                 445

His Lys Gln Phe Asp Ala Gly Lys Ile Thr Pro Phe Ala Gly Arg Leu
    450                 455                 460

Asn Ile Glu Ile Ile Glu Thr Leu His Ala Val Lys Thr Arg Arg Pro
465                 470                 475                 480

Arg Gly Glu Asn Glu Arg Ser Tyr Ile Ser Gly Thr Gly Lys Thr Arg
                485                 490                 495

Tyr Val His Phe Leu Leu Asn Gln Arg Thr Val Pro Leu His Ala Ser
            500                 505                 510

Phe Lys Glu Cys Glu Tyr Arg Asp Asp Gly Trp Cys Glu Leu Ser Thr
        515                 520                 525

Phe Leu Glu Val Gln Lys Gly Ser Leu Gly Lys Ala Gln Phe Gln Tyr
    530                 535                 540

Ser Cys Glu Gly Asn Trp Thr Val Gly Pro Tyr Gly Ser Val Thr Asp
545                 550                 555                 560

Gly Val Pro Pro Gln
                565

<210>  30
<211>  552
<212>  PRT
<213>  aspergillus niger

<400>  30

Met His Pro Thr Ile Ala Thr Leu Ser Ile Leu Ala Val Ala Ser Leu
1               5                   10                  15

Ala Glu Ala Val Ala Ile Glu Ala Arg Ser Gln Asn Ala Thr Pro Leu
            20                  25                  30

Thr Pro Ser Pro Ser Ala Thr Thr Thr Gly Ile Asp Trp Phe Gln Thr
        35                  40                  45

Thr Pro Glu Ser Tyr Gln Gly Thr Thr Ala Thr Gly Val Ala Pro Phe
    50                  55                  60

Leu Ala Glu Thr Asn Pro Ala Pro Phe Gly Gln Lys Thr Leu Ser Pro
65                  70                  75                  80

Asn Asp Pro Leu Glu Thr Ser Glu Pro Ile Glu Gly Ala Ala Gly Arg
                85                  90                  95

Asn Ile Phe His Tyr Met Gly Asn Leu Ser Pro Tyr His Val Pro Asp
            100                 105                 110

Gly Phe Gly Val Asp Glu Tyr Pro Leu Pro Lys Gly Ser Asn Ile Thr
        115                 120                 125

Gln Met His Met Ile His Arg His Gly Ser Arg Tyr Pro Ser Ser Ser
    130                 135                 140

Glu Gly Leu Ala Ser Trp Ala Gln Lys Ile Ile Asn Ser Thr Ala Ser

145                 150                 155                 160

Gly Asn Asn Phe Thr Gly Ala Leu Ser Phe Leu Asn Asp Trp Asp Tyr
                165                 170                 175

Gly Leu Gly Leu Glu Ile Leu Val Pro Lys Gly Arg Gln Glu Leu Tyr
            180                 185                 190

Asp Ser Gly Val Leu Asn Phe Tyr Asn Tyr Gly His Leu Tyr Asn Ala
        195                 200                 205

Ser Ser Pro His Lys Leu Val Ala Arg Thr Thr Thr Gln Asp Arg Met
    210                 215                 220

Leu Lys Ser Ala Glu Asn Phe Leu Ala Gly Phe Phe Gly Leu Glu Trp
225                 230                 235                 240

Thr Glu Lys Ala Asn Leu Leu Pro Ile Ile Glu Gly Val Gly Tyr Asn
                245                 250                 255

Asn Ser Leu Ile Gly Thr Tyr Ser Cys Thr Arg Ala Leu Glu Tyr Met
            260                 265                 270

Ala Tyr Asn Ala Thr Thr Pro Leu Ser Thr Trp Lys Asn Ile Tyr Leu
        275                 280                 285

Lys Ala Arg Thr Glu Ala Leu Arg Thr Leu Thr Gly Ser Tyr Asn Trp
    290                 295                 300

Thr Thr Thr Asp Ser Phe Asn Ala Gln Asp Met Cys Ala Tyr Glu Thr
305                 310                 315                 320

Ile Ser Tyr Gly Tyr Ser Gln Phe Cys Glu Leu Phe Thr Phe Glu Glu
                325                 330                 335

Phe Glu Asn Phe Gly Tyr Ala Phe Asp Ile Glu Phe Ala Asn Met Val
            340                 345                 350

Gly Phe Ala Cys Pro Ala Gly Arg Ala Gln Gly Ile Ala Trp Val Glu
        355                 360                 365

Glu Phe Leu Ala Arg Val Glu Gly His Leu Leu Gln Thr Thr Gly Thr
    370                 375                 380

Asn Ala Asn Met Thr Leu Asp Thr Asn Pro Val Thr Phe Pro Thr Asp
385                 390                 395                 400

Gln Asn Leu Tyr Leu Asp Phe Ser His Asp Ala Gly Ile Val Ala Val
                405                 410                 415

Leu Thr Ala Phe Gly Phe Arg Gln Phe Ala Glu Ser Leu Pro Ala Thr
            420                 425                 430

Gly Pro Pro Leu Tyr Gln Gln Phe Lys Ser Ser Lys Ile Val Pro Phe
        435                 440                 445

Ala Gly Arg Thr Asn Ile Glu Ile Ile Lys Ala Pro Asn Gln Val Ala
    450                 455                 460

Ala Ile Arg Pro Ala Gly Asp Gln Ser Asp Ala Tyr Val Asp Gly Thr
465                 470                 475                 480

Gly Glu Thr Phe Tyr Val His Phe Leu Gln Asn Gln Arg Thr Leu Pro
                485                 490                 495

Leu His Ser Ser Phe Glu Glu Cys Glu Tyr Arg Asp Asp Gly Trp Cys
            500                 505                 510

Glu Leu Ser Thr Phe Met Ala Val Gln Lys Lys Gly Leu Glu Arg Ser
        515                 520                 525

Gln Phe Glu Tyr Ala Cys Phe Gly Asn Trp Thr Val Thr Glu Tyr Gly
    530                 535                 540

Thr Val Thr Asp Gly Val Pro Val
545                 550

<210>  31
<211>  547
<212>  PRT
<213>  Aspergillus niger

<400>  31

Met Arg Ser Arg Ile Tyr Trp Ala Ile Leu Phe Leu Leu Leu Val Ser
1               5                   10                  15

Cys Val Ser Ala Asn Asp Gln Tyr Val Met Gly Val Ala Pro Thr Thr
            20                  25                  30

Thr Glu Pro Glu Trp Phe Lys Thr Arg Pro Gln Ser Phe Gln Gly Tyr
        35                  40                  45

Thr Ala Thr Gly Ala Ala Pro Phe Leu Ala Gln Thr Asn Pro Ala Pro
    50                  55                  60

Phe Gly Asn Pro Ala Thr Tyr Thr Ala Asn His Pro Leu Glu Thr Ser
65                  70                  75                  80

Gln Pro Ile Arg Gly Gly Lys Asp Arg Asn Ile Phe His His Met Gly
                85                  90                  95

Ile Leu Ser Pro Tyr Tyr Pro Arg Ala Asp Gly Phe Gly Val Asp Glu
            100                 105                 110

Phe Pro Arg Pro Lys Gly Ser Asn Ile Thr Gln Met His Met Leu His
        115                 120                 125

Arg His Gly Ser Arg Tyr Pro Asn Lys Asp Glu Gly Asp Asp Phe Ala
    130                 135                 140

Asn Trp Ile Lys Ala Ile Thr Asn Ala Thr Ala His Gly Ala Val Phe
145                 150                 155                 160

Arg Asp Glu Leu Ser Phe Ile His Asp Trp Thr Tyr Ser Leu Gly Ala
                165                 170                 175

Asp Met Leu Thr Thr Arg Gly Arg Glu Asp Leu Leu Glu Ser Gly Ile
            180                 185                 190

Leu Asn Phe Tyr Asn Tyr Gly His Leu Tyr Thr Pro Gly Thr Lys Ile
        195                 200                 205

Val Ala Arg Thr Thr Thr Gln Asp Arg Met Leu Lys Ser Ala Glu Asn
    210                 215                 220

Phe Leu Ala Gly Phe Phe His Leu Asp Trp Asp Glu His Val Asn Leu
225                 230                 235                 240

Leu Ala Met Ile Glu Glu Lys Asn Phe Asn Ser Ser Leu Gln Ala Lys

                245                 250                 255

Asn Ala Cys Pro Asn Ala Met Lys Ile Ser Phe Asp Asp Tyr Val Ser
            260                 265                 270

Asp Thr Val Thr Lys Trp Lys Thr His Tyr Leu Ser His Arg Thr His
        275                 280                 285

His Leu Asn Tyr Leu Ser Thr Asp Tyr His Trp Thr Ser Asn Asp Ser
    290                 295                 300

Phe Asn Ala Gln Thr Leu Cys Ala Tyr Glu Thr Val Ala Leu Gly Tyr
305                 310                 315                 320

Ser Pro Trp Cys Ser Leu Phe Thr Phe Pro Glu Trp Glu Gly Phe Ser
                325                 330                 335

Tyr Thr Tyr Asp Leu Thr Phe Gly Gly Asn Ala Gly Phe Gln Cys Pro
            340                 345                 350

Ile Ser Arg Ala Met Gly Ile Thr Trp Val Gln Glu Phe Leu Ala Arg
        355                 360                 365

Val Glu Asn Arg Ser Phe Ser Thr Pro Gly Ser Ser Ser Ala Ala Asn
    370                 375                 380

Leu Thr Leu Asn Thr Asn Pro Val Thr Phe Pro Thr Asn Gln Ser Leu
385                 390                 395                 400

Tyr Phe Asp Phe Ala His Asp Lys Ile Leu Leu Gly Val Leu Thr Ala
                405                 410                 415

Phe Gly Leu Arg Gln Phe Ala Asp Leu Pro Phe Pro Asp Tyr Thr Asp
            420                 425                 430

Gln Tyr Phe Met Asp Val Phe Pro Pro Arg His His Ala Phe Gln Thr
        435                 440                 445

Ala Lys Leu Ile Pro Phe Ala Gly Arg Leu Asn Ile Glu Ile Ile Arg
    450                 455                 460

Ala Pro His Lys Ile Asn Pro Arg Gln Thr Glu Tyr Val His Phe Leu
465                 470                 475                 480

Leu Asn Gln Arg Thr Val Pro Leu His Lys Ser Leu Pro Glu Cys Ser
                485                 490                 495

Phe Arg Ser Asp Gly Trp Cys Glu Leu Asp Ala Phe Leu Arg Ala Gln
            500                 505                 510

Lys Asp Ser Leu Arg Lys Ala Glu Tyr Asp Phe Ser Cys Val Gly Glu
        515                 520                 525

Trp Asp Leu Gly Glu Phe Gly Asp Val Trp Asp Gly Val Pro Val Leu
    530                 535                 540

Arg Gly Glu
545

<210>  32
<211>  484
<212>  PRT
<213>  Phaeosphaeria nodorum

<400>  32

Met Leu Gly Ile Phe Glu Leu Ser Leu Gln Val Leu Asn Ser Phe Asn
1               5                   10                  15

Pro Ala Lys Tyr Gly Pro Thr Gln Thr Gly Asp Ala Pro Trp Leu Ala
            20                  25                  30

Gln Thr Asn Ala Ala Pro Phe Ala Gly Val Ser Tyr Ile Pro Asn Thr
        35                  40                  45

Pro Leu Glu Thr Gln Ile Pro Ile Ala Gly Asn Val Asn Asn Thr Asn
    50                  55                  60

Ile Phe Gln Arg Leu Ala Ile Leu Ser Pro Tyr Phe Pro Asn Pro Arg
65                  70                  75                  80

Gly Phe Gly Val Asn Glu Tyr Ser Val Pro Phe Gly Ser Asn Val Thr
                85                  90                  95

Trp Leu Asn Met Val His Arg His Gly Ser Arg Tyr Pro Glu Val Ser
            100                 105                 110

Gly Asp Ala Ala Glu Arg Thr Leu Gly Leu Lys Leu Ala Ala Ala Glu
        115                 120                 125

Gly Lys Phe Ala Gly His Gly Pro Leu Ser Phe Leu Asn Asp Trp Lys
    130                 135                 140

Phe Met Leu Gly Ala Glu Ile Leu Val Pro Asn Gly Lys Ala Glu Leu
145                 150                 155                 160

Phe Gln Ser Gly Thr Leu His Tyr Tyr Gln Tyr Gly His Leu Tyr Pro
                165                 170                 175

Asn Asn Gly Ser Lys Ile Ile Val Arg Ser Thr Thr Gln Arg Arg Met
            180                 185                 190

Tyr Glu Ser Ala Glu Tyr Phe Leu Ala Gly Phe Phe Gly Leu Ser Trp
        195                 200                 205

Thr Gln Asn Ala Thr Leu Glu Leu Ala Ile Glu Trp Pro Gly Phe Asn
    210                 215                 220

Asn Thr Leu Ala Gly Tyr Lys Gln Cys Asn His Thr Gly Trp Leu Thr
225                 230                 235                 240

Ala Arg Glu Gly Asp Leu Asp Trp Thr Leu Ser Asp Thr Tyr Asn Ala
                245                 250                 255

Gln Ala Leu Cys Ala Tyr Glu Thr Val Gly Leu Gly Phe Ser His Phe
            260                 265                 270

Cys Gly Leu Phe Thr Tyr Glu Glu Trp Glu Gly Tyr Glu Tyr Ala Leu
        275                 280                 285

Asp Ile Ala Phe Ser Ala Gly Thr Ala Phe Gly Ser Pro Val Gly Arg
    290                 295                 300

Ala Ile Gly Val Gly Tyr Val Gln Glu Val Leu Ala Arg Met Gln His
305                 310                 315                 320

His Val Ile Thr Asp Pro Ala Ala Gln Ile Asn Thr Thr Leu Asp Asn
                325                 330                 335

Asn Thr Val Thr Phe Pro Val Asp Gln Asn Ile Asn Leu Asp Phe Ser

340                 345                 350

His Asp Ala Asn Ile Ile Ser Ile Leu Ala Ala Phe Gly Leu Thr Gln
        355                 360                 365

Phe Ala Asp Tyr Leu Pro Thr Ser His Ile Lys Lys Asp Arg Glu Phe
    370                 375                 380

Val Met Ser Tyr Leu Glu Pro Phe Ala Gly Arg Leu Asp Ile Glu Leu
385                 390                 395                 400

Ile Lys Thr Pro Thr Pro Val Asn Pro Asp Arg Ser Ala Lys Lys Ile
                405                 410                 415

Tyr Leu Asp Gly Glu Pro Thr Thr Tyr Val His Phe Ile Leu Asn Gln
            420                 425                 430

Arg Leu Arg Glu Gln Gly Arg Arg Gly Val Cys Glu Met Ser Ile Phe
        435                 440                 445

Leu Arg Val Met Glu Lys Glu Ile Glu Lys Ala Asp Tyr Asp Tyr Ala
    450                 455                 460

Cys Phe Gly Asp Tyr Asp Leu Pro Ala Tyr Gly Glu Ile Lys Asp Gly
465                 470                 475                 480

Arg Pro Ile Arg

<210>  33
<211>  531
<212>  PRT
<213>  aspergillus oryzae

<400>  33

Met Gln Asp Met Val Phe Ala Leu Pro Leu Ile Thr Ala Ser Trp Val
1               5                   10                  15

Gly Val Thr Ala Ala Ser Ser Ala Arg Ala Thr Gly Thr Thr Gln Pro
            20                  25                  30

Asn Trp Phe Gln Thr Ser Pro Gln Ser Tyr Gln Gly Pro Thr Ala Thr
        35                  40                  45

Gly Ser Ala Pro Phe Leu Ala Val Thr Asn Pro Ala Phe Ser Ser His
    50                  55                  60

Gly Thr Tyr Ile Ala Asn Glu Pro Leu Val Thr Glu Gln Pro Ile Ser
65                  70                  75                  80

Gly Ala His Gly Arg Asn Ile Phe His His Ala Gly Asn Leu Ser Pro
                85                  90                  95

Tyr Phe Ser Ser Glu Glu Gly Phe Gly Val Asp Glu Tyr Pro Leu Pro
            100                 105                 110

Ala Gly Ala Asn Ile Thr Gln Met His Met Pro His Arg His Gly Ser
        115                 120                 125

Arg Tyr Pro Ser Ser Ser Glu Gly Tyr Pro Ser Trp Ala Glu Gly Ile
    130                 135                 140

Lys Asn Ser Thr Thr Ala Gly Asn Arg Phe Lys Ala Glu Ile Leu Val
145                 150                 155                 160

Pro Lys Gly Arg Gln Glu Met Phe Asp Ser Gly Val Leu Asn Tyr Tyr
                165                 170                 175

Asn Tyr Gly His Leu Tyr Asn Glu Ser Leu Gly His Lys Leu Val Ala
            180                 185                 190

Arg Thr Thr Thr Gln Asn Arg Met Leu Lys Ser Ala Glu Asn Phe Leu
        195                 200                 205

Ala Gly Phe Phe Gly Leu Asp Trp Thr Asp Lys Ala Asn Leu Leu Ala
    210                 215                 220

Met Ile Glu Asn Val Gly Phe Asn Asn Ser Leu Ile Gly Thr Tyr Ser
225                 230                 235                 240

Cys Pro Asn Ala Met Thr Val Met Ala Asn Thr Ser Ile Tyr Glu Pro
                245                 250                 255

Met Asn Gln Trp Ile Asn Ile Tyr Leu Lys Asn Arg Thr Thr Thr Leu
            260                 265                 270

Lys Glu Leu Ser Gly Ser Tyr Asn Trp Thr Ala Thr Asp Ser His Asn
        275                 280                 285

Ala Gln Ala Leu Cys Val Tyr Glu Thr Ile Ser Phe Gly Tyr Ser Gln
    290                 295                 300

Phe Cys Gln Leu Phe Thr Tyr Lys Glu Phe Glu Gln Phe Ser Tyr Ala
305                 310                 315                 320

Tyr Asp Leu Met Phe Thr Ala Met Val Gly Phe Gln Asn Pro Ala Gly
                325                 330                 335

Arg Ala Gln Gly Ile Ala Trp Val Glu Glu Phe Leu Ala Arg Val Glu
            340                 345                 350

Gly His Val Leu Gln Thr Thr Gly Thr Asn Ala Asn Met Thr Leu Ala
        355                 360                 365

Thr Asn Pro Val Thr Phe Pro Met Asp Gln Asn Leu Tyr Leu Asp Phe
    370                 375                 380

Thr His Asp Ser Asp Ile Phe Ala Thr Leu Thr Ala Phe Gly Phe Arg
385                 390                 395                 400

Gln Phe Ala Gln Phe Leu Pro Pro Thr Gly Pro Pro Lys Asn Gln Gln
                405                 410                 415

Phe Ser Thr Ser Lys Val Val Pro Phe Gly Gly Arg Thr Asn Ile Glu
            420                 425                 430

Ile Ile Arg Ala Pro His Lys Val Ser Thr Lys Arg Ser Arg Asn Glu
        435                 440                 445

Thr Gln Ser Val Tyr Val Lys Asp Thr Lys Asp Thr Tyr Tyr Val His
    450                 455                 460

Phe Leu Gln Asn Gln Arg Thr Leu Pro Leu His Ala Ser Phe Pro Glu
465                 470                 475                 480

Cys Glu Tyr Arg Asp Asp Gly Trp Cys Glu Leu Asp Thr Phe Met Lys
                485                 490                 495

Val Gln Arg Lys Ser Leu Gln Arg Ser Gln Phe Glu Tyr Ala Cys Phe

500                 505                 510

Gly Asn Trp Ser Thr Pro Ala Tyr Gly Asp Val Thr Asp Gly Val Pro
        515                 520                 525

Pro Arg Ser
    530

<210>  34
<211>  436
<212>  PRT
<213>  Sclerotinia sclerotiorum

<400>  34

Met Leu Leu Asp Gln Ile Leu Leu Leu Ser Leu Thr Gly Val Thr Thr
1               5                   10                  15

Ala Ala Leu Leu Pro Glu Lys Gln Gln Ser Ser Thr Ser Thr Ser Asp
            20                  25                  30

Val Pro Gln Tyr Phe Gln Thr Thr Pro Glu Leu Trp Ala Gly Pro Thr
        35                  40                  45

Ala Thr Gly Arg Ala Pro Phe Leu Ala Gln Thr Asn Pro Val Ser Phe
    50                  55                  60

Ala Pro Thr Val Thr Phe Val Pro Asn Thr Pro Leu Glu Thr Ala Gln
65                  70                  75                  80

Pro Ile Val Gly Gln Gly Gln Asn Gln Ser Ile Phe Gln Leu Met Gly
                85                  90                  95

Asn Leu Ser Pro Tyr Leu Pro Asn Pro Ser Gly Phe Gly Val Ala Glu
            100                 105                 110

Tyr Pro Leu Pro Pro Gly Ala Lys Ile Ala Gln Leu Gln Met Leu Ser
        115                 120                 125

Arg His Gly Ser Arg Tyr Pro Thr Val Gly Ser Asn Val Glu Val Phe
    130                 135                 140

Gly Glu Lys Val Ala Asn Asn Thr Glu Lys Leu Asn Ala Thr Gly Ala
145                 150                 155                 160

Leu Ser Phe Leu Asn Asp Trp Lys Tyr Gly Leu Gly His Glu Ile Leu
                165                 170                 175

Val Pro Arg Gly Arg Gln Glu Leu Tyr Asp Ser Gly Ile Leu His Tyr
            180                 185                 190

Tyr Gln Tyr Ala Gln Leu Tyr Asn Pro His Ser Lys Ile Ile Ala Arg
        195                 200                 205

Thr Thr Thr Gln Asp Arg Met Leu Lys Ser Ala Glu Tyr Phe Met Ala
    210                 215                 220

Gly Phe Phe Gly Leu Glu Trp Thr Asn Asn Ala Thr Ile Glu Val Ile
225                 230                 235                 240

Ile Glu Ser Gln Gly Tyr Asn Asn Ser Leu Ala Gly Tyr Asp Asn Cys
                245                 250                 255

Pro Asn Ser Asn Asn Tyr Arg Ser Ala Gly Gly Ser Asn Ala Ser Asp
            260                 265                 270

Gln Trp Val Ala Val Tyr Leu Gln Asp Ala Thr Thr Arg Phe Gln Ser
        275                 280                 285

Met Val Ser Pro Glu Phe Asn Trp Thr Val Thr Asp Thr Tyr Ala Ala
    290                 295                 300

Gln Thr Leu Cys Pro Tyr Glu Thr Pro Phe Gly Ala Arg Leu Asp Ile
305                 310                 315                 320

Glu Leu Ile Lys Thr Pro Lys Pro Leu Ala Ala Asn Arg Thr Tyr Ile
                325                 330                 335

Asp Gly Lys Glu Thr Thr Tyr Ile His Phe Ile Leu Asn Gln Arg Thr
            340                 345                 350

Ile Pro Leu Gly Phe Ser Phe Leu Glu Cys Gly Val Asp Arg Leu Asp
        355                 360                 365

Gly Trp Cys Glu Leu Glu Thr Phe Leu Lys Val Gln Ser Lys Ser Thr
    370                 375                 380

Glu Glu Ala Gln Tyr Glu Tyr Ala Cys Phe Gly Asp Tyr Pro Ala Glu
385                 390                 395                 400

Pro Tyr Gly Ser Val Lys Asn Glu Asn Gln Gly Leu Arg Ala Ala Gln
                405                 410                 415

Ile Gly Phe Leu Ser Ser Pro Ser Leu Ser Val Tyr Gly Thr Gly Thr
            420                 425                 430

Thr Glu Arg Leu
        435

SEQUENCE LISTING

<110>  Nederlandse Organisatie voor
       toegepast-natuurwetenschappelijk onderzoek TNO

<120>  Novel phytases

<130>  P83267EP00

<160>  34

<170>  PatentIn version 3.3

<210>  1
<211>  24
<212>  PRT
<213>  Artificial

<220>
<223>  signal sequence

<400>  1

Met Val Ala Ser Ser Ile Gly Val Leu Leu Ala Ala Val Leu Leu Gly
1               5                   10                  15

Gly Thr Ser Thr Ile Val Thr Ala
            20

<210>  2
<211>  2262
<212>  DNA
<213>  Magnaporthe grisea

<400>  2
atggtcgcca gcagcatcgg cgtcctcctc gccgccgtcc tcctcggcgg caccagcacc      60

atcgtcaccg ccgccccggt cgagctggag ctgaacgtca ccgccctcac ccccggcatc     120

atcgacagcg actggaccgc cgcctactac cccaagagcg gcgccgcccc cctcctcctc     180

gccaacgacg ccggcaccgc cacgggcggc ttccgcgtgt tcggcctgga cggcgccccc     240

accctcagcc aggccggcca cgtcatcgcc ggtcgcacca agctcgtcgc ccccctctac     300

aacgcctccg gcgccggcga cgacttcctc gtcaccatcg ccaccaccga cagcgtcgtc     360

cgcctctaca gcctccccga cctcgccgtc gtccccatcg ccgagcccct cgtcaccctc     420

ggcgactgga acgccgtctg cagctggggc agctacttct tcctcttcgg caagggcggt     480

cgggccttcc agtacctcgt ccgccccgcc gacggcggca gcttcgaggt cctcgaggtc     540

cgcgccctcc ccgcccccgt cgtgttcgtc ggctgcgccg tcagcccccg cgacgccaag     600

ctctacctca ccgaggacgt cgaccaggac gtctacgtca tggacgtcgg cgacatggcc     660

ggccagccga ccctcaagaa ggtcaagggc gtcaaggacg ccaccggcat cgccctctac     720

agcagcggcg accaggagta cgtgttcgtc gcccaggagt cccagctcag cgtctacggc     780

cccgactgga ccctcctcgg caccgtccag ctcgccggcc tggacgacat cgagatccag     840

ggcctcagca tctaccaggc ccagaccagc acctacccca gcggcgccat caccttcgcc     900

atcgaggccg acgccacgga gggcttcggc accacctccc tccagcccat cctcgacgcc     960

ctcagcatcc cccccaacac cgcctacacc ccccgctccg agcccaccgg cccccgcacc 1020

tgcgcccgct gcagcgacaa cggcttctgc cccaccgtcg gctgccccaa gacctgcacc 1080

agctgcctcc tcggcttcag cggccccacc tgcagcaccc cccgctgcga ggccgactgc 1140

tccggtcgcg gcacctgcgt cggccccaac aagtgcgcct gcgacgccgg ctggggcggc 1200

ctccactgca gcttcgtcct ggtggaggcc gaccgcgaga ccgacgccgc cggtggtgac 1260

ggtgacgacc ccgccgtctg gatcagcccc gtcagcaagg agctgagccg cgtcatcacc 1320

accgtcaaga gcgaggccgg tgccggcctc aacgtgttcg acctcgaggg caagctcgtc 1380

cagcagttca ccgccggcca gcccaacaac gtcgacgtca tctacggctt caaggccggc 1440

gaccgcgtcg tcgacctcgc cttcgccggc tgccgcagcg acgagaccct ctgcctcttc 1500

gagatcacca gcaacggcac catcaccacc atccccggcg gcatccagcc cctccccaag 1560

ggctacaagg tctacggcag ctgcgtctac cgcagcccca agagcaaccg ccagtacctc 1620

ttcgtcaaca gcaagaaggc ccgctacctc cagtacgagc tgaccagcac cgagagcggc 1680

accctccaga ccaccctcgt ccgcgacttc accggcggca gcggcggcca ggtcgagggc 1740

tgcgtcgtgg acgaccgcaa ctcctggctc ttcctcggcg aggagccccg cgccctctgg 1800

cgctacggcg ccgagccgga cgacggcacc gagggcctcg agatcgccgt gttcggcgac 1860

ggcaagctga acgccgaggt cgagggcgtc accctcgtcg agggcaagac cccccaggag 1920

ggctacatcc tcgtcagcaa ccagggcgtc agcagctacg ccgtctaccg ccgcgccgag 1980

ccccacgagt acgtcgccac cttcagcatc gtcgccagcg ccgacggcaa gatcgacgcc 2040

gtcagcaaca ccgacggcat caccgccgtg gccacgggcc tcggccccaa cttcccccac 2100

ggcctcgtcg tcgtccatga cgacgccaac cagctccccg agggcggcac ctccaccgag 2160

gccagcttca agatgatcag cctcgagaag atcttcggcg gccccctcaa ggagctgaac 2220

ctcctcgacc acgtcgacgc caactgggac ccccgcgcct aa 2262

<210> 3
<211> 753
<212> PRT
<213> Magnaporthe grisea

<400> 3

Met Val Ala Ser Ser Ile Gly Val Leu Leu Ala Ala Val Leu Leu Gly
1               5                   10                  15

Gly Thr Ser Thr Ile Val Thr Ala Ala Pro Val Glu Leu Glu Leu Asn
            20                  25                  30

Val Thr Ala Leu Thr Pro Gly Ile Ile Asp Ser Asp Trp Thr Ala Ala
        35                  40                  45

Tyr Tyr Pro Lys Ser Gly Ala Ala Pro Leu Leu Leu Ala Asn Asp Ala
    50                  55                  60

Gly Thr Ala Thr Gly Gly Phe Arg Val Phe Gly Leu Asp Gly Ala Pro

65                  70                  75                  80

Thr Leu Ser Gln Ala Gly His Val Ile Ala Gly Arg Thr Lys Leu Val
                85                  90                  95

Ala Pro Leu Tyr Asn Ala Ser Gly Ala Gly Asp Asp Phe Leu Val Thr
            100                 105                 110

Ile Ala Thr Thr Asp Ser Val Val Arg Leu Tyr Ser Leu Pro Asp Leu
        115                 120                 125

Ala Val Val Pro Ile Ala Glu Pro Leu Val Thr Leu Gly Asp Trp Asn
    130                 135                 140

Ala Val Cys Ser Trp Gly Ser Tyr Phe Phe Leu Phe Gly Lys Gly Gly
145                 150                 155                 160

Arg Ala Phe Gln Tyr Leu Val Arg Pro Ala Asp Gly Gly Ser Phe Glu
                165                 170                 175

Val Leu Glu Val Arg Ala Leu Pro Ala Pro Val Val Phe Val Gly Cys
            180                 185                 190

Ala Val Ser Pro Arg Asp Ala Lys Leu Tyr Leu Thr Glu Asp Val Asp
        195                 200                 205

Gln Asp Val Tyr Val Met Asp Val Gly Asp Met Ala Gly Gln Pro Thr
    210                 215                 220

Leu Lys Lys Val Lys Gly Val Lys Asp Ala Thr Gly Ile Ala Leu Tyr
225                 230                 235                 240

Ser Ser Gly Asp Gln Glu Tyr Val Phe Val Ala Gln Glu Ser Gln Leu
                245                 250                 255

Ser Val Tyr Gly Pro Asp Trp Thr Leu Leu Gly Thr Val Gln Leu Ala
            260                 265                 270

Gly Leu Asp Asp Ile Glu Ile Gln Gly Leu Ser Ile Tyr Gln Ala Gln
        275                 280                 285

Thr Ser Thr Tyr Pro Ser Gly Ala Ile Thr Phe Ala Ile Glu Ala Asp
    290                 295                 300

Ala Thr Glu Gly Phe Gly Thr Thr Ser Leu Gln Pro Ile Leu Asp Ala
305                 310                 315                 320

Leu Ser Ile Pro Pro Asn Thr Ala Tyr Thr Pro Arg Ser Glu Pro Thr
                325                 330                 335

Gly Pro Arg Thr Cys Ala Arg Cys Ser Asp Asn Gly Phe Cys Pro Thr
            340                 345                 350

Val Gly Cys Pro Lys Thr Cys Thr Ser Cys Leu Leu Gly Phe Ser Gly
        355                 360                 365

Pro Thr Cys Ser Thr Pro Arg Cys Glu Ala Asp Cys Ser Gly Arg Gly
    370                 375                 380

Thr Cys Val Gly Pro Asn Lys Cys Ala Cys Asp Ala Gly Trp Gly Gly
385                 390                 395                 400

Leu His Cys Ser Phe Val Leu Val Glu Ala Asp Arg Glu Thr Asp Ala
                405                 410                 415

Ala Gly Gly Asp Gly Asp Asp Pro Ala Val Trp Ile Ser Pro Val Ser
            420                 425                 430

Lys Glu Leu Ser Arg Val Ile Thr Thr Val Lys Ser Glu Ala Gly Ala
        435                 440                 445

Gly Leu Asn Val Phe Asp Leu Glu Gly Lys Leu Val Gln Gln Phe Thr
    450                 455                 460

Ala Gly Gln Pro Asn Asn Val Asp Val Ile Tyr Gly Phe Lys Ala Gly
465                 470                 475                 480

Asp Arg Val Val Asp Leu Ala Phe Ala Gly Cys Arg Ser Asp Glu Thr
                485                 490                 495

Leu Cys Leu Phe Glu Ile Thr Ser Asn Gly Thr Ile Thr Thr Ile Pro
            500                 505                 510

Gly Gly Ile Gln Pro Leu Pro Lys Gly Tyr Lys Val Tyr Gly Ser Cys
        515                 520                 525

Val Tyr Arg Ser Pro Lys Ser Asn Arg Gln Tyr Leu Phe Val Asn Ser
    530                 535                 540

Lys Lys Ala Arg Tyr Leu Gln Tyr Glu Leu Thr Ser Thr Glu Ser Gly
545                 550                 555                 560

Thr Leu Gln Thr Thr Leu Val Arg Asp Phe Thr Gly Gly Ser Gly Gly
                565                 570                 575

Gln Val Glu Gly Cys Val Val Asp Asp Arg Asn Ser Trp Leu Phe Leu
            580                 585                 590

Gly Glu Glu Pro Arg Ala Leu Trp Arg Tyr Gly Ala Glu Pro Asp Asp
        595                 600                 605

Gly Thr Glu Gly Leu Glu Ile Ala Val Phe Gly Asp Gly Lys Leu Asn
    610                 615                 620

Ala Glu Val Glu Gly Val Thr Leu Val Glu Gly Lys Thr Pro Gln Glu
625                 630                 635                 640

Gly Tyr Ile Leu Val Ser Asn Gln Gly Val Ser Ser Tyr Ala Val Tyr
                645                 650                 655

Arg Arg Ala Glu Pro His Glu Tyr Val Ala Thr Phe Ser Ile Val Ala
            660                 665                 670

Ser Ala Asp Gly Lys Ile Asp Ala Val Ser Asn Thr Asp Gly Ile Thr
        675                 680                 685

Ala Val Ala Thr Gly Leu Gly Pro Asn Phe Pro His Gly Leu Val Val
    690                 695                 700

Val His Asp Asp Ala Asn Gln Leu Pro Glu Gly Gly Thr Ser Thr Glu
705                 710                 715                 720

Ala Ser Phe Lys Met Ile Ser Leu Glu Lys Ile Phe Gly Gly Pro Leu
                725                 730                 735

Lys Glu Leu Asn Leu Leu Asp His Val Asp Ala Asn Trp Asp Pro Arg
            740                 745                 750

Ala

<210>  4
<211>  340
<212>  PRT
<213>  Magnaporthe grisea

<400>  4

Met Val Ala Ser Ser Ile Gly Val Leu Leu Ala Ala Val Leu Leu Gly
1               5                   10                  15

Gly Thr Ser Thr Ile Val Thr Ala Ala Pro Val Glu Leu Glu Leu Asn
            20                  25                  30

Val Thr Ala Leu Thr Pro Gly Ile Ile Asp Ser Asp Trp Thr Ala Ala
        35                  40                  45

Tyr Tyr Pro Lys Ser Gly Ala Ala Pro Leu Leu Leu Ala Asn Asp Ala
    50                  55                  60

Gly Thr Ala Thr Gly Gly Phe Arg Val Phe Gly Leu Asp Gly Ala Pro
65                  70                  75                  80

Thr Leu Ser Gln Ala Gly His Val Ile Ala Gly Arg Thr Lys Leu Val
                85                  90                  95

Ala Pro Leu Tyr Asn Ala Ser Gly Ala Gly Asp Asp Phe Leu Val Thr

            100                 105                 110

Ile Ala Thr Thr Asp Ser Val Val Arg Leu Tyr Ser Leu Pro Asp Leu
        115                 120                 125

Ala Val Val Pro Ile Ala Glu Pro Leu Val Thr Leu Gly Asp Trp Asn
    130                 135                 140

Ala Val Cys Ser Trp Gly Ser Tyr Phe Phe Leu Phe Gly Lys Gly Gly
145                 150                 155                 160

Arg Ala Phe Gln Tyr Leu Val Arg Pro Ala Asp Gly Gly Ser Phe Glu
                165                 170                 175

Val Leu Glu Val Arg Ala Leu Pro Ala Pro Val Val Phe Val Gly Cys
            180                 185                 190

Ala Val Ser Pro Arg Asp Ala Lys Leu Tyr Leu Thr Glu Asp Val Asp
        195                 200                 205

Gln Asp Val Tyr Val Met Asp Val Gly Asp Met Ala Gly Gln Pro Thr
    210                 215                 220

Leu Lys Lys Val Lys Gly Val Lys Asp Ala Thr Gly Ile Ala Leu Tyr
225                 230                 235                 240

Ser Ser Gly Asp Gln Glu Tyr Val Phe Val Ala Gln Glu Ser Gln Leu
                245                 250                 255

Ser Val Tyr Gly Pro Asp Trp Thr Leu Leu Gly Thr Val Gln Leu Ala
            260                 265                 270

Gly Leu Asp Asp Ile Glu Ile Gln Gly Leu Ser Ile Tyr Gln Ala Gln
        275                 280                 285

Thr Ser Thr Tyr Pro Ser Gly Ala Ile Thr Phe Ala Ile Glu Ala Asp
    290                 295                 300

Ala Thr Glu Gly Phe Gly Thr Thr Ser Leu Gln Pro Ile Leu Asp Ala
305                 310                 315                 320

Leu Ser Ile Pro Pro Asn Thr Ala Tyr Thr Pro Arg Ser Glu Pro Thr
                325                 330                 335

Gly Pro Arg Thr
            340

<210> 5
<211> 374
<212> PRT
<213> Magnaporthe grisea

<400> 5

Met Val Ala Ser Ser Ile Gly Val Leu Leu Ala Ala Val Leu Leu Gly
1               5                   10                  15

Gly Thr Ser Thr Ile Val Thr Ala Ser Phe Val Leu Val Glu Ala Asp
            20                  25                  30

Arg Glu Thr Asp Ala Ala Gly Gly Asp Gly Asp Asp Pro Ala Val Trp
        35                  40                  45

Ile Ser Pro Val Ser Lys Glu Leu Ser Arg Val Ile Thr Thr Val Lys
    50                  55                  60

Ser Glu Ala Gly Ala Gly Leu Asn Val Phe Asp Leu Glu Gly Lys Leu
65                  70                  75                  80

Val Gln Gln Phe Thr Ala Gly Gln Pro Asn Asn Val Asp Val Ile Tyr
                85                  90                  95

Gly Phe Lys Ala Gly Asp Arg Val Val Asp Leu Ala Phe Ala Gly Cys
            100                 105                 110

Arg Ser Asp Glu Thr Leu Cys Leu Phe Glu Ile Thr Ser Asn Gly Thr
        115                 120                 125

Ile Thr Thr Ile Pro Gly Gly Ile Gln Pro Leu Pro Lys Gly Tyr Lys
    130                 135                 140

Val Tyr Gly Ser Cys Val Tyr Arg Ser Pro Lys Ser Asn Arg Gln Tyr
145                 150                 155                 160

Leu Phe Val Asn Ser Lys Lys Ala Arg Tyr Leu Gln Tyr Glu Leu Thr
                165                 170                 175

Ser Thr Glu Ser Gly Thr Leu Gln Thr Thr Leu Val Arg Asp Phe Thr
            180                 185                 190

Gly Gly Ser Gly Gly Gln Val Glu Gly Cys Val Val Asp Asp Arg Asn
        195                 200                 205

Ser Trp Leu Phe Leu Gly Glu Glu Pro Arg Ala Leu Trp Arg Tyr Gly
    210                 215                 220

Ala Glu Pro Asp Asp Gly Thr Glu Gly Leu Glu Ile Ala Val Phe Gly
225                 230                 235                 240

Asp Gly Lys Leu Asn Ala Glu Val Glu Gly Val Thr Leu Val Glu Gly
                245                 250                 255

Lys Thr Pro Gln Glu Gly Tyr Ile Leu Val Ser Asn Gln Gly Val Ser
            260                 265                 270

Ser Tyr Ala Val Tyr Arg Arg Ala Glu Pro His Glu Tyr Val Ala Thr
        275                 280                 285

Phe Ser Ile Val Ala Ser Ala Asp Gly Lys Ile Asp Ala Val Ser Asn
    290                 295                 300

Thr Asp Gly Ile Thr Ala Val Ala Thr Gly Leu Gly Pro Asn Phe Pro
305                 310                 315                 320

His Gly Leu Val Val Val His Asp Asp Ala Asn Gln Leu Pro Glu Gly
                325                 330                 335

Gly Thr Ser Thr Glu Ala Ser Phe Lys Met Ile Ser Leu Glu Lys Ile
            340                 345                 350

Phe Gly Gly Pro Leu Lys Glu Leu Asn Leu Leu Asp His Val Asp Ala
        355                 360                 365

Asn Trp Asp Pro Arg Ala
    370

<210>  6
<211>  63
<212>  PRT
<213>  Magnaporthe grisea

<400>  6

Cys Ala Arg Cys Ser Asp Asn Gly Phe Cys Pro Thr Val Gly Cys Pro
1               5                   10                  15

Lys Thr Cys Thr Ser Cys Leu Leu Gly Phe Ser Gly Pro Thr Cys Ser
            20                  25                  30

Thr Pro Arg Cys Glu Ala Asp Cys Ser Gly Arg Gly Thr Cys Val Gly
        35                  40                  45

Pro Asn Lys Cys Ala Cys Asp Ala Gly Trp Gly Gly Leu His Cys
    50                  55                  60

<210>  7
<211>  539
<212>  PRT
<213>  Magnaporthe grisea

<400>  7

Met Ala Ala Val Val Gly Phe Ile Ala Val Cys Phe Leu Ala Trp Phe
1               5                   10                  15

Ala Thr Ser Lys Arg Leu Leu Phe Ala Thr Thr Ser Thr Gly Ser Arg
            20                  25                  30

Cys Pro Cys Glu Pro Asp Pro Asp Val Pro Gln Tyr Phe Arg Thr Ser
        35                  40                  45

Pro Glu Leu Trp Ala Gly Pro Thr Ala Thr Gly Lys Ala Pro Phe Leu
    50                  55                  60

Gln Gln Thr Val Thr Phe Asp Pro Thr Ala Thr Tyr Val Pro Asn Ala
65                  70                  75                  80

Pro Leu Gln Thr Ala Ile Pro Ile Gln Gly Met Gly Ser Gly Asn Lys
                85                  90                  95

Ser Ile Phe Gln Met Met Gly His Leu Ser Pro Tyr Ser Pro Ser Pro
            100                 105                 110

Gly Phe Gly Val Arg Glu Tyr Pro Leu Pro Ala Gly Ala Glu Ile Val
        115                 120                 125

Gln Val Gln Met Leu Ser Arg His Gly Ser Arg Tyr Pro Thr Ser Asn
    130                 135                 140

Ser Asp Val Ala Ala Leu Gly Lys His Ile Ser Asn Ala Lys Lys Asp
145                 150                 155                 160

Lys Ser Phe Lys Ala Ser Gly Pro Leu Ser Phe Leu Asn Asp Trp Glu
                165                 170                 175

Tyr Gln Leu Gly His Glu Ile Leu Val Pro Arg Gly Arg Gln Glu Leu
            180                 185                 190

Phe Asp Ser Gly Arg Arg Ile Pro Val Leu Ile His Cys Gly Lys Ile
        195                 200                 205

Ile Val Arg Thr Thr Thr Gln Asp Arg Met Leu Lys Ser Ala Glu Tyr
    210                 215                 220

Phe Met Ala Gly Phe Phe Gly Leu Glu Trp Pro Arg Asn Ala Thr Ile
225                 230                 235                 240

Glu Val Ile Ile Glu Glu Ala Gly Phe Asn Asn Ser Leu Ala Gly Tyr
                245                 250                 255

Leu Asn Cys Pro Ser Gly Ala Gly Gln His Val Gly Asp Asp Ala Arg
            260                 265                 270

Lys Ile Trp Val Ser Asn Tyr Leu Gln Asn Ala Thr Ala Arg Ile Gln
        275                 280                 285

Thr Met Ile Glu Gly Tyr His Trp Thr Ile Glu Asp Thr Tyr Ala Ala
    290                 295                 300

Gln Asn Met Cys Pro Tyr Glu Thr Val Ala Tyr Gly Phe Ser Arg Phe
305                 310                 315                 320

Cys Asp Leu Phe Asn Tyr Glu Glu Trp Ile Gly Phe Gly Tyr Ser Ile
                325                 330                 335

Asp Leu Trp Phe Ala Gly Ile Ser Gly Phe Gln Ser Pro Ile Gly Arg
            340                 345                 350

Ala Leu Gly Ile Gly Tyr Gln Gln Glu Val Val Ala Arg Leu Lys Asn
        355                 360                 365

His Thr Leu Gly Tyr Ser Gly Ser Gln Ile Asn Val Thr Leu Asp Asn
    370                 375                 380

Asn Thr Glu Thr Phe Pro Leu Asn Gln Thr Leu Tyr Phe Asp Phe Ser
385                 390                 395                 400

His Asp Thr Asn Ile Met Ala Ile Leu Thr Ala Phe Gly Leu Arg Gln
                405                 410                 415

Phe Ala His Leu Met Gln Pro Thr Thr His Pro Gly Glu His Asn Leu
            420                 425                 430

Thr Val Ala Asn Leu Thr Pro Phe Gly Ala Arg Leu Asp Ile Glu Ile
        435                 440                 445

Ile Lys Thr Pro Lys Pro Val Lys Pro Asp Arg Ser Gly Tyr Met Val
    450                 455                 460

Asn Gly Gly Glu Thr Lys Tyr Val His Phe Val Leu Asn Gln Arg Thr
465                 470                 475                 480

Leu Pro Leu Gly Trp Ser Leu Pro Glu Cys Asp Val Ser Arg Val Asp
                485                 490                 495

Gly Trp Cys Glu Leu Gly Ala Phe Leu Ser Ala Gln Asp Lys Met Pro
            500                 505                 510

Ala Arg Ala Arg Phe Asp Tyr Ala Cys Phe Gly Ser Tyr Asp Pro Gly
        515                 520                 525

Pro Tyr Gly Ser Val Met Asp Gly Ala Pro Pro
    530                 535

<210>  8
<211>  459
<212>  PRT
<213>  Magnaporthe grisea

<400>  8

Met Leu Pro Ile Phe Ile Ile Leu Phe Gly Leu Val Met Pro Ala Ile
1               5                   10                  15

Ser Thr Asn Asn Tyr Phe Asp Leu Asn Lys Tyr Asp Thr Pro Asp Leu
            20                  25                  30

Gly Phe Leu Tyr Arg Pro Glu Ile Thr Tyr Lys Trp Gly His Phe Thr
        35                  40                  45

Pro Phe His Ser Thr Ser Ser Glu Phe Asn Pro Ser Pro Ser Leu Pro
    50                  55                  60

Gly Cys Thr Val Thr Phe Val Gln Ser Leu Met Arg His Gly Ala Arg
65                  70                  75                  80

Tyr Pro Lys Thr Gly Ser Pro Tyr Arg Asp Ile Ile Gln Arg Ile Gln
                85                  90                  95

Ser Ser Val Asp Lys Tyr Ser Ala Lys Tyr Glu Phe Ile Arg Asn Tyr
            100                 105                 110

Glu Tyr Asn Leu Pro Thr Glu Asp Leu Asn Asp Leu Gly Arg His Gln
        115                 120                 125

Leu Ile Asn Ala Gly Ile His Phe Tyr Arg Arg Tyr Gln Asn Leu Ala
    130                 135                 140

Lys Asp Asn Glu Pro Phe Ile Arg Ser Asp Gly Ala Glu Arg Val Ala
145                 150                 155                 160

Glu Ser Ala Glu Lys Trp Ala Asp Gly Phe His Gln Thr Ser Leu Thr
                165                 170                 175

Asp Lys Thr Arg Ile Gln Pro His Gln Phe Pro Val Ser Ile Phe Lys
            180                 185                 190

Phe Pro Val Gly Lys Gln Tyr Asn Asn Thr Leu Asn Asn Leu Ala Cys
        195                 200                 205

Thr Lys Tyr Asp Lys Ser Lys Ala Gln Lys Ile Ala Lys Glu Leu Pro
    210                 215                 220

Lys Arg Ile Leu Thr Glu Thr Ala Lys Gln Leu Asn Gln Asn Leu Pro
225                 230                 235                 240

Gly Ala Asn Leu Ser Val Tyr Glu Ile Leu Gln Leu Met Glu Leu Cys
                245                 250                 255

Gly Met Glu Thr Thr Ala Asn Ile Val Lys Thr Gly Ile Leu Ser Pro
            260                 265                 270

Ile Cys Asn Ile Phe Asp Asn Lys Asp Trp Glu Ala Phe Gln Arg Phe
        275                 280                 285

His His Glu Val Lys Trp Tyr Thr His Phe Tyr Gly Asn Pro Leu Gly
    290                 295                 300

Pro Ser Leu Gly Ile Gly Trp Val Asn Glu Leu Ile Ala Arg Leu Leu
305                 310                 315                 320

Arg Arg Pro Val Gln Asp Ser Thr Ser Thr Asn Thr Thr Ile Thr Ala
                325                 330                 335

Asn Pro Lys Thr Phe Pro Leu Asp Lys Lys Leu Tyr Ala Asp Phe Thr
            340                 345                 350

His Ala Thr Asp Phe Leu Ala Ile Gln Ala Ala Leu Gly Leu Leu Gly
        355                 360                 365

Ser Ala His Thr Pro Val Pro Ala Asn Arg Ile Val Ser Asp Gln Arg
    370                 375                 380

Ile Ala Glu Phe Ser Ala Ser Glu Val Ser Pro Phe Ala Ala Arg Met
385                 390                 395                 400

Tyr Val Glu Lys Met Thr Cys Ser Gly His Thr Glu Glu Leu Val Arg
                405                 410                 415

Ile Leu Val Asn Asn Arg Val Val Trp Leu Pro Asn Cys Gly Val Asp
            420                 425                 430

Gly Gln Gly Arg Cys Lys Leu Gly Ala Phe Val Glu Ser Leu Ser Phe
        435                 440                 445

Ala Arg Ser Gly Gly Phe Trp Asn Lys Cys Phe
    450                 455

<210>  9
<211>  6054
<212>  DNA
<213>  Artificial

<220>
<223>  plug-in expression cassette

<400>  9
ctcgaggcgg ccgctcagta gtgtatcgcc ccgcataaca gtgctctcac ggccattcat      60

gccatgtgaa caaccaggcc gtccggctgt tcgttccggc gcgcccgcaa gaccccgttg     120

aagggacttc tcggaatggg gtgattatcg ctgagccggg cccacttgat ccactgccga     180

cgctgctgca ttcactttgg tgcatacgag acagctccaa gccgcttgga aaatggggtg     240

aagggtaact atcttaatgg agaacggtaa tcgcatactt gcttgccctc gtcgaggccc     300

ggggctggga gaggagggga tgaaacaaaa gtcaatgccg ctgccgagag accgacagcg     360

cgatagacta cctgatgaag catggtggtt gttgacacaa ggatagaacg gagggaggta     420

tcacagtcta acgcagatag tatagtgtac tccgtaattc atgtaaggat tggtaactgc     480

atcagaccac gacgggaccc catcatcgtg gcggacctca cgggcagaag ctggaggcgc     540

ccgggagaga gtggatcgag ttgcatgcgc aagcgccctt catctcttca gccgagaatc     600

tgttgcctaa ttacatagta gatgctggga acagtctgag aagcctgcgc aggttcttca 660
ttaattacca agaacgcggg agaggtggga agacgccgaa aactcgacgc ggaccattcg 720
ctgttttccg atcttacaaa aaaaaggtat ccgatttggg gaacgtcgat gaaagtattg 780
caaaagtgac gagagttgcg caactaactc gctgccgaag aagctgcgga agaaagagaa 840
ggcctaaagt ggaataacgt tacggatgtc ctgacctcaa agttgaaacc agcccttcct 900
gctctatttg ggaaagcggc ttgcccttga atgcgctgca ctgtggcacg actaccagtg 960
atcgggagga gcaaactacc ctggtccgtt ccttggtggg gcggcactag gcccaactta 1020
gggtgatcgg aggtcgatgc cgcggtcctc gttggtctgg gctcttctca tttcccggtt 1080
tgcacccccc gttgcacctg ctgatcgccc gccaacgccg atgaggttgc gcccagaccg 1140
acaatcaccg cggctgcatt cccaagtata ttgaagatgg caccaggtac ccggttttgc 1200
gtcccagtcg tttggtgcca aatttgggag tttttgagcc tcaagatctg gggaaatcga 1260
cctcaacttc catacaagtt aaagtcgcac acacggcgag ttccacgaag agacacattt 1320
ttttctgacg gccgaatatt ttgaatagct cgcccgctgg agagcatcct gaatgcaagt 1380
aacaaccgta gaggctgaca cggcaggtgt tgctagggag cgtcgtgttc tacaaggcca 1440
gacgtcttcg cggttgatat atatgtatgt ttgactgcag gctgctcagc gacgacagtc 1500
aagttcgccc tcgctgcttg tgcaataatc gcagtgggga agccacaccg tgactcccat 1560
ctttcagtaa agctctgttg gtgtttatca gcaatacacg taatttaaac tcgttagcat 1620
ggggctgata gcttaattac cgtttaccag tgccgcggtt ctgcagcttt ccttggcccg 1680
taaaattcgg cgaagccagc caatcaccag ctaggcacca gctaaaccct ataattagtc 1740
tcttatcaac accatccgct cccccgggat caatgaggag aatgaggggg atgcggggct 1800
aaagaagcct acataaccct catgccaact cccagtttac actcgtcgag ccaacatcct 1860
gactataagc taacacagaa tgcctcaatc ctgggaagaa ctggccgctg ataagcgggc 1920
ccgcctcgca aaaaccatcc ctgatgaatg gaaagtccag acgctgcctg cggaagacag 1980
cgttattgat ttcccaaaga aatcgggcat cctttcagag gccgaactga agatcacaga 2040
ggcgtccgcc gcagaccttg tgtccaagct ggccgccgga gagttgacct cggtggaagt 2100
tacgctagca ttctgtaaac gggcagcaat cgcccagcag ttgacaaact gcgcccacga 2160
gttcttccct gacgccgctc tcgcgcaggc aagggaactc gatgaatact acgcaaagca 2220
caagagaccc gttggtccac tgcatggcct ccccatctct ctcaaagacc agcttcgagt 2280
caagggctac gaaacatcaa tgggctacat ctcatggcta aacaagtacg acgaagggga 2340
ctcggttctg acaaccatgc tccgcaaagc cggtgccgtc ttctacgtca agacctctgt 2400
cccgcagacc ctgatggtct gcgagacagt caacaacatc atcgggcgca ccgtcaaccc 2460
acgcaacaag aactggtcgt gcggcggcag ttctggtggt gagggtgcca tcgttgggat 2520
tcgtggtggc gtcatcggtg taggaacgga catcggtggc tcgattcgag tgccggcggc 2580
gttcaacttc ctgtacggtc taaggccgag tcatgggcgg ctgccgtatg caaagatggc 2640

gaacagcatg gagggtcagg agacggtgca cagcgttgtc gggccgatta cgcactctgt 2700
tgaggacctc cgcctcttca ccaaatccgt cctcggtcag gagccctgga aatacgactc 2760
caaggtcatc cccatgccct ggcgccagtc cgagtcggac attattgcct ccaagatcaa 2820
gaacggcggg ctcaatatcg gctactacaa cttcgacggc aatgtccttc cacaccctcc 2880
tatcctgcgc ggcgtggaaa ccaccgtcgc cgcactcgcc aaagccggtc acaccgtgac 2940
cccgtggacg ccatacaagc acgatttcgg ccacgatctc atctcccata tctacgcggc 3000
tgacggcagc gccgacgtaa tgcgcgacat cagtgcatcc ggcgagccgg cgattccaaa 3060
tatcaaagac ctactgaacc cgaacatcaa agctgtgaac atgaacgagc tgtgggacac 3120
gcatctccag aagtggaatt accagatgga gtaccttgag aaatggcggg aggctgaaga 3180
aaaggccggg aaggaactgg acgccatcat cgcgccgatt acgcctaccg ctgcggtacg 3240
gcatgaccag ttccggtact atgggtatgc ctctgtcatc aacctgctgg atttcacgag 3300
cgtggttgtt cccgttacct ttgcggataa gaacatcgac aagaagaatg agagtttcaa 3360
ggcggttagt gagcttgatg ccctcgtgca ggaagagtat gatccggagg cgtaccacgg 3420
ggcaccggtt gcagtgcagg ttatcggacg gagactcagt gaagagagga cgttggcgat 3480
tgcagaggaa gtggggaagt tgctgggaaa tgtggtgact ccatagctaa taagtgtcag 3540
atagcaattt gcacaagaaa tcaataccag caactgtaaa taagcgctga agtgaccatg 3600
ccatgctacg aaagagcaga aaaaaacctg ccgtagaacc gaagagatat gacacgcttc 3660
catctctcaa aggaagaatc ccttcagggt tgcgtttcca gtctagacac gtataacggc 3720
acaagtgtct ctcaccaaat gggttatatc tcaaatgtga tctaaggatg gaaagcccag 3780
aatattgcgg ccgaagagaa caccgaaagt ggaataacgt tacggatgtc ctgacctcaa 3840
agttgaaacc agcccttcct gctctatttg ggaaagcggc ttgcccttga atgcgctgca 3900
ctgtggcacg actaccagtg atcgggagga gcaaactacc ctggtccgtt ccttggtggg 3960
gcggcactag gcccaactta gggtgatcgg aggtcgatgc cgcggtcctc gttggtctgg 4020
gctcttctca tttcccggtt tgcacccccc gttgcacctg ctgatcgccc gccaacgccg 4080
atgaggttgc gcccagaccg acaatcaccg cggctgcatt cccaagtata ttgaagatgg 4140
caccaggtac ccggttttgc gtcccagtcg tttggtgcca aatttgggag tttttgagcc 4200
tcaagatctg gggaaatcga cctcaacttc catacaagtt aaagtcgcac acacggcgag 4260
ttccacgaag agacacattt ttttctgaag gcctctctcc ccgcacatca gaaaccacca 4320
aataccaaga ctgcagaagc cggggtaagt gggccaccgg gactacacta aaatgcgggg 4380
agaagcgaga tccgttgcga agggaaggga tggggtgtgc tgcggctttc tccgctctcg 4440
tgcgcctttt gcttgaatct agtgtacacc agggtaggct ccgaaggagt atctacggca 4500
gcgctgttcg tgctgcgttg agagtcaggg cggagacgag caggcgacag gagcctcgca 4560
ccggcacttc ggatcgcatt tgcgcggagc gtcaaatacg ctcttctgcg gtcatcagag 4620
agcatcgtga accaaggttc ttccgcaggg cggcctgggc ttcgcagagt cgcactcggc 4680
ggacgccttc cgtgtcaccc ctgataacct ggctgccgcg cccagactcc tccaatgagg 4740

tgtgtggttg ccctcgccga cccttcagca accttaatcg cttccatcgc acggctccac 4800
gtcctcgaac gatgccctca gtccgtgccc ggccgtggca accataacgt gacatcgccg 4860
cccagcctac tagccgctat cgaccggtta ggcttgtcac cgcagcgccc attctccatc 4920
gggcctctac tctgatccac ctcacccacc gcaagcacta gcgagcctca ccagagtgca 4980
agcgacacga cccgcttggc ccttcgtcct tgactatctc ccagacctct tgccatcttg 5040
ccgacgccgc cccctttttt ttctcctccc cctgccggca ggtcggtggc cccagtcccg 5100
agatggcatt gctccgttgt ccatgacgac ccatcattcg atggctgact ggcacactcg 5160
tcttgtttga gcatcgacgg cccgcggccc gtctcccacg gtacggaacc tcgttgtaca 5220
gtacctctcg taatgatacc caacaccggg gccgagcgct gggagggcgg cgttcccgag 5280
aagccgggaa ggcggctggc cggctgacct ttgtgacttg gcgatggatg cggccatggt 5340
gcatgcttcg aattcgatat cggatcctaa gtaagtaaac gaacctctct gaaggaggtt 5400
ctgagacacg cgcgattctt ctgtatatag ttttattttt cactctggag tgcttcgctc 5460
caccagtaca taaacctttt ttttcacgta acaaaatggc ttcttttcag accatgtgaa 5520
ccatcttgat gccttgacct cttcagttct cactttaacg tagttcgcgt tagtctgtat 5580
gtcccagttg catgtagttg agataaatac ccctggaagt gggtctgggc ctttgtggga 5640
cggagccctc tttctgtggt ctggagagcc cgctctctac cgcctacctt cttaccacag 5700
tacactactc acacattgct gaactgaccc atcataccgt actttatcct gttaattcgt 5760
ggtgctgtcg actattctat ttgctcaaat ggagagcaca ttcatcggcg cagggataca 5820
cggtttatgg accccaagag tgtaaggact attattagta atattatatg cctctaggcg 5880
ccttaacttc aacaggcgag cactactaat caacttttgg tagacccaat tacaaacgac 5940
catacgtgcc ggaaattttg ggattccgtc cgctctcccc aaccaagcta gaagaggcaa 6000
cgaacagcca atcccggtgc taattaaatt atatggttca gcggccgcga gctc 6054

<210> 10
<211> 2429
<212> DNA
<213> Artificial

<220>
<223> synthetic expression cassette for phy7789ab

<400> 10
ggtaccccat ggagaatgtc cgtccgaagc gacgcgacaa ttagcctggc taccatcgat 60
ataaattggg tgattcccag ctcttgatgg gcgtgtcttc tgcctggcag ccctcgtctt 120
cagatcaagc aactgtgtgc tgatcctctt ccgtcatggt cgccagcagc atcggcgtcc 180
tcctcgccgc cgtcctcctc ggcggcacca gcaccatcgt caccgccgcc ccggtcgagc 240
tggagctgaa cgtcaccgcc ctcacccccg gcatcatcga cagcgactgg accgccgcct 300
actaccccaa gagcggcgcc gcccccctcc tcctcgccaa cgacgccggc accgccacgg 360
gcggcttccg cgtgttcggc ctggacggcg cccccaccct cagccaggcc ggccacgtca 420

tcgccggtcg caccaagctc gtcgcccccc tctacaacgc ctccggcgcc ggcgacgact 480
tcctcgtcac catcgccacc accgacagcg tcgtccgcct ctacagcctc cccgacctcg 540
ccgtcgtccc catcgccgag cccctcgtca ccctcggcga ctggaacgcc gtctgcagct 600
ggggcagcta cttcttcctc ttcggcaagg gcggtcgggc cttccagtac ctcgtccgcc 660
ccgccgacgg cggcagcttc gaggtcctcg aggtccgcgc cctccccgcc cccgtcgtgt 720
tcgtcggctg cgccgtcagc ccccgcgacg ccaagctcta cctcaccgag gacgtcgacc 780
aggacgtcta cgtcatggac gtcggcgaca tggccggcca gccgaccctc aagaaggtca 840
agggcgtcaa ggacgccacc ggcatcgccc tctacagcag cggcgaccag gagtacgtgt 900
tcgtcgccca ggagtcccag ctcagcgtct acggccccga ctggaccctc ctcggcaccg 960
tccagctcgc cggcctggac gacatcgaga tccagggcct cagcatctac caggcccaga 1020
ccagcaccta ccccagcggc gccatcacct tcgccatcga ggccgacgcc acggagggct 1080
tcggcaccac ctccctccag cccatcctcg acgccctcag catccccccc aacaccgcct 1140
acaccccccg ctccgagccc accggccccc gcacctgcgc ccgctgcagc gacaacggct 1200
tctgccccac cgtcggctgc cccaagacct gcaccagctg cctcctcggc ttcagcggcc 1260
ccacctgcag caccccccgc tgcgaggccg actgctccgg tcgcggcacc tgcgtcggcc 1320
ccaacaagtg cgcctgcgac gccggctggg gcggcctcca ctgcagcttc gtcctggtgg 1380
aggccgaccg cgagaccgac gccgccggtg gtgacggtga cgaccccgcc gtctggatca 1440
gccccgtcag caaggagctg agccgcgtca tcaccaccgt caagagcgag gccggtgccg 1500
gcctcaacgt gttcgacctc gagggcaagc tcgtccagca gttcaccgcc ggccagccca 1560
acaacgtcga cgtcatctac ggcttcaagg ccggcgaccg cgtcgtcgac ctcgccttcg 1620
ccggctgccg cagcgacgag accctctgcc tcttcgagat caccagcaac ggcaccatca 1680
ccaccatccc cggcggcatc cagcccctcc ccaagggcta caaggtctac ggcagctgcg 1740
tctaccgcag ccccaagagc aaccgccagt acctcttcgt caacagcaag aaggcccgct 1800
acctccagta cgagctgacc agcaccgaga gcggcaccct ccagaccacc ctcgtccgcg 1860
acttcaccgg cggcagcggc ggccaggtcg agggctgcgt cgtggacgac cgcaactcct 1920
ggctcttcct cggcgaggag ccccgcgccc tctggcgcta cggcgccgag ccggacgacg 1980
gcaccgaggg cctcgagatc gccgtgttcg gcgacggcaa gctgaacgcc gaggtcgagg 2040
gcgtcaccct cgtcgagggc aagacccccc aggagggcta catcctcgtc agcaaccagg 2100
gcgtcagcag ctacgccgtc taccgccgcg ccgagcccca cgagtacgtc gccaccttca 2160
gcatcgtcgc cagcgccgac ggcaagatcg acgccgtcag caacaccgac ggcatcaccg 2220
ccgtggccac gggcctcggc cccaacttcc cccacggcct cgtcgtcgtc catgacgacg 2280
ccaaccagct ccccgagggc ggcacctcca ccgaggccag cttcaagatg atcagcctcg 2340
agaagatctt cggcggcccc ctcaaggagc tgaacctcct cgaccacgtc gacgccaact 2400
gggacccccg cgcctaagat atcgagctc 2429

<210>  11
<211>  1190
<212>  DNA
<213>  Artificial

<220>
<223>  synthetic expression cassette for phy7789a

<400>  11
ggtaccccat ggagaatgtc cgtccgaagc gacgcgacaa ttagcctggc taccatcgat      60

ataaattggg tgattcccag ctcttgatgg gcgtgtcttc tgcctggcag ccctcgtctt     120

cagatcaagc aactgtgtgc tgatcctctt ccgtcatggt cgccagcagc atcggcgtcc     180

tcctcgccgc cgtcctcctc ggcggcacca gcaccatcgt caccgccgcc ccggtcgagc     240

tggagctgaa cgtcaccgcc ctcacccccg gcatcatcga cagcgactgg accgccgcct     300

actaccccaa gagcggcgcc gcccccctcc tcctcgccaa cgacgccggc accgccacgg     360

gcggcttccg cgtgttcggc ctggacggcg cccccaccct cagccaggcc ggccacgtca     420

tcgccggtcg caccaagctc gtcgcccccc tctacaacgc ctccggcgcc ggcgacgact     480

tcctcgtcac catcgccacc accgacagcg tcgtccgcct ctacagcctc cccgacctcg     540

ccgtcgtccc catcgccgag cccctcgtca ccctcggcga ctggaacgcc gtctgcagct     600

ggggcagcta cttcttcctc ttcggcaagg gcggtcgggc cttccagtac ctcgtccgcc     660

ccgccgacgg cggcagcttc gaggtcctcg aggtccgcgc cctccccgcc cccgtcgtgt     720

tcgtcggctg cgccgtcagc ccccgcgacg ccaagctcta cctcaccgag gacgtcgacc     780

aggacgtcta cgtcatggac gtcggcgaca tggccggcca gccgaccctc aagaaggtca     840

agggcgtcaa ggacgccacc ggcatcgccc tctacagcag cggcgaccag gagtacgtgt     900

tcgtcgccca ggagtcccag ctcagcgtct acggccccga ctggaccctc ctcggcaccg     960

tccagctcgc cggcctggac gacatcgaga tccagggcct cagcatctac caggcccaga    1020

ccagcaccta ccccagcggc gccatcacct tcgccatcga ggccgacgcc acggagggct    1080

tcggcaccac ctccctccag cccatcctcg acgccctcag catccccccc aacaccgcct    1140

acaccccccg ctccgagccc accggccccc gcacctaaga tatcgagctc               1190

<210>  12
<211>  1292
<212>  DNA
<213>  Artificial

<220>
<223>  synthetic expression cassette for phy7789b

<400>  12
ggtaccccat ggagaatgtc cgtccgaagc gacgcgacaa ttagcctggc taccatcgat      60

ataaattggg tgattcccag ctcttgatgg gcgtgtcttc tgcctggcag ccctcgtctt     120

cagatcaagc aactgtgtgc tgatcctctt ccgtcatggt cgccagcagc atcggcgtcc     180

tcctcgccgc cgtcctcctc ggcggcacca gcaccatcgt caccgccagc ttcgtcctgg     240

tggaggccga ccgcgagacc gacgccgccg gtggtgacgg tgacgacccc gccgtctgga     300

tcagccccgt cagcaaggag ctgagccgcg tcatcaccac cgtcaagagc gaggccggtg 360

ccggcctcaa cgtgttcgac ctcgagggca agctcgtcca gcagttcacc gccggccagc 420

ccaacaacgt cgacgtcatc tacggcttca aggccggcga ccgcgtcgtc gacctcgcct 480

tcgccggctg ccgcagcgac gagaccctct gcctcttcga gatcaccagc aacggcacca 540

tcaccaccat ccccggcggc atccagcccc tccccaaggg ctacaaggtc tacggcagct 600

gcgtctaccg cagccccaag agcaaccgcc agtacctctt cgtcaacagc aagaaggccc 660

gctacctcca gtacgagctg accagcaccg agagcggcac cctccagacc accctcgtcc 720

gcgacttcac cggcggcagc ggcggccagg tcgagggctg cgtcgtggac gaccgcaact 780

cctggctctt cctcggcgag gagccccgcg ccctctggcg ctacggcgcc gagccggacg 840

acggcaccga gggcctcgag atcgccgtgt tcggcgacgg caagctgaac gccgaggtcg 900

agggcgtcac cctcgtcgag ggcaagaccc cccaggaggg ctacatcctc gtcagcaacc 960

agggcgtcag cagctacgcc gtctaccgcc gcgccgagcc ccacgagtac gtcgccacct 1020

tcagcatcgt cgccagcgcc gacggcaaga tcgacgccgt cagcaacacc gacggcatca 1080

ccgccgtggc cacgggcctc ggccccaact tcccccacgg cctcgtcgtc gtccatgacg 1140

acgccaacca gctccccgag ggcggcacct ccaccgaggc cagcttcaag atgatcagcc 1200

tcgagaagat cttcggcggc cccctcaagg agctgaacct cctcgaccac gtcgacgcca 1260

actgggaccc ccgcgcctaa gatatcgagc tc 1292

<210> 13
<211> 1787
<212> DNA
<213> Artificial

<220>
<223> synthetic expression cassette for phy538

<400> 13
ggtaccccat ggagaatgtc cgtccgaagc gacgcgacaa ttagcctggc taccatcgat 60

ataaattggg tgattcccag ctcttgatgg gcgtgtcttc tgcctggcag ccctcgtctt 120

cagatcaagc aactgtgtgc tgatcctctt ccgtcatggc cgccgtcgtc ggcttcatcg 180

ccgtctgctt cctcgcctgg ttcgccacct ccaagcgcct cctcttcgcc accacctcca 240

ccggcagccg ctgcccctgc gagcccgacc ccgacgtccc ccagtacttc cgcaccagcc 300

ccgagctgtg ggccggcccg accgccaccg gcaaggcccc cttcctccag cagaccgtca 360

ccttcgaccc caccgccacc tacgtcccca acgcccccct ccagaccgcc atccccatcc 420

agggcatggg cagcggcaac aagagcatct tccagatgat gggccacctc agcccctaca 480

gccccagccc cggcttcggc gtccgcgagt accccctccc cgccggcgcc gagatcgtcc 540

aggtccagat gctcagccgc cacggctccc gctaccccac ctccaacagc gacgtggccg 600

ccctcggcaa gcacatcagc aacgccaaga aggacaagag cttcaaggcc agcggccccc 660

tcagcttcct caacgactgg gagtaccagc tcggccacga gatcctcgtc cccccgggcc 720

gccaggagct gttcgactcg ggccgccgca tccccgtcct catccactgc ggcaagatca 780

tcgtccgcac caccacccag gaccgcatgc tcaagagcgc cgagtacttc atggccggct 840

tcttcggcct cgagtggccc cgcaacgcca ccatcgaggt catcatcgag gaggccggct 900

tcaacaacag cctcgccggc tacctcaact gccccagcgg cgccggccag cacgtcggcg 960

acgacgcccg caagatctgg gtctccaact acctccagaa cgccaccgcc cgcatccaga 1020

ccatgatcga gggctaccac tggaccatcg aggacaccta cgccgcccag aacatgtgcc 1080

cctacgagac cgtcgcctac ggcttcagcc gcttctgcga cctcttcaac tacgaggagt 1140

ggatcggctt cggctacagc atcgacctct ggttcgccgg catcagcggc ttccagagcc 1200

ccatcggtcg cgccctcggc atcggctacc agcaggaggt cgtcgcccgc ctcaagaacc 1260

acaccctcgg ctacagcggc agccagatca acgtcaccct cgacaacaac accgagacct 1320

tccccctcaa ccagaccctc tacttcgact tcagccacga caccaacatc atggccatcc 1380

tgaccgcctt cggcctccgc cagttcgccc acctcatgca gcccaccacc caccccggcg 1440

agcacaacct caccgtcgcc aacctcaccc ccttcggcgc ccgcctcgac atcgagatca 1500

tcaagacccc caagcccgtc aagcccgacc gcagcggcta catggtcaac ggcggcgaga 1560

ccaagtacgt ccacttcgtc ctcaaccagc gcaccctccc cctcggctgg tccctccccg 1620

agtgcgacgt cagccgcgtc gacggctggt gcgagctggg cgccttcctc agcgcccagg 1680

acaagatgcc cgcccgcgcc cgcttcgact acgcctgctt cggcagctac gaccccggcc 1740

cctacggcag cgtcatggac ggcgcccccc cctaagatat cgagctc 1787

<210> 14
<211> 1547
<212> DNA
<213> Artificial

<220>
<223> synthetic expression cassette for phy1900

<400> 14
ggtaccccat ggagaatgtc cgtccgaagc gacgcgacaa ttagcctggc taccatcgat 60

ataaattggg tgattcccag ctcttgatgg gcgtgtcttc tgcctggcag ccctcgtctt 120

cagatcaagc aactgtgtgc tgatcctctt ccgtcatgct ccccatcttc atcatcctct 180

tcggcctcgt catgcccgcc atcagcacca acaactactt cgacctcaac aagtacgaca 240

cccccgacct cggcttcctc taccgccccg agatcaccta caagtggggc cacttcaccc 300

ccttccacag caccagcagc gagttcaacc ccagccccag cctccccggc tgcaccgtca 360

ccttcgtcca gagcctcatg cgccacggcg cccgctaccc caagaccggc agcccctacc 420

gcgacatcat ccagcgcatc cagagcagcg tcgacaagta cagcgccaag tacgagttca 480

tccgcaacta cgagtacaac ctccccaccg aggacctgaa cgacctgggc cggcaccagc 540

tcatcaacgc cggcatccac ttctaccgcc gctaccagaa cctcgccaag gacaacgagc 600

ccttcatccg cagcgacggc gccgagcgcg tcgccgagag cgccgagaag tgggccgacg 660

gcttccacca gaccagcctc accgacaaga cgcgcatcca gccccaccag ttccccgtca 720

gcatcttcaa gttccccgtc ggcaagcagt acaacaacac cctcaacaac ctcgcctgca 780

ccaagtacga caagagcaag gcccagaaga tcgccaagga gctgcccaag cgcatcctca 840

ccgagaccgc caagcagctc aaccagaacc tccccggcgc caacctcagc gtctacgaga 900

tcctccagct catggagctg tgcggcatgg agaccaccgc caacatcgtc aagaccggca 960

tcctcagccc catctgcaac atcttcgaca acaaggactg ggaggccttc cagcgcttcc 1020

accacgaggt caagtggtac acccacttct acggcaaccc cctcggcccc agcctcggca 1080

tcggctgggt caacgagctg atcgcccgcc tcctccgccg ccccgtccag gacagcacct 1140

ccaccaacac caccatcacc gccaacccca agaccttccc cctcgacaag aagctctacg 1200

ccgacttcac ccacgccacc gacttcctcg ccatccaggc cgccctcggc ctcctcggca 1260

gcgcccacac ccccgtcccc gccaaccgca tcgtcagcga ccagcgcatc gccgagttca 1320

gcgccagcga ggtctccccc ttcgccgccc gcatgtacgt cgagaagatg acctgcagcg 1380

gccacaccga ggagctggtc cgcatcctcg tcaacaaccg cgtcgtctgg ctccccaact 1440

gcggcgtcga cggccagggc cgctgcaagc tcggcgcctt cgtcgagtcc ctcagcttcg 1500

cccgcagcgg cggcttctgg aacaagtgct tctaagatat cgagctc 1547

<210> 15
<211> 1265
<212> PRT
<213> Artificial

<220>
<223> glucoamylase phytase fusion protein

<400> 15

Met Ser Phe Arg Ser Leu Leu Ala Leu Ser Gly Leu Val Cys Thr Gly
1               5                   10                  15

Leu Ala Asn Val Ile Ser Lys Arg Ala Thr Leu Asp Ser Trp Leu Ser
            20                  25                  30

Asn Glu Ala Thr Val Ala Arg Thr Ala Ile Leu Asn Asn Ile Gly Ala
        35                  40                  45

Asp Gly Ala Trp Val Ser Gly Ala Asp Ser Gly Ile Val Val Ala Ser
    50                  55                  60

Pro Ser Thr Asp Asn Pro Asp Tyr Phe Tyr Thr Trp Thr Arg Asp Ser
65                  70                  75                  80

Gly Leu Val Leu Lys Thr Leu Val Asp Leu Phe Arg Asn Gly Asp Thr
                85                  90                  95

Ser Leu Leu Ser Thr Ile Glu Asn Tyr Ile Ser Ala Gln Ala Ile Val
            100                 105                 110

Gln Gly Ile Ser Asn Pro Ser Gly Asp Leu Ser Ser Gly Ala Gly Leu
        115                 120                 125

Gly Glu Pro Lys Phe Asn Val Asp Glu Thr Ala Tyr Thr Gly Ser Trp
    130                 135                 140

Gly Arg Pro Gln Arg Asp Gly Pro Ala Leu Arg Ala Thr Ala Met Ile
145                 150                 155                 160

Gly Phe Arg Gln Trp Leu Leu Asp Asn Gly Tyr Thr Ser Thr Ala Thr
                165                 170                 175

Asp Ile Val Trp Pro Leu Val Arg Asn Asp Leu Ser Tyr Val Ala Gln
            180                 185                 190

Tyr Trp Asn Gln Thr Gly Tyr Asp Leu Trp Glu Glu Val Asn Gly Ser
        195                 200                 205

Ser Phe Phe Thr Ile Ala Val Gln His Arg Ala Leu Val Glu Gly Ser
    210                 215                 220

Ala Phe Ala Thr Ala Val Gly Ser Ser Cys Ser Trp Cys Asp Ser Gln
225                 230                 235                 240

Ala Pro Glu Ile Leu Cys Tyr Leu Gln Ser Phe Trp Thr Gly Ser Phe
                245                 250                 255

Ile Leu Ala Asn Phe Asp Ser Ser Arg Ser Gly Lys Asp Ala Asn Thr
            260                 265                 270

Leu Leu Gly Ser Ile His Thr Phe Asp Pro Glu Ala Ala Cys Asp Asp
        275                 280                 285

Ser Thr Phe Gln Pro Cys Ser Pro Arg Ala Leu Ala Asn His Lys Glu
    290                 295                 300

Val Val Asp Ser Phe Arg Ser Ile Tyr Thr Leu Asn Asp Gly Leu Ser
305                 310                 315                 320

Asp Ser Glu Ala Val Ala Val Gly Arg Tyr Pro Glu Asp Thr Tyr Tyr
                325                 330                 335

Asn Gly Asn Pro Trp Phe Leu Cys Thr Leu Ala Ala Ala Glu Gln Leu
            340                 345                 350

Tyr Asp Ala Leu Tyr Gln Trp Asp Lys Gln Gly Ser Leu Glu Val Thr
        355                 360                 365

Asp Val Ser Leu Asp Phe Phe Lys Ala Leu Tyr Ser Asp Ala Ala Thr
    370                 375                 380

Gly Thr Tyr Ser Ser Ser Ser Ser Thr Tyr Ser Ser Ile Val Asp Ala
385                 390                 395                 400

Val Lys Thr Phe Ala Asp Gly Phe Val Ser Ile Val Glu Thr His Ala
                405                 410                 415

Ala Ser Asn Gly Ser Met Ser Glu Gln Tyr Asp Lys Ser Asp Gly Glu
            420                 425                 430

Gln Leu Ser Ala Arg Asp Leu Thr Trp Ser Tyr Ala Ala Leu Leu Thr
        435                 440                 445

Ala Asn Asn Arg Arg Asn Ser Val Val Pro Ala Ser Trp Gly Glu Thr
    450                 455                 460

Ser Ala Ser Ser Val Pro Gly Thr Cys Ala Ala Thr Ser Ala Ile Gly
465                 470                 475                 480

Thr Tyr Ser Ser Val Thr Val Thr Ser Trp Pro Ser Ile Val Ala Thr
                485                 490                 495

Gly Gly Thr Thr Thr Thr Ala Thr Pro Thr Gly Ser Gly Ser Val Thr
            500                 505                 510

Ser Thr Ser Lys Thr Thr Ala Thr Ala Ser Lys Thr Ser Thr Ser Thr
        515                 520                 525

Ser Ser Asn Val Ile Ser Lys Arg Ala Pro Val Glu Leu Glu Leu Asn
    530                 535                 540

Val Thr Ala Leu Thr Pro Gly Ile Ile Asp Ser Asp Trp Thr Ala Ala
545                 550                 555                 560

Tyr Tyr Pro Lys Ser Gly Ala Ala Pro Leu Leu Leu Ala Asn Asp Ala
                565                 570                 575

Gly Thr Ala Thr Gly Gly Phe Arg Val Phe Gly Leu Asp Gly Ala Pro
            580                 585                 590

Thr Leu Ser Gln Ala Gly His Val Ile Ala Gly Arg Thr Lys Leu Val
        595                 600                 605

Ala Pro Leu Tyr Asn Ala Ser Gly Ala Gly Asp Asp Phe Leu Val Thr
    610                 615                 620

Ile Ala Thr Thr Asp Ser Val Val Arg Leu Tyr Ser Leu Pro Asp Leu
625                 630                 635                 640

Ala Val Val Pro Ile Ala Glu Pro Leu Val Thr Leu Gly Asp Trp Asn
                645                 650                 655

Ala Val Cys Ser Trp Gly Ser Tyr Phe Phe Leu Phe Gly Lys Gly Gly
            660                 665                 670

Arg Ala Phe Gln Tyr Leu Val Arg Pro Ala Asp Gly Gly Ser Phe Glu
        675                 680                 685

Val Leu Glu Val Arg Ala Leu Pro Ala Pro Val Val Phe Val Gly Cys
    690                 695                 700

Ala Val Ser Pro Arg Asp Ala Lys Leu Tyr Leu Thr Glu Asp Val Asp
705                 710                 715                 720

Gln Asp Val Tyr Val Met Asp Val Gly Asp Met Ala Gly Gln Pro Thr
                725                 730                 735

Leu Lys Lys Val Lys Gly Val Lys Asp Ala Thr Gly Ile Ala Leu Tyr
            740                 745                 750

Ser Ser Gly Asp Gln Glu Tyr Val Phe Val Ala Gln Glu Ser Gln Leu
        755                 760                 765

Ser Val Tyr Gly Pro Asp Trp Thr Leu Leu Gly Thr Val Gln Leu Ala
    770                 775                 780

Gly Leu Asp Asp Ile Glu Ile Gln Gly Leu Ser Ile Tyr Gln Ala Gln
785                 790                 795                 800

Thr Ser Thr Tyr Pro Ser Gly Ala Ile Thr Phe Ala Ile Glu Ala Asp
                805                 810                 815

Ala Thr Glu Gly Phe Gly Thr Thr Ser Leu Gln Pro Ile Leu Asp Ala
            820                 825                 830

Leu Ser Ile Pro Pro Asn Thr Ala Tyr Thr Pro Arg Ser Glu Pro Thr
        835                 840                 845

Gly Pro Arg Thr Cys Ala Arg Cys Ser Asp Asn Gly Phe Cys Pro Thr
    850                 855                 860

Val Gly Cys Pro Lys Thr Cys Thr Ser Cys Leu Leu Gly Phe Ser Gly
865                 870                 875                 880

Pro Thr Cys Ser Thr Pro Arg Cys Glu Ala Asp Cys Ser Gly Arg Gly
                885                 890                 895

Thr Cys Val Gly Pro Asn Lys Cys Ala Cys Asp Ala Gly Trp Gly Gly
            900                 905                 910

Leu His Cys Ser Phe Val Leu Val Glu Ala Asp Arg Glu Thr Asp Ala
        915                 920                 925

Ala Gly Gly Asp Gly Asp Asp Pro Ala Val Trp Ile Ser Pro Val Ser
    930                 935                 940

Lys Glu Leu Ser Arg Val Ile Thr Thr Val Lys Ser Glu Ala Gly Ala

945                    950                    955                    960

Gly Leu Asn Val Phe Asp Leu Glu Gly Lys Leu Val Gln Gln Phe Thr
                965                 970                 975

Ala Gly Gln Pro Asn Asn Val Asp Val Ile Tyr Gly Phe Lys Ala Gly
            980                 985                 990

Asp Arg Val Val Asp Leu Ala Phe  Ala Gly Cys Arg Ser  Asp Glu Thr
        995                 1000                1005

Leu Cys  Leu Phe Glu Ile Thr  Ser Asn Gly Thr Ile  Thr Thr Ile
    1010                 1015                 1020

Pro Gly  Gly Ile Gln Pro Leu  Pro Lys Gly Tyr Lys  Val Tyr Gly
    1025                 1030                 1035

Ser Cys  Val Tyr Arg Ser Pro  Lys Ser Asn Arg Gln  Tyr Leu Phe
    1040                 1045                 1050

Val Asn  Ser Lys Lys Ala Arg  Tyr Leu Gln Tyr Glu  Leu Thr Ser
    1055                 1060                 1065

Thr Glu  Ser Gly Thr Leu Gln  Thr Thr Leu Val Arg  Asp Phe Thr
    1070                 1075                 1080

Gly Gly  Ser Gly Gly Gln Val  Glu Gly Cys Val Val  Asp Asp Arg
    1085                 1090                 1095

Asn Ser  Trp Leu Phe Leu Gly  Glu Glu Pro Arg Ala  Leu Trp Arg
    1100                 1105                 1110

Tyr Gly  Ala Glu Pro Asp Asp  Gly Thr Glu Gly Leu  Glu Ile Ala
    1115                 1120                 1125

Val Phe  Gly Asp Gly Lys Leu  Asn Ala Glu Val Glu  Gly Val Thr
    1130                 1135                 1140

Leu Val  Glu Gly Lys Thr Pro  Gln Glu Gly Tyr Ile  Leu Val Ser
    1145                 1150                 1155

Asn Gln  Gly Val Ser Ser Tyr  Ala Val Tyr Arg Arg  Ala Glu Pro
    1160                 1165                 1170

His Glu  Tyr Val Ala Thr Phe  Ser Ile Val Ala Ser  Ala Asp Gly
    1175                 1180                 1185

Lys Ile  Asp Ala Val Ser Asn  Thr Asp Gly Ile Thr  Ala Val Ala
    1190                 1195                 1200

Thr Gly  Leu Gly Pro Asn Phe  Pro His Gly Leu Val  Val Val His
    1205                 1210                 1215

Asp Asp Ala Asn Gln Leu Pro Glu Gly Gly Thr Ser Thr Glu Ala
    1220                1225                1230

Ser Phe Lys Met Ile Ser Leu Glu Lys Ile Phe Gly Gly Pro Leu
    1235                1240                1245

Lys Glu Leu Asn Leu Leu Asp His Val Asp Ala Asn Trp Asp Pro
    1250                1255                1260

Arg Ala
    1265

<210> 16
<211> 4083
<212> DNA
<213> Artificial

<220>
<223> glucoamylase phytase fusion gene

<400> 16
atgggcttcc gatctctact cgccctgagc ggcctcgtct gcacagggtt ggcaaatgtg      60

atttccaagc gcgcgacctt ggattcatgg ttgagcaacg aagcgaccgt ggctcgtact     120

gccatcctga ataacatcgg ggcggacggt gcttgggtgt cgggcgcgga ctctggcatt     180

gtcgttgcta gtcccagcac ggataacccg gactgtatgt ttcgagctca gatttagtat     240

gagtgtgtca ttgattgatt gatgctgact ggcgtgtcgt ttgttgtaga cttctacacc     300

tggactcgcg actctggtct cgtcctcaag accctcgtcg atctcttccg aaatggagat     360

accagtctcc tctccaccat tgagaactac atctccgccc aggcaattgt ccagggtatc     420

agtaacccct ctggtgatct gtccagcggc gctggtctcg gtgaacccaa gttcaatgtc     480

gatgagactg cctacactgg ttcttgggga cggccgcagc gagatggtcc ggctctgaga     540

gcaactgcta tgatcggctt cgggcagtgg ctgcttgtat gttctccacc cccttgcgtc     600

tgatctgtga catatgtagc tgactggtca ggacaatggc tacaccagca ccgcaacgga     660

cattgtttgg cccctcgtta ggaacgacct gtcgtatgtg gctcaatact ggaaccagac     720

aggatatggt gtgtttgttt tattttaaat ttccaaagat gcgccagcag agctaacccg     780

cgatcgcaga tctctgggaa gaagtcaatg gctcgtcttt ctttacgatt gctgtgcaac     840

accgcgccct tgtcgaaggt agtgccttcg cgacggccgt cggctcgtcc tgctcctggt     900

gtgattctca ggcacccgaa attctctgct acctgcagtc cttctggacc ggcagcttca     960

ttctggccaa cttcgatagc agccgttccg gcaaggacgc aaacaccctc ctgggaagca    1020

tccacacctt tgatcctgag gccgcatgcg acgactccac cttccagccc tgctccccgc    1080

gcgcgctcgc caaccacaag gaggttgtag actctttccg ctcaatctat accctcaacg    1140

atggtctcag tgacagcgag gctgttgcgg tgggtcggta ccctgaggac acgtactaca    1200

acggcaaccc gtggttcctg tgcaccttgg ctgccgcaga gcagttgtac gatgctctat    1260

accagtggga caagcagggg tcgttggagg tcacagatgt gtcgctggac ttcttcaagg 1320
cactgtacag cgatgctgct actggcacct actcttcgtc cagttcgact tatagtagca 1380
ttgtagatgc cgtgaagact ttcgccgatg gcttcgtctc tattgtggta agtctacgct 1440
agacaagcgc tcatgttgac agagggtgcg tactaacaga agtaggaaac tcacgccgca 1500
agcaacggct ccatgtccga gcaatacgac aagtctgatg gcgagcagct ttccgctcgc 1560
gacctgacct ggtcttatgc tgctctgctg accgccaaca accgtcgtaa ctccgtcgtg 1620
cctgcttctt ggggcgagac ctctgccagc agcgtgcccg gcacctgtgc ggccacatct 1680
gccattggta cctacagcag tgtgactgtc acctcgtggc cgagtatcgt ggctactggc 1740
ggcaccacta cgacggctac ccccactgga tctggcagcg tgacctcgac cagcaagacc 1800
accgcgactg ctagcaagac cagcaccagt acgtcatcaa cctcctgtac cactcccacg 1860
gcgaatgtga tatccaagcg cgccccggtc gagctggagc tgaacgtcac cgccctcacc 1920
cccggcatca tcgacagcga ctggaccgcc gcctactacc ccaagagcgg cgccgccccc 1980
ctcctcctcg ccaacgacgc cggcaccgcc acgggcggct tccgcgtgtt cggcctggac 2040
ggcgccccca ccctcagcca ggccggccac gtcatcgccg gtcgcaccaa gctcgtcgcc 2100
cccctctaca acgcctccgg cgccggcgac gacttcctcg tcaccatcgc caccaccgac 2160
agcgtcgtcc gcctctacag cctccccgac ctcgccgtcg tccccatcgc cgagcccctc 2220
gtcaccctcg gcgactggaa cgccgtctgc agctggggca gctacttctt cctcttcggc 2280
aagggcggtc gggccttcca gtacctcgtc cgccccgccg acggcggcag cttcgaggtc 2340
ctcgaggtcc gcgccctccc cgcccccgtc gtgttcgtcg gctgcgccgt cagcccccgc 2400
gacgccaagc tctacctcac cgaggacgtc gaccaggacg tctacgtcat ggacgtcggc 2460
gacatggccg gccagccgac cctcaagaag gtcaagggcg tcaaggacgc caccggcatc 2520
gccctctaca gcagcggcga ccaggagtac gtgttcgtcg cccaggagtc ccagctcagc 2580
gtctacggcc ccgactggac cctcctcggc accgtccagc tcgccggcct ggacgacatc 2640
gagatccagg gcctcagcat ctaccaggcc cagaccagca cctaccccag cggcgccatc 2700
accttcgcca tcgaggccga cgccacggag ggcttcggca ccacctccct ccagcccatc 2760
ctcgacgccc tcagcatccc ccccaacacc gcctacaccc cccgctccga gcccaccggc 2820
ccccgcacct gcgcccgctg cagcgacaac ggcttctgcc ccaccgtcgg ctgccccaag 2880
acctgcacca gctgcctcct cggcttcagc ggccccacct gcagcacccc ccgctgcgag 2940
gccgactgct ccggtcgcgg cacctgcgtc ggccccaaca agtgcgcctg cgacgccggc 3000
tggggcggcc tccactgcag cttcgtcctg gtggaggccg accgcgagac cgacgccgcc 3060
ggtggtgacg gtgacgaccc cgccgtctgg atcagccccg tcagcaagga gctgagccgc 3120
gtcatcacca ccgtcaagag cgaggccggt gccggcctca acgtgttcga cctcgagggc 3180
aagctcgtcc agcagttcac cgccggccag cccaacaacg tcgacgtcat ctacggcttc 3240
aaggccggcg accgcgtcgt cgacctcgcc ttcgccggct gccgcagcga cgagaccctc 3300
tgcctcttcg agatcaccag caacggcacc atcaccacca tccccggcgg catccagccc 3360

ctccccaagg gctacaaggt ctacggcagc tgcgtctacc gcagccccaa gagcaaccgc 3420

cagtacctct tcgtcaacag caagaaggcc cgctacctcc agtacgagct gaccagcacc 3480

gagagcggca ccctccagac caccctcgtc cgcgacttca ccggcggcag cggcggccag 3540

gtcgagggct gcgtcgtgga cgaccgcaac tcctggctct tcctcggcga ggagccccgc 3600

gccctctggc gctacggcgc cgagccggac gacggcaccg agggcctcga gatcgccgtg 3660

ttcggcgacg gcaagctgaa cgccgaggtc gagggcgtca ccctcgtcga gggcaagacc 3720

ccccaggagg gctacatcct cgtcagcaac cagggcgtca gcagctacgc cgtctaccgc 3780

cgcgccgagc cccacgagta cgtcgccacc ttcagcatcg tcgccagcgc cgacggcaag 3840

atcgacgccg tcagcaacac cgacggcatc accgccgtgg ccacgggcct cggccccaac 3900

ttcccccacg gcctcgtcgt cgtccatgac gacgccaacc agctccccga gggcggcacc 3960

tccaccgagg ccagcttcaa gatgatcagc ctcgagaaga tcttcggcgg ccccctcaag 4020

gagctgaacc tcctcgacca cgtcgacgcc aactgggacc cccgcgccta agatatcgag 4080

ctc 4083

<210> 17
<211> 4083
<212> DNA
<213> Magnaporthe grisea

<400> 17
atgggcttcc gatctctact cgccctgagc ggcctcgtct gcacagggtt ggcaaatgtg 60

atttccaagc gcgcgacctt ggattcatgg ttgagcaacg aagcgaccgt ggctcgtact 120

gccatcctga ataacatcgg ggcggacggt gcttgggtgt cgggcgcgga ctctggcatt 180

gtcgttgcta gtcccagcac ggataacccg gactgtatgt ttcgagctca gatttagtat 240

gagtgtgtca ttgattgatt gatgctgact ggcgtgtcgt ttgttgtaga cttctacacc 300

tggactcgcg actctggtct cgtcctcaag accctcgtcg atctcttccg aaatggagat 360

accagtctcc tctccaccat tgagaactac atctccgccc aggcaattgt ccagggtatc 420

agtaacccct ctggtgatct gtccagcggc gctggtctcg gtgaacccaa gttcaatgtc 480

gatgagactg cctacactgg ttcttgggga cggccgcagc gagatggtcc ggctctgaga 540

gcaactgcta tgatcggctt cgggcagtgg ctgcttgtat gttctccacc cccttgcgtc 600

tgatctgtga catatgtagc tgactggtca ggacaatggc tacaccagca ccgcaacgga 660

cattgtttgg cccctcgtta ggaacgacct gtcgtatgtg gctcaatact ggaaccagac 720

aggatatggt gtgtttgttt tattttaaat ttccaaagat gcgccagcag agctaacccg 780

cgatcgcaga tctctgggaa gaagtcaatg gctcgtcttt ctttacgatt gctgtgcaac 840

accgcgccct tgtcgaaggt agtgccttcg cgacggccgt cggctcgtcc tgctcctggt 900

gtgattctca ggcacccgaa attctctgct acctgcagtc cttctggacc ggcagcttca 960

ttctggccaa cttcgatagc agccgttccg gcaaggacgc aaacaccctc ctgggaagca 1020

tccacacctt tgatcctgag gccgcatgcg acgactccac cttccagccc tgctccccgc 1080

gcgcgctcgc caaccacaag gaggttgtag actctttccg ctcaatctat accctcaacg 1140
atggtctcag tgacagcgag gctgttgcgg tgggtcggta ccctgaggac acgtactaca 1200
acggcaaccc gtggttcctg tgcaccttgg ctgccgcaga gcagttgtac gatgctctat 1260
accagtggga caagcagggg tcgttggagg tcacagatgt gtcgctggac ttcttcaagg 1320
cactgtacag cgatgctgct actggcacct actcttcgtc cagttcgact tatagtagca 1380
ttgtagatgc cgtgaagact ttcgccgatg gcttcgtctc tattgtggta agtctacgct 1440
agacaagcgc tcatgttgac agagggtgcg tactaacaga agtaggaaac tcacgccgca 1500
agcaacggct ccatgtccga gcaatacgac aagtctgatg gcgagcagct ttccgctcgc 1560
gacctgacct ggtcttatgc tgctctgctg accgccaaca accgtcgtaa ctccgtcgtg 1620
cctgcttctt ggggcgagac ctctgccagc agcgtgcccg gcacctgtgc ggccacatct 1680
gccattggta cctacagcag tgtgactgtc acctcgtggc cgagtatcgt ggctactggc 1740
ggcaccacta cgacggctac ccccactgga tctggcagcg tgacctcgac cagcaagacc 1800
accgcgactg ctagcaagac cagcaccagt acgtcatcaa cctcctgtac cactcccacg 1860
gcgaatgtga tatccaagcg cgccccggtc gagctggagc tgaacgtcac cgccctcacc 1920
cccggcatca tcgacagcga ctggaccgcc gcctactacc ccaagagcgg cgccgccccc 1980
ctcctcctcg ccaacgacgc cggcaccgcc acgggcggct tccgcgtgtt cggcctggac 2040
ggcgccccca ccctcagcca ggccggccac gtcatcgccg gtcgcaccaa gctcgtcgcc 2100
cccctctaca acgcctccgg cgccggcgac gacttcctcg tcaccatcgc caccaccgac 2160
agcgtcgtcc gcctctacag cctccccgac ctcgccgtcg tccccatcgc cgagcccctc 2220
gtcaccctcg gcgactggaa cgccgtctgc agctggggca gctacttctt cctcttcggc 2280
aagggcggtc gggccttcca gtacctcgtc cgccccgccg acggcggcag cttcgaggtc 2340
ctcgaggtcc gcgccctccc cgcccccgtc gtgttcgtcg gctgcgccgt cagcccccgc 2400
gacgccaagc tctacctcac cgaggacgtc gaccaggacg tctacgtcat ggacgtcggc 2460
gacatggccg gccagccgac cctcaagaag gtcaagggcg tcaaggacgc caccggcatc 2520
gccctctaca gcagcggcga ccaggagtac gtgttcgtcg cccaggagtc ccagctcagc 2580
gtctacggcc ccgactggac cctcctcggc accgtccagc tcgccggcct ggacgacatc 2640
gagatccagg gcctcagcat ctaccaggcc cagaccagca cctaccccag cggcgccatc 2700
accttcgcca tcgaggccga cgccacggag ggcttcggca ccacctccct ccagcccatc 2760
ctcgacgccc tcagcatccc ccccaacacc gcctacaccc cccgctccga gcccaccggc 2820
ccccgcacct gcgcccgctg cagcgacaac ggcttctgcc ccaccgtcgg ctgccccaag 2880
acctgcacca gctgcctcct cggcttcagc ggccccacct gcagcacccc ccgctgcgag 2940
gccgactgct ccggtcgcgg cacctgcgtc ggccccaaca agtgcgcctg cgacgccggc 3000
tggggcggcc tccactgcag cttcgtcctg gtggaggccg accgcgagac cgacgccgcc 3060
ggtggtgacg gtgacgaccc cgccgtctgg atcagccccg tcagcaagga gctgagccgc 3120

gtcatcacca ccgtcaagag cgaggccggt gccggcctca acgtgttcga cctcgagggc 3180

aagctcgtcc agcagttcac cgccggccag cccaacaacg tcgacgtcat ctacggcttc 3240

aaggccggcg accgcgtcgt cgacctcgcc ttcgccggct gccgcagcga cgagaccctc 3300

tgcctcttcg agatcaccag caacggcacc atcaccacca tccccggcgg catccagccc 3360

ctccccaagg gctacaaggt ctacggcagc tgcgtctacc gcagccccaa gagcaaccgc 3420

cagtacctct tcgtcaacag caagaaggcc cgctacctcc agtacgagct gaccagcacc 3480

gagagcggca ccctccagac caccctcgtc cgcgacttca ccggcggcag cggcggccag 3540

gtcgagggct gcgtcgtgga cgaccgcaac tcctggctct tcctcggcga ggagccccgc 3600

gccctctggc gctacggcgc cgagccggac gacggcaccg agggcctcga gatcgccgtg 3660

ttcggcgacg gcaagctgaa cgccgaggtc gagggcgtca ccctcgtcga gggcaagacc 3720

ccccaggagg gctacatcct cgtcagcaac cagggcgtca gcagctacgc cgtctaccgc 3780

cgcgccgagc cccacgagta cgtcgccacc ttcagcatcg tcgccagcgc cgacggcaag 3840

atcgacgccg tcagcaacac cgacggcatc accgccgtgg ccacgggcct cggccccaac 3900

ttcccccacg gcctcgtcgt cgtccatgac gacgccaacc agctccccga gggcggcacc 3960

tccaccgagg ccagcttcaa gatgatcagc ctcgagaaga tcttcggcgg ccccctcaag 4020

gagctgaacc tcctcgacca cgtcgacgcc aactgggacc cccgcgccta agatatcgag 4080

ctc 4083

<210> 18
<211> 1380
<212> DNA
<213> Magnaporthe grisea

<400> 18
atgctcccca tcttcatcat cctcttcggc ctcgtcatgc ccgccatcag caccaacaac 60

tacttcgacc tcaacaagta cgacaccccc gacctcggct tcctctaccg ccccgagatc 120

acctacaagt ggggccactt cacccccttc cacagcacca gcagcgagtt caaccccagc 180

cccagcctcc ccggctgcac cgtcaccttc gtccagagcc tcatgcgcca cggcgcccgc 240

taccccaaga ccggcagccc ctaccgcgac atcatccagc gcatccagag cagcgtcgac 300

aagtacagcg ccaagtacga gttcatccgc aactacgagt acaacctccc caccgaggac 360

ctgaacgacc tgggccggca ccagctcatc aacgccggca tccacttcta ccgccgctac 420

cagaacctcg ccaaggacaa cgagcccttc atccgcagcg acggcgccga gcgcgtcgcc 480

gagagcgccg agaagtgggc cgacggcttc caccagacca gcctcaccga caagacgcgc 540

atccagcccc accagttccc cgtcagcatc ttcaagttcc ccgtcggcaa gcagtacaac 600

aacaccctca acaacctcgc ctgcaccaag tacgacaaga gcaaggccca gaagatcgcc 660

aaggagctgc ccaagcgcat cctcaccgag accgccaagc agctcaacca gaacctcccc 720

ggcgccaacc tcagcgtcta cgagatcctc cagctcatgg agctgtgcgg catggagacc 780

accgccaaca tcgtcaagac cggcatcctc agccccatct gcaacatctt cgacaacaag 840

gactgggagg ccttccagcg cttccaccac gaggtcaagt ggtacaccca cttctacggc 900

aaccccctcg gccccagcct cggcatcggc tgggtcaacg agctgatcgc ccgcctcctc 960

cgccgccccg tccaggacag cacctccacc aacaccacca tcaccgccaa ccccaagacc 1020

ttccccctcg acaagaagct ctacgccgac ttcacccacg ccaccgactt cctcgccatc 1080

caggccgccc tcggcctcct cggcagcgcc cacacccccg tccccgccaa ccgcatcgtc 1140

agcgaccagc gcatcgccga gttcagcgcc agcgaggtct cccccttcgc cgcccgcatg 1200

tacgtcgaga agatgacctg cagcggccac accgaggagc tggtccgcat cctcgtcaac 1260

aaccgcgtcg tctggctccc caactgcggc gtcgacggcc agggccgctg caagctcggc 1320

gccttcgtcg agtccctcag cttcgcccgc agcggcggct tctggaacaa gtgcttctaa 1380

<210> 19
<211> 774
<212> PRT
<213> Gibberella zeae

<400> 19

Met Thr Ala Ser Lys Thr Lys Asp Gly Ser Pro Pro Lys Lys Gln Ser
1               5                   10                  15

Thr Phe Thr Cys Leu Ala Ile Met Val Gly Ser Gly Leu Ile Ala Leu
            20                  25                  30

Leu Ala Val Ala Phe Gln Ile Ser Phe Ala Lys Ala Val Glu Val Asp
        35                  40                  45

Val Lys Val Asp Ala Tyr Thr Asn Thr Val Glu Ser Asp Trp Thr Thr
    50                  55                  60

Val Tyr Tyr Ser Asn Ser Asn Pro Leu Leu Ile Gly Asn Asp Gly Gly
65                  70                  75                  80

Pro Asp Lys Gly Gly Phe His Ala Tyr Asp Leu Ile Ser Lys Ser Pro
                85                  90                  95

Leu Arg Glu Val Thr Ala Gln Thr Pro Gly Arg Ser Lys Leu Val Thr
            100                 105                 110

Thr Met Tyr Asp Val Asp Asp Lys Asp Val Leu Val Thr Ile Ala Gln
        115                 120                 125

Pro Asp Ser Val Leu Arg Val Tyr Lys Met Pro Glu Phe Lys Gln Ile
    130                 135                 140

Lys Asp Ala Asp Phe Lys Val Leu Gly Asp Trp Ser Ala Leu Cys Ser
145                 150                 155                 160

Trp Lys Ser Pro Ala Gly Asn Asp Tyr Val Tyr Leu Phe Gly Lys Gly
                165                 170                 175

Gln Ala Val Gln Leu Leu Leu Arg Ala Thr Lys Asn Ser Ile Glu Phe
            180                 185                 190

Val Glu Tyr Gln Ile Gln Thr Phe Pro Thr Asp Phe Glu Thr Ser Gly
        195                 200                 205

Cys Ala Ala Ser Arg Ser Glu Ser Arg Met Tyr Ile Ser Thr Asp Asp
    210                 215                 220

Asp Lys Asn Val Tyr Ser Phe Ser Leu Lys Glu Ser Thr Gly Ala Pro
225                 230                 235                 240

Lys Ile Thr Lys Val Gly Lys Ala Glu Asp Asp Val Thr Gly Leu Ala
                245                 250                 255

Val Tyr Val Pro Asn Lys Gly Ser Asp Tyr Leu Phe Val Ala Gln Thr
            260                 265                 270

Asp Lys Ile Ala Val Tyr Asp Gln Ser Phe Lys Leu Lys Gly Thr Leu
        275                 280                 285

Ser Leu Thr Gly Phe Glu Asp Ile Glu Val Gln Gly Leu Asn Ile Tyr
    290                 295                 300

Gln Gly Ser Ala Gly Lys Tyr Pro Ala Gly Ala Leu Thr Tyr Ala Leu
305                 310                 315                 320

Glu Ser Glu Glu Val Asn Gly Phe Gly Val Ser Ser Leu Glu Thr Thr
                325                 330                 335

Leu Lys Lys Leu Lys Ile Glu Pro Asn Thr Lys Tyr Asn Pro Arg Lys
            340                 345                 350

Leu Lys Ser Glu Ser Glu Lys Glu Pro Ile Cys Lys Thr Cys Gly Gly
        355                 360                 365

Ser Gly Phe Cys Val Lys Asp Lys Lys Gln Lys Cys Glu Cys Phe Ser
    370                 375                 380

Gly Phe Ala Gly Lys Thr Cys Asn Ser Phe Thr Cys Val Asp Asn Cys
385                 390                 395                 400

Ser Gly His Gly Lys Cys Val Gly Ala Asn Asn Cys Lys Cys Asp Lys
                405                 410                 415

Gly Trp Gly Gly Leu His Cys Ser Phe Leu Leu Val Glu Pro Thr Tyr
            420                 425                 430

Glu Thr Glu Ser Arg Pro Gly Asp Gly Asp Asp Pro Ala Ile Trp Ile
        435                 440                 445

Ser Pro Glu Gly Pro Glu Lys Ser Arg Ile Val Thr Thr Met Lys Ser
    450                 455                 460

Gly Lys Glu Ala Gly Leu Gly Val Phe Asp Leu Lys Gly Asn Leu Val
465                 470                 475                 480

Gln Thr Phe Ala Ala Gly Glu Pro Asn Asn Val Asp Met Ile Tyr Ser
                485                 490                 495

Phe Lys Ala Gly Asp Arg Lys Val Asp Leu Ala Phe Ala Ala Cys Arg
            500                 505                 510

Ala Asp Asp Thr Leu Cys Leu Phe Glu Met Leu Pro Asn Gly Thr Leu
        515                 520                 525

Thr Asn Ile Pro Gly Gly Ser His Pro Val Val Glu Asp Tyr Lys Val
    530                 535                 540

Tyr Gly Ser Cys Thr Tyr Arg Ser Pro Lys Thr Gly Lys Gln Tyr Leu
545                 550                 555                 560

Phe Val Asn Glu Lys Ser Ala Arg Tyr Leu Gln Tyr Glu Leu Thr Ser
                565                 570                 575

Thr Ser Glu Gly Glu Leu Gln Thr Lys Leu Val Arg Glu Phe Gln Gly
            580                 585                 590

Gly Ser Gly Gly Gln Val Glu Gly Cys Val Thr Asp Lys Glu Asn Gly
        595                 600                 605

Trp Ile Phe Leu Gly Glu Glu Pro Ser Ala Leu Trp Arg Tyr Gly Ala
    610                 615                 620

Glu Pro Asp Ser Lys Asp Glu Gly Val Val Ile Gly Lys Val Gly Asp
625                 630                 635                 640

Gly Lys Leu Tyr Gly Asp Val Glu Gly Val Thr Leu Val Tyr Gly Ser
                645                 650                 655

Lys Ala Asp Glu Gly Tyr Ile Leu Val Ser Cys Gln Gly Val Ser Ala
            660                 665                 670

Tyr Asn Val Tyr Arg Arg Ala Glu Pro His Glu Tyr Val Thr Thr Phe
        675                 680                 685

Thr Leu Val Glu Ser Ser Asp Gly Lys Ile Asp Pro Val Ser Asn Thr
    690                 695                 700

Asp Gly Ile Thr Ala Val Gly Thr Ala Leu Ser Lys Asp Phe Pro His
705                 710                 715                 720

Gly Leu Val Val Val His Asp Asp Ala Asn Gln Leu Pro Asn Gly Lys

725                 730                 735

Thr Ser Ala Glu Ala Ser Phe Lys Met Val Ser Leu Glu Lys Ile Leu
            740                 745                 750

Gly Ser Lys Val Leu Gly Lys Lys Gly Leu Leu Asp Gln Val Asp Lys
        755                 760                 765

Asp Trp Asp Pro Arg Lys
    770

<210>  20
<211>  736
<212>  PRT
<213>  Aspergillus nidulans

<400>  20

Met His Leu Pro Tyr Ala Leu Gly Val Leu Leu Val Ser Ala Ala Gly
1               5                   10                  15

Ser Ile Ala Ser Pro Leu Thr Gly Asn Leu Ala Val Ser Ser Leu Ser
            20                  25                  30

Thr Glu Val Glu Ser Asp Gln Thr Ala Phe Tyr Tyr Gly Thr Ala Arg
        35                  40                  45

Ser Arg Ser Phe Leu Ile Gly Asn Asp Gly Ser Ala Ala Thr Gly Gly
    50                  55                  60

Ile Arg Ser Phe Ser Leu Gln Asn Leu Asn Glu Thr Ala Arg Val Lys
65                  70                  75                  80

Pro Gly Arg Thr Lys Val Ala Gly Leu Leu Tyr Asp Val Gly His Arg
                85                  90                  95

Asp Leu Val Val Ser Ile Ala Ala Pro Asp Ser Ile Ile Arg Val Phe
            100                 105                 110

Asp Val Asn Gly Leu Gly Glu Ile Pro Ser Thr Arg Lys Lys Ala Leu
        115                 120                 125

Gly Asp Trp Ser Cys Leu Cys Thr Trp Thr Ser Ser Ser Gly Gly Lys
    130                 135                 140

Tyr Phe Tyr Leu Leu Gly Lys Lys Gln Gly Ile Gln Phe Leu Val Arg
145                 150                 155                 160

Glu Lys Gly Ser Lys Val Glu Val Leu Glu Val Gln Thr Phe Pro Leu
                165                 170                 175

Pro Val Glu Pro Ser Ser Cys Ala Val Ser Pro Glu Asp Asn Val Val
            180                 185                 190

Tyr Phe Ala Ala Glu Asp Lys Thr Ile Tyr Ser Phe Ala Ala Ala Glu
        195                 200                 205

Ser Thr Thr Ala Pro Asp Ile Gln Thr Leu Gly Gln Val Ser Asp Glu
    210                 215                 220

Ile Ser Gly Leu Ala Val Tyr Val Ser Ala Ala Thr Gln Tyr Leu Phe
225                 230                 235                 240

Val Thr Gln Ser Asp Lys Val Glu Val Tyr Thr Pro Glu Leu Glu Gln
                245                 250                 255

Val Gly Ser Leu Ala Val Thr Gly Val Glu Asp Leu Glu Ile Ala Gly
            260                 265                 270

Thr Ser Ile Tyr Gln Ser Asn Ser Ser Gln Tyr Pro Tyr Gly Leu Leu
        275                 280                 285

Gly Phe Ala Ile Glu Ser Asp Ser Gly Asn Ala Phe Gly Val Ala Ser
    290                 295                 300

Leu Glu Pro Ala Phe Thr Ser Leu Lys Leu Gln Pro Asn Thr Ser Tyr
305                 310                 315                 320

Thr Pro Arg Arg Ser Ser Gly Gln Ser Gly Pro Lys Gln Asn Gly Phe
                325                 330                 335

Pro Ser Ala Asn Asn Thr Leu Ser Cys Phe Ala Gly Trp Thr Gly Ser
            340                 345                 350

Asp Cys Thr Glu Ile Thr Cys His Asn Asn Cys Ser Arg His Gly Thr
        355                 360                 365

Cys Leu Gly Pro Asn Glu Cys Lys Cys Arg Ser His Trp Ala Gly Pro
    370                 375                 380

Glu Cys Ser Trp Ile Gly Val Glu Ala Lys Tyr Glu Thr Asp Ala Asn
385                 390                 395                 400

Gly Gly Asp Gly Asp Asp Pro Ala Ile Trp Ile Ser Pro Ala Asn Leu
                405                 410                 415

Asn Arg Ser Thr Ile Ile Thr Thr Thr Lys Ser Glu Ile Gly Ala Gly
            420                 425                 430

Leu Ala Val Phe Asp Leu Lys Gly Asn Leu Leu Gln Thr Val Ala Ala
        435                 440                 445

Gly Glu Pro Asn Asn Val Asp Ile Ile Tyr Gly Phe Gln Ala Gly Arg
    450                 455                 460

Arg Thr Ile Asp Leu Ala Tyr Ala Ala Cys Arg Glu Asp Asp Thr Leu
465                 470                 475                 480

Cys Leu Phe Glu Ile Thr Pro Asp Gly Leu Leu Thr Ser Ile Pro Gly
                485                 490                 495

Gly Arg Gln Pro Thr Pro Glu Asp Tyr Thr Val Tyr Gly Ser Cys Ser
            500                 505                 510

Tyr Arg Ser Pro Ser Asn Gly Lys Gln Tyr Leu Phe Val Asn Glu Lys
        515                 520                 525

Ser Gly Leu Tyr Leu Gln Tyr Glu Leu Thr Ser Ser Pro Asn Gly Thr
    530                 535                 540

Leu Ala Thr Thr Leu Val Arg Lys Phe Thr Gly Gly Ser Gly Gly Gln
545                 550                 555                 560

Pro Glu Gly Cys Val Ala Asp Glu Glu Asn Gly Tyr Ile Phe Leu Gly
                565                 570                 575

Glu Glu Pro Leu Gly Leu Trp Arg Tyr Glu Ala Glu Pro Thr Gly Ser
            580                 585                 590

Pro Asn Gly Thr Leu Ile Ala Lys Val Gly Asp Gly Thr Ile Tyr Ala
        595                 600                 605

Asp Val Glu Gly Val Thr Leu Leu Pro Gly Gln Thr Pro Glu Gln Gly
    610                 615                 620

Leu Ile Ile Val Ser Cys Gln Gly Val Ser Ala Tyr Ser Val Tyr Arg
625                 630                 635                 640

Arg Ala Glu Pro His Asp His Val Leu Thr Phe Thr Ile Gly Glu Ser
                645                 650                 655

Gly Asp Gly Ser Val Asp Gly Val Thr Asn Thr Asp Gly Val Thr Gly
            660                 665                 670

Val Ser Thr Gly Leu Asn Asp Asp Phe Pro Arg Gly Leu Leu Val Val
        675                 680                 685

His Asp Asp Ala Asn Gln Leu Ser Thr Gly Glu Thr Ala Glu Leu Ala
    690                 695                 700

Ser Phe Lys Leu Val Ser Leu Glu Asp Val Leu Ala Ala Ala Gly Lys
705                 710                 715                 720

Arg Thr Trp Leu Phe Glu Glu Val Asp Glu Thr Trp Asp Pro Arg Ala
                725                 730                 735

<210>  21

<211>  745
<212>  PRT
<213>  aspergillus terreus

<400>  21

Met Ser Gly Arg Val Pro Glu Ala Asn Lys Arg Gln Leu Arg Gly Lys
1               5                   10                  15

Arg His Lys Val Asp Lys Met Arg Leu Ala Cys Ala Leu Gly Ser Leu
            20                  25                  30

Phe Ala Leu Phe Leu Ser Val Ala Ala Ser Ala Pro Thr Val Lys Val
        35                  40                  45

Ser Ile Ser Ala Leu Thr Ala Gly Ile Glu Ser Asp Trp Ala Ala Val
    50                  55                  60

Tyr Tyr Gly Pro Arg Pro Gln Asp Ser Leu Leu Val Gly Asn Asp Gly
65                  70                  75                  80

Ser Ala Ala Thr Gly Gly Leu Arg Thr Tyr Gly Leu Leu Asp Arg Asn
                85                  90                  95

Leu Asn Glu Thr Ala Arg Arg Thr Pro Gly Arg Thr Lys Val Val Gly
            100                 105                 110

Val Leu Tyr Asp Ile Gly Gly Arg Asp Leu Leu Val Ser Ile Thr Thr
        115                 120                 125

Thr Glu Ser Ile Ile Arg Leu Tyr Asp Val Asp Gly Leu Arg Glu Ile
    130                 135                 140

Ser Gly Val Lys Lys Lys Val Leu Gly Asp Trp Ser Cys Leu Cys Thr
145                 150                 155                 160

Trp Arg Ser Pro Arg Gly Ala Asp Tyr Val Phe Leu Leu Gly Lys Lys
                165                 170                 175

Gln Ala Val Gln Phe Leu Val Arg Gly Asn Arg Lys Glu Leu Glu Ile
            180                 185                 190

Leu Glu Val Gln Ala Phe Pro Leu Pro Val Glu Pro Ser Ser Cys Ala
        195                 200                 205

Val Asp Pro Glu Gly Val Val Tyr Phe Ala Ala Glu Asn Thr Met Leu
    210                 215                 220

Tyr Ser Phe Gln Ala Ala Glu Ser Thr Val Ala Pro Glu Ile Lys Ala
225                 230                 235                 240

Phe Gly Glu Val Gly Asp Asp Ile Ser Gly Leu Ala Val Tyr Val Ser
                245                 250                 255

His Arg Ser His Tyr Leu Phe Val Ala Gln Thr Asp Val Ile Glu Ile
            260                 265                 270

Tyr Ser Pro Ser Leu Glu Leu Lys Gly Ser Leu Ala Val Thr Gly Ala
        275                 280                 285

Glu Asp Ile Glu Ile Ala Gly Thr Ala Ile Tyr Gln Ser Ser Ser Ser
    290                 295                 300

Gln Tyr Pro Tyr Gly Leu Ile Ser Tyr Ala Ile Glu Ser Asp Ser Gly
305                 310                 315                 320

Val Gly Phe Gly Val Ser Ser Leu Glu Pro Val Phe Thr Glu Leu Arg
                325                 330                 335

Leu Glu Pro Asn Thr Arg Tyr Thr Pro Arg Ser Ala Arg Pro Pro Gln
            340                 345                 350

Ser Glu Ser Lys Leu Asn Gly Phe Arg Asn Arg Asp Gly Ser Leu Ser
        355                 360                 365

Cys Phe Ala Gly Phe Thr Gly Arg Asn Cys Asn Arg Val Ala Cys Ser
    370                 375                 380

Asn Asp Cys Ser Gly His Gly Ser Cys Val Gly Pro Asn Glu Cys Gln
385                 390                 395                 400

Cys Arg Asp Pro Trp Ala Gly Pro Asp Cys Ser Trp Ile Gly Val Glu
                405                 410                 415

Ala Lys Tyr Glu Thr Asp Ala Asn Gly Gly Asp Gly Asp Asp Pro Ala
            420                 425                 430

Ile Trp Ile Ser Pro Thr Ser Pro Asn Leu Ser Thr Ile Ile Thr Thr
        435                 440                 445

Thr Lys Ser Glu Thr Ile Ser Ala Gly Glu Pro Asn Asn Val Asp Val
    450                 455                 460

Ile Tyr Ser Phe Gln Leu Gly Asn Arg Thr Val Asp Leu Ala Tyr Ala
465                 470                 475                 480

Ala Cys Arg Ala Asp Asp Thr Leu Cys Leu Phe Glu Val Thr Ser Asp
                485                 490                 495

Gly Leu Leu Ala Glu Val Pro Gly Gly Ile Gln Pro Val Pro Glu Asp
            500                 505                 510

Tyr Thr Val Tyr Gly Ser Cys Ala Tyr Arg Ser Pro Ser Ser Gly Lys
        515                 520                 525

Gln Tyr Leu Phe Val Asn Ala Lys Ser Gly Gln Tyr Leu Gln Tyr Glu
    530                 535                 540

Leu Thr Ser Arg Thr Asn Gly Thr Leu Ser Thr Thr Leu Val Arg Thr
545                 550                 555                 560

Phe Thr Gly Gly Thr Gly Gly Gln Pro Glu Gly Cys Val Ala Asp Glu
                565                 570                 575

Glu Asn Gly Ser Leu Phe Leu Gly Glu Glu Pro Tyr Gly Leu Trp Arg
            580                 585                 590

Tyr Asp Ala Glu Pro Thr Gly Ser Ser Asn Gly Thr Leu Val Ala Lys
        595                 600                 605

Ala Gly Asp Gly Thr Leu Phe Ala Asp Val Glu Gly Val Thr Leu Leu
    610                 615                 620

Pro Gly Lys Thr Ala Asp Gln Gly Phe Ile Val Val Ser Cys Gln Gly
625                 630                 635                 640

Val Ser Ala Tyr Ser Val Tyr Arg Arg Ala Glu Pro His Glu Tyr Val
                645                 650                 655

Met Thr Phe Thr Ile Gly Glu Ser Lys Asp Gly Arg Val Asp Gly Val
            660                 665                 670

Thr Asn Thr Asp Gly Val Ala Gly Val Pro Asn Arg Leu Asn Ala Asp
        675                 680                 685

Phe Pro His Gly Leu Leu Val Val His Asp Asp Ala Asn Gln Leu Ala
    690                 695                 700

Glu Gly Gly Thr Ala Glu Leu Ala Ser Phe Lys Leu Val Ser Leu Glu
705                 710                 715                 720

Asp Val Leu Gly Pro Thr Val Gly Gly Met Leu Leu Leu Glu Leu Asp
                725                 730                 735

Ala Glu Trp Asn Pro Arg Thr Thr Val
            740                 745


<210>  22
<211>  729
<212>  PRT
<213>  Phaeosphaeria nodorum

<400>  22

Met Met Ile Phe Ala Asn Ser Leu Leu Trp Val Ser Thr Val Phe Ala
1               5                   10                  15

Val Ala Ser Ala Gln Ser Val Asn Leu Thr Leu Val Pro Val Ala Thr
            20                  25                  30

Gly Phe Lys Asp Glu Asn Thr Gly Phe Val Tyr Gly Gly Ser Pro Ile
        35                  40                  45

Leu Val Ala Asn Asp Lys Ser Ala Asp Asp Gly Gly Phe Arg Thr Phe
    50                  55                  60

Ala Val Ala Pro Pro Pro Ile Phe Lys Gln Ile Ala His Gln Lys Thr
65                  70                  75                  80

Gly Arg Ser Lys Val Val Val Pro Val His Asp Ile Gly Gly Arg Asp
                85                  90                  95

Leu Ile Ile Asn Ile Pro Ser Pro Asp Ser Leu Ile Arg Val Phe Asp
            100                 105                 110

Thr Lys Thr Gly Lys Lys Val Asp Ser Asn Asp Lys Ile Gln Leu Gly
        115                 120                 125

Asp Trp Ser Thr Ala Cys Val Trp Arg Ser Gln Lys Ser Gly Ala Ser
    130                 135                 140

Tyr Leu Phe Leu Phe Gly Lys Ser Lys Val Val Gln Phe Leu Ile Arg
145                 150                 155                 160

Asp Lys Lys Lys Asp Val Glu Ile Val Glu Thr Phe Pro Val Pro Ile
                165                 170                 175

Glu Gly Glu Thr Cys Thr Val Phe Leu Asn Gly Gln Val Phe Phe Ser
            180                 185                 190

Ala Glu Asp Arg Pro Leu Tyr Ser Phe Gln Ala Thr Glu Ser Thr Arg
        195                 200                 205

Ala Pro Glu Val Lys Thr Val Ser Asp Lys Ile Glu Val Ala Gly Leu
    210                 215                 220

Ala Thr Tyr His Ser Asn Ser Ser Asp Tyr Leu Phe Val Phe His Gly
225                 230                 235                 240

Glu Val Met Asp Val Tyr Asp Ser Lys Ile Ala Pro Lys Gly Thr Val
                245                 250                 255

Glu Phe Asn Gly Val Ala Asp Leu Ser Ile Glu Gly Gly Leu Ser Leu
            260                 265                 270

Leu Gln Ser Ser Thr Glu Ser Tyr Pro Ala Gly Ile Ile Ala Phe Ala
        275                 280                 285

Phe Glu Gly Glu Asp Gly Asn Gly Val Val Ala Ala Ser Leu Ala Ser
    290                 295                 300

Val Leu Ala Pro Leu Gly Ile Ala Thr Asn Thr Ala Tyr Ser Pro Lys
305                 310                 315                 320

Thr Lys Pro Cys Thr Arg Cys Glu Ser Ala Ile Ser Glu Lys Cys Ser
                325                 330                 335

Asn Asn Gly Phe Ser Ala Gly Gly Gly Cys Ser Cys Phe Ala Gly Phe
            340                 345                 350

Ser Gly Lys Glu Cys Asn Lys Asn Ala Cys Leu Asn Asp Cys Ser Gly
        355                 360                 365

His Gly Lys Cys Asp Gly Pro Asn Val Cys Lys Cys Lys Asp Gly Trp
    370                 375                 380

Thr Gly Pro Asp Cys Ser Phe Val Ala Val Lys Ala Lys Tyr Glu Thr
385                 390                 395                 400

Glu Ala Asn Gly Gly Asp Gly Asp Asp Pro Ala Val Trp Ile His Ala
                405                 410                 415

Thr Lys Ala Asp Gln Ser Lys Ile Ile Thr Thr Thr Lys Ser Ser Asp
            420                 425                 430

Gly Glu Gly Phe Gly Val Phe Asp Leu Gln Gly Lys Leu Leu Gln His
        435                 440                 445

Phe Thr Ala Glu Glu Pro Asn Asn Val Asp Ile Ile Tyr Asn Val Thr
    450                 455                 460

Val Gly Ser Arg Lys Thr Asp Leu Ala Phe Ala Ala Cys Arg Gly Asp
465                 470                 475                 480

Asn Thr Leu Cys Leu Val Glu Val Asn Ser Ser Gly Leu Ile Gln Pro
                485                 490                 495

Ile Ala Gly Gly Lys Gln Ser Leu Pro Glu Asp Tyr Glu Pro Tyr Gly
            500                 505                 510

Ser Cys Thr Tyr His Ser Arg Lys Ser Gly Lys Asp Tyr Leu Phe Val
        515                 520                 525

Asn Asn Lys Lys Ala Glu Tyr Leu Gln Tyr Glu Leu Ser Ser Thr Val
    530                 535                 540

Asn Gly Thr Leu Gln Thr Lys Leu Val Arg Gln Phe Thr Gly Gly Ser
545                 550                 555                 560

Gly Gly Gln Val Glu Gly Cys Val Gly Asp Asp Gly Ala Gly Tyr Ile
                565                 570                 575

Phe Leu Gly Glu Glu Pro Leu Gly Ile Trp Arg Tyr Glu Ala Glu Pro
            580                 585                 590

Thr Gly Ser Asn Thr Gly Val Gln Val Ala Lys Val Gly Asp Ala Ser
        595                 600                 605

Gly Leu Ser Ala Asp Val Glu Gly Ile Thr Leu Val Pro Ala Lys Ser
    610                 615                 620

Gly Pro Gly Gly Tyr Ile Leu Val Ser Ser Gln Gly Ile Ser Ala Tyr
625                 630                 635                 640

Lys Val Tyr Glu Arg Ala Pro Pro His Lys Tyr Val Thr Thr Phe Thr
                645                 650                 655

Ile Val Asn Asn Glu Lys Lys Gly Val Asp His Val Ser Asn Thr Asp
            660                 665                 670

Gly Cys Ala Ala Val Gly Asn Arg Leu Asn Lys Asp Phe Pro Tyr Gly
        675                 680                 685

Leu Phe Val Thr His Asp Asp Ala Asn Glu Leu Ala Glu Gly Gly Thr
    690                 695                 700

Ala Lys Glu Ala Ser Phe Lys Leu Ala Ser Leu Val Asp Ile Leu Gly
705                 710                 715                 720

Glu Glu Arg Ala Lys Gly Leu Gly Tyr
                725


<210>  23
<211>  616
<212>  PRT
<213>  Chaetomium globosum

<400>  23

Met Ala Ile Ser Arg Arg Ile Thr Ala Ala Ala Leu Leu Ala Ser Thr
1               5                   10                  15

Leu Ser Ile Gln Ala Ala Ala Val Asp Val Glu Ile Pro Val Ser Ala
            20                  25                  30

Ala Thr Ser Asp Ile Glu Ser Asp Trp Ala Ser Val Tyr Tyr Gly Lys
        35                  40                  45

Thr Pro Leu Leu Leu Gly Asn Asp Gly Thr Ala Ala Thr Gly Gly Trp
    50                  55                  60

Leu Ala Trp Asp Leu Asn Ser Ala Ser Pro Leu Asn Gln Val His Ala
65                  70                  75                  80

Glu Thr Pro Gly Arg Arg Thr Lys Leu Val Thr Thr Ile His Gly Arg
                85                  90                  95

Asp Asp Asn Lys Lys Pro Thr Gly Leu Ala Val Ser Ile Ser Gln Pro
            100                 105                 110

Asp Ser Val Leu Arg Ala Trp Glu Leu Pro Asp Phe Asp Glu Val Lys
        115                 120                 125

Ser Ala Arg Ser Thr Val Leu Gly Asp Trp Ser Ala Leu Cys Ser Trp
    130                 135                 140

Lys Ser Pro Ser Gly His Asp Tyr Val Tyr Leu Phe Gly Lys Lys Glu
145                 150                 155                 160

Ala Lys Val Phe Leu Val Arg Lys Asp Lys Asp Glu Asp Asp Tyr Glu
                165                 170                 175

Asp Ala Lys Phe Val Glu Ile Gln Thr Phe Pro Leu Ser Val Glu Ala
            180                 185                 190

Ser Gly Cys Ala Thr Ser Pro Ser Leu Gly Lys Met Phe Val Ser Ala
        195                 200                 205

Asp Asp Asp Lys Asp Val Tyr Val Phe Asp Leu Val Glu Ser Thr Ala
    210                 215                 220

Ala Pro Ala Ile Ser Lys Ile Gly Glu Ala Glu Asp Asp Val Thr Gly
225                 230                 235                 240

Val Ala Val Tyr Val Ser Ala Thr Thr Gly Val Asp Tyr Leu Leu Val
                245                 250                 255

Ala Met Glu Asp Thr Val Ala Val Tyr Gly Pro Pro Phe Asp Phe Ile
            260                 265                 270

Gly Asn Leu Lys Leu Val Gly Gln Glu Asp Ile Glu Ile Gln Gly Leu
        275                 280                 285

Ala Val Tyr Gln Gly Ser Thr Ser Lys Tyr Pro Ser Gly Val Leu Ala
    290                 295                 300

Tyr Ala Ile Glu Ser Asp Asp Val Thr Gly Phe Gly Val Ser Ser Leu
305                 310                 315                 320

Asp Gly Ala Ile Gln Glu Leu Gly Leu Glu Gly Asn Thr Lys Tyr Asp
                325                 330                 335

Pro Ser Leu Glu Cys Lys Lys Ser Ser Pro Ile Cys Ser Ser Cys Ser
            340                 345                 350

Asn Asn Gly Phe Cys Ala Gln Asn Gly Lys Gly Ile Glu Cys Ser Cys
        355                 360                 365

Phe Ala Gly Trp Gln Gly Lys Thr Cys Asn Thr Tyr Thr Cys Glu Asp
    370                 375                 380

Asn Cys Ser Gly Asn Gly Gln Cys Val Gly Pro Asn Gln Cys Lys Cys
385                 390                 395                 400

Lys Thr Gly Trp Gly Gly Leu His Cys Ser Phe Val Val Val Gln Pro
                405                 410                 415

Val Ala Glu Thr Asp Ala Asn Gly Gly Asp Gly Asp Asp Pro Ala Ile
            420                 425                 430

Trp Ile Ser Pro Ser Asp Arg Ser Gln Ser Arg Ile Ile Thr Thr Thr
        435                 440                 445

Lys Ser Glu Gln Gly Ala Gly Leu Gly Val Phe Asp Leu Ser Gly Lys
    450                 455                 460

Leu Leu Gln Thr Ile Pro Ala Gly Glu Pro Asn Asn Val Asp Val Ile
465                 470                 475                 480

Tyr Asn Phe Gln Ala Gly Pro Arg Lys Ile Asp Leu Ala Tyr Ala Ala
                485                 490                 495

Cys Arg Ala Asp Asp Thr Leu Cys Leu Phe Glu Ile Thr Pro Asn Gly
            500                 505                 510

Thr Leu Thr Pro Ile Pro Gly Gly Ser Gln Pro Thr Pro Pro Asp Tyr
        515                 520                 525

Glu Val Tyr Gly Ser Cys Thr Tyr Arg Ser Pro Thr Thr Gln Lys Gln
    530                 535                 540

Tyr Leu Phe Val Asn Ala Lys Ser Ala Gln Tyr Leu Gln Tyr Glu Leu
545                 550                 555                 560

Gly Ala Thr Ala Asn Gly Thr Leu Thr Thr Thr Leu Leu Ala Gly Gly
                565                 570                 575

Lys Gly Thr Ser Glu Glu Ala Ser Phe Lys Leu Val Pro Leu Glu Arg
            580                 585                 590

Ile Leu Gly Ala Glu Val Val Lys Gln Leu Gly Leu Met Glu Glu Val
        595                 600                 605

Asp Ser Asp Trp Asp Pro Arg Ala
    610                 615

<210>  24
<211>  710
<212>  PRT
<213>  Coprinus cinereus

<400>  24

Met Val Ala Phe Phe Ser Ala Ala Leu Ala Ala Phe Cys Thr Leu Arg
1               5                   10                  15

Ile Gln Ser Ile Leu Ala Gln Thr Ala Leu Glu Ile Pro Ile Gln Glu
            20                  25                  30

Tyr Thr Pro Ser Asp Asp Thr Gly Pro Leu Gly Val Tyr Tyr Asp Ser
        35                  40                  45

Ser Glu Pro Leu Val Phe Ala Ala Asn Val Ser Gly Leu Phe Ser Tyr
    50                  55                  60

Ser Leu Pro Ala Leu Thr Gly Thr Ser Thr Val Arg Arg Asp Thr Lys
65                  70                  75                  80

Ser Val Thr Thr Leu Tyr Gly Glu Arg Asp Arg Asp Asp Trp Leu Val
                85                  90                  95

Ser Leu Val Ile Glu Asp Ser Leu Leu Ala Val Thr Asn Leu Lys Asn
            100                 105                 110

Pro Arg Arg Thr Ser His Leu Arg Leu Trp Gly Asp Tyr Ser Ala Val
        115                 120                 125

Cys Ala Val Gly Ser Ser Phe Val Tyr Ile Phe Gly Lys Gly His Ala
    130                 135                 140

Lys Leu Val Gln Ile Glu Arg Gly Asn Ile Lys Glu Ile Cys Glu Phe
145                 150                 155                 160

Ala Val Pro Val Glu Ala Glu Ala Cys Ala Ala Thr Ala Ala Gly Thr
                165                 170                 175

Val Phe Phe Gly Gly Asn Asp Gly Val Val Tyr Ser Phe Ala Gly Asn
            180                 185                 190

Ala Val Gln Pro Gly Ser Ser Ile Gln Gln Thr Ser Ile Thr Val Asp
        195                 200                 205

Gly Glu Ile Ala Gly Met Ala Val Tyr Glu Ser Ser Asn Asn Ala Ser
    210                 215                 220

Ser Leu Phe Val Ser Val Glu Ser Gly Thr Val Glu Val Phe Ser Thr
225                 230                 235                 240

Ala Glu Trp Ala His Gln Tyr Thr Leu Ser Phe Ser Glu Glu Asp Val
                245                 250                 255

Glu Leu Glu Gly Ile Ala Ile Tyr Gln Arg Ala Leu Pro Gly Tyr Pro
            260                 265                 270

Asp Gly Ser Leu Val Val Ser Phe Thr Glu Asp Asp Gly Arg Thr Gly
        275                 280                 285

Thr Gly Ile Val Ser Leu Thr Gln Ile Pro Leu Pro Arg Asn Ala Asn
    290                 295                 300

Phe Asn Pro Arg Asp Ala Ser Ser Asn Pro Asp Ala Ser Asn Gln Cys
305                 310                 315                 320

Thr Arg Arg Gly Phe Arg Asn Gly Ser Glu Cys Ile Cys Phe Thr Gly
                325                 330                 335

Tyr Thr Gly Ser Arg Cys Asn Gln Arg Thr Cys Pro Asn Asp Cys Ser
            340                 345                 350

Ser Arg Gly Arg Cys Val Gly Pro Asn Thr Cys Arg Cys Asp Asn Gly
        355                 360                 365

Trp Thr Gly Pro Asp Cys Ser Phe Lys Val Val Leu Ala Asp Ile Glu
    370                 375                 380

Thr Asp Ala Asn Gly Gly Asp Gly Asp Asp Pro Ala Ile Trp Ile His
385                 390                 395                 400

Pro Thr Asp Arg Thr Lys Ser Arg Val Leu Thr Thr Thr Lys Ser Lys
                405                 410                 415

Gln Gly Ala Gly Phe Ala Leu Phe Asp Leu Ala Gly Gln Met Ile Ala
            420                 425                 430

Gln Leu Pro Ala Gly Glu Pro Asn Asn Val Asp Val Ile Tyr Asn Phe
        435                 440                 445

Glu Leu Glu Asp Gly Arg Thr Val Asp Leu Gly Val Ala Ala Cys Arg
    450                 455                 460

Asp Glu Asp Thr Leu Cys Leu Met Gln Val Thr Glu Gly Gly Leu Thr
465                 470                 475                 480

Asp Leu Val Asn Leu Pro Val Lys Pro Asp Phe Ser Val Tyr Gly Ser
                485                 490                 495

Cys Val Tyr His Ser Arg Ser Ser Gly Lys Tyr Tyr Ile Phe Val Asn
            500                 505                 510

Ser Lys Thr Ala Glu Tyr Leu Gln Tyr Glu Leu Thr Pro Ser Leu Glu
        515                 520                 525

Ala Val Leu Val Arg Glu Phe Ile Gly Gly Asn Gly Gly Gln Val Glu
    530                 535                 540

Gly Cys Val Gly Asp Asp Glu Asn Gly Phe Val Tyr Ile Gly Glu Glu
545                 550                 555                 560

Pro Tyr Gly Leu Trp Arg Tyr Gly Ala Glu Pro Asp Ala Gly Thr Asp
                565                 570                 575

Arg Val Leu Val Asp Ser Ile Gln Gly Asn Leu Phe Pro Asp Val Glu
            580                 585                 590

Gly Val Thr Leu Val His Gly Pro Thr Pro Asp Lys Gly Leu Leu Ile
        595                 600                 605

Val Ser Ala Gln Gly Val Ser Ala Phe Asn Ile Tyr Arg Arg Gly Thr
    610                 615                 620

His Glu Phe Val Gly Thr Phe Thr Val Gln Ala Gly Gln Val Asp Lys
625                 630                 635                 640

Val Thr Asn Thr Asp Gly Val Ala Ala Val Gly Thr Asn Leu Gly Val
                645                 650                 655

Glu Gly Phe Glu Met Gly Val Val Val Val His Asp Asp Val Asn Ser
            660                 665                 670

Ala Pro Asp Gly Ser Arg Glu Asp Gly Ala Ser Phe Lys Ile Val Gly
        675                 680                 685

Leu Gly Gln Ile Leu Glu Thr Phe Gly Leu Glu Asp Glu Val Asp Thr
    690                 695                 700

Asn Trp Asp Pro Arg Arg
705                 710


<210>  25
<211>  595
<212>  PRT
<213>  Gibberella zeae

<400>  25

Met Ala Ala Ala Ser Gly Leu Leu Lys Gly Lys Ala Gly Ser Gly Ser
1               5                   10                  15

Ala Gly Tyr Gln Pro Val Tyr Gln Asp Glu Gly Gly Asp Gly His Asp
            20                  25                  30

Glu Gly His Pro Thr Pro His Val Gln Pro Ile Ala Pro Arg Gly Pro
        35                  40                  45

Met Arg Thr Tyr Arg Asn Met Val Ile Val Leu Ser Gly Leu Val Val
    50                  55                  60

Ile Leu Met Ala Met Asn Leu Tyr Leu Ser Leu Pro Leu Ala Phe Ser

65                  70                  75                  80

Ser Gly Ser Gly Ser Gly Ser Cys Pro Gly Asp Pro His Arg Val Pro
                85                  90                  95

Gln Tyr Phe Gln Thr Ser Pro Asn Leu Trp Pro Gly Val Thr Glu Thr
            100                 105                 110

Gly Lys Pro Ala Phe Met Ala Gln Thr His Val Phe Glu Pro Thr Ala
        115                 120                 125

Thr Phe Val Pro Asn Asp Pro Leu Gln Thr Ser Ile Pro Ile Glu Gly
    130                 135                 140

Met Lys Glu Gly Asn Arg Ser Ile Phe Gln Met Met Gly Tyr Leu Ser
145                 150                 155                 160

Pro Tyr Ser Pro Ser Thr Gly Phe Gly Val Asp Glu Tyr Pro Ile Pro
                165                 170                 175

Pro Gly Ala Glu Ile Val Gln Val Gln Met Leu Ser Arg His Gly Ala
            180                 185                 190

Arg Tyr Pro Thr Pro Gly Ala Asn Val Ala Thr Leu Gly Glu Arg Ile
        195                 200                 205

Ala Asn Ala Ser Ala Ser Leu Lys Thr Ser Gly Ala Leu Glu Phe Leu
    210                 215                 220

Asn Asn Trp Lys Tyr Glu Leu Gly Lys Ala Ile Leu Val Pro Arg Gly
225                 230                 235                 240

Arg Gln Glu Leu Phe Asp Ser Gly Ile Leu His Ser Tyr Met Tyr Gly
                245                 250                 255

Ser Leu Tyr Asn Pro Gln Ser Lys Leu Ile Val Arg Thr Thr Thr Gln
            260                 265                 270

Asp Arg Met Leu Lys Ser Ala Glu Asn Trp Met Ala Gly Phe Phe Gly
        275                 280                 285

Leu Glu Trp Thr Asn Asn Ala Thr Ile Glu Val Ile Ile Glu Ala Pro
    290                 295                 300

Gly Phe Asn Asn Ser Leu Ala Gly Ser Leu Asn Cys Pro Asn Ala Asp
305                 310                 315                 320

Lys Ala Asp Tyr Val Thr Pro Val Ala Ala Trp Tyr Glu Ile Tyr Leu
                325                 330                 335

Lys Asp Ala Thr Ala Arg Phe Asn Asn Val Thr Glu Gly Phe Thr Trp
            340                 345                 350

Thr Ala Ala Asp Val Trp Ala Ala Gln Asn Met Cys Pro Tyr Glu Thr
        355                 360                 365

Val Ala Tyr Gly Phe Ser Arg Phe Cys Asp Leu Phe Thr Tyr Glu Glu
    370                 375                 380

Trp Glu His Phe Gly Tyr Ser Ile Asp Leu Gly Phe Ser Ser Gly Ala
385                 390                 395                 400

Gly Phe Gln Ser Pro Ile Gly Arg Ala Thr Gly Leu Gly Tyr Gln Gln
                405                 410                 415

Glu Val Met Ala Arg Leu Lys Asn His Thr Leu Gly Tyr Ser Gly Ser
            420                 425                 430

Gln Ile Asn Thr Thr Leu Asp Gly Met Thr Glu Thr Phe Pro Leu Asn
        435                 440                 445

Gln Ser Leu Tyr Phe Asp Phe Ser His Asp Thr Asn Ile Ile Ser Ile
    450                 455                 460

Leu Thr Ala Phe Gly Phe Arg Gln Phe Ala Glu Asp Leu Pro Thr Asp
465                 470                 475                 480

Lys Tyr Pro Gly Asp His Glu Phe Val Val Ser Lys Ile Thr Pro Phe
                485                 490                 495

Gly Ala Arg Leu Asp Ile Glu Ile Ile Lys Ala Pro Gln Pro Ile Ser
            500                 505                 510

Pro Asn Arg Glu Asp Tyr Gln Glu Gly Lys Glu Thr Lys Tyr Ile His
        515                 520                 525

Phe Val Leu Asn Gln Arg Thr Val Pro Leu Gly Lys Ser Phe Pro Glu
    530                 535                 540

Cys Asp Val Asn Arg Lys Asp Gly Trp Cys Glu Leu Asp Thr Phe Ile
545                 550                 555                 560

Glu Val Gln Asp Lys Met Ala Glu Lys Ala Gln Phe Asp Tyr Ala Cys
                565                 570                 575

Phe Gly Asp Tyr Thr Ser Pro Pro Tyr Gly Lys Val Thr Asp Gly Val
            580                 585                 590

Pro Pro Ser
        595

<210>  26
<211>  599
<212>  PRT

<213>  Neurospora crassa

<400>  26

Met Arg Ser Pro Ile Asn Pro Gln Ala Pro Gly His Asn Pro Val Gly
1               5                   10                  15

Leu Ala Glu Lys Glu Glu Leu Val Leu Arg Pro Leu Leu Ala His Asp
            20                  25                  30

Thr Gln Pro Ser Pro Gly Gln Arg Leu Lys Arg Lys Leu Ser Ile Leu
        35                  40                  45

Leu Ala Ser Cys Ala Val Ser Leu Ala Val Leu Phe Ala Phe Asn Ile
    50                  55                  60

Leu Ala Cys Asn Gly Thr Leu Phe Gly Ile Lys Pro Arg Pro Ser Glu
65                  70                  75                  80

Leu Ala Ser Pro Thr Pro Leu Gln Ala Arg Asp Asp Gln Arg Ser Ser
                85                  90                  95

Gly Glu Lys Asp Cys Pro Cys Lys Pro Thr Ser Thr Val Pro Asp Tyr
            100                 105                 110

Phe Asn Thr Ser Pro Gly Pro Trp Ile Gly Lys Thr Ala Thr Gly Lys
        115                 120                 125

Ala Pro Phe Met Ala Gln Thr Arg Thr Phe Asp His Ala Ala Thr Tyr
    130                 135                 140

Val Pro Asn Ala Pro Leu Gln Thr Gln Val Pro Ile Gln Gly Trp His
145                 150                 155                 160

Pro Gly Asn Leu Ser Ile Phe Gly Met Met Gly Phe Leu Thr Pro Tyr
                165                 170                 175

Thr Pro Ser Thr Gly Phe Gly Val Asp Glu Trp Pro Leu Pro Asp Gly
            180                 185                 190

Ala Glu Ile Ile Trp Leu Gln Met Val Ser Arg His Gly Ser Arg Tyr
        195                 200                 205

Pro Thr Gly Gly Ser Asn Val Glu Ser Phe Gly Ala Arg Leu Ala Asn
    210                 215                 220

Ala Thr Gly Lys Phe Asn Ala Thr Gly Glu Leu Glu Phe Leu Asn Asn
225                 230                 235                 240

Trp Lys Tyr Gln Met Gly Thr Glu Ile Leu Val Pro Arg Gly Arg Gln
                245                 250                 255

Glu Leu Phe Asp Ser Gly Val Leu His Ala Tyr Met Tyr Ser Ser Leu

            260                 265                 270

Tyr Asp Pro Asn Thr Lys Ile Ile Ala Arg Thr Thr Thr Gln Asp Arg
        275                 280                 285

Met Leu Arg Ser Ala Glu Asn Phe Leu Ala Gly Met Phe Gly Leu Glu
    290                 295                 300

Trp Pro Asn Asn Val Thr Leu Glu Val Ile Ile Glu Gly Ser Asn Leu
305                 310                 315                 320

Asn Asn Ser Leu Ala Gly Tyr Met Asn Cys Pro Asn Glu Arg Glu Asp
                325                 330                 335

Gly Leu Gly Ser Ala Ala Arg Asp Ile Trp Val Gly His Tyr Leu Gln
            340                 345                 350

Asn Ala Thr Glu Arg Phe Ser Lys Leu Val Thr Gly Tyr Asn Trp Thr
        355                 360                 365

Leu Asp Asp Thr Tyr Ala Ala Gln Thr Leu Cys Ala Tyr Asp Thr Val
    370                 375                 380

Ala Ser Gly Tyr Ser Arg Phe Cys Ser Leu Phe Thr Tyr Glu Glu Trp
385                 390                 395                 400

Ile Gly Phe Gly Tyr Ser His Asp Leu Gln Phe Tyr Gly Asn Asn Ala
                405                 410                 415

Phe Gly Ser Glu Thr Gly Arg Ala Ile Gly Ile Gly Phe Gln Gln Glu
            420                 425                 430

Val Leu Ala Arg Leu Gln Asn His Thr Ile Pro Tyr Ser Glu Thr Gln
        435                 440                 445

Val Asn Val Thr Leu Asp Asn Asn Thr Val Thr Phe Pro Leu Asn Gln
    450                 455                 460

Ser Leu Tyr Leu Asp Phe Ser His Asp Thr Asn Ile Val Ser Ile Leu
465                 470                 475                 480

Ala Ala Phe Gly Leu Thr Gln Phe Glu Glu Asp Leu Pro Ala Asp Lys
                485                 490                 495

Tyr Pro Gly Glu His Asn Phe Thr Val Ser His Met Thr Pro Phe Gly
            500                 505                 510

Ala Arg Leu Asp Ile Glu Ile Ile Lys Thr Pro Lys Pro Leu Lys Ala
        515                 520                 525

Asp Arg Ser Gly Tyr Glu Asp Glu Gly Glu Glu Thr Lys Tyr Val His
    530                 535                 540

Phe Val Leu Asn Gln Arg Thr Val Pro Leu Gly Trp Ser His Pro Glu
545                 550                 555                 560

Cys Asp Ala Glu Arg Val Asp Gly Trp Cys Glu Phe Glu Ala Phe Leu
                565                 570                 575

Lys Val Gln Glu Lys Met Pro Gly Leu Ala Arg Tyr Glu Glu Val Cys
            580                 585                 590

Phe Ala Asp Gly Gly Ser Pro
        595

<210>  27
<211>  468
<212>  PRT
<213>  Botryotinia fuckeliana

<400>  27

Met Leu Leu Asp Gln Ile Leu Leu Leu Ser Leu Thr Gly Val Thr Ala
1               5                   10                  15

Ser Ala Leu Leu Pro Glu Lys Gln Pro Cys Ser Thr Ser Thr Asn Asp
            20                  25                  30

Val Pro Gln Tyr Phe Gln Thr Thr Pro Glu Leu Trp Ala Gly Pro Thr
        35                  40                  45

Ala Thr Gly Arg Ala Pro Phe Leu Ala Gln Thr Asn Pro Val Ser Phe
    50                  55                  60

Ala Pro Thr Val Thr Phe Val Pro Asn Thr Pro Leu Glu Thr Ala Gln
65                  70                  75                  80

Pro Ile Val Gly Gln Gly Gln Asn Gly Ser Ile Phe Gln Leu Met Gly
                85                  90                  95

His Leu Ser Pro Tyr Leu Pro Asn Pro Ser Gly Phe Gly Val Ala Glu
            100                 105                 110

Tyr Pro Leu Pro Pro Gly Ala Lys Ile Ala Gln Leu Gln Met Leu Ser
        115                 120                 125

Arg His Gly Ala Arg Tyr Pro Thr Ser Gly Ser Asn Val Ala Thr Phe
    130                 135                 140

Gly Glu Lys Val Ala Asn Tyr Thr Gly Lys Met Asp Ala Thr Gly Ala
145                 150                 155                 160

Leu Ser Phe Leu Asn Asp Trp Lys Tyr Glu Leu Gly His Glu Ile Leu
                165                 170                 175

Val Pro Arg Gly Arg Gln Glu Leu Tyr Asp Ser Gly Ile Leu His Tyr
            180                 185                 190

Tyr Gln Tyr Ala Gln Leu Tyr Asn Pro His Ser Lys Ile Ile Ala Arg
        195                 200                 205

Thr Thr Thr Gln Asp Arg Met Leu Lys Ser Ala Glu Tyr Phe Met Ala
    210                 215                 220

Gly Phe Phe Gly Leu Glu Trp Thr Asn Asn Val Thr Ile Glu Val Ile
225                 230                 235                 240

Ile Glu Ser Asn Gly Tyr Asn Asn Ser Leu Ala Gly Tyr Asp Asn Cys
                245                 250                 255

Pro Asn Ser Asn Asn Tyr Arg Ser Ala Gly Gly Asn Asn Ala Thr Asn
            260                 265                 270

Gln Trp Val Ala Thr Tyr Leu Gln Asn Ala Thr Ala Arg Phe Gln Ser
        275                 280                 285

Met Val Ser Pro Glu Phe Asn Trp Thr Asp Val Asp Thr Tyr Ala Ala
    290                 295                 300

Gln Thr Met Cys Pro Tyr Glu Thr Val Ala Tyr Gly Tyr Ser Ala Phe
305                 310                 315                 320

Cys Asn Leu Phe Thr Tyr Glu Glu Trp Val Gly Phe Glu Tyr Ala Ile
                325                 330                 335

Asp Leu Tyr Phe Ala Gly Gly Ser Ser Phe Ala Ser Pro Thr Gly Arg
            340                 345                 350

Ala Val Gly Ile Gly Tyr Val Gln Glu Val Val Ala Arg Leu Gln Asn
        355                 360                 365

His Thr Leu Gly Tyr Ser Gly Ser Gln Ile Asn Thr Thr Leu Asp Asn
    370                 375                 380

Asn Thr Ala Thr Phe Pro Leu Asn Gln Ser Ile Tyr Phe Asp Phe Ser
385                 390                 395                 400

His Asp Thr Asn Ile Met Ser Ile Leu Thr Ala Phe Gly Phe Thr Gln
                405                 410                 415

Phe Ala Asp Ala Leu Pro Ala Thr His His Pro Gly Pro His Asn Leu
            420                 425                 430

Thr Val Ser His Met Glu Pro Phe Gly Ala Arg Leu Asp Ile Glu Ile
        435                 440                 445

Ile Lys Thr Pro Lys Pro Leu Ala Ala Asp Arg Thr Tyr Val Asp Gly

450                 455                 460

Lys Glu Thr Thr
465

<210>  28
<211>  540
<212>  PRT
<213>  aspergillus terreus

<400>  28

Met Arg Leu Ala Ser Gln Ser Ala Leu Val Ala Leu Ala Thr Ala Gly
1               5                   10                  15

His Ala Ala Val Ile Ser Pro Ser Arg Thr Ser Ser Val Pro Glu Tyr
            20                  25                  30

Phe Gln Thr Ser Phe Gly Pro Trp Ala Gly Pro Thr Glu Ala Gly Ala
        35                  40                  45

Pro Pro Phe Leu Ala Gln Thr Asn Pro Ala Pro Phe Gly Asn Pro Thr
    50                  55                  60

Tyr Val Pro Asn Thr Pro Val Val Thr Asp Val Pro Ile Pro Gly Arg
65                  70                  75                  80

Ser Asp Gly Ala Glu Ile Phe Lys Leu Met Gly Asn Leu Ser Pro Tyr
                85                  90                  95

Met Pro Asn Pro Ser Gly Phe Gly Val Asn Glu Tyr Pro Leu Pro Ala
            100                 105                 110

Gly Ala Asn Ile Thr Gln Met His Met Ile His Arg His Gly Ser Arg
        115                 120                 125

Tyr Pro Thr Thr Gly Asn Ala Val Ala Ser Leu Gln Asp Arg Val Ser
    130                 135                 140

Glu Leu Leu Gln Gly Gly Thr Lys Phe Lys Gly Asp Leu Glu Phe Leu
145                 150                 155                 160

Asn Ser Trp Arg Tyr Glu Leu Gly Lys Glu Glu Leu Thr Ala Leu Gly
                165                 170                 175

Arg Gln Gln Leu Phe Asp Ser Gly Val Leu Asn Trp Phe Asn Tyr Gly
            180                 185                 190

Arg Leu Tyr Asp Pro Ser Lys Pro Leu Ile Ala Arg Thr Thr Thr Gln
        195                 200                 205

Val Arg Met Leu Gln Ser Ala Glu Asn Trp Leu Asn Gly Phe Phe Gly
    210                 215                 220

Pro Asp Trp Thr Lys Asn Val Thr Leu Glu Val Leu Ile Glu Ala Asn
225                 230                 235                 240

Gly Phe Asn Asn Ser Leu Ala Gly Asp Lys Gln Cys Val Asn Ser Lys
                245                 250                 255

Asn Asp Arg Ser Ser Ala Gly Asp Thr Ala Ser Ser Glu Trp Gln Lys
            260                 265                 270

Ile Tyr Leu Lys Asn Ala Thr Glu Arg Phe Arg Arg Ser Met Thr Gly
        275                 280                 285

His Ala Asn Trp Thr Val Asp Asp Thr Tyr Asn Ala Gln Thr Met Cys
    290                 295                 300

Pro Tyr Glu Thr Val Gly Leu Gly Tyr Ser Ser Phe Cys Ser Leu Phe
305                 310                 315                 320

Thr Tyr Glu Glu Trp Leu Gly Phe Glu Tyr Ser Leu Asp Leu Ser Tyr
                325                 330                 335

Tyr Gly Ser Asn Ser Phe Gly Ser Pro Thr Gly Arg Ala Val Gly Val
            340                 345                 350

Gly Tyr Val Glu Glu Leu Val Ala Arg Leu Gln Arg His Phe Pro Gln
        355                 360                 365

Pro Ala Glu Gly Ser Val Ala Ile Asn Glu Thr Leu Asp Asp Ser Ala
    370                 375                 380

Thr Thr Phe Pro Leu Asp Gln Ser Leu Tyr Leu Asp Phe Ser His Asp
385                 390                 395                 400

Thr Asp Ile Met Ser Met Leu Thr Ala Met Gly Leu Thr Gln Phe Ala
                405                 410                 415

Gln Phe Leu Pro Ile Ser Gly Pro Pro Lys Asp Gln Gln Leu Ile Val
            420                 425                 430

Ser His Ile Val Pro Phe Ala Gly Arg Phe Asn Ile Glu Val Ile Lys
        435                 440                 445

Ala Pro Gln Pro Val Arg Ala Lys Arg Pro Gln Asn Ala His Ala Ser
    450                 455                 460

Ala Tyr Glu Ser Gly Glu Glu Thr Thr Tyr Val His Met Leu Met Asn
465                 470                 475                 480

Gln Arg Thr Ile Pro Leu Gly Arg Ser Leu Lys Glu Cys Gly Asp Arg
                485                 490                 495

Asp Asp Gly Trp Cys Glu Leu Gln Thr Phe Val Ser Ala Leu Lys Glu
            500                 505                 510

Asn Ile Pro Lys Ala Lys Phe Asp Glu Ser Cys Tyr Gly Asn Tyr Ser
        515                 520                 525

Val Pro Ala Tyr Gly Asp Ile Thr Asp Gly Ala Ile
    530                 535                 540

<210>  29
<211>  565
<212>  PRT
<213>  aspergillus terreus

<400>  29

Met Arg Tyr Ser Gly Phe Ile Leu Leu Ala Ala Ala Ser Gly Ile Glu
1               5                   10                  15

Ala Val Ala Leu Asp Pro Arg Val Thr Ser Leu Gly Pro Ser Ala Thr
            20                  25                  30

Ala Thr Glu Ala Asp Trp Phe Gln Thr Arg Pro Gln Ser Tyr Gln Gly
        35                  40                  45

Lys Lys His Cys His Tyr Ile Val Lys Thr Val Ser His Thr Ser Asn
    50                  55                  60

Thr Gly Val Thr Ala Thr Gly Thr Ala Pro Met Leu Ala Glu Thr Asn
65                  70                  75                  80

Pro Ala Pro Phe Gly Gln Lys Thr Tyr Val Pro Asn Ala Pro Leu Glu
                85                  90                  95

Thr Ser Glu Pro Ile Lys Gly Ala Lys Asn Arg Asn Ile Phe His Leu
            100                 105                 110

Met Gly Asn Leu Ser Pro Tyr Phe Thr Ser Glu Glu Gly Phe Gly Val
        115                 120                 125

Ala Glu Tyr Pro Leu Pro Glu Gly Ser Asp Ile Thr Gln Val His Leu
    130                 135                 140

Ile His Arg His Gly Ser Arg Tyr Pro Ser Asp Ser Glu Thr Ile Ala
145                 150                 155                 160

Ala Phe Ala Gln Arg Ile Val Asn Ala Thr Ala His Gly Thr Lys Phe
                165                 170                 175

Thr Gly Asp Leu Ala Phe Leu Asn Glu Tyr Ser Trp Ser Gln Gly Met
            180                 185                 190

Asn Val Leu Val Pro Met Gly Arg Gln Glu Leu Phe Asp Ser Gly Val
        195                 200                 205

Leu His Tyr Tyr Asn Tyr Gly Ser Leu Tyr Arg Asn Gly Ser Lys Leu
    210                 215                 220

Val Val Arg Ser Thr Thr Ser Asp Arg Ala Leu Lys Ser Ala Glu Asn
225                 230                 235                 240

Phe Leu Ala Gly Phe Phe Gly Leu Glu Trp Arg Asp Tyr Ala Asn Ile
                245                 250                 255

Leu Pro Leu Ile Ser Gly Gly Gly Phe Asn Ser Ser Leu Thr Gly Met
            260                 265                 270

Ser Val Cys Arg Asn Ala Gly Lys Ile Phe Ser Ser Leu Glu Asn Asp
        275                 280                 285

Ala Met Arg Thr Trp Gln Gly Ile Tyr Leu Lys Asp Lys Thr Gln Lys
    290                 295                 300

Leu Arg Lys Met Ala Ser Gly Phe Asn Trp Thr Val Ser Asp Ser Ala
305                 310                 315                 320

Asp Ala Gln Thr Leu Cys Ala Tyr Glu Thr Val Ser Phe Gly Tyr Ser
                325                 330                 335

Ser Phe Cys Gln Leu Phe Asp Tyr Glu Glu Trp Glu Gly Tyr Ser Tyr
            340                 345                 350

Ala Ser Ser Ile Val Ser Ser Ser Leu Met Gly Phe Gly Ser Pro Ile
        355                 360                 365

Ser Arg Ala Thr Gly Ile Ala Trp Val Gln Glu Phe Leu Ala Arg Val
    370                 375                 380

Glu Ser His Leu Leu Asp Val Ala Pro Gly Gln Thr Ser Ala Asn Leu
385                 390                 395                 400

Thr Leu Ala Thr Asn Pro Val Thr Phe Pro Leu Asp Gln Pro Leu Tyr
                405                 410                 415

Phe Asp Phe Gly His Asp Ala Ser Ile Leu Ala Ala Leu Ser Ala Phe
            420                 425                 430

Gly Ile Arg Gln Phe Ala Asp Tyr Leu Pro Pro Thr Gly Pro Pro Thr
        435                 440                 445

His Lys Gln Phe Asp Ala Gly Lys Ile Thr Pro Phe Ala Gly Arg Leu
    450                 455                 460

Asn Ile Glu Ile Ile Glu Thr Leu His Ala Val Lys Thr Arg Arg Pro
465                 470                 475                 480

Arg Gly Glu Asn Glu Arg Ser Tyr Ile Ser Gly Thr Gly Lys Thr Arg
                485                 490                 495

Tyr Val His Phe Leu Leu Asn Gln Arg Thr Val Pro Leu His Ala Ser
            500                 505                 510

Phe Lys Glu Cys Glu Tyr Arg Asp Asp Gly Trp Cys Glu Leu Ser Thr
        515                 520                 525

Phe Leu Glu Val Gln Lys Gly Ser Leu Gly Lys Ala Gln Phe Gln Tyr
    530                 535                 540

Ser Cys Glu Gly Asn Trp Thr Val Gly Pro Tyr Gly Ser Val Thr Asp
545                 550                 555                 560

Gly Val Pro Pro Gln
                565


<210>  30
<211>  552
<212>  PRT
<213>  aspergillus niger

<400>  30

Met His Pro Thr Ile Ala Thr Leu Ser Ile Leu Ala Val Ala Ser Leu
1               5                   10                  15

Ala Glu Ala Val Ala Ile Glu Ala Arg Ser Gln Asn Ala Thr Pro Leu
            20                  25                  30

Thr Pro Ser Pro Ser Ala Thr Thr Thr Gly Ile Asp Trp Phe Gln Thr
        35                  40                  45

Thr Pro Glu Ser Tyr Gln Gly Thr Thr Ala Thr Gly Val Ala Pro Phe
    50                  55                  60

Leu Ala Glu Thr Asn Pro Ala Pro Phe Gly Gln Lys Thr Leu Ser Pro
65                  70                  75                  80

Asn Asp Pro Leu Glu Thr Ser Glu Pro Ile Glu Gly Ala Ala Gly Arg
                85                  90                  95

Asn Ile Phe His Tyr Met Gly Asn Leu Ser Pro Tyr His Val Pro Asp
            100                 105                 110

Gly Phe Gly Val Asp Glu Tyr Pro Leu Pro Lys Gly Ser Asn Ile Thr
        115                 120                 125

Gln Met His Met Ile His Arg His Gly Ser Arg Tyr Pro Ser Ser Ser
    130                 135                 140

Glu Gly Leu Ala Ser Trp Ala Gln Lys Ile Ile Asn Ser Thr Ala Ser

145                 150                 155                 160

Gly Asn Asn Phe Thr Gly Ala Leu Ser Phe Leu Asn Asp Trp Asp Tyr
                165                 170                 175

Gly Leu Gly Leu Glu Ile Leu Val Pro Lys Gly Arg Gln Glu Leu Tyr
            180                 185                 190

Asp Ser Gly Val Leu Asn Phe Tyr Asn Tyr Gly His Leu Tyr Asn Ala
        195                 200                 205

Ser Ser Pro His Lys Leu Val Ala Arg Thr Thr Thr Gln Asp Arg Met
    210                 215                 220

Leu Lys Ser Ala Glu Asn Phe Leu Ala Gly Phe Phe Gly Leu Glu Trp
225                 230                 235                 240

Thr Glu Lys Ala Asn Leu Leu Pro Ile Ile Glu Gly Val Gly Tyr Asn
                245                 250                 255

Asn Ser Leu Ile Gly Thr Tyr Ser Cys Thr Arg Ala Leu Glu Tyr Met
            260                 265                 270

Ala Tyr Asn Ala Thr Thr Pro Leu Ser Thr Trp Lys Asn Ile Tyr Leu
        275                 280                 285

Lys Ala Arg Thr Glu Ala Leu Arg Thr Leu Thr Gly Ser Tyr Asn Trp
    290                 295                 300

Thr Thr Thr Asp Ser Phe Asn Ala Gln Asp Met Cys Ala Tyr Glu Thr
305                 310                 315                 320

Ile Ser Tyr Gly Tyr Ser Gln Phe Cys Glu Leu Phe Thr Phe Glu Glu
                325                 330                 335

Phe Glu Asn Phe Gly Tyr Ala Phe Asp Ile Glu Phe Ala Asn Met Val
            340                 345                 350

Gly Phe Ala Cys Pro Ala Gly Arg Ala Gln Gly Ile Ala Trp Val Glu
        355                 360                 365

Glu Phe Leu Ala Arg Val Glu Gly His Leu Leu Gln Thr Thr Gly Thr
    370                 375                 380

Asn Ala Asn Met Thr Leu Asp Thr Asn Pro Val Thr Phe Pro Thr Asp
385                 390                 395                 400

Gln Asn Leu Tyr Leu Asp Phe Ser His Asp Ala Gly Ile Val Ala Val
                405                 410                 415

Leu Thr Ala Phe Gly Phe Arg Gln Phe Ala Glu Ser Leu Pro Ala Thr
            420                 425                 430

Gly Pro Pro Leu Tyr Gln Gln Phe Lys Ser Ser Lys Ile Val Pro Phe
        435                 440                 445

Ala Gly Arg Thr Asn Ile Glu Ile Ile Lys Ala Pro Asn Gln Val Ala
    450                 455                 460

Ala Ile Arg Pro Ala Gly Asp Gln Ser Asp Ala Tyr Val Asp Gly Thr
465                 470                 475                 480

Gly Glu Thr Phe Tyr Val His Phe Leu Gln Asn Gln Arg Thr Leu Pro
                485                 490                 495

Leu His Ser Ser Phe Glu Glu Cys Glu Tyr Arg Asp Asp Gly Trp Cys
            500                 505                 510

Glu Leu Ser Thr Phe Met Ala Val Gln Lys Lys Gly Leu Glu Arg Ser
        515                 520                 525

Gln Phe Glu Tyr Ala Cys Phe Gly Asn Trp Thr Val Thr Glu Tyr Gly
    530                 535                 540

Thr Val Thr Asp Gly Val Pro Val
545                 550


<210>  31
<211>  547
<212>  PRT
<213>  Aspergillus niger

<400>  31

Met Arg Ser Arg Ile Tyr Trp Ala Ile Leu Phe Leu Leu Leu Val Ser
1               5                   10                  15

Cys Val Ser Ala Asn Asp Gln Tyr Val Met Gly Val Ala Pro Thr Thr
            20                  25                  30

Thr Glu Pro Glu Trp Phe Lys Thr Arg Pro Gln Ser Phe Gln Gly Tyr
        35                  40                  45

Thr Ala Thr Gly Ala Ala Pro Phe Leu Ala Gln Thr Asn Pro Ala Pro
    50                  55                  60

Phe Gly Asn Pro Ala Thr Tyr Thr Ala Asn His Pro Leu Glu Thr Ser
65                  70                  75                  80

Gln Pro Ile Arg Gly Gly Lys Asp Arg Asn Ile Phe His His Met Gly
                85                  90                  95

Ile Leu Ser Pro Tyr Tyr Pro Arg Ala Asp Gly Phe Gly Val Asp Glu
            100                 105                 110

Phe Pro Arg Pro Lys Gly Ser Asn Ile Thr Gln Met His Met Leu His
        115                 120                 125

Arg His Gly Ser Arg Tyr Pro Asn Lys Asp Glu Gly Asp Asp Phe Ala
    130                 135                 140

Asn Trp Ile Lys Ala Ile Thr Asn Ala Thr Ala His Gly Ala Val Phe
145                 150                 155                 160

Arg Asp Glu Leu Ser Phe Ile His Asp Trp Thr Tyr Ser Leu Gly Ala
                165                 170                 175

Asp Met Leu Thr Thr Arg Gly Arg Glu Asp Leu Leu Glu Ser Gly Ile
            180                 185                 190

Leu Asn Phe Tyr Asn Tyr Gly His Leu Tyr Thr Pro Gly Thr Lys Ile
        195                 200                 205

Val Ala Arg Thr Thr Thr Gln Asp Arg Met Leu Lys Ser Ala Glu Asn
    210                 215                 220

Phe Leu Ala Gly Phe Phe His Leu Asp Trp Asp Glu His Val Asn Leu
225                 230                 235                 240

Leu Ala Met Ile Glu Glu Lys Asn Phe Asn Ser Ser Leu Gln Ala Lys
                245                 250                 255

Asn Ala Cys Pro Asn Ala Met Lys Ile Ser Phe Asp Asp Tyr Val Ser
            260                 265                 270

Asp Thr Val Thr Lys Trp Lys Thr His Tyr Leu Ser His Arg Thr His
        275                 280                 285

His Leu Asn Tyr Leu Ser Thr Asp Tyr His Trp Thr Ser Asn Asp Ser
    290                 295                 300

Phe Asn Ala Gln Thr Leu Cys Ala Tyr Glu Thr Val Ala Leu Gly Tyr
305                 310                 315                 320

Ser Pro Trp Cys Ser Leu Phe Thr Phe Pro Glu Trp Glu Gly Phe Ser
                325                 330                 335

Tyr Thr Tyr Asp Leu Thr Phe Gly Gly Asn Ala Gly Phe Gln Cys Pro
            340                 345                 350

Ile Ser Arg Ala Met Gly Ile Thr Trp Val Gln Glu Phe Leu Ala Arg
        355                 360                 365

Val Glu Asn Arg Ser Phe Ser Thr Pro Gly Ser Ser Ser Ala Ala Asn
    370                 375                 380

Leu Thr Leu Asn Thr Asn Pro Val Thr Phe Pro Thr Asn Gln Ser Leu

385                 390                 395                 400

Tyr Phe Asp Phe Ala His Asp Lys Ile Leu Leu Gly Val Leu Thr Ala
                405                 410                 415

Phe Gly Leu Arg Gln Phe Ala Asp Leu Pro Phe Pro Asp Tyr Thr Asp
            420                 425                 430

Gln Tyr Phe Met Asp Val Phe Pro Pro Arg His His Ala Phe Gln Thr
        435                 440                 445

Ala Lys Leu Ile Pro Phe Ala Gly Arg Leu Asn Ile Glu Ile Ile Arg
    450                 455                 460

Ala Pro His Lys Ile Asn Pro Arg Gln Thr Glu Tyr Val His Phe Leu
465                 470                 475                 480

Leu Asn Gln Arg Thr Val Pro Leu His Lys Ser Leu Pro Glu Cys Ser
                485                 490                 495

Phe Arg Ser Asp Gly Trp Cys Glu Leu Asp Ala Phe Leu Arg Ala Gln
            500                 505                 510

Lys Asp Ser Leu Arg Lys Ala Glu Tyr Asp Phe Ser Cys Val Gly Glu
        515                 520                 525

Trp Asp Leu Gly Glu Phe Gly Asp Val Trp Asp Gly Val Pro Val Leu
    530                 535                 540

Arg Gly Glu
545

<210>  32
<211>  484
<212>  PRT
<213>  Phaeosphaeria nodorum

<400>  32

Met Leu Gly Ile Phe Glu Leu Ser Leu Gln Val Leu Asn Ser Phe Asn
1               5                   10                  15

Pro Ala Lys Tyr Gly Pro Thr Gln Thr Gly Asp Ala Pro Trp Leu Ala
            20                  25                  30

Gln Thr Asn Ala Ala Pro Phe Ala Gly Val Ser Tyr Ile Pro Asn Thr
        35                  40                  45

Pro Leu Glu Thr Gln Ile Pro Ile Ala Gly Asn Val Asn Asn Thr Asn
    50                  55                  60

Ile Phe Gln Arg Leu Ala Ile Leu Ser Pro Tyr Phe Pro Asn Pro Arg
65                  70                  75                  80

Gly Phe Gly Val Asn Glu Tyr Ser Val Pro Phe Gly Ser Asn Val Thr
                85                  90                  95

Trp Leu Asn Met Val His Arg His Gly Ser Arg Tyr Pro Glu Val Ser
            100                 105                 110

Gly Asp Ala Ala Glu Arg Thr Leu Gly Leu Lys Leu Ala Ala Ala Glu
        115                 120                 125

Gly Lys Phe Ala Gly His Gly Pro Leu Ser Phe Leu Asn Asp Trp Lys
    130                 135                 140

Phe Met Leu Gly Ala Glu Ile Leu Val Pro Asn Gly Lys Ala Glu Leu
145                 150                 155                 160

Phe Gln Ser Gly Thr Leu His Tyr Tyr Gln Tyr Gly His Leu Tyr Pro
                165                 170                 175

Asn Asn Gly Ser Lys Ile Ile Val Arg Ser Thr Thr Gln Arg Arg Met
            180                 185                 190

Tyr Glu Ser Ala Glu Tyr Phe Leu Ala Gly Phe Phe Gly Leu Ser Trp
        195                 200                 205

Thr Gln Asn Ala Thr Leu Glu Leu Ala Ile Glu Trp Pro Gly Phe Asn
    210                 215                 220

Asn Thr Leu Ala Gly Tyr Lys Gln Cys Asn His Thr Gly Trp Leu Thr
225                 230                 235                 240

Ala Arg Glu Gly Asp Leu Asp Trp Thr Leu Ser Asp Thr Tyr Asn Ala
                245                 250                 255

Gln Ala Leu Cys Ala Tyr Glu Thr Val Gly Leu Gly Phe Ser His Phe
            260                 265                 270

Cys Gly Leu Phe Thr Tyr Glu Glu Trp Glu Gly Tyr Glu Tyr Ala Leu
        275                 280                 285

Asp Ile Ala Phe Ser Ala Gly Thr Ala Phe Gly Ser Pro Val Gly Arg
    290                 295                 300

Ala Ile Gly Val Gly Tyr Val Gln Glu Val Leu Ala Arg Met Gln His
305                 310                 315                 320

His Val Ile Thr Asp Pro Ala Ala Gln Ile Asn Thr Thr Leu Asp Asn
                325                 330                 335

Asn Thr Val Thr Phe Pro Val Asp Gln Asn Ile Asn Leu Asp Phe Ser
            340                 345                 350

His Asp Ala Asn Ile Ile Ser Ile Leu Ala Ala Phe Gly Leu Thr Gln
        355                 360                 365

Phe Ala Asp Tyr Leu Pro Thr Ser His Ile Lys Lys Asp Arg Glu Phe
    370                 375                 380

Val Met Ser Tyr Leu Glu Pro Phe Ala Gly Arg Leu Asp Ile Glu Leu
385                 390                 395                 400

Ile Lys Thr Pro Thr Pro Val Asn Pro Asp Arg Ser Ala Lys Lys Ile
                405                 410                 415

Tyr Leu Asp Gly Glu Pro Thr Thr Tyr Val His Phe Ile Leu Asn Gln
            420                 425                 430

Arg Leu Arg Glu Gln Gly Arg Arg Gly Val Cys Glu Met Ser Ile Phe
        435                 440                 445

Leu Arg Val Met Glu Lys Glu Ile Glu Lys Ala Asp Tyr Asp Tyr Ala
    450                 455                 460

Cys Phe Gly Asp Tyr Asp Leu Pro Ala Tyr Gly Glu Ile Lys Asp Gly
465                 470                 475                 480

Arg Pro Ile Arg

<210>  33
<211>  531
<212>  PRT
<213>  aspergillus oryzae

<400>  33

Met Gln Asp Met Val Phe Ala Leu Pro Leu Ile Thr Ala Ser Trp Val
1               5                   10                  15

Gly Val Thr Ala Ala Ser Ser Ala Arg Ala Thr Gly Thr Thr Gln Pro
            20                  25                  30

Asn Trp Phe Gln Thr Ser Pro Gln Ser Tyr Gln Gly Pro Thr Ala Thr
        35                  40                  45

Gly Ser Ala Pro Phe Leu Ala Val Thr Asn Pro Ala Phe Ser Ser His
    50                  55                  60

Gly Thr Tyr Ile Ala Asn Glu Pro Leu Val Thr Glu Gln Pro Ile Ser
65                  70                  75                  80

Gly Ala His Gly Arg Asn Ile Phe His His Ala Gly Asn Leu Ser Pro
                85                  90                  95

Tyr Phe Ser Ser Glu Glu Gly Phe Gly Val Asp Glu Tyr Pro Leu Pro
            100                 105                 110

Ala Gly Ala Asn Ile Thr Gln Met His Met Pro His Arg His Gly Ser
        115                 120                 125

Arg Tyr Pro Ser Ser Ser Glu Gly Tyr Pro Ser Trp Ala Glu Gly Ile
    130                 135                 140

Lys Asn Ser Thr Thr Ala Gly Asn Arg Phe Lys Ala Glu Ile Leu Val
145                 150                 155                 160

Pro Lys Gly Arg Gln Glu Met Phe Asp Ser Gly Val Leu Asn Tyr Tyr
                165                 170                 175

Asn Tyr Gly His Leu Tyr Asn Glu Ser Leu Gly His Lys Leu Val Ala
            180                 185                 190

Arg Thr Thr Thr Gln Asn Arg Met Leu Lys Ser Ala Glu Asn Phe Leu
        195                 200                 205

Ala Gly Phe Phe Gly Leu Asp Trp Thr Asp Lys Ala Asn Leu Leu Ala
    210                 215                 220

Met Ile Glu Asn Val Gly Phe Asn Asn Ser Leu Ile Gly Thr Tyr Ser
225                 230                 235                 240

Cys Pro Asn Ala Met Thr Val Met Ala Asn Thr Ser Ile Tyr Glu Pro
                245                 250                 255

Met Asn Gln Trp Ile Asn Ile Tyr Leu Lys Asn Arg Thr Thr Thr Leu
            260                 265                 270

Lys Glu Leu Ser Gly Ser Tyr Asn Trp Thr Ala Thr Asp Ser His Asn
        275                 280                 285

Ala Gln Ala Leu Cys Val Tyr Glu Thr Ile Ser Phe Gly Tyr Ser Gln
    290                 295                 300

Phe Cys Gln Leu Phe Thr Tyr Lys Glu Phe Glu Gln Phe Ser Tyr Ala
305                 310                 315                 320

Tyr Asp Leu Met Phe Thr Ala Met Val Gly Phe Gln Asn Pro Ala Gly
                325                 330                 335

Arg Ala Gln Gly Ile Ala Trp Val Glu Glu Phe Leu Ala Arg Val Glu
            340                 345                 350

Gly His Val Leu Gln Thr Thr Gly Thr Asn Ala Asn Met Thr Leu Ala
        355                 360                 365

Thr Asn Pro Val Thr Phe Pro Met Asp Gln Asn Leu Tyr Leu Asp Phe
    370                 375                 380

Thr His Asp Ser Asp Ile Phe Ala Thr Leu Thr Ala Phe Gly Phe Arg
385                 390                 395                 400

Gln Phe Ala Gln Phe Leu Pro Pro Thr Gly Pro Pro Lys Asn Gln Gln
                405                 410                 415

Phe Ser Thr Ser Lys Val Val Pro Phe Gly Gly Arg Thr Asn Ile Glu
            420                 425                 430

Ile Ile Arg Ala Pro His Lys Val Ser Thr Lys Arg Ser Arg Asn Glu
        435                 440                 445

Thr Gln Ser Val Tyr Val Lys Asp Thr Lys Asp Thr Tyr Tyr Val His
    450                 455                 460

Phe Leu Gln Asn Gln Arg Thr Leu Pro Leu His Ala Ser Phe Pro Glu
465                 470                 475                 480

Cys Glu Tyr Arg Asp Asp Gly Trp Cys Glu Leu Asp Thr Phe Met Lys
                485                 490                 495

Val Gln Arg Lys Ser Leu Gln Arg Ser Gln Phe Glu Tyr Ala Cys Phe
            500                 505                 510

Gly Asn Trp Ser Thr Pro Ala Tyr Gly Asp Val Thr Asp Gly Val Pro
        515                 520                 525

Pro Arg Ser
    530

<210>  34
<211>  436
<212>  PRT
<213>  Sclerotinia sclerotiorum

<400>  34

Met Leu Leu Asp Gln Ile Leu Leu Leu Ser Leu Thr Gly Val Thr Thr
1               5                   10                  15

Ala Ala Leu Leu Pro Glu Lys Gln Gln Ser Ser Thr Ser Thr Ser Asp
            20                  25                  30

Val Pro Gln Tyr Phe Gln Thr Thr Pro Glu Leu Trp Ala Gly Pro Thr
        35                  40                  45

Ala Thr Gly Arg Ala Pro Phe Leu Ala Gln Thr Asn Pro Val Ser Phe
    50                  55                  60

Ala Pro Thr Val Thr Phe Val Pro Asn Thr Pro Leu Glu Thr Ala Gln
65                  70                  75                  80

Pro Ile Val Gly Gln Gly Gln Asn Gln Ser Ile Phe Gln Leu Met Gly

                85                  90                  95

Asn Leu Ser Pro Tyr Leu Pro Asn Pro Ser Gly Phe Gly Val Ala Glu
            100                 105                 110

Tyr Pro Leu Pro Pro Gly Ala Lys Ile Ala Gln Leu Gln Met Leu Ser
        115                 120                 125

Arg His Gly Ser Arg Tyr Pro Thr Val Gly Ser Asn Val Glu Val Phe
    130                 135                 140

Gly Glu Lys Val Ala Asn Asn Thr Glu Lys Leu Asn Ala Thr Gly Ala
145                 150                 155                 160

Leu Ser Phe Leu Asn Asp Trp Lys Tyr Gly Leu Gly His Glu Ile Leu
                165                 170                 175

Val Pro Arg Gly Arg Gln Glu Leu Tyr Asp Ser Gly Ile Leu His Tyr
            180                 185                 190

Tyr Gln Tyr Ala Gln Leu Tyr Asn Pro His Ser Lys Ile Ile Ala Arg
        195                 200                 205

Thr Thr Thr Gln Asp Arg Met Leu Lys Ser Ala Glu Tyr Phe Met Ala
    210                 215                 220

Gly Phe Phe Gly Leu Glu Trp Thr Asn Asn Ala Thr Ile Glu Val Ile
225                 230                 235                 240

Ile Glu Ser Gln Gly Tyr Asn Asn Ser Leu Ala Gly Tyr Asp Asn Cys
                245                 250                 255

Pro Asn Ser Asn Asn Tyr Arg Ser Ala Gly Gly Ser Asn Ala Ser Asp
            260                 265                 270

Gln Trp Val Ala Val Tyr Leu Gln Asp Ala Thr Thr Arg Phe Gln Ser
        275                 280                 285

Met Val Ser Pro Glu Phe Asn Trp Thr Val Thr Asp Thr Tyr Ala Ala
    290                 295                 300

Gln Thr Leu Cys Pro Tyr Glu Thr Pro Phe Gly Ala Arg Leu Asp Ile
305                 310                 315                 320

Glu Leu Ile Lys Thr Pro Lys Pro Leu Ala Ala Asn Arg Thr Tyr Ile
                325                 330                 335

Asp Gly Lys Glu Thr Thr Tyr Ile His Phe Ile Leu Asn Gln Arg Thr
            340                 345                 350

Ile Pro Leu Gly Phe Ser Phe Leu Glu Cys Gly Val Asp Arg Leu Asp
        355                 360                 365

Gly Trp Cys Glu Leu Glu Thr Phe Leu Lys Val Gln Ser Lys Ser Thr
    370                 375                 380

Glu Glu Ala Gln Tyr Glu Tyr Ala Cys Phe Gly Asp Tyr Pro Ala Glu
385                 390                 395                 400

Pro Tyr Gly Ser Val Lys Asn Glu Asn Gln Gly Leu Arg Ala Ala Gln
                405                 410                 415

Ile Gly Phe Leu Ser Ser Pro Ser Leu Ser Val Tyr Gly Thr Gly Thr
            420                 425                 430

Thr Glu Arg Leu
        435